# EUROPEAN PATENT SPECIFICATION

(11) **EP 3 108 014 B1**
(45) Date of publication and mention of the grant of the patent: **05.12.2018**
(21) Application number: 15719021.6
(22) Date of filing: 18.02.2015
(51) Int. Cl.: C12Q 1/70

(54) **METHODS AND SYSTEMS FOR RAPID DETECTION OF MICROORGANISMS USING RECOMBINANT BACTERIOPHAGE**
VERFAHREN UND SYSTEME ZUM SCHNELLEN NACHWEIS VON MIKROORGANISMEN MITTELS REKOMBINANTER BAKTERIOPHAGEN
PROCÉDÉS ET SYSTÈMES POUR UNE DÉTECTION RAPIDE DE MICRO-ORGANISMES À L'AIDE D'UN BACTÉRIOPHAGE RECOMBINANT

(30) Priority: 18.02.2014 US 201461940959 P
(43) Date of publication of application: 28.12.2016
(73) Proprietor: Laboratory Corporation of America Holdings, Burlington, North Carolina 27215 (US)
(72) Inventor: ANDERSON, Dwight Lyman, Minneapolis, Minnesota 55406 (US); GIL, Jose S., Winnetka, California 91306 (US); HOPKINS, Ben Barrett, Sherman Oaks, CA 91423 (US); ERICKSON, Stephen Eric, White Bear Township, Minnesota 55110 (US)
(74) Representative: Donald, Jenny Susan
(86) International application number: PCT/US2015/016415
(87) International publication number: WO 2015/126966

(56) References cited:
- LOESSNER M J ET AL: "Construction of luciferase reporter bacteriophage A511::luxAB for rapid and sensitive detection of viable Listeria cells", APPLIED AND ENVIRONMENTAL MICROBIOLOGY, AMERICAN SOCIETY FOR MICROBIOLOGY, US, vol. 62, no. 4, 1 April 1996 (1996-04-01), pages 1133-1140, XP002079442, ISSN: 0099-2240
- LOESSNER M J ET AL: "Evaluation of luciferase reporter bacteriophage A511:luxAB for detection of listeria monocytogenes in contaminated foods", APPLIED AND ENVIRONMENTAL MICROBIOLOGY, AMERICAN SOCIETY FOR MICROBIOLOGY, US, vol. 63, no. 8, 1 August 1997 (1997-08-01) , pages 2961-2965, XP002966690, ISSN: 0099-2240
- STEVEN HAGENS ET AL: "Reporter bacteriophage A511:: celB transduces a hyperthermostable glycosidase from Pyrococcus furiosus for rapid and simple detection of viable Listeria cells", BACTERIOPHAGE, vol. 1, no. 3, 1 May 2011 (2011-05-01), pages 143-151, XP055192917, DOI: 10.4161/bact.1.3.16710
- R. EDGAR: "High-sensitivity bacterial detection using biotin-tagged phage and quantum-dot nanocomplexes", PROCEEDINGS OF THE NATIONAL ACADEMY OF SCIENCES, vol. 103, no. 13, 28 March 2006 (2006-03-28), pages 4841-4845, XP055056103, ISSN: 0027-8424, DOI: 10.1073/pnas.0601211103
- LINA WU ET AL: "Trace Detection of Specific Viable Bacteria Using Tetracysteine-Tagged Bacteriophages", ANALYTICAL CHEMISTRY, vol. 86, no. 1, 7 January 2014 (2014-01-07), pages 907-912, XP055192910, ISSN: 0003-2700, DOI: 10.1021/ac403572z & Lina Wu ET AL: "Sensitive and Selective Bacterial Detection Using Tetracysteine-Tagged Phages in Conjunction with Biarsenical Dye", Angewandte Chemie International Edition, vol. 50, no. 26, 10 May 2011 (2011-05-10), pages 5873-5877, XP055192921, ISSN: 1433-7851, DOI: 10.1002/anie.201100334

## Description

### FIELD OF THE INVENTION

This invention relates to a recombinant bacteriophage and related methods and systems for the detection of microorganisms using bacteriophages.

### BACKGROUND

There is a strong interest in improving speed and sensitivity for detection of bacteria, viruses, and other microorganisms in biological, food, water, and clinical samples. Microbial pathogens can cause substantial morbidity among humans and domestic animals, as well as immense economic loss. Also, detection of microorganisms is a high priority for the Food and Drug Administration (FDA) and Centers for Disease Control (CDC) given outbreaks of life-threatening or fatal illness caused by ingestion of food contaminated with certain microorganisms, e.g., *Escherichia coli* or *Salmonella spp.*

Traditional microbiological tests for the detection of bacteria rely on non-selective and selective enrichment cultures followed by plating on selective media and further testing to confirm suspect colonies. Such procedures can require several days. A variety of rapid methods have been investigated and introduced into practice to reduce the time requirement. However, these methods have drawbacks. For example, techniques involving direct immunoassays or gene probes generally require an enrichment step in order to obtain adequate sensitivity. Polymerase chain reaction (PCR) tests also include an amplification step and therefore are capable of both very high sensitivity and selectivity; however, the sample size that can be economically subjected to PCR testing is limited. With dilute bacterial suspensions, most small subsamples will be free of cells and therefore purification and/or enrichment steps are still required.

The time required for traditional biological enrichment is dictated by the growth rate of the target bacterial population of the sample, by the effect of the sample matrix, and by the required sensitivity. For instance, a magnetic-capture PCR system for verotoxigenic *E. coli* can require about 5, 7 and 10 hours of culturing for enrichment to detect 1000, 100, and 1 colony forming unit per milliliter (CFU/mL), respectively, in a model system, and 15 hours of culturing for enrichment to detect 1 CFU per gram (g) in ground beef. In practice, most high sensitivity methods employ an overnight incubation and take about 24 hours overall. Due to the time required for cultivation, these methods can take up to three days, depending upon the organism to be identified and the source of the sample. This lag time is generally unsuitable as the contaminated food, water (or other product) may have already made its way into livestock or humans. In addition, increases in antibiotic-resistant bacteria and biodefense considerations make rapid identification of bacterial pathogens in water, food and clinical samples critical priorities worldwide.

Therefore, there is a need for more rapid, simple and sensitive detection and identification of microorganisms, such as bacteria, viruses, and other potentially pathogenic microorganisms.

Loessner M J et al., Applied and Environmental Microbiology, American Society for Microbiology, US, vol.62, no.4, 1996, page 1133-1140 discloses a recombinant bacteriophage comprising a luciferase A and luciferase B gene fusion; A511::*luxAB*.

Loessner M J et al., Applied and Environmental Microbiology, American Society for Microbiology, US, vol. 63, no.8, 1997, pages 2961-2965 discloses the use of the A511::*luxAB* recombinant bacteriophage for testing of contaminated foods and environmental samples.

Steven Hagens et al., Bacteriophage, vol.1, no.3, 2011, pages 143-151 discloses the bacteriophage A511::celB, which incorporates the gene for thermostable glycosidase from *Pyrococcus furiosus* into phage-infected Listeria cells..

Edgar R., Proceedings of the National Academy of Sciences, vol. 103, no.13, 2006, pages 4831-4845 discloses combining *in vivo* biotinylation of engineered host-specific bacteriophage and conjugation of the phage to streptavidin-coated quantum dots.

Lina Wu et al., Analytical Chemistry, vol.86, no.1, 2014, pages 907-912 discloses a tetracysteine (TC)-tagged bacteriophage.

### SUMMARY

Embodiments of the invention comprise a recombinant bacteriophage, methods, systems for the detection of microorganisms. The invention is defined in the claims..

The invention comprises a recombinant bacteriophage comprising an indicator gene inserted into a late gene region of the bacteriophage, wherein the indicator gene encodes a luciferase enzyme, wherein the indicator gene does not encode a fusion protein and wherein the indicator protein product does not comprise polypeptides of the natural bacteriophage.

In some embodiments, the invention comprises a method for detecting a microorganism of interest in a sample comprising the steps of incubating the sample with a recombinant bacteriophage that infects the microorganism of interest, wherein the recombinant bacteriophage comprises an indicator gene inserted into a late gene region of the bacteriophage such that expression of the indicator gene during bacteriophage replication following infection of host bacteria results in a soluble indicator protein product, wherein the indicator gene encodes a luciferase enzyme, and wherein the indicator gene does not encode a fusion protein and wherein the indicator protein product does not comprise polypeptides of the natural bacteriophage; and detecting the indicator protein product, wherein positive detection of the indicator protein product indicates that the microorganism of interest is present in the sample.

In other embodiments, the invention comprises a system for rapid detection of a microorganism of interest in a sample, comprising a recombinant bacteriophage that infects the microorganism of interest, wherein the recombinant bacteriophage comprises an indicator gene inserted into a late gene region of the bacteriophage as the indicator moiety such that the expression of the indicator gene during bacteriophage replication following infection of host bacteria results in a soluble indicator protein product, wherein the indicator gene encodes a luciferase enzyme, and wherein the indicator gene does not encode a fusion protein and wherein the indicator protein product does not comprise polypeptides of the natural bacteriophage; a component for incubating the sample with the recombinant bacteriophage specific for the microorganism of interest; and a component for detecting the indicator moiety.

In still other embodiments, the disclosure comprises non-transient computer readable media for use with methods or systems according to the disclosure.

### BRIEF DESCRIPTION OF THE FIGURES

The present invention may be better understood by referring to the following nonlimiting figures.

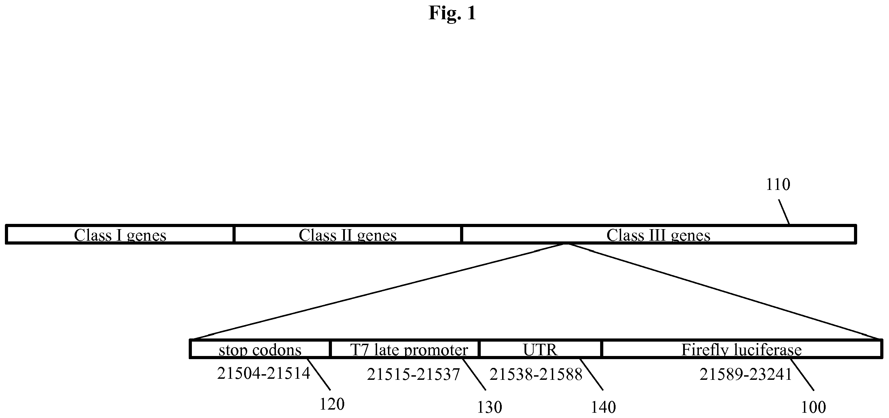

**Figure 1** depicts an indicator phage construct according to an embodiment of the invention illustrating insertion of a genetic construct comprising Firefly luciferase gene and a T7 late promoter inserted into the late (class III) region of a bacteriophage. Also depicted is a sequence comprising stop codons in all three reading frames to prevent read-through and an untranslated region (UTR).

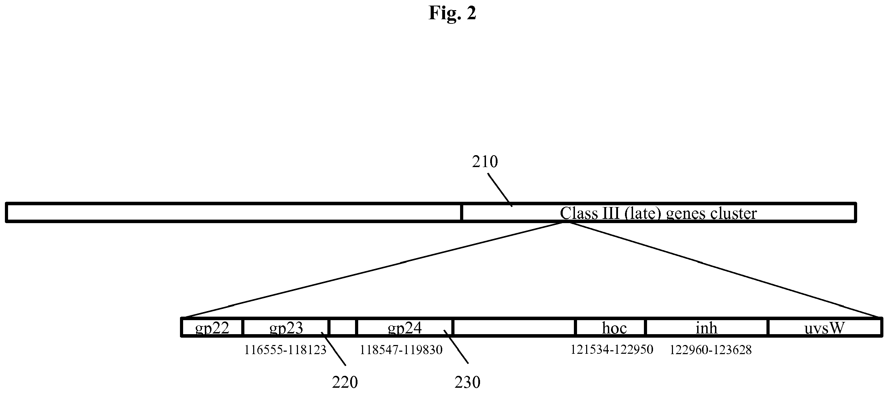

**Figure 2** shows the genome of bacteriophage JG04, a T4-like bacteriophage which was isolated from sewage treatment plant samples and shares ∼98% homology with T4-like phage RB69. Capsid proteins gp23 and gp24 are within the late gene region, consisting of structural genes, which code for virion proteins. As these virion proteins are expressed at a very high level, any genes inserted into this region can be expected to have similar expression levels, as long as late gene promoters and/or other similar control elements are used.

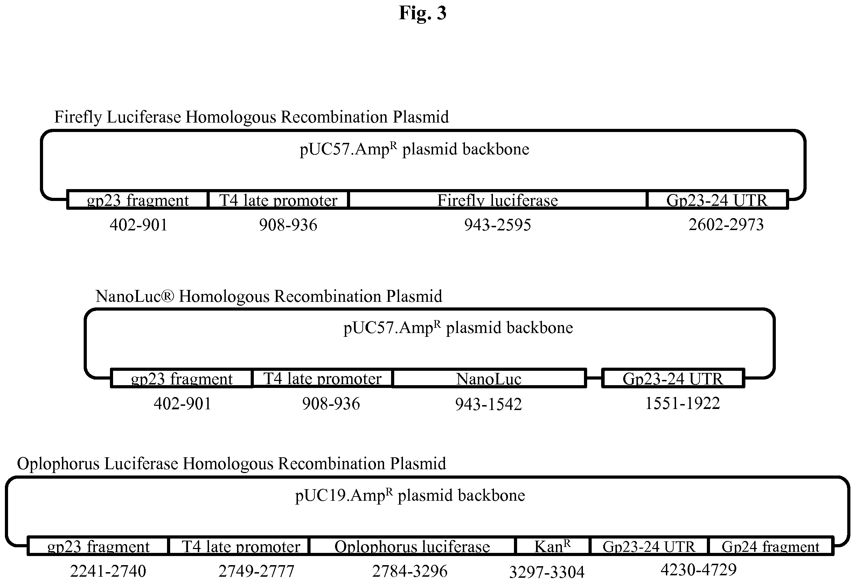

**Figure 3** shows three homologous recombination plasmid constructs carrying three different luciferase genes. Firefly luciferase, NANOLUC® luciferase, and Oplophorus luciferase genes are each inserted into a pUC57.Amp^{R} plasmid backbone. In each construct, a fragment of the gp23 capsid protein gene is followed by a T4 late promoter, the respective luciferase gene, and a gp23-24 untranslated region. The Oplophorus construct additionally comprises a fragment of the gp24 gene downstream of the untranslated region.

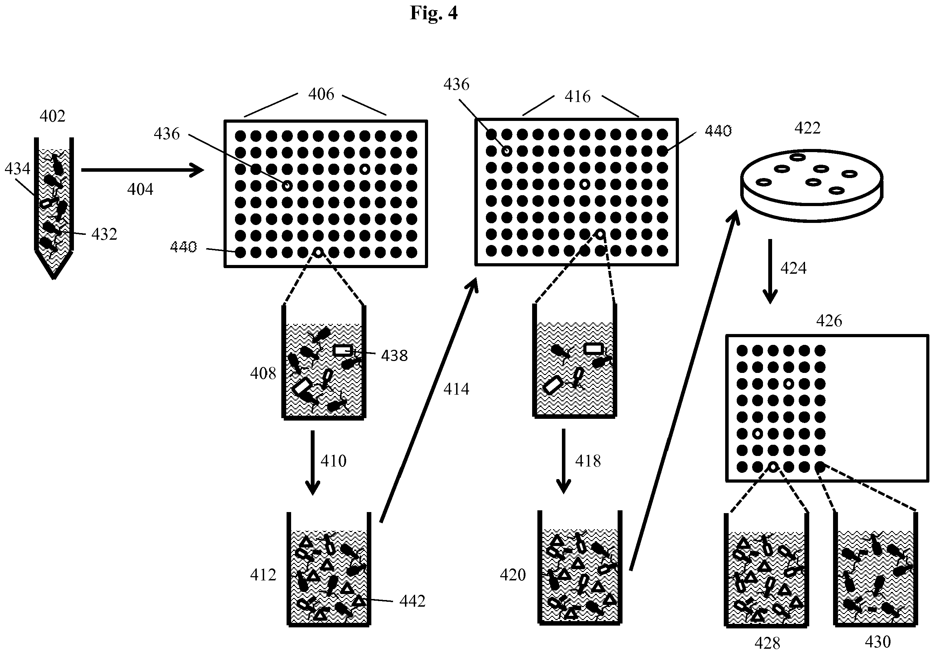

**Figure 4** depicts the isolation of recombinant phage from modifications of JG04 bacteriophage using the plasmid constructs such as those shown in Figure 3 using a series of sequential infection and dilution steps to identify recombinant phage that express an indicator gene.

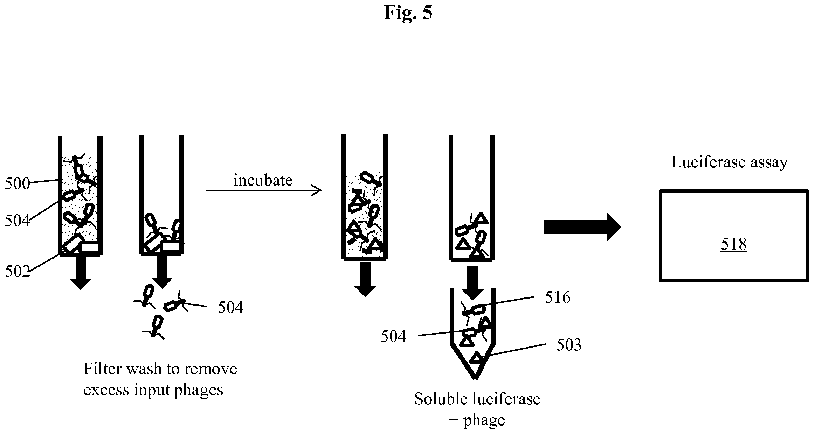

**Figure 5** depicts the use of indicator phage encoding a soluble luciferase to detect bacterial cells via detection of luciferase generated from replication of progeny phage during infection of the bacterial cells, according to an embodiment of the invention

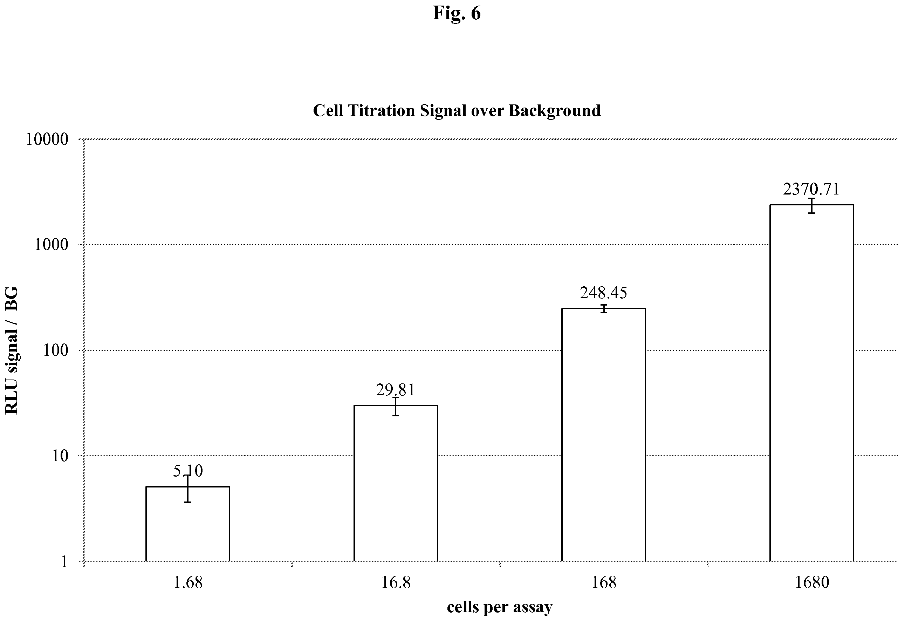

**Figure 6** demonstrates sensitivity for detection of target bacteria using indicator phage with spin columns, according to an embodiment of the invention. The approximate number of *E. coli* O157:H7 cells in each assay is indicated on the X-axis. The signal provided by the soluble luciferase produced upon infection of the bacteria is shown on the Y-axis as relative luminal units (RLU) over background (BG) signal (i.e., no cells) (RLU/BG).

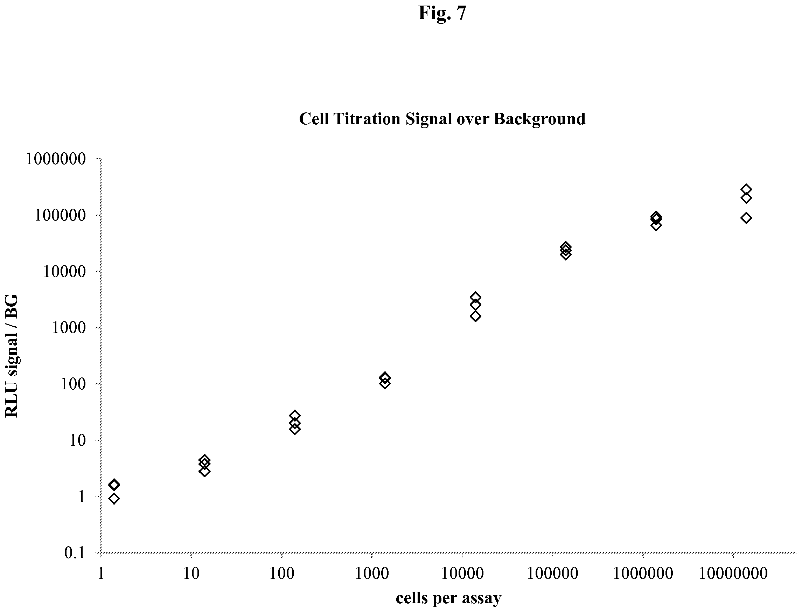

**Figure 7** demonstrates detection of target bacteria over a range of titers using indicator phage with spin columns, according to an embodiment of the invention. The approximate number *of E. coli* O157:H7 cells in each assay is indicated on the X-axis. The signal provided by the soluble luciferase produced upon infection of the bacteria is shown on the Y-axis as relative luminal units (RLU) over background (BG) signal (i.e., no cells) (RLU/BG).

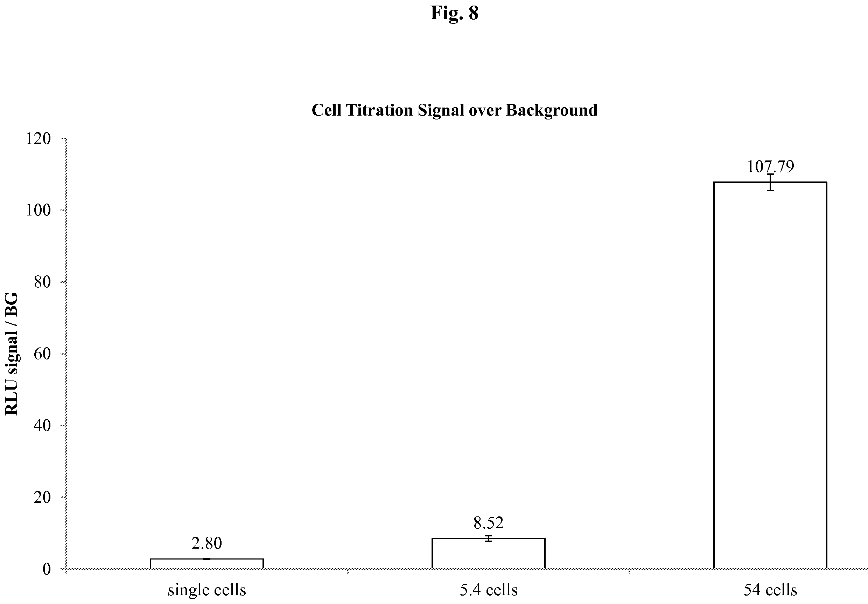

**Figure 8** demonstrates sensitivity for detection of target bacteria using indicator phage with 96-well filter plates, according to an embodiment of the invention. The approximate number of *E. coli* O157:H7 cells in each assay is indicated on the X-axis. The signal provided by the soluble luciferase produced upon infection of the bacteria is shown on the Y-axis as relative luminal units (RLU) over background (BG) signal (i.e., no cells) (RLU/BG).

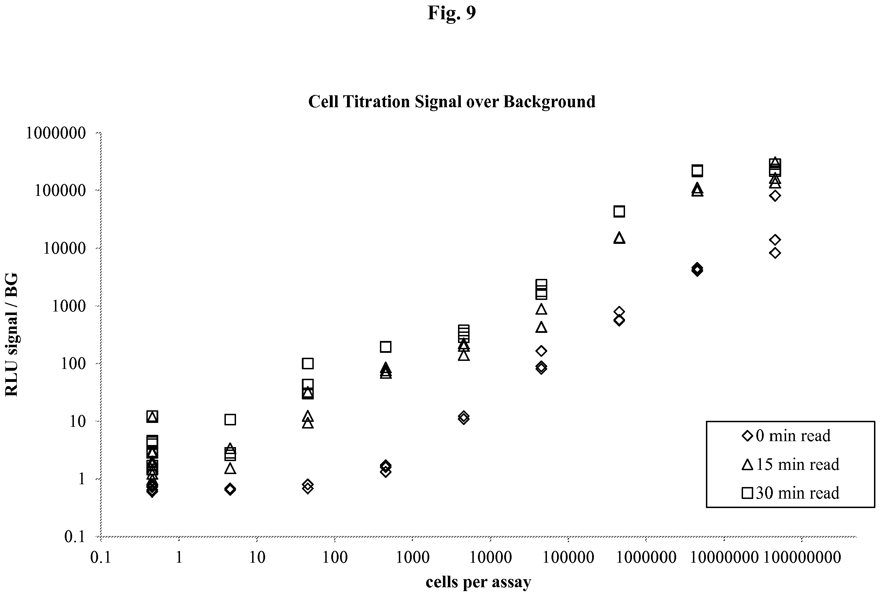

**Figure** 9 demonstrates detection of target bacteria over a range of titers using indicator phage with 96-well filter plates, according to an embodiment of the invention. The approximate number of *E. coli* O157:H7 cells in each assay is indicated on the X-axis. The signal provided by the soluble luciferase produced upon infection of the bacteria is shown on the Y-axis as relative luminal units (RLU) over background (BG) signal (i.e., no cells) (RLU/BG). The detection of luciferase activity may be continued for an extended period of time. Here, reading ◇ represents 0 time (i.e., a baseline control); Δ after 15 minutes (min); and □ after 30 minutes.

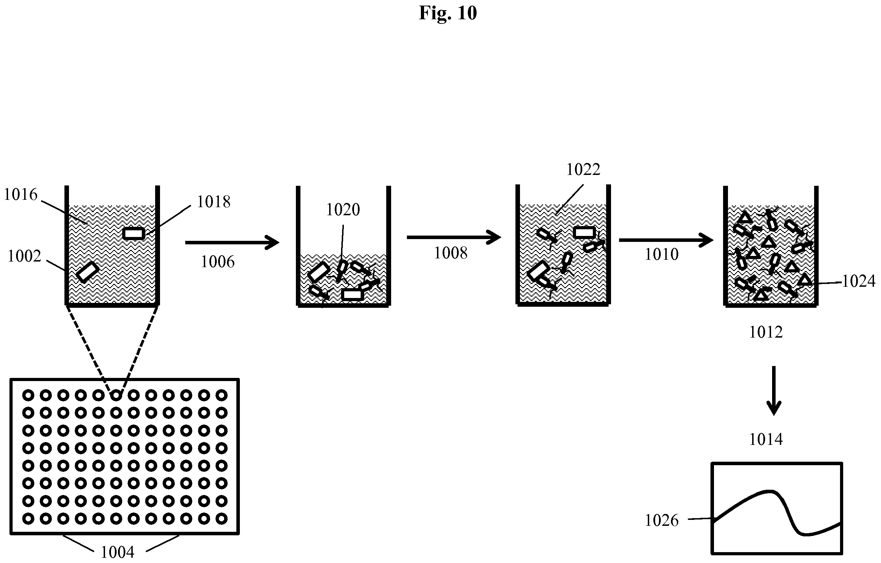

**Figure 10** depicts a filter plate assay for detecting bacteria of interest using a modified bacteriophage according to an embodiment of the invention where bacteria and recombinant phage are incubated on filter plates and after generation of progeny bacteriophage the indicator protein is detected directly without removal of the incubation medium.

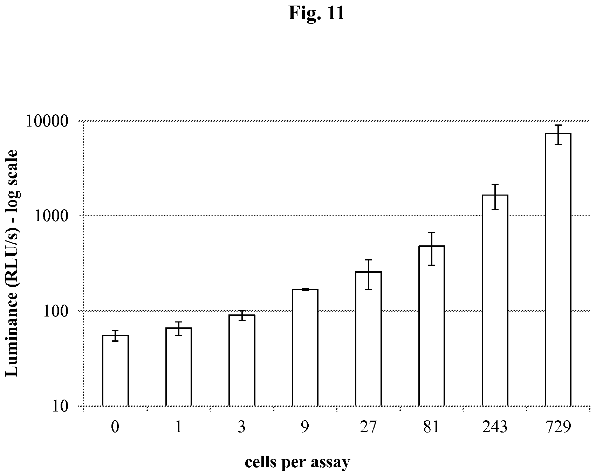

**Figure 11** shows results from a filter plate assay using JG04-NANOLUC® phage to detect *E. coli* O157:H7 cells in samples with known cell numbers. The approximate number of *E. coli* O157:H7 cells in each assay is indicated on the X-axis. The signal provided by the soluble luciferase produced upon infection of the bacteria is shown on the Y-axis as relative luminal units (RLU) over background (BG) signal (i.e., no cells) (RLU/BG).

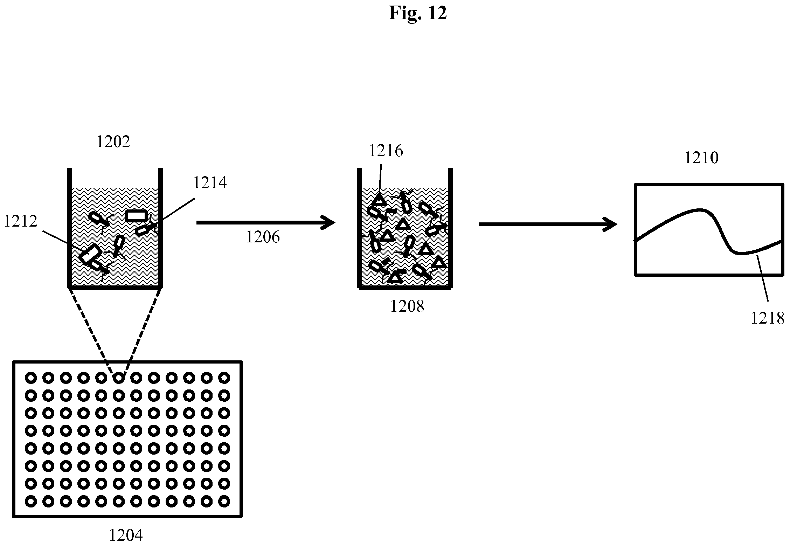

**Figure 12** depicts a "No Concentration Assay" for detecting a bacterium of interest using a modified bacteriophage according to an embodiment of the invention.

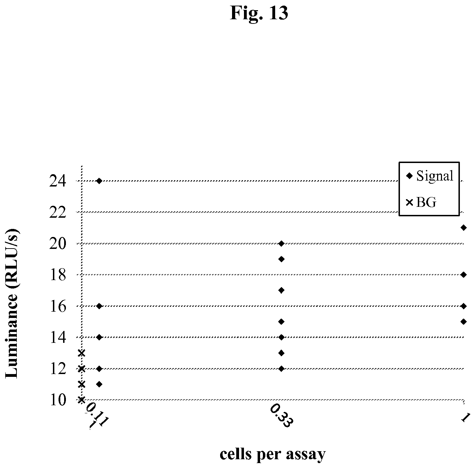

**Figure 13** shows results from a No Concentration Assay using JG04-OpLuc phage to detect *E. coli* O157:H7 cells in samples with known cell numbers in the low number range. The approximate number of *E. coli* 0157:H7 cells in each assay is indicated on the X-axis. The signal provided by the soluble luciferase produced upon infection of the bacteria is shown on the Y-axis as relative luminal units (RLU) as compared to background (X) signal (i.e., no cells).

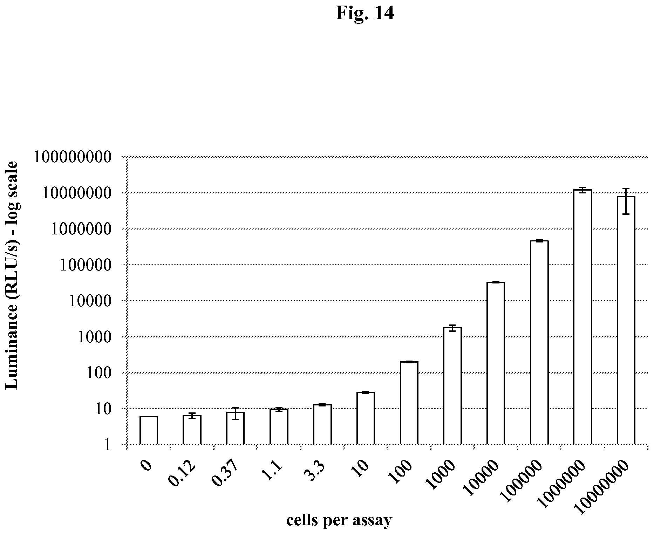

**Figure 14** shows results from a No Concentration Assay using JG04-OpLuc phage to detect *E. coli* O157:H7 cells in samples with known cell numbers in the low to high number range. The approximate number of *E. coli* 0157:H7 cells in each assay is indicated on the X-axis. The signal provided by the soluble luciferase produced upon infection of the bacteria is shown on the Y-axis as relative luminal units (RLU).

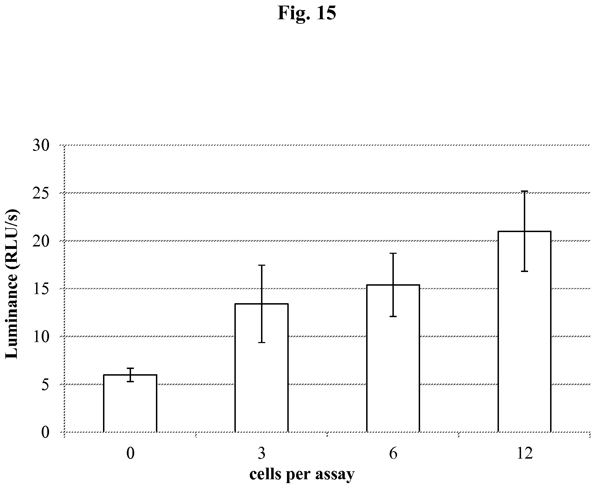

**Figure 15** shows results from a No Concentration Assay using JG04-OpLuc phage to detect *E. coli* O157:H7 cells in Vegetable Wash samples with known cell numbers. The approximate number of *E. coli* O157:H7 cells in each assay is indicated on the X-axis. The signal provided by the soluble luciferase produced upon infection of the bacteria is shown on the Y-axis as relative luminal units (RLU).

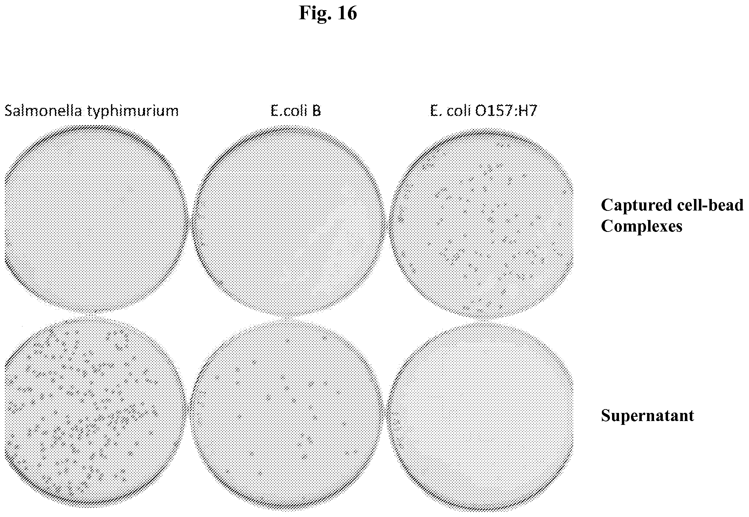

**Figure 16** demonstrates specific and quantitative capture of *E. coli* O157:H7 using affinity-purified, surface-specific antibodies, according to an embodiment of the invention.

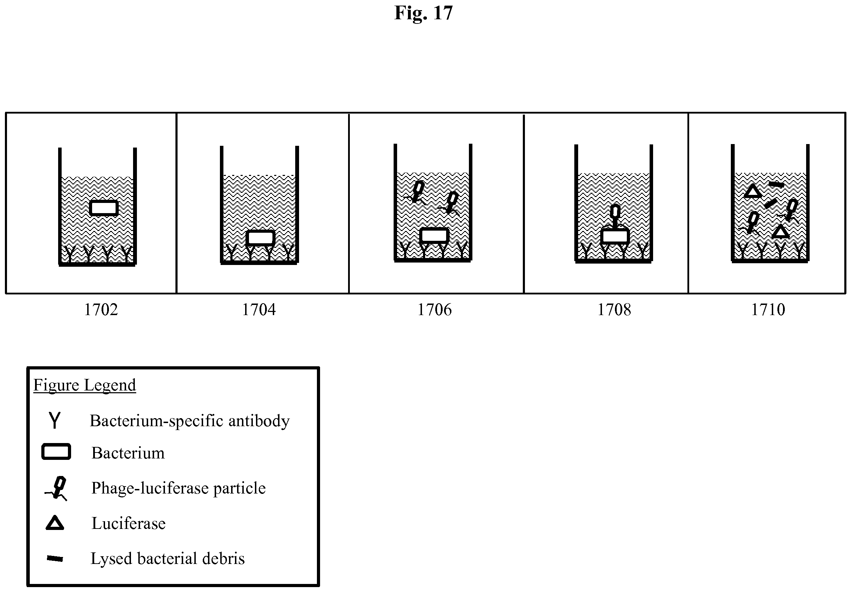

**Figure 17** depicts a Hybrid Immuno-Phage (HIP) Assay for detecting a bacterium of interest using a modified bacteriophage according to an embodiment of the invention wherein antibodies to the microorganism of interest are used to capture the microorganism on the surface of the assay well prior to incubation with a recombinant infectious agent having an indicator gene.

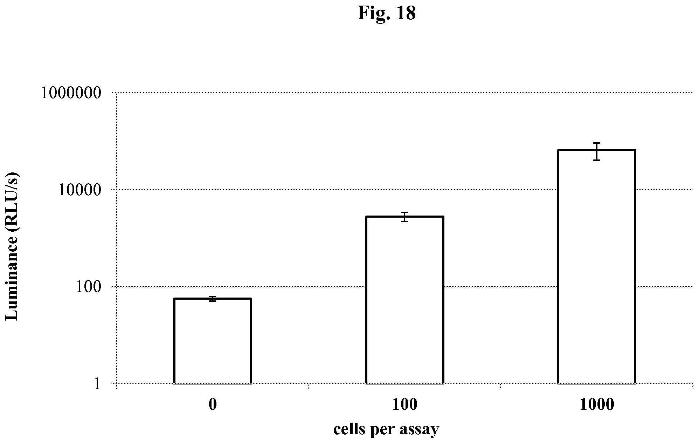

**Figure 18** shows results from a HIP assay using JG04-NANOLUC® phage to detect *E. coli* O157:H7 cells in samples with known cell numbers, on a logarithmic scale. The approximate number of *E. coli* O157:H7 cells in each assay is indicated on the X-axis. The signal provided by the soluble luciferase produced upon infection of the bacteria is shown on the Y-axis as relative luminal units (RLU).

### DETAILED DESCRIPTION OF THE INVENTION

Disclosed herein are compositions, methods and systems that demonstrate surprising sensitivity for detection of a microorganism of interest in test samples (e.g., biological, food, water, and clinical samples). Detection can be achieved in a shorter timeframe than was previously thought possible using genetically modified infectious agents in assays performed without any culturing for enrichment, or in some embodiments with minimal incubation times during which microorganisms could potentially multiply. Also surprising is the success of using high multiplicity of infection (MOI), or high concentrations of plaque forming units (PFU), for incubation with a test sample. Such high phage concentrations (PFU/mL) were previously purported to be detrimental to microorganism detection assays, as they were purported to cause "lysis from without."

The compositions, methods, systems and kits of the disclosure may comprise infectious agents for use in detection of such microorganisms. The invention comprises a a recombinant bacteriophage having an indicator gene inserted into a late gene region of the bacteriophage, wherein the indicator gene encodes a luciferase enzyme, and wherein the indicator gene does not encode a fusion protein and wherein the indicator protein product does not comprise polypeptides of the natural bacteriophage. In certain embodiments, expression of the indicator gene during bacteriophage replication following infection of a host bacterium results in a soluble indicator protein product. The indicator gene is inserted into a late gene (i.e., class III) region of the bacteriophage. The bacteriophage may be derived from T7, T4, JG04, or another natural bacteriophage.

In some aspects, the invention comprises a method for detecting a microorganism of interest. The method uses an infectious agent for detection of the microorganism of interest; the microorganism of interest is a bacterium and the infectious agent is a bacteriophage. Thus, the method comprises detection of a microorganism of interest in a sample by incubating the sample with a recombinant bacteriophage that infects the microorganism of interest. The recombinant bacteriophage comprises an indicator gene, wherein the indicator gene encodes a luciferase enzyme, and wherein the indicator gene does not encode a fusion protein and wherein the indicator protein product does not comprise polypeptides of the natural bacteriophage. The indicator gene is inserted into a late gene region of the bacteriophage such that expression of the indicator gene during bacteriophage replication following infection of host bacteria results in a soluble indicator protein product. The method comprises detecting the indicator protein product, wherein positive detection of the indicator protein product indicates that the microorganism of interest is present in the sample.

In certain embodiments, the invention may comprise a system. The system contains the recombinant bacteriophage of the invention. Also, the system may comprise at least some of the components for performing the method. In certain embodiments of the disclosure, the system is formulated as a kit. Thus, in certain embodiments, the invention comprises a system for rapid detection of a microorganism of interest in a sample, comprising: a recombinant bacteriophage that infects the microorganism of interest, wherein the recombinant bacteriophage comprises an indicator gene inserted into a late gene region of the bacteriophage as the indicator moiety such that the expression of the indicator gene during bacteriophage replication following infection of host bacteria results in a soluble indicator protein product, wherein the indicator gene encodes a luciferase enzyme, and wherein the indicator gene does not encode a fusion protein and wherein the indicator protein product does not comprise polypeptides of the natural bacteriophage; a component for incubating the sample with the recombinant bacteriophage specific for the microorganism of interest, ; and a component for detecting the indicator moiety. In yet other embodiments, the disclosure comprises software for use with the methods or systems of the disclosure.

Thus, the present invention solves a need by using bacteriophage-based methods for amplifying a detectable signal indicating the presence of bacteria. In certain embodiments as little as a single bacterium is detected. The principles applied herein can be applied to the detection of a variety of microorganisms. Because of numerous binding sites for an infectious agent on the surface of a microorganism, the capacity to produce one hundred or more agent progeny during infection, and the potential for high level expression of an encoded indicator moiety, the infectious agent or an indicator moiety can be more readily detectable than the microorganism itself. In this way, embodiments of the present invention can achieve tremendous signal amplification from even a single infected cell.

Aspects of the present invention utilize the high specificity of binding agents that can bind to particular microorganisms, i.e. the binding component of bacteriophage, as a means to detect and/or quantify the specific microorganism in a sample. The present invention utilizes the high specificity of bacteriophage.

In some embodiments of the disclosure, detection is achieved through an indicator moiety associated with the binding agent specific for the microorganism of interest. For example, an infectious agent may comprise an indicator moiety. In some embodiments the indicator may be encoded by the infectious agent which is a bacteriophage, and the bacteriophage is designated an indicator phage as defined in the claims.

Some embodiments of the disclosure disclosed and described herein utilize the discovery that a single microorganism is capable of binding specific recognition agents, such as phage. Following infection and replication of the phage, progeny phage may be detected via an indicator moiety expressed during phage replication. This principle allows amplification of indicator signal from one or a few cells based on specific recognition of microorganism surface receptors. For example, by exposing even a single cell of a microorganism to a plurality of phage, thereafter allowing amplification of the phage and high-level expression of an encoded indicator gene product during replication, the indicator signal is amplified such that the single microorganism is detectable.

Embodiments of the methods and systems of the disclosure can be applied to detection and quantification of a variety of microorganisms (e.g., bacteria, fungi, yeast) in a variety of circumstances, including but not limited to detection of pathogens from food, water, clinical and commercial samples. The methods of the present disclosure provide high detection sensitivity and specificity rapidly and without the need for traditional biological enrichment (e.g., culturing for enrichment), which is a surprising aspect as all available methods require culturing. In some embodiments detection is possible within 1-2 replication cycles of the bacteriophage or virus, which goes against conventional wisdom, as it doesn't take advantage of the phage's natural ability to amplify itself and the luciferase signal.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. Known methods and techniques are generally performed according to conventional methods well known in the art and as described in various general and more specific references that are discussed throughout the present specification unless otherwise indicated. Enzymatic reactions and purification techniques are performed according to manufacturer's specifications, as commonly accomplished in the art or as described herein. The nomenclatures used in connection with the laboratory procedures and techniques described herein are those well-known and commonly used in the art.

The following terms, unless otherwise indicated, shall be understood to have the following meanings:
As used herein, the terms "a", "an", and "the" can refer to one or more unless specifically noted otherwise.

The use of the term "or" is used to mean "and/or" unless explicitly indicated to refer to alternatives only or the alternatives are mutually exclusive, although the disclosure supports a definition that refers to only alternatives and "and/or." As used herein "another" can mean at least a second or more.

Throughout this application, the term "about" is used to indicate that a value includes the inherent variation of error for the device, the method being employed to determine the value, or the variation that exists among samples.

The term "solid support" or "support" means a structure that provides a substrate and/or surface onto which biomolecules may be bound. For example, a solid support may be an assay well (i.e., such as a microtiter plate or multi-well plate), or the solid support may be a location on a filter, an array, or a mobile support, such as a bead or a membrane (e.g., a filter plate or lateral flow strip).

The term "antibody" includes monoclonal antibodies, polyclonal antibodies, synthetic antibodies and chimeric antibodies, e.g., generated by combinatorial mutagenesis and phage display. The term "antibody" also includes mimetics or peptidomimetics of antibodies. Peptidomimetics are compounds based on, or derived from, peptides and proteins. The peptidomimetics of the present invention typically can be obtained by structural modification of a known peptide sequence using unnatural amino acids, conformational restraints, isosteric replacement, and the like. Fragments of antibodies may serve in place of antibodies in some embodiments. "Surface-specific antibodies" as used herein bind to molecules exposed on the outer surface of a specific microorganism.

As used herein, the term "free antibodies" refers to antibodies which are in solution and may move freely through a liquid; i.e., they are not initially bound to a solid support or otherwise constrained.

As used herein, "affinity-purified" or "affinity-purification" refers to a series of steps used to prepare and treat antibodies such that they exhibit optimal specificity and sensitivity, including minimal cross-reactivity with undesired epitopes. For example, removal of undesired lipids and proteins from antiserum may first be achieved by salt precipitation steps. Further positive selection (also called "affinity-purification") and/or negative selection (also called "reverse-purification") may be achieved by passing the remaining antibodies over a support (e.g., agarose columns) comprising surface antigens designed to retain antibodies with particular epitope affinities. In some examples, where the starting antiserum is polyclonal, the purified antibodies that remain after these selection steps are able to recognize many different epitopes on the surface of the microorganism of interest, but they do not recognize the surface epitopes of other microorganisms.

The term "binding agent" refers to a molecule that can specifically and selectively bind to a second (i.e., different) molecule of interest. The interaction may be non-covalent, for example, as a result of hydrogen-bonding, van der Waals interactions, or electrostatic or hydrophobic interactions, or it may be covalent. The term "soluble binding agent" refers to a binding agent that is not associated with (i.e., covalently or non-covalently bound) to a solid support.

As used herein, an "analyte" refers to a molecule, compound or cell that is being measured. The analyte of interest may, in certain embodiments, interact with a binding agent. As described herein, the term "analyte" may refer to a protein or peptide of interest. An analyte may be an agonist, an antagonist, or a modulator. Or, an analyte may not have a biological effect. Analytes may include small molecules, sugars, oligosaccharides, lipids, peptides, peptidomimetics, organic compounds and the like.

The term "detectable moiety" or "detectable biomolecule" or "reporter" or "indicator moiety" refers to a molecule that can be measured in a quantitative assay. For example, an indicator moiety may comprise an enzyme that may be used to convert a substrate to a product that can be measured. An indicator moiety may be an enzyme that catalyzes a reaction that generates bioluminescent emissions (e.g., luciferase). Or, an indicator moiety may be a radioisotope that can be quantified. Or, an indicator moiety may be a fluorophore. Or, other detectable molecules may be used.

As used herein, "bacteriophage" or "phage" includes one or more of a plurality of bacterial viruses. In this disclosure, the terms "bacteriophage" and "phage" include viruses such as mycobacteriophage (such as for TB and paraTB), mycophage (such as for fungi), mycoplasma phage, and any other term that refers to a virus that can invade living bacteria, fungi, mycoplasma, protozoa, yeasts, and other microscopic living organisms and uses them to replicate itself. Here, "microscopic" means that the largest dimension is one millimeter or less. Bacteriophages are viruses that have evolved in nature to use bacteria as a means of replicating themselves. A phage does this by attaching itself to a bacterium and injecting its DNA (or RNA) into that bacterium, and inducing it to replicate the phage hundreds or even thousands of times. This is referred to as phage amplification.

As used herein, "late gene region" refers to a region of a viral genome that is transcribed late in the viral life cycle. The late gene region typically includes the most abundantly expressed genes (e.g., structural proteins assembled into the bacteriophage particle). Late genes are synonymous with class III genes and include genes with structure and assembly functions. For example, the late genes (synonymous with class III,) are transcribed in T7, e.g., from 8 minutes after infection until lysis, class I (e.g., RNA polymerase) is early from 4-8 minutes, and class II from 6-15 minutes, so there is overlap in timing of II and III. A late promoter is one that is naturally located and active in such a late gene region.

As used herein, "culturing for enrichment" refers to traditional culturing, such as incubation in media favorable to propagation of microorganisms, and should not be confused with other possible uses of the word "enrichment," such as enrichment by removing the liquid component of a sample to concentrate the microorganism contained therein, or other forms of enrichment that do not include traditional facilitation of microorganism propagation. Culturing for enrichment for very short periods of time may be employed in some embodiments of methods described herein, but is not necessary and is for a much shorter period of time than traditional culturing for enrichment, if it is used at all.

As used herein "recombinant" refers to genetic (i.e., nucleic acid) modifications as usually performed in a laboratory to bring together genetic material that would not otherwise be found. This term is used interchangeably with the term "modified" herein.

### Samples

Each of the embodiments of the methods and systems of the invention can allow for the rapid detection and quantification of microbes in a sample. For example, methods according to the present invention can be performed, most preferably, in about two hours or less.

Microbes detected by the methods and systems of the present disclosure include pathogens that are of commercial, medical or veterinary concern. Such pathogens of the disclosure include Gram-negative bacteria, Gram-positive bacteria, mycoplasmas and viruses. Any microbe for which an infectious agent that is specific for the particular microbe has been identified can be detected by the methods of the present disclosure. Those skilled in the art will appreciate that there is no limit to the application of the present methods of the disclosure other than the availability of the necessary specific infectious agent/microbe pairs.

Bacterial cells detectable by the present invention include, but are not limited to, bacterial cells that are food or water borne pathogens. Bacterial cells detectable by the present invention include, but are not limited to, all species of *Salmonella,* all strains of *Escherichia coli,* including, but not limited to *E. coli* 0157:H7, all species of *Listeria,* including, but not limited to *L. monocytogenes,* and all species of *Campylobacter.* Bacterial cells detectable by the present invention include, but are not limited to, bacterial cells that are pathogens of medical or veterinary significance. Such pathogens include, but are not limited to, *Bacillus spp., Bordetella pertussis, Camplyobacterjejuni, Chlamydia pneumoniae, Clostridium perfringens, Enterobacter spp*., *Klebsiella pneumoniae, Mycoplasma pneumoniae, Salmonella typhi, Shigella sonnei, Staphylococcus aureus, and Streptococcus spp.*

The sample may be environmental or food or water samples and medical or veterinary samples. Samples may be liquid, solid, or semi-solid. Samples may be swabs of solid surfaces. Samples may include environmental materials, such as the water samples, or the filters from air samples or aerosol samples from cyclone collectors. Samples may be of meat, poultry, processed foods, milk, cheese, or other dairy products. Medical or veterinary samples include, but are not limited to, blood, sputum, cerebrospinal fluid, and fecal samples and different types of swabs.

Samples may be used directly in the detection methods of the present invention, without preparation, concentration, or dilution. For example, liquid samples, including but not limited to, milk and juices, may be assayed directly. Samples may be diluted or suspended in solution, which may include, but is not limited to, a buffered solution or a bacterial culture medium. A sample that is a solid or semi-solid may be suspending in a liquid by mincing, mixing or macerating the solid in the liquid. A sample should be maintained within a pH range that promotes bacteriophage attachment to the host bacterial cell. A sample should also contain the appropriate concentrations of divalent and monovalent cations, including but not limited to Na⁺, Mg²⁺, and K⁺. Preferably a sample is maintained at a temperature that maintains the viability of any pathogen cells contained within the sample.

Preferably throughout detection assays, the sample is maintained at a temperature that maintains the viability of any pathogen cell present in the sample. During steps in which bacteriophages are attaching to bacterial cells, it is preferable to maintain the sample at a temperature that facilitates bacteriophage attachment. During steps in which bacteriophages are replicating within an infected bacterial cell or lysing such an infected cell, it is preferable to maintain the sample at a temperature that promotes bacteriophage replication and lysis of the host. Such temperatures are at least about 25 degrees Celsius (C), more preferably no greater than about 45 degrees C, most preferably about 37 degrees C. It is also preferred that the samples be subjected to gentle mixing or shaking during bacteriophage attachment, replication and cell lysis.

Assays may include various appropriate control samples. For example, control samples containing no bacteriophages or control samples containing bacteriophages without bacteria may be assayed as controls for background signal levels.

### Indicator Infectious Agents

As described in more detail herein, the compositions, methods, systems and kits of the disclosure may comprise infectious agents for use in detection of such microorganisms. The invention comprises a recombinant bacteriophage having an indicator gene inserted into a late gene region of the bacteriophage, wherein the indicator gene encodes a luciferase enzyme, and the indicator gene does not encode a fusion protein and wherein the indicator protein product does not comprise polypeptides of the natural bacteriophage. For example, in certain embodiments, expression of the indicator gene during bacteriophage replication following infection of a host bacterium results in a soluble indicator protein product. The indicator gene is inserted into a late gene region of the bacteriophage. In a recombinant bacteriophage the late gene region may be a class III gene region.

In some embodiments, the indicator phage is derived from T7, T4 or another phage. An indicator bacteriophage may also be derived from T7-like, T4-like, JG04, JG04-like, or any other bacteriophage having a genome with at least 99, 98, 97, 96, 95, 94, 93, 92, 91 90, 89, 88, 87, 86, 85, 84, 83, 82, 81, 80, 79, 78, 77, 76, 75, 74, 73, 72, 71, or 70 % homology to T7, T7-like, T4, T4-like, JG04, or JG04-like phages. In some embodiments, the indicator phage is derived from a natural phage isolated from the environment, such as JG04 phage, a T4-like phage isolated as described in the Examples. The genetic modifications may avoid deletions of wild-type genes and thus remain more similar to the wild-type infectious agent than many commercially available phage, e.g. T7SELECT®415. Such naturally derived bacteriophage may be more specific for bacteria that are found in the environment than bacteriophage that are commercially available and as such, genetically distinct from phage found in the environment.

Moreover, phage genes thought to be nonessential may have unrecognized function. For example, an apparently nonessential gene may have an important function in elevating burst size with subtle cutting, fitting, or trimming functions in assembly. As such, deleting genes to insert an indicator may be detrimental. Most phages can package a DNA that is a few percent larger than their natural genome. With this consideration, a smaller indicator gene may be a more appropriate choice for modifying a bacteriophage, especially one with a smaller genome. OpLuc and NANOLUC® proteins are only about 20 kDa (approximately 500-600 bp to encode), while FLuc is about 62 kDa (approximately 1,700 bp to encode). For comparison, the genome of T7 is around 40 kbp, while the T4 genome is about 170 kbp.

In some indicator phage embodiments, the indicator gene may be inserted into an untranslated region to avoid disruption of functional genes, leaving wild-type phage genes intact, which may lead to greater fitness when infecting non-laboratory strain bacteria. Additionally, stop codons at all three reading frames may help to increase expression by reducing read-through, also known as leaky expression. This strategy may also eliminate the possibility of a fusion protein being made at low levels, which would manifest as background signal (e.g., luciferase) that cannot be separated from the phage.

An indicator gene may express a variety of biomolecules. In an embodiment of the disclosure, the indicator gene is a gene that expresses a detectable product or an enzyme that produces a detectable product. The indicator gene of the invention encodes a luciferase enzyme. Various type of luciferase may be used. In alternate embodiments, and as described in more detail herein, the luciferase is one of Oplophorus luciferase, Firefly luciferase, or an engineered luciferase.

Thus, the present invention comprises a modified bacteriophage comprising a non-bacteriophage indicator gene in the late (class III) gene region. In some embodiments, the non-native indicator gene is under the control of a late promoter. Using a viral late gene promoter insures the reporter gene (luciferase) is not only expressed at high levels, like viral capsid proteins, but also does not shut down like endogenous bacterial genes or even early viral genes.

In some embodiments, the late promoter is a T4- or T7-like promoter, or another phage promoter similar to that found in the natural phage without genetic modification. The late gene region may be a class III gene region, and the bacteriophage may be derived from T7, T4, T4-like, JG04, or another natural bacteriophage having a genome with at least 90% homology to T7, T4 or T4-like phages. In preferred embodiments, the indicator gene does not encode a fusion protein.

Genetic modifications to infectious agents may include insertions, deletions, or substitutions of a small fragment of nucleic acid, a substantial part of a gene, or an entire gene. In some embodiments, inserted or substituted nucleic acids comprise non-native sequences. A non-native indicator gene may be inserted into a bacteriophage genome such that it is under the control of a bacteriophage promoter. In embodiments, the non-native indicator gene is not part of a fusion protein. That is, in embodiments, a genetic modification may be configured such that the indicator protein product does not comprise polypeptides of the natural bacteriophage. In some embodiments, the indicator protein product is soluble. In some embodiments, the invention comprises a method for detecting a microorganism of interest comprising the step of incubating a test sample with such a modified bacteriophage.

In some embodiments, the non-native indicator gene is not contiguous with a gene encoding a structural phage protein and therefore does not yield a fusion protein. In some embodiments, expression of the indicator gene in progeny bacteriophage following infection of host bacteria results in a soluble protein product.

Unlike systems which employ a fusion of a detection moiety to the capsid protein (i.e., a fusion protein), some embodiments of the present invention express a soluble luciferase. This may greatly increase the sensitivity of the assay (down to a single bacterium), and simplifies the assay, allowing the assay to be completed in less than an hour for some embodiments, as opposed to several hours due to additional purification steps required with constructs that produce detectable fusion proteins. Further, fusion proteins may be less active than soluble proteins due, e.g., to protein folding constraints that may alter the conformation of the enzyme active site or access to the substrate.

Furthermore, fusion proteins by definition limit the number of the moieties attached to subunits of a protein in the bacteriophage. For example, using a commercially available system designed to serve as a platform for a fusion protein would result in about 415 copies of the fusion moiety, corresponding to the about 415 copies of the gene 10B capsid protein in each T7 bacteriophage particle. Without this constraint, infected bacteria can be expected to express many more copies of the detection moiety (e.g., luciferase) than can fit on the bacteriophage. Additionally, large fusion proteins, such as a capsid-luciferase fusion, may inhibit assembly of the bacteriophage particle, thus yielding fewer bacteriophage progeny. Thus a soluble, non-fusion indicator gene product may be preferable.

In some embodiments of the disclosure, the indicator phage encodes a detectable enzyme. The indicator may emit light and/or may be detectable by a color change. Various appropriate enzymes are commercially available, such as alkaline phosphatase (AP), horseradish peroxidase (HRP), or luciferase (Luc). In some embodiments of the disclosure, these enzymes may serve as the indicator moiety. In some embodiments, Firefly luciferase is the indicator moiety. In some embodiments, Oplophorus luciferase is the indicator moiety. In some embodiments, NANOLUC® is the indicator moiety. Other engineered luciferases or other enzymes that generate detectable signals may also be appropriate indicator moieties.

Also, the use of a soluble detection moiety eliminates the need to isolate contaminating parental phage from the lysate of the infected sample cells. With a fusion protein system, any bacteriophage used to infect sample cells would have the detection moiety attached, and would be indistinguishable from the daughter bacteriophage also containing the detection moiety. As detection of sample bacteria relies on the detection of newly created (*de novo* synthesized) detection moiety, using fusion constructs requires additional steps to separate old (parental) moieties from newly created (daughter bacteriophage) moieties. This may be accomplished by washing the infected cells multiple times, prior to the completion of the bacteriophage life cycle, inactivating excess parental phage after infection by physical or chemical means, and/or chemically modifying the parental bacteriophage with a binding moiety (such as biotin), which can then be bound and separated (such as by streptavidin-coated sepharose beads). However, even with all these attempts at isolation, parental phage typically remain when a high multiplicity of infection (MOI) is used to assure infection of a low number of sample cells, creating background signal that may obscure detection of signal from infected cell progeny phage.

By contrast, with the soluble detection moiety expressed in some embodiments of the present invention, purification of the parental phage from the final lysate is unnecessary, as the parental phage will not have any detection moiety attached. Thus any detection moiety present after infection must have been created *de novo,* indicating the presence of an infected bacterium or bacteria. To take advantage of this benefit, the production and preparation of parental phage may include purification of the phage from any free detection moiety produced during the production of parental bacteriophage in bacterial culture. Standard bacteriophage purification techniques may be employed to purify some embodiments of phage according to the present invention, such as sucrose density gradient centrifugation, cesium chloride isopycnic density gradient centrifugation, HPLC, size exclusion chromatography, and dialysis or derived technologies (such as Amicon brand concentrators - Millipore, Inc.). The Examples herein describe the use cesium chloride isopycnic ultracentrifugation as part of the preparation of recombinant phage of the invention so as to separate parental phage particles from contaminating luciferase protein produced upon propagation of the phage in the bacteria stock. In this way, the recombinant bacteriophage of the invention is substantially free of any luciferase generated during production of the bacteria. Removal of residual luciferase present in the phage stock can substantially reduce background signal seen when the recombinant bacteriophage are incubated with a test sample.

In some embodiments of modified bacteriophage, the late promoter (class III promoter, e.g., from T7 or T4) has high affinity for RNA polymerase of the same native bacteriophage (e.g., T7 or T4, respectively) that transcribes genes for structural proteins assembled into the bacteriophage particle. These proteins are the most abundant proteins made by the phage, as each bacteriophage particle comprises dozens or hundreds of copies of these molecules. The use of a viral late promoter can ensure optimally high level of expression of the luciferase detection moiety. The use of a late viral promoter derived from, specific to, or active under the original wild-type bacteriophage the Indicator Phage is derived from (e.g., the T4 or T7 late promoter with a T4- or T7-based system) can further ensure optimal expression of the detection moiety. The use of a standard bacterial (non-viral/non-bacteriophage) promoter may in some cases be detrimental to expression, as these promoters are often down-regulated during bacteriophage infection (in order for the bacteriophage to prioritize the bacterial resources for phage protein production). Thus, in some embodiments, the phage is preferably engineered to encode and express at high level a soluble (free) indicator moiety, which does not limit expression to the number of subunits of a phage structural component.

Embodiments employing modified bacteriophage of the invention may allow rapid detection of specific bacterial strains, with total assay times as fast as 45 minutes - 1.5 hours. The amount of time required may be somewhat shorter or longer depending on the strain of bacteriophage and the strain of bacteria to be detected in the assay.

**Figure 1** depicts a schematic representation of the genomic structure of a recombinant bacteriophage of the invention, Indicator Phage T7SELECT®415-Luc. For the embodiment depicted in **Figure 1****,** the detection moiety is encoded by a Firefly luciferase gene 100 inserted within the late (class III) gene region 110, which is expressed late in the viral life cycle. Late genes are generally expressed at higher levels than other phage genes, as they code for structural proteins. Thus, in the embodiment of the recombinant phage depicted by **Figure 1****,** the indicator gene (i.e., Firefly luciferase) is inserted into the late gene region, just after gene 10B (major capsid protein), and is a construct comprising the Firefly luciferase gene 100. The construct depicted in **Figure 1** was designed to include stop codons 120 in all 3 reading frames to ensure luciferase is not incorporated into the gene 10B product. Also as depicted by **Figure 1****,** the construct may comprise the consensus T7 late promoter 130 to drive transcription and expression of the luciferase gene. The construct may also comprise a composite untranslated region synthesized from several T7 UTRs 140. This construct ensures soluble Firefly luciferase is produced such that expression is not limited to the number of capsid proteins inherent in the phage display system.

As noted herein, in certain embodiments, it may be preferred to utilize infectious agents that have been isolated from the environment for production of the infectious agents of the invention. In this way, infectious agents that are specific to naturally derived microorganisms may be generated.

For example, **Figure 2** shows the genome of bacteriophage JG04, a natural phage having about 98 % sequence homology to a T4-like bacteriophage, RB69. Isolation of the JG04 bacteriophage from a sewage treatment plant sample is described with particularity in the Examples herein. As discussed in the Examples, the capsid proteins, gp23 (220) and gp24 (230) are within the late gene region (210), consisting of structural genes, which code for virion proteins. As these virion proteins are expressed at a very high level, any genes inserted into this region can be expected to have similar expression levels, as long as late gene promoters and/or other similar control elements are used. The bacteriophage construct depicted in **Figure 2** has the sequence as set forth in SEQ ID NO: 3.

The compositions of the invention may comprise various infectious agents and/or indicator genes. For example, **Figure 3** shows three homologous recombination plasmid constructs carrying three different luciferase genes. Three constructs were made and used in recombination with JG04 to generate recombinant bacteriophage of the invention. Thus, the top construct in **Figure 3** shows a recombination plasmid having Firefly luciferase construct used for homologous recombination insertion of the Firefly luciferase into JG04: homologous recombination plasmid pUC57.FIR.Fluc, corresponding to SEQ ID NO. 5. The middle construct in **Figure 3** shows a recombination plasmid used for homologous recombination insertion of the NANOLUC® luciferase into JG04: homologous recombination plasmid pUC57.HR.NANOLUC® corresponding to SEQ ID NO. 6; and the lower construct in **Figure 3** shows a recombination plasmid used for homologous recombination insertion of the Oplophorus luciferase into JG04: homologous recombination plasmid pUC19.HR.OpLuc.KanR, corresponding to SEQ ID NO. 7.

In some embodiments, indicator phage according to the invention comprise JG04 genetically engineered to comprise any one of the three constructs shown in Figure 3. That is, an indicator phage comprising the sequence of SEQ ID NO. 3, further comprising additional sequence inserted between nucleotides 116,555 and 119,830 of SEQ ID NO. 3 corresponding to nucleotides 402 - 2,973 of SEQ ID NO. 5 (or a portion thereof); or nucleotides 402 - 1,922 of SEQ ID NO. 6 (or a portion thereof), also called JG04-NANOLUC® Indicator Phage in Examples 6 and 11 herein; or nucleotides 2,241 - 4,729 of SEQ ID NO. 7 (or a portion thereof), also called JG04-OpLuc Indicator Phage in Examples 7-9 herein. For example, a portion thereof may comprise the luciferase portion only (i.e., nucleotides 943 - 2,595 of SEQ ID NO. 5; or nucleotides 943 - 1,542 of SEQ ID NO. 6; or nucleotides 2,784 - 3,296 of SEQ ID NO. 7). A portion thereof may further comprise the T4 late promoter (i.e., nucleotides 908 - 936 of either SEQ ID NO. 5 or NO. 6, or nucleotides 2,749 - 2,777 of SEQ ID NO. 7). In other embodiments, such indicator phage are comprised in systems or kits according to the invention. Methods described herein may also utilize such indicator phage.

**Figure 4** depicts the isolation of recombinant phage from modifications of JG04 bacteriophage using the plasmid constructs shown in Figure 3 and described in Example 6.

In the first step 402, bacteria transformed with homologous recombination plasmid are infected with bacteriophage, resulting in progeny phage with a mixture of parental and recombinant phage with a ratio of approximately 20,000 wild-type 432 :1 recombinant phage 434. The resulting recombinant phage mix is diluted 404 into 96-well plates 406 to give an average of 3 recombinant transducing units (TU) per plate, which corresponds to about 625 infectious units (IU) of mostly wild-type phage per well. The 96-well plate is assayed for luciferase activity to identify wells 436 containing recombinant phage as compared to wells 440 containing wild-type bacteriophage. Bacteria 438 are added 408; for example, each well may contain about 50 µL of turbid *E. coli* O157:H7. This allows the phage to replicate and produce the luciferase enzyme 442. After 2 hours of incubation at 37°C shown in 410, wells may be screened for the presence of luciferase 442. Any positive wells are likely to have been inoculated with a single recombinant phage, and at this stage the mixture may contain a ratio of approximately 600 wild-type phage: 1 recombinant, an enrichment over the original 20,000:1 ratio. In one embodiment, soluble luciferase and phage were present at approximate ratio of 625 wild-type: 1 recombinant. Progeny from this enriched culture 412 may be subjected to additional limiting dilution assay(s) 414 to verify the ratio and determine the actual concentration of recombinant phage transducing units. For example, about 3 recombinant TU per 96-well plate 416 may be aliquoted 414 from the first purification stock, leading to an approximate inoculation of ∼20 mostly wild-type phage per well of a second dilution assay plate 420. Any positive luciferase wells are likely to have been inoculated with a single recombinant along with ∼20 wild-type phage. These wells may be analyzed for presence of luciferase 442.

After addition of bacteria and incubation (e.g., 37°C for 2 hours) 418, soluble luciferase and phage are present at approximately 20 wild-type: 1 recombinant 420. Finally, a plaque assay may be performed 422 to screen for recombinants that express luciferase 446. A small number of individual (e.g., n=48) plaques may be individually picked and screened on a third multiwell plate 426 for luciferase activity 436. In an embodiment, this approach should insure that about 3 recombinants would be in the mix of plaques being screened. One plaque may be removed from the plate to each well of a 96-well plate 424 and a luciferase assay performed 426 to determine which wells contained phage exhibiting luciferase activity 442. Wells 428 demonstrating luciferase activity represent pure recombinant phage 434, while wells without luciferase activity 430 represent pure wild-type phage 432.

Individual plaques may then be suspended in buffer or media (e.g., 100 µL TMS), and an aliquot (e.g., about 5 µL) added to a well containing a turbid *E. coli* O157:H7 culture, and assayed after incubation (e.g., about 45 minutes to 1 hour at 37°C). Positive wells are expected to contain a pure culture of recombinant phage. Still, it may be preferred, in certain embodiments to include an additional round of plaque purification.

Thus, as exemplified by Figure 4, recombinant phage generated by homologous recombination of the appropriate recombination plasmid with JG04 can be isolated from a mixture comprising 0.005% of total phage. Following isolation, large scale production may be performed to obtain high titer stocks appropriate for use in the *E. coli* 0157:H7 detection assay. For example, as described in more detail in the Examples herein, cesium chloride isopycnic density gradient centrifugation may be used to separate phage particles from contaminating luciferase protein to reduce background.

In this way, and as described in more detail in the Examples below, recombinant bacteriophage having the luciferase gene of interest (e.g., Firefly, Oplophorus or an engineered luciferase such as NANOLUC®) inserted into a bacteriophage derived from the environment may be generated.

### Methods of Using Infectious Agents for Detecting Microorganisms

As noted herein, in certain embodiments, the disclosure may comprise methods of using infectious particles for detecting microorganisms. The methods of the invention may be embodied in a variety of ways.

Thus, the methods of the present disclosure utilizes the high specificity of binding agents that recognize and bind to a particular microorganism of interest as a means to amplify a signal and thereby detect low levels of a microorganism (e.g., a single microorganism) present in a sample. For example, infectious agents (e.g., bacteriophage) specifically recognize surface receptors of particular microorganisms and thus specifically infect those microorganisms. As such, these infectious agents may be appropriate binding agents for targeting a microorganism of interest. Some embodiments of the disclosure utilize the specificity of binding and high-level genetic expression capacity of infectious agents for rapid and sensitive targeting to infect and facilitate detection of a microorganism of interest.

Thus, in an embodiment, the invention may comprise a method for detecting a microorganism of interest in a sample comprising the steps of: incubating the sample with an recombinant bacteriophage that infects the microorganism of interest, wherein the recombinant bacteriophage comprises an indicator gene inserted into a later gene region of the bacteriophage such that expression of the indicator gene during bacteriophage replication following infection of microorganism of interest results in a soluble indicator protein product, wherein the indicator gene encodes a luciferase enzyme, and wherein the indicator gene does not encode a fusion protein and wherein the indicator protein product does not comprise polypeptides of the natural bacteriophage; and detecting the indicator protein product, wherein positive detection of the indicator protein product indicates that the microorganism of interest is present in the sample.

A variety of infectious agents may be used as part of the disclosure. In alternate embodiments of the disclosure, bacteriophages, phages, mycobacteriophages (such as for TB and paraTB), mycophages (such as for fungi), mycoplasma phages, and any other virus that can invade living bacteria, fungi, mycoplasma, protozoa, yeasts, and other microscopic living organisms can be employed to target a microorganism of interest. In embodiments, the microorganism of interest is a bacterium, the infectious agent comprises a bacteriophage. As discussed herein, such bacteriophage may replicate inside of the bacteria to generate hundreds of progeny bacteria. Detection of the indicator gene inserted into the bacteriophage can be used as a measure of the bacteria in the sample. For example, well-studied phages of *E. coli* include T1, T2, T3, T4, T5, T7, and lambda; other *E. coli* phages available in the ATCC collection, for example, include phiX174, S13, Ox6, MS2, phiV1, fd, PR772, and ZIK1. Alternatively, natural bacteriophage may be isolated from a variety of environmental sources. A source for phage isolation may be selected based on the location where a microorganism of interest is expected to be found. Thus, in some embodiments of the disclosure, the indicator bacteriophage comprises an indicator moiety and infection of a single *E. coli* cell may be detected in an amplified signal generated via the indicator moiety. Thus the method may comprise detecting an indicator moiety produced during phage replication, wherein detection of the indicator indicates that the bacterium of interest is present in the sample.

In an embodiment, the invention may comprise a method for detecting a bacteria of interest in a sample comprising the steps of: incubating the sample with a recombinant bacteriophage that infects the bacteria of interest, wherein the recombinant bacteriophage comprises an indicator gene inserted into a late gene region of the bacteriophage such that expression of the indicator gene during bacteriophage replication following infection of host bacteria results in a soluble indicator protein product wherein the indicator gene encodes a luciferase enzyme and wherein the indicator gene does not encode a fusion protein; and wherein the indicator protein product does not comprise polypeptides of the natural bacteriophage; and detecting the indicator protein product, wherein positive detection of the indicator protein product indicates that the bacteria of interest is present in the sample. In an embodiment, and as described in detail herein, the amount of indicator moiety detected corresponds to the amount of the bacteria of interest present in the sample.

In an embodiment, the late gene region is a class III gene region. As described in more detail herein, insertion of the indicator gene into the late class III gene region may ensure that the indicator gene is expressed in high quantities upon replication in the bacterium.

As described above for the compositions of the invention, the bacteriophage is derived from T7, T4, T4-like, JG04, or another natural bacteriophage having a genome with at least 90% homology to T7, T4 or other T4-like phage.

Also, in embodiments, the indicator gene does not encode a fusion protein. Thus, in certain embodiments, expression of the indicator gene during bacteriophage replication following infection of host bacteria results in a soluble indicator protein product.

A variety of indicator moieties may be used as part of the disclosure. In embodiments, the indicator gene may encode a luciferase enzyme. For example, the luciferase may be one of Oplophorus luciferase, Firefly luciferase, or an engineered luciferase.

As described in more detail herein, the methods and systems of the invention may utilize a variety of multiplicities of infection (MOI). In certain embodiments, the MOI is higher that standard assays. Such a relatively high MOI may allow for infection of microorganisms that are present at very low amounts in the sample. For example, in certain embodiments, the bacteriophage concentration for the incubating step is greater than 1 x 10⁷ PFU/mL.

In certain embodiments, the recombinant infectious agent may be purified so as to be free of any residual indicator protein that may be generated upon production of the infectious agent stock. Thus, in certain embodiments, and as described in more detail herein, the recombinant bacteriophage may be purified using cesium chloride isopycnic density gradient centrifugation prior to incubation with the sample. When the infectious agent is a bacteriophage, this purification may have the added benefit of removing bacteriophage that do not have DNA (i.e., empty phage).

As described further below, in certain embodiments, the method may employ a step whereby the microorganism of interest is concentrated or captured from a large volume of sample. Thus, in certain embodiments, the method may comprise a step for capturing the microorganism from the sample on a solid support before the incubating step.

In some embodiments, the method may include contacting a microorganism captured on a solid support (e.g., a magnetic bead or a filter substrate) with a plurality of the specific infectious agent (indicator bacteriophage) and allowing the bacteriophage to bind and infect the bacteria. In other embodiments, capture of the microorganism is not necessary for detection. A variety of solid supports may be used. In certain embodiments, the solid support may comprise a multi-well plate, a filter, a bead, or a lateral flow strip, a filter strip, filter disc, or filter paper, or thin films designed for culturing cells (e.g., PetriFilm by 3M). Other solid supports may also be appropriate.

The microorganism of interest may be purified from the sample by using a binding agent. For example, in certain embodiments, the capturing step further comprises binding microorganism with a capture antibody. The antibody may be used in conjunction with the solid support. For example, in certain embodiments, the capture antibody facilitates binding of the microorganism to the solid support.

The method of the invention may comprise various steps to increase sensitivity. For example, as discussed in more detail herein, the method may comprise a step for washing the captured and infected microorganism, after adding the bacteriophage but before incubating, to remove excess parental bacteriophage and/or luciferase or other reporter protein contaminating the bacteriophage preparation.

In contrast to assays known in the art, detection of the microorganism of interest may be completed without the need for culturing the sample as a way to increase the population of the microorganisms. Thus, in certain embodiments, detection of the microorganism of interest is completed in less time than a time period required for increasing the number of microorganisms by 4-fold or 10-fold using culturing for enrichment. For example, in certain embodiments the total time required for detection is less than 6.0 hours, 5.0 hours, 4.0 hours, 3.0 hours, 2.5 hours, 2.0 hours, 1.5 hours, 1.0 hour, 45 minutes, or less than 30 minutes.

Also, in contrast to assays known in the art, the method of the invention can detect individual microorganisms. Thus, in certain embodiments, the method may detect ≤ 10 cells of the microorganism (i.e., 1, 2, 3, 4, 5, 6, 7, 8, 9 microorganisms) present in a sample.

Thus, aspects of the present disclosure provide methods for detection of microorganisms in a test sample via an indicator moiety. In some embodiments, where the microorganism of interest is a bacteria, the indicator moiety may be associated with an infectious agent is an indicator bacteriophage. The indicator moiety of the disclosure may react with a substrate to emit a detectable signal or may emit an intrinsic signal (e.g., fluorescent protein). In some embodiments, the detection sensitivity can reveal the presence of as few as 50, 20, 10, 9, 8, 7, 6, 5, 4, 3, or 2 cells of the microorganism of interest in a test sample. In some embodiments, even a single cell of the microorganism of interest may yield a detectable signal.

In some embodiments of the disclosure, the indicator moiety associated with the infectious agent may be detectable during or after replication of the infectious agent. Many different types of detectable biomolecules suitable for use as indicator moieties are known in the art, and many are commercially available. In some embodiments of the disclosure the indicator phage comprises an enzyme, which serves as the indicator moiety. In some embodiments, the genome of the indicator phage is modified to encode a soluble protein. In some embodiments of the disclosure, the indicator phage encodes a detectable enzyme. The indicator may emit light and/or may be detectable by a color change. Various appropriate enzymes are commercially available, such as alkaline phosphatase (AP), horseradish peroxidase (HRP), or luciferase (Luc). In some embodiments of the disclosure, these enzymes may serve as the indicator moiety. In some embodiments, Firefly luciferase is the indicator moiety. In some embodiments, Oplophorus luciferase is the indicator moiety. In some embodiments, NANOLUC® is the indicator moiety. Other engineered luciferases or other enzymes that generate detectable signals may also be appropriate indicator moieties.

Detecting the indicator may include detecting emissions of light. In some embodiments, a luminometer may be used to detect the indicator (e.g., luciferase). However, other machines or devices may also be used. For example, a spectrophotometer, CCD camera, or CMOS camera may detect color changes and other light emissions.

In some embodiments, indicator phage is genetically engineered to contain the gene for an enzyme, such as a luciferase, which is only produced upon infection of bacteria that the phage specifically recognizes and infects. In some embodiments, the indicator moiety is expressed late in the viral life cycle. In some embodiments, as described herein, the indicator is a soluble protein (soluble luciferase) and is not fused with a phage structural protein that limits its copy number.

In various embodiments of the methods of the invention, the microorganism may be detected without any purification of the microorganisms from a sample. For example, in certain embodiments, a sample containing one or a few microorganisms of interest may be applied directly to an assay container such as a spin column, an microtiter well, or a filter and the assay is conducted in that assay container. Various embodiments of such assays are disclosed herein.

For example, aliquots of a test sample comprising bacteria may be applied to a spin column and after infection with a recombinant bacteriophage and an optional washing to remove any excess bacteriophage, the amount of soluble indicator detected will be proportional to the amount of bacteriophage that are produced by infected bacteria. Example 4 describes such an assay.

Or, aliquots of a test sample may be distributed directly into wells of a multi-well plate, indicator phage may be added, and after a period of time sufficient for infection, a lysis buffer may be added as well as a substrate for the indicator moiety (e.g., luciferase substrate for a luciferase indicator) and assayed for detection of the indicator signal. Examples 5-6 describe embodiments of the method performed on filter plates. Examples 7-9 herein describe assay variations called the "No concentration assay."

For example, in many embodiments, multi-well plates are used to conduct the assays. The choice of plates (or any other container in which detecting may be performed) may affect the detecting step. For example, some plates may include a colored or white background, which may affect the detection of light emissions. Generally speaking, white plates have higher sensitivity but also yield a higher background signal. Other colors of plates may generate lower background signal but also have a slightly lower sensitivity. Additionally, one reason for background signal is the leakage of light from one well to another, adjacent well. There are some plates that have white wells but the rest of the plate is black. This allows for a high signal inside the well but prevents well-to-well light leakage and thus may decrease background. Thus the choice of plate or other assay vessel may influence the sensitivity and background signal for the assay.

Thus in some embodiments of the disclosure utilizing indicator phage, the invention comprises a method for detecting a microorganism of interest comprising the steps of capturing at least one sample microorganism; incubating the at least one microorganism with a plurality of indicator phage; allowing time for infection and replication to generate progeny phage and express soluble indicator moiety; and detecting the progeny phage, or preferably the indicator, wherein detection of the indicator demonstrates that the microorganism is present in the sample.

For example, in some embodiments the test sample microorganism may be captured by binding to the surface of a plate, or by filtering the sample through a bacteriological filter (e.g., 0.45 µm pore size spin filter or plate filter). In an embodiment, the infectious agent (indicator phage) is added in a minimal volume to the captured sample directly on the filter. In an embodiment, the microorganism captured on the filter or plate surface is subsequently washed one or more times to remove excess unbound infectious agent. In an embodiment, media (e.g., Luria-Bertani also called LB broth herein) is added for further incubation time, to allow replication of phage and high-level expression of the gene encoding the indicator moiety. However, a surprising aspect of embodiments of the assays is that the incubation step only needs to be long enough for a single phage life cycle. The amplification power of using bacteriophage was previously thought to require more time, such that the phage would replicate for several cycles. A single replication of indicator phage is sufficient to facilitate sensitive and rapid detection according to some embodiments of the present invention.

Soluble indicator (luciferase) is released into the surrounding liquid upon lysis of the bacteria, which may then be measured and quantified. In an embodiment, the solution is then spun through the filter, and the filtrate collected for assay by addition of a substrate for the indicator enzyme (luciferase substrate). The filtrate may thus be removed from the capture solid support and analyzed in a new receptacle (e.g., in a luminometer), or the indicator signal may be measured directly on the filter.

In various embodiments, the purified parental indicator phage does not comprise the detectable indicator itself, because the parental phage may be purified before it is used for incubation with a test sample. Expression of late (Class III) genes occurs late in the viral life cycle. In some embodiments of the present invention, parental phage may be purified to exclude any existing indicator protein (luciferase). In some embodiments, expression of the indicator gene during progeny bacteriophage replication following infection of host bacteria results in a soluble indicator protein product. Thus, in many embodiments, it is not necessary to separate parental from progeny phage in the detecting step. In an embodiment, the microorganism is a bacterium and the indicator phage is a bacteriophage. In an embodiment, the indicator moiety is soluble luciferase, which is released upon lysis of the host microorganism.

Thus, in an alternate embodiment, the indicator substrate may be incubated with the portion of the sample that remains bound to a filter or bound to a plate surface. Accordingly, in some embodiments the solid support is a 96-well filter plate (or regular 96-well plate), and the substrate reaction may be detected by placing the plate directly in the luminometer.

For example, in an embodiment, the invention may comprise a method for detecting *E. coli* O157:H7 comprising the steps of: infecting cells captured on a 96-well filter plate with a plurality of parental indicator phage capable of expressing luciferase upon infection; washing excess phage away; adding LB broth and allowing time for phage to replicate and lyse the specific *E. coli* target (e.g., 30-90 minutes); and detecting the indicator luciferase by adding luciferase substrate and measuring luciferase activity directly in the 96-well plate, wherein detection of luciferase activity indicates that the *E. coli* 0157:H7 is present in the sample.

In another embodiment, the invention may comprise a method for detecting *E. coli* O157:H7 comprising the steps of: infecting cells in liquid solution or suspension in a 96-well plate with a plurality of parental indicator phage capable of expressing luciferase upon infection; allowing time for phage to replicate and lyse the specific *E. coli* target (e.g., 30-90 minutes); and detecting the indicator luciferase by adding luciferase substrate and measuring luciferase activity directly in the 96-well plate, wherein detection of luciferase activity indicates that the *E. coli* O157:H7 is present in the sample. In such an embodiment no capturing step is necessary. In some embodiments, the liquid solution or suspension may be a consumable test sample, such as a vegetable wash. In some embodiments, the liquid solution or suspension may be vegetable wash fortified with concentrated LB broth or Nutrient Broth. In some embodiments, the liquid solution or suspension may be bacteria diluted in LB Broth.

In some embodiments, lysis of the microorganism may occur before, during, or after the detection step. Experiments suggest that infected unlysed cells may be detectable upon addition of luciferase substrate in some embodiments. Presumably, luciferase may exit cells and/or luciferase substrate may enter cells without complete cell lysis. Thus, for embodiments utilizing the spin filter system, where only luciferase released into the lysate (and not luciferase still inside intact bacteria) is analyzed in the luminometer, lysis is required for detection. However, for embodiments utilizing filter plates or 96-well plates with sample in solution or suspension, where the original plate full of intact and lysed cells is directly assayed in the luminometer, lysis is not necessary for detection.

In some embodiments, the reaction of indicator moiety (luciferase) with substrate may continue for 30 minutes or more, and detection at various time points may be desirable for optimizing sensitivity. For example, in embodiments using 96-well filter plates as the solid support and luciferase as the indicator, luminometer readings may be taken initially and at 10- or 15-minute intervals until the reaction is completed.

Surprisingly, high concentrations of phage utilized for infecting test samples (i.e., high MOI) have successfully achieved detection of very low numbers of target microorganism in a very short timeframe. The incubation of phage with a test sample in some embodiments need only be long enough for a single phage life cycle. In some embodiments, the bacteriophage concentration for this incubating step is greater than 7 x 10⁶, 8 x 10⁶, 9 x 10⁶, 1.0 x 10⁷, 1.1 x 10⁷, 1.2 x 10⁷, 1.3 x 10⁷, 1.4 x 10⁷, 1.5 x 10⁷, 1.6 x 10⁷, 1.7 x 10⁷, 1.8 x 10⁷, 1.9 x 10⁷, 2.0 x 10⁷, 3.0 x 10⁷, 4.0 x 10⁷, 5.0 x 10⁷, 6.0 x 10⁷, 7.0 x 10⁷, 8.0 x 10⁷, 9.0 x 10⁷, or 1.0 x 10⁸ PFU/mL.

Success with such high concentrations of phage is surprising because the large numbers of phage were previously associated with "lysis from without," which killed target cells and thereby prevented generation of useful signal from earlier phage assays. It is possible that the clean-up of prepared phage stock described herein helps to alleviate this problem (e.g., clean-up by cesium chloride isopycnic density gradient ultracentrifugation), because in addition to removing any contaminating luciferase associated with the phage, this clean-up may also remove ghost particles (particles that have lost DNA). The ghost particles can lyse bacterial cells via "lysis from without," killing the cells prematurely and thereby preventing generation of indicator signal. Electron microscopy demonstrates that a crude JG04 lysate (i.e., before cesium chloride clean-up) may have greater than 50% ghosts. These ghost particles may contribute to premature death of the microorganism through the action of many phage particles puncturing the cell membrane. Thus ghost particles may have contributed to previous problems where high PFU concentrations were reported to be detrimental. Moreover, a very clean phage prep allows the assay to be performed with no wash steps, which makes the No Concentration assay possible.

### Spin Column Assays

**Figure 5** shows a strategy of using indicator phage that produce soluble luciferase according to an embodiment of the invention. In this method, the phage (e.g., T7, T4, or JG04 phage) may be engineered to express a soluble luciferase during replication of the phage. Expression of luciferase is driven by a viral capsid promoter (e.g., the bacteriophage T7 or T4 late promoter), yielding high expression. Parental phage will be free of luciferase, so the luciferase detected in the assay must come from replication of progeny phage during infection of the bacterial cells. Thus, there is generally no need to separate out the parental phage from the progeny phage.

In these experiments, at least part of the sample 500 comprising the *E. coli* bacteria 502 to be quantified is placed in a spin column filter and centrifuged to remove the LB broth, and an appropriate multiplicity of T7 phage 504 genetically engineered to express soluble luciferase 503 are added. The infected cells may be incubated for a time sufficient for replication of progeny phage and cell lysis to occur (e.g., 30-90 minutes at 37°C). The parental 504 and progeny phage 516 plus free luciferase 503 in the lysate may then be collected, e.g., by centrifugation, and the level of luciferase in the filtrate quantified using a luminometer 518. Alternatively, a high through-put method may be employed where bacterial samples are applied to a 96-well filter plate, and after all manipulations listed above are performed, may be directly assayed for luciferase in the original 96-well filter plate without a final centrifugation step.

Data from example experiments of embodiments of the invention are shown in Figures 6-9. The results demonstrate alternative embodiments of the invention utilizing indicator phage to assay sample bacteria via detection of soluble luciferase produced by the infection of bacteria with indicator phage. Indicator detection level calibrated as relative light units (RLU) is plotted against cell concentrations determined from the standard overnight colony forming unit (CFU) assay and expressed as "cells per assay," thus demonstrating similar sensitivity. Increasing luciferase signal corresponds to increasing input sample cells, demonstrating a dose-dependent response.

As shown in **Figure 6** the method of the invention may demonstrate high sensitivity for detection of target bacteria using indicator phage with spin column filters. In this assay, a test sample may be collected on a spin filter by centrifugation and incubated with indicator phage comprising a gene for soluble luciferase (e.g., as shown in **Figure 1**). Following incubation for a time sufficient for infection (e.g., 10 minutes at room temperature), the filter may be washed and spun to remove excess input phage. Media (e.g., LB broth) may be added and incubated (e.g., for 30 minutes at 37°C) to allow replication of progeny phage and lysis of bacteria. Filters may be spun again to remove the filtrate, which may be transferred to a luminometer plate, and a luciferase assay was performed (e.g., using a Promega® luminometer with injection of Luciferase Assay Reagent, Promega, Inc.). The cell count may be corrected according to the number of colonies in the parallel CFU assay.

As shown in Figure 6, approximately 1,700 bacterial cells may be detected in the original sample, and further assay of serial dilutions may demonstrate detection down to an average of 1.7 cells, corresponding to actual detection of 1 or 2 cells. This shows that infection of as few as 1 to 3 *E. coli* cells can provide a measurable signal, via luciferase activity. This also shows statistical significance for the presence or absence of even a single cell over background (p value = 0.018).

**Figure 7** demonstrates the very large detection range of the same method described for the assay of Figure 5 using serial dilutions across a broader range of cell numbers. This shows detection may range from an average of 1.4 cells to 14 million cells.

### Filter Plate Assays

As noted above, in certain embodiments, the assay may be conducted directly in the well of a microtiter assay plate. For example, **Figure 8** demonstrates the use of indicator phage with a 96-well filter plate for capture and detection. The method is the same as described above for Figure 5, except that the entire assay is performed in a 96-well filter plate, including the luciferase reaction. This embodiment reduces manipulations and materials compared to the spin filter method. The reduced manipulations and use of a 96-well filter plate is amenable to a high throughput assay situation, and may be adapted for use with a liquid handling robot according to embodiments of the invention. **Figure 8** shows that in this embodiment, a 96-well filter plate may be used to detect single *E. coli* cells (0.5 cells average assayed, confirming that about one-half of the wells received single cells), 5.4 and 54 cells.

**Figure 9** demonstrates that in certain embodiments, a very large detection range of cell concentrations may be achieved for the same assay, using the 96-well system, from less than 1 cell per assay on average (single cells) to at least 14 million cells per assay. Multiple reads at different time points after addition of the luciferase substrate may demonstrate varied sensitivity. Sensitivity for detecting <10 cells was achieved with a 15 minute read, and sensitivity down to single cell levels may be achieved at the 30 minute read. Thus time may be saved if tens of cells or less need not be detected. Note signal increases in response to increased number of input sample cells in both experiments, again demonstrating a dose-dependent response.

**Figure 10** depicts a filter plate assay for detecting bacteria of interest using a modified bacteriophage according to an embodiment of the invention. An actual experiment utilizing this assay is described in Example 6. Briefly, samples 1016 that include a bacterium of interest 1018 may be added to wells 1002 of a multi-well filter plate 1004 and spun 1006 to concentrate the samples by removal of liquid from the sample. Genetically modified phage 1020 are added to wells and incubated with additional media added for enough time sufficient for adsorption 1008 followed by infection of target bacteria and advancement of the phage life cycle 1010 (e.g., ∼ 45 minutes). Finally, luciferase substrate is added and reacts with any luciferase present 1024. The resulting emission is measured in a luminometer 1014 which detects luciferase activity 1026. **Figure 11** shows results from a filter plate assay as described in **Figure 10****,** using JG04-NANOLUC® phage to detect *E. coli* 0157:H7 cells in samples with known cell numbers. Student's t-Test showed a p value of 0.034 between 0 cells and 1 cell per assay, demonstrating statistical significance.

In certain embodiments, the assay may be performed without concentrating the bacterium on or near the capture surface. **Figure 12** illustrates a "No Concentration Assay" for detecting a bacterium of interest using a modified bacteriophage according to an embodiment of the invention. Aliquots of indicator phage 1214 are distributed to the individual wells 1202 of a multi-well plate 1204, and then test sample aliquots containing bacteria 1212 are added and incubated 1206 (e.g., 45 minutes at 37°C) for a period of time sufficient for phage to replicate and generate soluble indicator 1216 (e.g., luciferase). The plate wells 1208 containing soluble indicator and phage may then be assayed 1210 to measure the indicator activity on the plate 1218 (e.g., luciferase assay). Actual experiments utilizing this method are described in Examples 7-9. In this embodiment, the test samples are not concentrated (e.g., by centrifugation) but are simply incubated directly with indicator phage for a period of time and subsequently assayed for luciferase activity.

**Figure 13** shows results from a No Concentration Assay type assay as depicted in Figure 12 using JG04-OpLuc phage to detect *E. coli* 0157:H7 cells in samples with known cell numbers in the low number range (i.e., very diluted cell samples). This experiment, as described in Example 7, demonstrates that statistically significant differences can be seen between the signal from 0 cells and 1 cell per assay (p value = 0.0024 by ANOVA test), demonstrating the ability to detect single cells. Thus the assay is surprisingly sensitive. Samples with fewer than 1 cell per well appear to show a proportional number of wells above the background signal.

**Figure 14** shows results from a No Concentration Assay using JG04-OpLuc phage to detect *E. coli* O157:H7 cells in samples with known cell numbers in the very low to very high number range (i.e., samples containing less than 1 cell per assay to millions of cells). This experiment, as described in Example 8, demonstrates statistically significant differences between the signal from 0 cells and 1.1 cell per assay (p value = 0.000702 by Student's t-Test), demonstrating the ability to detect single cells. More bacterial cells per assay show increasing signal in a dose dependent manner, up to at least 10⁶ bacterial cells / assay, surprisingly demonstrating a very wide range of detection.

**Figure 15** shows results from a No Concentration Assay using JG04-OpLuc phage to detect *E. coli* O157:H7 cells in Vegetable Wash samples with known cell numbers, as described in Example 9.

To prepare the vegetable wash, vegetable leaves (e.g., spinach or lettuce) may be weighed and added to a clean plastic bag. One mL of LB (+/- 0.01 - 0.05% Tween20) was added per each gram (g) of vegetable. Leaves and solution are mixed manually for a few minutes. Liquid is then extracted from the plastic bag and used as the "vegetable wash." Using this method, ∼ 1 million bacteria were found to reside on a single spinach leaf (1-2 g).

The assay is quantitative in that the signal detected is proportional to the amount of the microorganism of interest in the sample. For example, in the experiment depicted in Figure 15, known numbers *of E. coli* 0157:H7 cells were added to vegetable wash samples to simulate contamination of vegetables with pathogenic bacteria. The experiment using vegetable wash samples demonstrates marked differences between the signal from 0 cells and 3 cells per assay, demonstrating the ability to detect single-digit cell numbers in vegetable wash. Using more bacterial cells per assay shows increasing signal in a dose dependent manner. The vegetable wash contains about 10⁶ non-target bacteria /mL, corresponding to at least 10⁵ non-target bacteria per sample in this assay (including the 0 cells *E. coli* 0157:H7 control). The ability to discern as few as 3 target bacterial cells from 10⁵ non-target bacteria is surprising and again demonstrates the specificity of the assay.

### Capture of the Microorganism Prior to Exposure to Infectious Agent

In some embodiments, the present invention comprises methods and systems that do not require a step for capturing microorganisms. Other embodiments allow physical isolation of bacterial cells from a sample. Methods described herein may serve as means to facilitate detection of low levels of a microorganism (e.g., a single microorganism) present in a sample. A capturing step may be based on a specific binding agent, such as an antibody that recognizes the microorganism of interest, or it may be based on selection for other features of the microorganism, such as size fractionation.

In some embodiments, a microorganism is captured based on physical features other than molecular specificity (e.g., size). In some embodiments, the present invention utilizes the physical size of the microorganism to capture it on a solid support. In some embodiments, the solid support is a filter. For example, filtering a sample through a bacteriological filter (e.g., 0.45 µm pore size spin filter) allows smaller substances to pass through while retaining intact bacteria. Alternatively, a plate filter may be used to capture a microorganism, or a variety of other filter devices may be used (e.g., 96-well filter plate).

For example, the method may include the step of collecting the microorganism on a solid support, such as for example by filtering a sample through a bacteriological filter. After size-based capture, any binding agent that specifically targets the microorganism of interest may be employed. For example, an infectious agent may be incubated with the captured microorganism, in order to specifically target and identify the microorganism of interest. Other methods of isolating the microorganisms in the sample may be used. In some embodiments, detection steps may be performed before, simultaneously with, or after such capture steps.

In some embodiments, binding agents with high specificity for a microorganism of interest may be employed as a means to facilitate specific capture of low levels of a microorganism (e.g., a single microorganism) present in a sample. In some embodiments, a large volume of liquid sample to be tested may need to be effectively concentrated before further testing.

For example, a single bacterium, which may have a volume of about one cubic micrometer, can be isolated from a one-milliliter sample having a volume of 10¹² cubic micrometers. In such embodiments the capturing step may comprise contacting the sample with a plurality (an excess) of affinity-purified capture antibodies or antibody fragments.

Some embodiments utilize affinity-purified and/or reverse-purified surface-specific antibodies or antibody fragments generated against antigenic molecules found on the surface of a specific microorganism of interest. Such antibodies or antibody fragments can specifically identify a microorganism for capture or detection purposes or both. Antibodies demonstrating specific recognition of surface antigens on a wide variety of bacteria or other microorganisms are available commercially from a number of sources, such as Kirkegaard & Perry Laboratories, Inc. (KPL) or Abcam.

In some embodiments of the invention, affinity-purified and/or reverse-purified surface-specific antibodies that recognize the microbial surface antigens of a particular microorganism (e.g., *E. coli* O157:H7) do not recognize other similar microorganisms (e.g., *E. coli* B). In some embodiments, antibodies specific to, e.g., *E. coli* B or *E. coli* O157:H7 do not recognize cells of *Salmonella typhimurium* or *Staphylococcus epidermidis.* This represents another surprising discovery, as many bacteria have, e.g., surface lipopolysaccharide (Gram-negative bacteria) or lipoteichoic acid (Gram-positive bacteria) molecules that were previously believed to be highly similar, especially between closely related species.

Methods for antibody-based capture disclosed herein may be adapted to any bacterium or other microorganism of interest (e.g. pathogenic microorganisms) for which surface-specific antibodies are available which do not cross-react with other microorganisms.

For example, in some embodiments, a capturing step of the invention may use capture antibodies or antibody fragments that are specific for the microorganism to facilitate capture of the microorganism. In some embodiments, capture antibodies may be conjugated to a chemical moiety that binds with another binding agent attached to a solid support (e.g. beads or a plate surface). For example, in some embodiments, the capture antibody may be biotinylated to facilitate binding to streptavidin bound to a solid support. In some embodiments, the solid support comprises magnetic beads. In other embodiments, a solid support comprises a plate surface or the surfaces of a multi-well plate (e.g., an ELISA plate). For example, an ELISA plate may be coated with an antibody that specifically recognizes the microorganism of interest.

In certain embodiments, the microorganism may be isolated from other components of the sample through binding of the microorganism to a free capture antibody or antibody fragment that subsequently binds to a solid support. In some embodiments, the capture antibody or antibody fragment comprises a binding agent (e.g., biotin) that binds to a second agent (e.g., streptavidin) bound to a solid support.

In some embodiments, e.g., if the capture antibody is labeled with biotin, the method may further comprise contacting the sample with a plurality of magnetic streptavidin-coated beads to bind the bacterium-antibody complex, and sequestering the bead-antibody-bacterium complex with a magnet to isolate the bacteria. Or, other methods of purifying the biotin-antibody: bacterium complex may be used. With such embodiments, a bacterium in a one-milliliter sample can be concentrated to about one microliter (∼1000-fold), facilitating further detection and/or quantification by methods described herein.

Thus, in some embodiments, the invention comprises a method for detecting a microorganism of interest wherein the capture step comprises specifically isolating the microorganism from other components in the sample.

Alternatively, in some embodiments the capturing step may be based on other features of the microorganism of interest, such as size. In embodiments utilizing size-based capture, the solid support may be a spin column filter. In some embodiments, the solid support comprises a 96-well filter plate. Or, the solid support for capture may be a location on an array, or a mobile support, such as a bead.

Thus, in some embodiments, a target microorganism may be captured and isolated from a large volume before detection using the methods described above. For example, the microorganism may be specifically isolated using attributes of a capture antibody or antibody fragment. In some embodiments, the capture antibody is biotinylated such that it facilitates subsequent binding of cell-antibody complexes to magnetic streptavidin beads. The biotin on the antibody can bind tightly to the streptavidin on the magnetic bead. Or the capture antibody may be conjugated to another protein or other molecule, which facilitates capture on beads or another solid support. Such embodiments may provide increased sensitivity, particularly where the initial sample volume is large. Thus, the method may comprise the steps of attaching a plurality of binding agents that can specifically bind to a surface antigen on the microorganism of interest, which thereby facilitates binding to a capture solid support.

Alternatively, another chemical moiety that binds the anti-bacterium antibody may be used to coat magnetic beads. For example, a bead coated with a secondary antibody that recognizes or binds the anti-bacterium antibody may be used. The bacteria bound to the beads may then be isolated. In an embodiment, the efficiency of capture may be quantified by plating the bacteria bound to the beads and the unbound supernatant fraction and counting the resultant colonies (CFU). In other embodiments, the signal generated by reaction with substrate (i.e., substrate reagent for the indicator moiety) is measured for detection.

In some embodiments, the specificity of antibodies is demonstrated using specific capture on a solid support. A method of the invention may comprise the step of retrieving and concentrating a microorganism (e.g., a bacterium) from a sample by the use of a substrate with a binding agent specific for the microorganism. In an embodiment, the binding agent is immobilized on a solid support (e.g. magnetic beads) or is free and subsequently immobilized on a solid support. The immobilized microorganism may then be removed from the sample (e.g., by aspiration, decanting, magnetic force, or other appropriate isolation techniques) and detected by a variety of techniques.

**Figure 16** depicts an example embodiment of specific capture of *E. coli* O157:H7 but not *E. coli* B or *S. typhimurium* from samples by the use of antibodies produced against intact *E. coli* O157:H7. Thus, in certain embodiments magnetic beads coated with streptavidin may be used to isolate *E. coli* O157:H7 preincubated with free biotinylated polyclonal antibodies (KPL), affinity-purified and reverse-purified to minimize cross-reactivity with other microbial species. In certain embodiments, the *E. coli* O157:H7 will only be present in the captured fraction (i.e., bead fraction) when specific *E. coli* O157:H7 antibodies were used, and no bacteria will be recovered in the supernatant (unbound) fraction. In certain embodiments, *E. coli* B and *S. typhimurium* cells are found only in the supernatant fraction when *E. coli* 0157:H7-specific antibodies are used, illustrating the remarkable specificity of these antibodies. In the absence of antibody, all three types of bacteria were found in the supernatant fraction.

### Hybrid Immuno-Phage (HIP) Assay

In certain embodiments, the methods of the present invention combine the use of a binding agent (e.g., antibody) to purify and/or concentrate a microorganism of interest from the sample in addition to detection with an infectious agent. For example, in certain embodiments, the present invention comprises a method for detecting a microorganism of interest in a sample comprising the steps of: capturing the microorganism from the sample on a prior support using a capture antibody specific to the microorganism of interest; incubating the sample with a recombinant bacteriophage that infects the microorganism of interest, wherein the recombinant bacteriophage comprises an indicator gene inserted into a late gene region of the bacteriophage such that expression of the indicator gene during bacteriophage replication following infection of host bacteria results in a soluble indicator protein product; and detecting the indicator protein product, wherein positive detection of the indicator protein product indicates that the microorganism of interest is present in the sample.

For example, **Figure 17** depicts a Hybrid Immuno-Phage (HIP) Assay for detecting a bacterium of interest using a modified bacteriophage according to an embodiment of the invention. The sample is first applied to the microtiter plate well coated with bacterium-specific antibodies 1702. The plate is then centrifuged to facilitate binding of the bacterium to the capture antibodies 1704. Following sufficient time to allow for complete bacteria capture, a solution containing bacterium-specific NANOLUC®-phage is added to each sample 1706. Incubation with the phage results in the binding and attachment of a single or multiple phages to the captured bacterium 1708. Finally, the sample is incubated to facilitate phage replication and luciferase expression, which leads to cell lysis and release of soluble luciferase 1710.

**Figure 18** shows results from a HIP assay using JG04-NANOLUC® phage to detect *E. coli* 0157:H7 cells in samples with known cell numbers, as described in Example 11, on a log scale. The HIP assay was able to detect 100 and 1,000 *E. coli* O157:H7 cells in LB media with approximately 2 × 10⁶ PFU JG04-NANOLUC® phage. The average signal over a no-cell sample ranged from approximately 50-fold for the 100 cell sample to over 1,000-fold for the 1,000 cell sample.

In some embodiments, the incubating step of the methods described herein comprises a final bacteriophage concentration of greater than 7 x 10⁶, 8 x 10⁶, 9 x 10⁶, 1.0 x 10⁷, 1.1 x 10⁷, 1.2 x 10⁷, 1.3 x 10⁷, 1.4 x 10⁷, 1.5 x 10⁷, 1.6 x 10⁷, 1.7 x 10⁷, 1.8 x 10⁷, 1.9 x 10⁷, 2.0 x 10⁷, 3.0 x 10⁷, 4.0 x 10⁷, 5.0 x 10⁷, 6.0 x 10⁷, 7.0 x 10⁷, 8.0 x 10⁷, 9.0 x 10⁷, or 1.0 x 10⁸ PFU/mL. This high phage concentration was previously purported to be detrimental to such an assay, and thus yields surprising results. In some embodiments, the methods of the invention require less than 3 hours, less than 2.5 hours, less than 2 hours, less than 1.5 hours, or less than 1 hour for detection of a microorganism of interest. In some embodiments, the methods can detect as few as 100, 50, 20, 10, 9, 8, 7, 6, 5, 4, 3, or 2 cells of the microorganism of interest. These are shorter timeframes than were previously thought possible. In some embodiments, even a single cell of the microorganism is detectable. In additional embodiments, the invention comprises systems (e.g., computer systems, automated systems or kits) comprising components for performing the methods disclosed herein, and/or using the modified infectious agents described herein.

### Systems and Kits of the Disclosure

In some embodiments, the disclosure comprises systems (e.g., automated systems or kits) comprising components for performing the methods disclosed herein. Some embodiments of the disclosure described herein are particularly suitable for automation or kits, given the minimal amount of reagents and materials required to perform the methods. In certain embodiments of the disclosure, each of the components of the a kit may comprise a self-contained unit that is deliverable from a first site to a second site.

In some embodiments of the disclosure, the invention comprises a systems or kits for rapid detection of a microorganism of interest in a sample. The systems or kits may in certain embodiments of the disclosure comprise a component for incubating the sample with an infectious agent specific for the microorganism of interest, wherein the infectious agent comprises an indicator moiety and a component for detecting the indicator moiety. In some embodiments of both the systems and the kits of the disclosure, the infectious agent is a recombinant bacteriophage that infects the microorganism of interest, and the recombinant bacteriophage comprises an indicator gene inserted into a late gene region of the bacteriophage as the indicator moiety such that expression of the indicator gene during bacteriophage replication following infection of host bacteria results in a soluble indicator protein product. Some systems further comprise a component for capturing the microorganism of interest on a solid support.

In certain embodiments of the disclosure, the systems and/or kits may further comprise a component for washing the captured microorganism sample. Additionally or alternatively, the systems and/or kits may further comprise a component for determining amount of the indicator moiety, wherein the amount of indicator moiety detected corresponds to the amount of microorganism in the sample. For example, in certain embodiments of the disclosure, the system or kit may comprise a luminometer or other device for measuring a luciferase enzyme activity.

In some systems and/or kits, the same component may be used for multiple steps. In some systems and/or kits, the steps are automated or controlled by the user via computer input and/or wherein a liquid-handling robot performs at least one step.

Thus in certain embodiments, the invention may comprise a system for rapid detection of a microorganism of interest in a sample, the system comprising: a recombinant bacteriophage that infects the microorganism of interest, wherein the recombinant bacteriophage comprises an indicator gene inserted into a late gene region of the bacteriophage as the indicator moiety such that the expression of the indicator gene during bacteriophage replication following infection of host bacteria results in a soluble indicator protein product, wherein the indicator gene encodes a luciferase enzyme, and wherein the indicator gene does not encode a fusion protein and wherein the indicator protein product does not comprise polypeptides of the natural bacteriophage; a component for incubating the sample with the recombinant bacteriophage specific for the microorganism of interest; a component for capturing the microorganism from the sample on a solid support; a component for washing the captured microorganism sample to remove unbound infectious agent; and a component for detecting the indicator moiety. In some embodiments, the same component may be used for steps of capturing and/or incubating and/or washing. Some embodiments additionally comprise a component for determining amount of the microorganism of interest in the sample, wherein the amount of indicator moiety detected corresponds to the amount of microorganism in the sample. Such systems can include various embodiments and subembodiments analogous to those described above for methods of rapid detection of microorganisms. The microorganism is a bacterium and the infectious agent is a bacteriophage. In a computerized system, the system may be fully automated, semi-automated, or directed by the user through a computer (or some combination thereof).

In some embodiments, the system may comprise a component for isloated the microorganism of interest from the other components in the sample.

In an embodiment, the disclosure comprises a system comprising components for detecting a microorganism of interest comprising: a component for isolating at least one microorganism from other components in the sample; a component for infecting the at least one microorganism with a plurality of a parental infectious agent; a component for lysing the at least one infected microorganism to release progeny infectious agents present in the microorganism; and a component for detecting the progeny infectious agents, or a constituent of the progeny infectious agents, wherein detection of the infectious agent or a constituent of the infectious agent, indicates that the microorganism is present in the sample.

The systems of the disclosure may comprise a variety of components for detection of progeny infectious agents. For example, in an embodiment of the disclosure, the progeny infectious agent (e.g., bacteriophage) may comprise an indicator moiety. In an embodiment of the disclosure, the indicator moiety in the progeny infectious agent (e.g., bacteriophage) may be a detectable moiety that is expressed during replication, such as a soluble luciferase protein.

In other embodiments, the disclosure may comprise a kit for rapid detection of a microorganism of interest in a sample, the system comprising: a component for incubating the sample with an infectious agent specific for the microorganism of interest, wherein the infectious agent comprises an indicator moiety; a component for capturing the microorganism from the sample on a solid support; a component for washing the captured microorganism sample to remove unbound infectious agent; and a component for detecting the indicator moiety. In some embodiments, the same component may be used for steps of capturing and/or incubating and/or washing. Some embodiments additionally comprise a component for determining amount of the microorganism of interest in the sample, wherein the amount of indicator moiety detected corresponds to the amount of microorganism in the sample. Such kits can include various embodiments and subembodiments analogous to those described above for methods of rapid detection of microorganisms. In an embodiment of the disclosure, the microorganism is a bacterium and the infectious agent is a bacteriophage.

In some embodiments of the disclosure, the kit may comprise a component for isloated the microorganism of interest from the other components in the sample.

In an embodiment, the disclosure comprises a kit comprising components for detecting a microorganism of interest comprising: a component for isolating at least one microorganism from other components in the sample; a component for infecting the at least one microorganism with a plurality of a parental infectious agent; a component for lysing the at least one infected microorganism to release progeny infectious agents present in the microorganism; and a component for detecting the progeny infectious agents, or a constituent of the progeny infectious agents, wherein detection of the infectious agent or a constituent of the infectious agent, indicates that the microorganism is present in the sample.

The kits of the disclosure may comprise a variety of components for detection of progeny infectious agents. For example, in an embodiment of the disclosure, the progeny infectious agent (e.g., bacteriophage) may comprise an indicator moiety. In an embodiment of the disclosure, the indicator moiety in the progeny infectious agent (e.g., bacteriophage) may be a detectable moiety that is expressed during replication, such as a soluble luciferase protein.

These systems and kits of the disclosure include various components. As used herein, the term "component" is broadly defined and includes any suitable apparatus or collections of apparatuses suitable for carrying out the recited method. The components need not be integrally connected or situated with respect to each other in any particular way. The invention includes any suitable arrangements of the components with respect to each other. For example, the components need not be in the same room. But in some embodiments of the disclosure, the components are connected to each other in an integral unit. In some embodiments, the same components may perform multiple functions.

### Computer Systems and Computer Readable Media

The system, as described in the present technique or any of its components, may be embodied in the form of a computer system. Typical examples of a computer system include a general-purpose computer, a programmed microprocessor, a microcontroller, a peripheral integrated circuit element, and other devices or arrangements of devices that are capable of implementing the steps that constitute the method of the present technique.

A computer system may comprise a computer, an input device, a display unit, and/or the Internet. The computer may further comprise a microprocessor. The microprocessor may be connected to a communication bus. The computer may also include a memory. The memory may include random access memory (RAM) and read only memory (ROM). The computer system may further comprise a storage device. The storage device can be a hard disk drive or a removable storage drive such as a floppy disk drive, optical disk drive, etc. The storage device can also be other similar means for loading computer programs or other instructions into the computer system. The computer system may also include a communication unit. The communication unit allows the computer to connect to other databases and the Internet through an I/O interface. The communication unit allows the transfer to, as well as reception of data from, other databases. The communication unit may include a modem, an Ethernet card, or any similar device which enables the computer system to connect to databases and networks such as LAN, MAN, WAN and the Internet. The computer system thus may facilitate inputs from a user through input device, accessible to the system through I/O interface.

A computing device typically will include an operating system that provides executable program instructions for the general administration and operation of that computing device, and typically will include a computer-readable storage medium (e.g., a hard disk, random access memory, read only memory, etc.) storing instructions that, when executed by a processor of the server, allow the computing device to perform its intended functions. Suitable implementations for the operating system and general functionality of the computing device are known or commercially available, and are readily implemented by persons having ordinary skill in the art, particularly in light of the disclosure herein.

The computer system executes a set of instructions that are stored in one or more storage elements, in order to process input data. The storage elements may also hold data or other information as desired. The storage element may be in the form of an information source or a physical memory element present in the processing machine.

The environment can include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers, or other network devices may be stored locally and/or remotely, as appropriate. Where a system includes computing devices, each such device can include hardware elements that may be electrically coupled via a bus, the elements including, for example, at least one central processing unit (CPU), at least one input device (e.g., a mouse, keyboard, controller, touch screen, or keypad), and at least one output device (e.g., a display device, printer, or speaker). Such a system may also include one or more storage devices, such as disk drives, optical storage devices, and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.), and working memory as described above. The computer-readable storage media reader can be connected with, or configured to receive, a computer-readable storage medium, representing remote, local, fixed, and/or removable storage devices as well as storage media for temporarily and/or more permanently containing, storing, transmitting, and retrieving computer-readable information. The system and various devices also typically will include a number of software applications, modules, services, or other elements located within at least one working memory device, including an operating system and application programs, such as a client application or Web browser. It should be appreciated that alternate embodiments may have numerous variations from that described above. For example, customized hardware might also be used and/or particular elements might be implemented in hardware, software (including portable software, such as applets), or both. Further, connection to other computing devices such as network input/output devices may be employed.

Non-transient storage media and computer readable media for containing code, or portions of code, can include any appropriate media known or used in the art, including storage media and communication media, such as but not limited to volatile and non-volatile, removable and non-removable media implemented in any method or technology for storage and/or transmission of information such as computer readable instructions, data structures, program modules, or other data, including RAM, ROM, EEPROM, flash memory or other memory technology, CD-ROM, digital versatile disk (DVD) or other optical storage, magnetic cassettes, magnetic tape, magnetic disk storage or other magnetic storage devices, or any other medium which can be used to store the desired information and which can be accessed by the a system device. Based on the disclosure and teachings provided herein, a person of ordinary skill in the art will appreciate other ways and/or methods to implement the various embodiments.

A computer-readable medium may comprise, but is not limited to, an electronic, optical, magnetic, or other storage device capable of providing a processor with computer-readable instructions. Other examples include, but are not limited to, a floppy disk, CD-ROM, DVD, magnetic disk, memory chip, ROM, RAM, SRAM, DRAM, content-addressable memory ("CAM"), DDR, flash memory such as NAND flash or NOR flash, an ASIC, a configured processor, optical storage, magnetic tape or other magnetic storage, or any other medium from which a computer processor can read instructions. In one embodiment, the computing device may comprise a single type of computer-readable medium such as random access memory (RAM). In other embodiments, the computing device may comprise two or more types of computer-readable medium such as random access memory (RAM), a disk drive, and cache. The computing device may be in communication with one or more external computer-readable mediums such as an external hard disk drive or an external DVD drive.

As discussed above, the embodiment comprises a processor which is configured to execute computer-executable program instructions and/or to access information stored in memory. The instructions may comprise processor-specific instructions generated by a compiler and/or an interpreter from code written in any suitable computer-programming language including, for example, C, C++, C#, Visual Basic, Java, Python, Perl, JavaScript, and ActionScript (Adobe Systems, Mountain View, Calif.). In an embodiment, the computing device comprises a single processor. In other embodiments, the device comprises two or more processors. Such processors may comprise a microprocessor, a digital signal processor (DSP), an application-specific integrated circuit (ASIC), field programmable gate arrays (FPGAs), and state machines. Such processors may further comprise programmable electronic devices such as PLCs, programmable interrupt controllers (PICs), programmable logic devices (PLDs), programmable read-only memories (PROMs), electronically programmable read-only memories (EPROMs or EEPROMs), or other similar devices.

The computing device comprises a network interface. In some embodiments, the network interface is configured for communicating via wired or wireless communication links. For example, the network interface may allow for communication over networks via Ethernet, IEEE 802.11 (Wi-Fi), 802.16 (Wi-Max), Bluetooth, infrared, etc. As another example, network interface may allow for communication over networks such as CDMA, GSM, UMTS, or other cellular communication networks. In some embodiments, the network interface may allow for point-to-point connections with another device, such as via the Universal Serial Bus (USB), 1394 FireWire, serial or parallel connections, or similar interfaces. Some embodiments of suitable computing devices may comprise two or more network interfaces for communication over one or more networks. In some embodiments, the computing device may include a data store in addition to or in place of a network interface.

Some embodiments of suitable computing devices may comprise or be in communication with a number of external or internal devices such as a mouse, a CD-ROM, DVD, a keyboard, a display, audio speakers, one or more microphones, or any other input or output devices. For example, the computing device may be in communication with various user interface devices and a display. The display may use any suitable technology including, but not limited to, LCD, LED, CRT, and the like.

The set of instructions for execution by the computer system may include various commands that instruct the processing machine to perform specific tasks such as the steps that constitute the method of the present technique. The set of instructions may be in the form of a software program. Further, the software may be in the form of a collection of separate programs, a program module with a larger program or a portion of a program module, as in the present technique. The software may also include modular programming in the form of object-oriented programming. The processing of input data by the processing machine may be in response to user commands, results of previous processing, or a request made by another processing machine.

It is intended that the present invention not be limited to the described embodiments, but that it have the full scope defined by the language of the following claims, and equivalents thereof.

### EXAMPLES

Results depicted in the following examples, except for example 11, were obtained without culturing for enrichment or without incubation of the sample to achieve replication of sample cells. Further, in the "no concentration assay," indicator phage added directly to a sample, without concentrating the cells, could infect and detect a low number of cells, even a single bacterium. Any examples falling outside the scope of the claims are provided for comparative purposes only.

### Example 1. Creation of Indicator Phage

Indicator Phage were created using the T7SELECT®415-1 phage display system from Millipore, Inc. Briefly, purified T7SELECT® DNA was purchased and digested with DNA restriction enzymes *Eco*RI and *Hind*III (New England Biolabs), and the cut DNA was subsequently purified. The gene for wild-type Firefly luciferase (from the common Eastern firefly, *Photinus pyralis*) was synthesized with a bacteriophage T7 upstream region including the late T7 promoter, to ensure high levels of expression of the luciferase gene.

The synthesized luciferase gene is designated SEQ ID NO. 1. This gene was amplified by PCR to include compatible restriction enzyme recognition sites for *Eco*RI and *Hind*III to ensure the new gene could be inserted into the T7SELECT®415-1 genome, using the following primers:
TATCTGAATTCTAAGTAACTGATAATACGACTCACTATAGGGAGACCACA AC, designated SEQ ID NO. 2, and
AATGA ***AAGCTT*** TTACAATTTGGACTTTCCGCCCTTCTTGG, designated SEQ ID NO. 3.

Additionally, stop codons in all 3 reading frames were added upstream of the luciferase start site, to terminate production of the bacteriophage major capsid protein, the gene 10B product. The T7SELECT®415-1 phage display system is designed to create a fusion product of the gene 10B major capsid protein and any small protein inserted downstream of it. The addition of the stop codons ensures no fusion product is made, and allows the relatively large luciferase gene to be expressed in soluble form. The PCR product was digested with *Eco*R1 and *Hind*III*,* purified, and ligated into the T7SELECT®415-1. The ligation product was inserted into MegaX DH10B electrocompetent cells (Invitrogen) using a BioRad MicroPulser electroporation system, and the culture plated on *E. coli* for plaques. Plaques were picked, and phage were grown in *E. coli* DH10B and purified via sucrose density gradient centrifugation for use as Indicator Phage, T7SFLFCT®415-Luc.

**Figure 1** depicts the genomic structure of an example Indicator Phage, T7SELECT®415-Luc. The detectable indicator moiety is encoded by the Firefly luciferase gene inserted within the Class III gene region, expressed late in the viral life cycle and at higher levels than other phage genes. The construct contains stop codons to ensure the luciferase is not incorporated into a native gene product, such as the capsid protein gene 10B, and is thus also not a fusion protein. Thus this construct allows progeny phage to express soluble Firefly luciferase as the indicator to be detected.

### Example 2. Isolation and purification of E. coli O157:H7 specific bacteriophage from the environment.

Samples from the Hyperion Sewage Treatment Plant were obtained along with water samples from the neighboring Ballona wetlands. The samples were mixed with powdered Nutrient Broth (Gibco, Inc.) to 1x and inoculated with *E*. *coli* 0157:H7 (ATCC 43888) from 3 mL of turbid culture. The sample was incubated for 3 hours @ 37°C with shaking to enrich for phage that infect *E. coli* 0157:H7, lysed with 120 µL chloroform, vortexed for 15 seconds, and a 1 mL sample was centrifuged for 2 minutes at 6800g. The supernatants were filtered (0.45 µm filter) and plated for plaques on *E. coli* O157:H7. This sample was plated out in plaque assays in various dilutions to obtain well isolated plaques. Individual plaques were stabbed with disposable pipette tips and resuspended in 100 µL TMS buffer (50mM Tris-HCl, pH 7.4, 10mM MgCl₂, and 100mM NaCl).

Aliquots of 5 µL of resuspended plaques were spot-tested in overlay agar on *E. coli* strains O157:H7, B, and DH10B, and on a strain of *Salmonella.* Phage that only cleared 0157:H7 were amplified in *E. coli* 0157:H7, the lysates clarified by centrifugation, and the particles put into TMS buffer using ZEBA® Buffer Exchange columns.

Eight closely related phage isolates were grown on *E. coli* 0157:H7, purified, and the genomes isolated and sequenced. Three genomic types all showed 98 % homology to the T4-like phage RB69. The late genes were mapped based on this homology, and phage JG04 was selected for further study. **Figure 2** shows JG04, and the genome of JG04 is designated SEQ ID NO. 4.

### Example 3. Creation and purification of recombinant indicator phage

Based on sequence analysis and location of the late gene region, homologous recombination sequences were synthesized with inserts for various reporter genes, consisting of different luciferase proteins, as shown in **Figure 3****.** Specifically, three constructs were used: Firefly luciferase homologous recombination plasmid, pUC57.HR.Fluc, corresponding to SEQ ID NO. 5; NANOLUC® Homologous Recombination Plasmid, pUC57.HR.NANOLUC® corresponding to SEQ ID NO. 6; and Oplophorus Luciferase Homologous Recombination Plasmid, pUC19.HR.OpLuc.KanR, corresponding to SEQ ID NO. 7.

Luciferase genes were inserted with an upstream T4 late gene promoter, to ensure expression during the viral late stage. In some cases, a kanamycin resistance marker was also inserted to allow for selection of infected cells under the kanamycin antibiotic. These regions were flanked with up to 500 bp of sequence matching the capsid proteins, gp23 and gp24. These synthesized sequences were carried by the Ampicillin resistant pUC57 or pUC19 plasmid.

Plasmids containing the synthesized sequences were transformed into electroporation competent *E. coli* 0157:H7 using a BIORAD® Gene Pulser II electroporator, conferring resistance to Ampicillin. Colonies were screened for positive transformation by assaying with the appropriate luciferase substrate (D-luciferin for Firefly luciferase, coelenterazine for Oplophorus luciferase, and either coelenterazine or NANOGLO® for NANOLUC®). *E. coli* O157:H7 harboring a plasmid with *luc* flanked by sequences homologous with phage JG04 were grown in Ampicillin-containing LB broth bacterial cultures were grown in Ampicillin containing LB broth to approximately 10⁷ cells/mL. Cultures were then infected with phage JG04 at an MOI of 1 and incubated for 45 minutes at 37°C with shaking to lyse cells. Lysates contained a mixture of mostly wild-type phage with a minority of recombinant phage created by homologous recombination of the wild-type phage genome with the homologous recombination plasmid.

In order to determine the ratio of recombinant to wild-type phage, limiting dilution assays based on the TCID50 (tissue culture infectious dose 50%) were used to both determine the concentration of infectious units (IU/mL), akin to number of virus particles or plaque forming units, and to determine the number of luciferase transducing units (TU/mL). In these assays, the sample was serially diluted, with each dilution aliquoted into replicates wells with *E. coli* O157:H7 bacteria. Any wells that show luciferase activity must have been infected with at least one recombinant phage. Any wells that showed cell lysis had been infected by at least one phage. Based on the highest dilution where each of these cases occurred, the original concentrations were back-calculated. These initial phage mixtures from transformed cells typically yielded a ratio of 20,000 wild-type IU for each recombinant phage TU. Steps were then taken to isolate and amplify the recombinant phage.

As illustrated in **Figure 4****,** recombinant phage were isolated from a mixture comprising 0.005% of total phage. The phage mixtures were diluted into 96 well plates to give an average of 3 recombinant TU per plate, which breaks down to about 625 IU of mostly wild-type phage per well. Each well contained 50 µL of turbid *E. coli* O157:H7. After 2 hours of incubation at 37°C, wells were sampled and screened for the presence of luciferase. Any positive wells would likely have been inoculated with a single recombinant phage, and ∼600 wild-type phage, which is an enrichment over the original 20,000:1 ratio. Progeny from this enriched culture was subjected to another limiting dilution assay to verify the ratio and determine the actual concentration of recombinant phage transducing units.

Again, 3 recombinant TU per 96-well plate were aliquoted from this stock, leading to an approximate inoculation of ∼20 mostly wild-type phage per well. Any positive luciferase wells were likely to have been inoculated with a single recombinant along with ∼20 wild-type phage. These wells were analyzed for luciferase activity, and any positive wells were subjected to the limiting dilution assay to determine the ratio of TU to IU, then to plaque assay to obtain well-isolated plaques.

At this point, the expected ratio of wild-type to recombinants was about 20:1. 48 plaques were individually picked and screened for luciferase transducing ability, insuring about 3 recombinants were in the mix of plaques being screened. Each plaque was suspended in 100 µL TMS, and 5 µL was added to a well containing a turbid *E. coli* 0157:H7 culture, and wells were assayed after incubation for 45 minutes to 1 hour at 37°C.

Positive wells were expected to contain a pure culture of recombinant phage, but an additional round of plaque purification was standard procedure. Large scale production was performed to obtain high titer stocks appropriate for use in the *E. coli* 0157:H7 detection assay. Cesium chloride isopycnic density gradient centrifugation was used to separate phage particles from contaminating luciferase protein to reduce background.

### Example 4. Bacterial Detection via Indicator Phage using Spin Column Filters

**Figure 5** illustrates use of indicator phage and spin column filters for bacterial detection, according to an embodiment of the invention. In an example experiment, *E. coli* DH10B was grown in Luria-Bertani broth (LB) at 37°C with shaking. T7SELECT® 415-Luc phage were diluted to 10⁸ PFU/40 µL (2.5 X 10⁹ PFU/mL). Cells were counted and diluted to 3000, 300, 30 and 3 cells/mL. A CFU assay was performed in parallel with the following luciferase assay to determine the actual number of input cells per assay.

For each cell dilution, 0.1 mL was added to filters in triplicate. Filters were spun at 600 g for 1 minute. Next, 40 µL of each of the phage dilution was added to each filter, followed by incubation for 10 min at room temperature. Filters were washed twice by addition of 400 µL PBST (0.05% Tween), followed by centrifugation at 600 g for 1 minute. Next 50 µL LB was added, followed by incubation for 30 minutes at 37°C. Filters were spun at 6800 g for 2 minutes. Next, 30 µL of the filtrate was transferred to a LUMITRAC® 200 96-well luminometer plate, and a luciferase assay was performed in a Promega luminometer with injection of 100 µL Luciferase Assay Reagent (Promega, Inc.). Cell count was corrected according to the number of colonies in the parallel CFU assay. The signal to background ratio was obtained by dividing each well's signal by the average of signal from zero cell controls. **Figure 6** shows results demonstrating high sensitivity of the assay for detecting as few as 1 to 3 *E. coli* cells, via luciferase activity. **Figure 7** shows results demonstrating the very large detection range of the same method using serial dilutions of the starting bacterial sample. This shows detection from an average of 1.4 cells to 14 million cells.

### Example 5. Bacterial Detection via Indicator Phage using 96-well Filter Plates

*E. coli* DH10B was grown in LB at 37°C with shaking. T7SELECT®415-Luc phage were diluted to 4 x 10⁷ PFU/20 µL in LB (2 X 10⁹ PFU/mL). Cells were counted and diluted to 500, 50 and 5 cells/mL (0.1 mL was added to each wells, giving ∼50, 5 and <1 cells shown in Figure 5). A CFU assay was performed in parallel with the following luciferase assay to determine the actual number of input cells per assay.

For each cell dilution, 0.1 mL was added to multiple wells in a 96-well filter plate: 9 wells for 0.5 cells and 3 wells for 50 cells, 5 cells and the zero cell control. The 96-well filter plate was spun at 1200 rpm (263 rcf) for 3 minutes. Next 20 µL of the phage dilution was added to each filter and incubated for 10 minutes at room temperature, followed by 30 minutes at 37°C. The luciferase assay was performed directly in the original filter plate with 100 µL Luciferase Assay Reagent (Promega, Inc.) injection using a Promega Luminometer, and plates were read immediately following injection and reread at 15 and 30 minutes thereafter. Cell count was corrected according to the number of colonies in the parallel CFU assay. Signal to background ratios were obtained by dividing each well's signal by the average of signal from zero cell controls.

**Figure 8** shows results demonstrating the use of the 96-well filter plate for detecting single *E. coli* cells in each well (0.5 cells on average were assayed, such that about one-half of the wells received single cells), and 5.4 and 54 cells per well.

**Figure 9** shows results demonstrating a very large cell detection range, using the 96-well filter plate system, from less than 1 cell per well on average (single cells) to at least 14 million cells per well.

### Example 6. Filter Plate Assay using JG04-NANOLUC® Indicator Phage and low cell concentration

*E. coli* O157:H7 cells were grown in LB at 37°C with 220 rpm shaking. JG04-NANOLUC® Indicator Phage were prepared to 10⁶ PFU/20 µL. Cells were counted and diluted to 7290, 2430, 810, 270, 90, 30, 10 and 0 cells /mL. Aliquots of 100 µL of each sample were deposited into wells of a PERKINELMER® Optiplate 96-well Grey Luminometer 0.45 µm filter plate in replicates.

As illustrated in **Figure 10****,** plates were loaded into a swinging bucket centrifuge and spun at 2400 rpm for 3 minutes. 20 µL of JG04-NANOLUC® phage dilution was added to each well, giving a final concentration of 5 x 10⁷ PFU/mL. Plates were incubated for 10 minutes at room temperature, 200 µL PBST was added to each well, and plates were spun down for 3 minutes at 2400 rpm to wash away excess parental JG04-NANOLUC® phage.

Next, 50 µL LB was added to each well and plates were incubated for 45 minutes in a 37°C incubator without shaking. Aliquots of 10 µL Promega Renilla Luciferase Lysis Buffer were added to wells, analyte was transferred to the wells, and luciferase assay was performed with 50 µL Promega NANOGLO® Reagent injection. Samples with cells were compared to 0 cell controls.

As seen in **Figure 11****,** the NANOLUC® Filter Plate results show statistically significant differences between the signal from 0 cells and 1 cell / assay (p value = 0.034 by Student's t-Test), demonstrating the ability to detect single cells. More bacterial cells per assay show increasing signal in a dose dependent manner.

### Example 7. No concentration assay with low cell concentrations

In preparation for an experiment similar to the assay illustrated in schematic **Figure 12****,** *E. coli* O157:H7 cells were grown in LB at 37°C with 220 rpm shaking. JG04-OpLuc Indicator Phage were prepared to 1.2 x 10⁷ PFU/20 µL, and 20 µL aliquots were distributed to wells of a PERKINELMER® Optiplate 96-well Grey Luminometer plate. Cells were counted and diluted to 10, 3.3, 1.1 and 0 cells /mL. Aliquots of 100 µL of each sample were distributed into wells in replicates (12x for 0.11 and 0.33 cells /assay and 5x for 1 cell /assay), giving a final phage concentration of 10⁸ PFU/mL.

Plates were incubated for 45 minutes in a 37°C incubator without shaking. Finally, 10 µL PROMEGA® Renilla Luciferase Lysis Buffer was added to each well, and the luciferase assay was performed with 50 µL PROMEGA® Renilla Luciferase Assay Reagent (coelenterazine) injection. Samples were compared to 0 cell controls.

As seen in **Figure 13****,** the No Concentration Assay with low cell concentration samples results show statistically significant differences between the signal from 0 cells and 1 cell / assay (p value = 0.0024 by ANOVA test), demonstrating the ability to detect single cells. Thus the assay is surprisingly sensitive. Samples with fewer than 1 cell per well appear to show a proportional number of wells above the background signal.

### Example 8. No concentration assay with wide cell concentration range

In a similar experiment with higher cell concentrations, *E. coli* 0157:H7 cells were grown in LB at 37°C with 220 rpm shaking. JG04-OpLuc Indicator Phage were prepared to 1.2 x 10⁷ PFU/20 µL, and 20 µL aliquots were distributed to wells of a PERKINELMER® Optiplate 96-well Grey Luminometer plate. Cells were counted and diluted to 10⁸, 10⁷, 10⁶, 10⁵, 10⁴, 10³, 10², 33, 11, 3.7, 1.2 and 0 cells /mL. Aliquots of 100 µL of each sample were distributed into wells in replicates, giving a final phage concentration of 10⁸ PFU/mL.

Plates were incubated for 45 minutes in a 37°C incubator without shaking. 10 µL PROMEGA® Renilla Luciferase Lysis Buffer was added to each well, and luciferase assay was performed with 50 µL PROMEGA® Renilla Luciferase Assay Reagent (coelenterazine) injection. Samples were compared to 0 cell controls.

As seen in **Figure 14****,** the experiment for the No Concentration Assay with a very wide range of cell concentration samples shows statistically significant differences between the signal from 0 cells and 1.1 cell / assay (p value = 0.000702 by Student's t-Test), demonstrating the ability to detect single cells. More bacterial cells per assay show increasing signal in a dose dependent manner, up to at least 10⁶ bacterial cells /mL, surprisingly demonstrating a very wide range of detection.

### Example 9. No Concentration Assay with vegetable wash samples

To prepare the vegetable wash, vegetable leaves (e.g., spinach or lettuce) were weighed and added to a clean plastic bag. One mL of LB (+/- 0.01 - 0.05% Tween20) was added per each gram (g) of vegetable. Leaves and solution were mixed manually for a few minutes. Liquid was then extracted from the plastic bag and used as the "vegetable wash." Using this method, ∼ 1 million bacteria were found by CFU to reside on a single spinach leaf (1-2 g).

*E. coli* O157:H7 cells were grown in LB at 37°C with 220 rpm shaking. JG04-OpLuc Indicator Phage were prepared to 1.2 x 10⁷ PFU/20 µL, and 20 µL aliquots were distributed to wells of a PERKINELMER® Optiplate 96-well Grey Luminometer plate. Cells were counted and diluted into Vegetable Wash at 120, 60, 30, and 0 cells /mL. Aliquots of 100 µL of each sample were distributed into wells in replicates, giving a final phage concentration of 10⁸ PFU/mL.

Plates were incubated for 45 minutes in a 37°C incubator without shaking. 10 µL PROMEGA® Renilla Luciferase Lysis Buffer was added to each well, and luciferase assay was performed with 50 µL PROMEGA® Renilla Luciferase Assay Reagent (coelenterazine) injection. Samples were compared to 0 cell controls.

As seen in **Figure 15****,** the experiment for the No Concentration Assay using vegetable wash samples shows marked differences between the signal from 0 cells and 3 cells/assay, demonstrating the ability to detect single-digit cell numbers in vegetable wash. Using more bacterial cells per assay shows increasing signal in a dose dependent manner. The vegetable wash contains about 10⁶ non-target bacteria /mL, corresponding to about 10⁵ non-target bacteria in this assay (including the 0 cell *E. coli* O157:H7 control). The ability to discern as few as 3 target bacterial cells from 10⁵ non-target bacteria is surprising and again demonstrates the specificity of the assay.

### Example 10. Specific and Quantitative Capture of E. coli O157:H7 Using Antibodies and Beads

**Figure 16** depicts an example experiment demonstrating that antibodies against *E. coli* O157:H7 and magnetic streptavidin-coated beads captured *E. coli* O157:H7 specifically and quantitatively from samples, but not *E. coli* B or *Salmonella typhimurium* in samples. In order to demonstrate specificity in capturing intact, viable bacterial cells from solution, polyclonal antibodies to surface epitopes of *E. coli* O157:H7 were purchased from KPL (affinity- and reverse-purified to minimize cross-reactivity).

Cultures of both *E. coli* species and *S. typhimurium* were grown in LB broth, harvested, and washed with phosphate buffer (1.1 mM KH₂PO₄, 5.6 mM Na₂HPO₄, 154 mM NaCl, pH 7.4). The washed cells were then counted and diluted to a concentration between 5-20 cells per mL.

A sample containing *E. coli* O157:H7 was combined with a solution containing BSA and biotin-conjugated antibody. Approximately 10 ng biotinylated, polyclonal anti-*E. coli* antibody (equivalent to about 4 x 10¹⁰ antibody molecules) produced by KPL was added to each of the cell suspensions. A control experiment, where antibody was not added to the cell suspension, was also performed in parallel. The BSA concentration was ∼1%, and the total biotin-antibody was 10 ng. The mixture was rotated end-over-end for 1 hour.

Following incubation with antibodies, 4 x 10⁷ streptavidin-coated magnetic microparticles (Invitrogen/Life Technologies) were added to the mixture and incubated a further 30 minutes. The cell-antibody-bead complexes were then collected using a magnetic stand, and the unbound fraction (supernatant) was removed. The beads were gently washed with phosphate buffer with Tween-20 (1.1 mM KH₂PO₄, 5.6 mM Na₂HPO₄, 154 mM NaCl, pH 7.4, 0.05% Tween-20). Both the supernatants and captured cell-bead complexes were then spread onto LB agar plates and the plates incubated overnight at 37° C to determine CFU. Capture of *E. coli* 0157:H7 with anti-O157:H7 antibodies, but not *E. coli* B or *Salmonella typhimurium,* was specific and quantitative.

### Example 11. Hybrid Immuno-Phage (HIP) Assay

As illustrated in schematic Figure 17, the Hybrid Immuno-Phage or "HIP" assay combines the benefits of bacterium-specific antibody capture with the benefits of a modified bacteriophage. The sample is first applied to the microtiter plate well coated with bacterium-specific antibodies (1702). The plate is then centrifuged to facilitate binding of the bacterium to the capture antibodies (1704). Following sufficient time to allow for complete capture of bacteria, a solution containing bacterium-specific Luc-phage is added to each sample (1706). Incubation with the phage results in the binding and attachment of a single or multiple phages to the captured bacterium (1708). Finally, the sample is incubated to facilitate phage replication and luciferase expression, which leads to cell lysis and release of soluble luciferase (1710).

In a HIP assay experiment, a white 96-well ELISA plate was coated with 300 ng monoclonal antibody (in 100 µL PBS) specific to the bacterium of interest at room temperature for 2 - 3 hours. Wells were washed with PBS (200 µL × 3 washes), blocked with 5% BSA/PBS (300 µL) at room temperature for 1 - 1.5 hours, and again washed with 300 µL PBS × 1. Test samples (100 µL) were applied to the wells.

The ELISA plate was centrifuged at 700 × g for 30 minutes and then incubated at room temperature for 1 hour. JG04-NANOLUC® phage was added to samples (2 - 4 × 10⁶ PFU in 10 µL LB) and incubated at room temperature for 10 minutes. Samples were washed with PBS (200 µL × 2 washes). LB medium was added to samples (100 µL) and incubated at 37°C for 1.5 hours.

NANOGLO® substrate (50 µL) was added directly to the samples, and the luminescence was measured in a luminometer. As seen in Figure 18, the HIP assay was able to detect 100 and 1,000 *E.coli* O157:H7 cells in LB medium with approximately 2 × 10⁶ PFU JG04-NANOLUC® phage. The average signal over a no-cell sample is shown on a log scale and ranged from approximately 50-fold for the 100 cell sample to over 1,000-fold for the 1,000 cell sample.

## Claims

1. A recombinant bacteriophage comprising an indicator gene inserted into a late gene region of the bacteriophage, wherein the indicator gene encodes a luciferase enzyme, and wherein the indicator gene does not encode a fusion protein and wherein the indicator protein product does not comprise polypeptides of the natural bacteriophage.

2. The recombinant bacteriophage of claim 1, wherein the indicator gene is adjacent to a major capsid gene and optionally wherein transcription of the indicator gene is controlled by a bacteriophage late promoter.

3. The recombinant bacteriophage of claim 1, wherein the bacteriophage is derived from T7, T4, T4-like, JG04, JG04-like or another natural Bacteriophage having a genome with at least 90% homology to T7, JG04, JG04-like, T4, or other T4-like phage.

4. The bacteriophage of claim 1, wherein expression of the indicator gene during bacteriophage replication following infection of a host bacterium results in a soluble indicator protein product and optionally wherein the luciferase is one of Oplophorus luciferase, Firefly luciferase, Lucia luciferase, or an engineered luciferase.

5. A method for detecting a microorganism of interest in a sample comprising the steps of:
incubating the sample with a recombinant bacteriophage that infects the microorganism of interest, wherein the recombinant bacteriophage comprises an indicator gene inserted into a late gene region of the bacteriophage such that expression of the indicator gene during bacteriophage replication following infection of host bacteria results in a soluble indicator protein product, wherein the indicator gene encodes a luciferase enzyme, and wherein the indicator gene does not encode a fusion protein and wherein the indicator protein product does not comprise polypeptides of the natural bacteriophage; and
detecting the indicator protein product, wherein positive detection of the indicator protein product indicates that the microorganism of interest is present in the sample.

6. The method of claim 5, wherein the amount of indicator moiety detected corresponds to the amount of the microorganism of interest present in the sample

7. The method of claim 5, wherein the indicator gene is adjacent to a major capsid gene.

8. The method of claim 5, wherein the bacteriophage is derived from T7, T4, T4-like, JG04, or another natural bacteriophage having a genome with at least 90% homology to T7, T4 or other T4-like phage.

9. The method of claim 5, wherein the bacteriophage concentration for the incubating step to infect bacteria is greater than 1 x 107 plaque forming units per milliliter of volume.

10. The method of claim 5, wherein the recombinant bacteriophage is purified using cesium chloride isopycnic density gradient centrifugation prior to incubating with the sample.

11. The method of claim 5, further comprising a step for capturing the microorganism from the sample on a solid support before incubating with the recombinant bacteriophage to infect bacteria.

12. The method of claim 11, wherein the solid support comprises a multi-well plate or a filter.

13. The method of claim 11, further comprising a step for washing the captured and infected microorganism, after adding the bacteriophage but before cell lysis, to remove excess bacteriophage and/or contaminating reporter protein, such as luciferase.

14. The method of claim 5, wherein the method can detect < 10 cells of the microorganism in the sample, and optionally wherein detection of the microorganism of interest is completed in less time that a time period required for increasing the number of microorganisms by 4-fold or 10-fold using culturing for enrichment, optionally wherein the total time required for detection is less than 2 hours.

15. A system for rapid detection of a microorganism of interest in a sample, comprising:
a recombinant bacteriophage that infects the microorganism of interest, wherein the recombinant bacteriophage comprises an indicator gene inserted into a late gene region of the bacteriophage as the indicator moiety such that the expression of the indicator gene during bacteriophage replication following infection of host bacteria results in a soluble indicator protein product , wherein the indicator gene encodes a luciferase enzyme, and wherein the indicator gene does not encode a fusion protein and wherein the indicator protein product does not comprise polypeptides of the natural bacteriophage;
a component for incubating the sample with the recombinant bacteriophage; and
a component for detecting the indicator moiety.

## Patentansprüche

1. Rekombinanter Bakteriophage, der ein Indikatorgen umfasst, das in eine späte Genregion des Bakteriophagen insertiert ist, wobei das Indikatorgen ein Luziferase-Enzym kodiert und wobei das Indikatorgen ein Fusionsprotein nicht kodiert und wobei das Indikatorproteinprodukt keine Polypeptide des natürlichen Bakteriophagen umfasst.

2. Rekombinanter Bakteriophage nach Anspruch 1, wobei das Indikatorgen zu einem Hauptkapsidgen benachbart ist und fakultativ wobei die Transkription des Indikatorgens durch einen späten Promotor des Bakteriophagen gesteuert wird.

3. Rekombinanter Bakteriophage nach Anspruch 1, wobei der Bakteriophage von einem T7-, T4-, T4-artigen, JG04-, JG04-artigen oder anderen natürlichen Bakteriophagen mit einem Genom mit mindestens 90 % Homologie zu einem T7-, JG04-, JG04-artigen, T4- oder anderen T4-artigen Phagen abgeleitet ist.

4. Bakteriophage nach Anspruch 1, wobei die Expression des Indikatorgens während der Bakteriophagenreplikation nach Infektion eines Wirtsbakteriums zu einem löslichen Indikatorproteinprodukt führt und fakultativ wobei die Luziferase eine von Oplophorus-Luziferase, Firefly-Luziferase, Lucia-Luziferase oder eine konstruierte Luziferase ist.

5. Verfahren zum Nachweis eines Mikroorganismus von Interesse in einer Probe, das die folgenden Schritte umfasst:
Inkubieren der Probe mit einem rekombinanten Bakteriophagen, der den Mikroorganismus von Interesse infiziert, wobei der rekombinante Bakteriophage ein Indikatorgen umfasst, das in eine späte Genregion des Bakteriophagen insertiert ist, so dass die Expression des Indikatorgens während der Bakteriophagenreplikation nach Infektion von Wirtsbakterien zu einem löslichen Indikatorproteinprodukt führt,
wobei das Indikatorgen ein Luziferase-Enzym kodiert und wobei das Indikatorgen ein Fusionsprotein nicht kodiert und wobei das Indikatorproteinprodukt keine Polypeptide des natürlichen Bakteriophagen umfasst; und
Nachweisen des Indikatorproteinprodukts, wobei ein positiver Nachweis des Indikatorproteinprodukts darauf hinweist, dass der Mikroorganismus von Interesse in der Probe vorliegt.

6. Verfahren nach Anspruch 5, wobei die Menge an nachgewiesener Indikatoreinheit der Menge des Mikroorganismus von Interesse, der in der Probe vorliegt, entspricht.

7. Verfahren nach Anspruch 5, wobei das Indikatorgen zu einem Hauptkapsidgen benachbart ist.

8. Verfahren nach Anspruch 5, wobei der Bakteriophage von einem T7-, T4-, T4-artigen, JG04- oder anderen natürlichen Bakteriophagen mit einem Genom mit mindestens 90 % Homologie zu einem T7-, T4- oder anderen T4-artigen Phagen abgeleitet ist.

9. Verfahren nach Anspruch 5, wobei die Bakteriophagenkonzentration für den Inkubationsschritt zum Infizieren von Bakterien höher als 1 x 10⁷ Plaque-bildende Einheiten pro Milliliter des Volumens ist.

10. Verfahren nach Anspruch 5, wobei der rekombinante Bakteriophage unter Verwendung von isopyknischer Cäsiumchlorid-Dichtegradientenzentrifugation vor Inkubieren mit der Probe aufgereinigt wird.

11. Verfahren nach Anspruch 5, das weiterhin einen Schritt zum Einfangen des Mikroorganismus aus der Probe auf einem festen Träger vor Inkubieren mit dem rekombinanten Bakteriophagen zum Infizieren von Bakterien umfasst.

12. Verfahren nach Anspruch 11, wobei der feste Träger eine Multi-Well-Platte oder einen Filter umfasst.

13. Verfahren nach Anspruch 11, das weiterhin einen Schritt zum Waschen des eingefangenen und infizierten Mikroorganismus nach Zugeben des Bakteriophagen, jedoch vor einer Zelllyse umfasst, um überschüssigen Bakteriophagen und/oder kontaminierendes Reporterprotein, wie Luziferase, zu entfernen.

14. Verfahren nach Anspruch 5, wobei das Verfahren < 10 Zellen des Mikroorganismus in der Probe nachweisen kann und fakultativ wobei der Nachweis des Mikroorganismus von Interesse in weniger Zeit als ein Zeitraum, der zum Erhöhen der Anzahl von Mikroorganismen um das 4-fache oder das 10-fache unter Verwendung von Kultivierung zur Anreicherung erforderlich ist, abgeschlossen wird, fakultativ wobei die zum Nachweis erforderliche Gesamtzeit weniger als 2 Stunden beträgt.

15. System zum schnellen Nachweis eines Mikroorganismus von Interesse in einer Probe, umfassend:
einen rekombinanten Bakteriophagen, der den Mikroorganismus von Interesse infiziert, wobei der rekombinante Bakteriophage ein Indikatorgen, das in eine späte Genregion des Bakteriophagen insertiert ist, als die Indikatoreinheit umfasst, so dass die Expression des Indikatorgens während der Bakteriophagenreplikation nach Infektion von Wirtsbakterien zu einem löslichen Indikatorproteinprodukt führt, wobei das Indikatorgen ein Luziferase-Enzym kodiert und wobei das Indikatorgen ein Fusionsprotein nicht kodiert und wobei das Indikatorproteinprodukt keine Polypeptide des natürlichen Bakteriophagen umfasst;
eine Komponente zum Inkubieren der Probe mit dem rekombinanten Bakteriophagen und
eine Komponente zum Nachweisen der Indikatoreinheit.

## Revendications

1. Un bactériophage recombinant comprenant un gène indicateur inséré dans une région de gènes tardifs du bactériophage, le gène indicateur codant pour une enzyme luciférase, et le gène indicateur ne codant pas pour une protéine de fusion et le produit protéique indicateur ne comprenant pas de polypeptides du bactériophage naturel.

2. Le bactériophage recombinant de la revendication 1, dans lequel le gène indicateur est adjacent à un gène capside majeur et éventuellement dans lequel la transcription du gène indicateur est contrôlée par un promoteur tardif du bactériophage.

3. Le bactériophage recombinant de la revendication 1, le bactériophage étant dérivé de T7, T4, type T4, JG04, type JG04 ou un autre bactériophage naturel ayant un génome avec au moins 90% d'homologie avec T7, JG04, type JG04, T4, ou un autre phage de type T4.

4. Le bactériophage de la revendication 1, dans lequel l'expression du gène indicateur au cours de la réplication du bactériophage à la suite de l'infection d'une bactérie hôte résulte en un produit protéique indicateur soluble et éventuellement dans lequel la luciférase est l'une des suivantes : luciférase Oplophorus, luciférase Firefly, luciférase Lucia ou une luciférase modifiée.

5. Un procédé de détection d'un microorganisme d'intérêt dans un échantillon comprenant les étapes consistant à :
incuber l'échantillon avec un bactériophage recombinant qui infecte le microorganisme d'intérêt, le bactériophage recombinant comprenant un gène indicateur inséré dans une région de gènes tardifs du bactériophage de manière à ce que l'expression du gène indicateur au cours de la réplication du bactériophage à la suite de l'infection de bactéries hôtes résulte en un produit protéique indicateur soluble, le gène indicateur codant pour une enzyme luciférase, et le gène indicateur ne codant pas pour une protéine de fusion et le produit protéique indicateur ne comprenant pas de polypeptides du bactériophage naturel ; et
détecter le produit protéique indicateur, la détection positive du produit protéique indicateur indiquant que le microorganisme d'intérêt est présent dans l'échantillon.

6. Le procédé de la revendication 5, dans lequel la quantité de fragment d'indicateur détecté correspond à la quantité de microorganisme d'intérêt présent dans l'échantillon.

7. Le procédé de la revendication 5, dans lequel le gène indicateur est adjacent à un gène capside majeur.

8. Le procédé de la revendication 5, dans lequel le bactériophage est dérivé de T7, T4, type T4, JG04 ou un autre bactériophage naturel ayant un génome avec au moins 90% d'homologie avec T7, T4, ou un autre phage de type T4.

9. Le procédé de la revendication 5, la concentration de bactériophages pour l'étape d'incubation permettant d'infecter des bactéries est supérieure à 1 x 10⁷ unités de formation de plaques par millilitre de volume.

10. Le procédé de la revendication 5, dans lequel le bactériophage recombinant est purifié par centrifugation en gradient de densité isopycnique de chlorure de césium avant l'incubation avec l'échantillon.

11. Le procédé de la revendication 5, comprenant en sus une étape consistant à capturer le microorganisme à partir de l'échantillon sur un support solide avant l'incubation avec le bactériophage recombinant pour infecter les bactéries.

12. Le procédé de la revendication 11, dans lequel le support solide comprend une plaque multi-puits ou un filtre.

13. Le procédé de la revendication 11, comprenant en sus une étape consistant à laver le microorganisme capturé et infecté, après avoir ajouté le bactériophage mais avant la lyse cellulaire, pour éliminer l'excès de bactériophage et/ou de protéine rapporteur contaminante, telle que la luciférase.

14. Le procédé de la revendication 5, le procédé pouvant détecter < 10 cellules du microorganisme dans l'échantillon, et éventuellement dans lequel la détection du microorganisme d'intérêt est terminée en moins de temps qu'une période requise pour augmenter le nombre de microorganismes par 4 fois ou 10 fois en utilisant une culture d'enrichissement, éventuellement dans lequel le temps total requis pour la détection est inférieur à 2 heures.

15. Un système de détection rapide d'un microorganisme d'intérêt dans un échantillon, comprenant :
un bactériophage recombinant qui infecte le microorganisme d'intérêt, le bactériophage recombinant comprenant un gène indicateur inséré dans une région de gènes tardifs du bactériophage en tant que fragment indicateur de manière à ce que l'expression du gène indicateur au cours de la réplication du bactériophage à la suite de l'infection de bactéries hôtes résulte en un produit protéique indicateur soluble, le gène indicateur codant pour une enzyme luciférase, et le gène indicateur ne codant pas pour une protéine de fusion, et le produit protéique indicateur soluble ne comprenant pas de polypeptides du bactériophage naturel ;
un composant pour incuber l'échantillon avec le bactériophage recombinant ; et
un composant pour détecter le fragment indicateur.


### SEQUENCE LISTING

<110> Laboratory Corporation of America Holdings Anderson, Dwight Lyman Gil, Jose S. Hopkins, Ben Barrett Erickson, Stephen
<120> Methods and Systems for Rapid Detection
   of Microorganisms using Infectious Agents
<130> 57618-929955
<150> US 61/940,959
   <151> 2014-02-18
<160> 7
<170> FastSEQ for Windows Version 4.0
<210> 1
   <211> 2196
   <212> DNA
   <213> Artificial Sequence
<220>
   <223> Synthesized luciferase gene sequence
<400> 1
   catatgagtc ttgtgatgta ctggctgatt tctacgacca gttcgctgac cagttgcacg 60
   agtctcaatt ggacaaaatg ccagcacttc cggctaaagg taacttgaac ctccgtgaca 120
   tcttagagtc ggacttcgcg ttcgcgtaac gccaaatcaa tacgactcac tatagaggga 180
   caaactcaag gtcattcgca agagtggcct ttatgattga ccttcttccg gttaatacga 240
   ctcactatag gagaacctta aggtttaact ttaagaccct taagtgttaa ttagagattt 300
   aaattaaaga attactaaga gaggacttta agtatgcgta acttcgaaaa gatgaccaaa 360
   cgttctaacc gtaatgctcg tgacttcgag gcaaccaaag gtcgcaagtt gaataagact 420
   aagcgtgacc gctctcacaa gcgtagctgg gagggtcagt aagtaatacg actcactata 480
   gggagaccac aacggtttcc tccctgtagt ctttttgttt aactttaagg aggtcaaatg 540
   gaagacgcca aaaacataaa gaaaggcccg gcgccattct atcctctaga ggatggaacc 600
   gctggagagc aactgcataa ggctatgaag agatacgccc tggttcctgg aacaattgct 660
   tttacagatg cacatatcga ggtgaacatc acgtacgcgg aatacttcga aatgtccgtt 720
   cggttggcag aagctatgaa acgatatggg ctgaatacaa atcacagaat cgtcgtatgc 780
   agtgaaaact ctcttcaatt ctttatgccg gtgttgggcg cgttatttat cggagttgca 840
   gttgcgcccg cgaacgacat ttataatgaa cgtgaattgc tcaacagtat gaacatttcg 900
   cagcctaccg tagtgtttgt ttccaaaaag gggttgcaaa aaattttgaa cgtgcaaaaa 960
   aaattaccaa taatccagaa aattattatc atggattcta aaacggatta ccagggattt 1020
   cagtcgatgt acacgttcgt cacatctcat ctacctcccg gttttaatga atacgatttt 1080
   gtaccagagt cctttgatcg tgacaaaaca attgcactga taatgaattc ctctggatct 1140
   actgggttac ctaagggtgt ggcccttccg catagaactg cctgcgtcag attctcgcat 1200
   gccagagatc ctatttttgg caatcaaatc attccggata ctgcgatttt aagtgttgtt 1260
   ccattccatc acggttttgg aatgtttact acactcggat atttgatatg tggatttcga 1320
   gtcgtcttaa tgtatagatt tgaagaagag ctgtttttac gatcccttca ggattacaaa 1380
   attcaaagtg cgttgctagt accaacccta ttttcattct tcgccaaaag cactctgatt 1440
   gacaaatacg atttatctaa tttacacgaa attgcttctg gggcgcaccc tctttcgaaa 1500
   gaagtcgggg aagcggttgc aaaacgcttc catcttccag ggatacgaca aggatatggg 1560
   ctcactgaga ctacatcagc tattctgatt acacccgagg gggatgataa accgggcgcg 1620
   gtcggtaaag ttgttccatt ttttgaagcg aaggttgtgg atctggatac cgggaaaacg 1680
   ctgggcgtta atcagagagg cgaattatgt gtcagaggac ctatgattat gtccggttat 1740
   gtaaacaatc cggaagcgac caacgccttg attgacaagg atggatggct acattctgga 1800
   gacatagctt actgggacga agacgaacac ttcttcatag ttgaccgctt gaagtcttta 1860
   attaaataca aaggatatca ggtggccccc gctgaattgg aatcgatatt gttacaacac 1920
   cccaacatct tcgacgcggg cgtggcaggt cttcccgacg atgacgccgg tgaacttccc 1980
   gccgccgttg ttgttttgga gcacggaaag acgatgacgg aaaaagagat cgtggattac 2040
   gtcgccagtc aagtaacaac cgcgaaaaag ttgcgcggag gagttgtgtt tgtggacgaa 2100
   gtaccgaaag gtcttaccgg aaaactcgac gcaagaaaaa tcagagagat cctcataaag 2160
   gccaagaagg gcggaaagtc caaattgtaa ctcgag                            2196
<210> 2
   <211> 52
   <212> DNA
   <213> Artificial Sequence
<220>
   <223> synthetic primer
<400> 2
   tatctgaatt ctaagtaact gataatacga ctcactatag ggagaccaca ac 52
<210> 3
   <211> 40
   <212> DNA
   <213> Artificial Sequence
<220>
   <223> synthetic primer
<400> 3
   aatgaaagct tttacaattt ggactttccg cccttcttgg 40
<210> 4
   <211> 169133
   <212> DNA
   <213> Unknown
<220>
   <223> bacteriophage JG04 sequence
<400> 4
   gtggtaatgc tagttatgac gcgcctggtg gtgccggtta cacttcccaa ttcggcggtg 60
   gtaatggtgg cgatgctggg ggtcggggcg gagatggatg gggtaatcac ttatctagat 120
   ctggcggtgg tgctcctggc agagcagtat tcggaagttc accgtcatgg ggtgctacag 180
   gcacaatcta cggctcctgg atttaaacga taatacctct caattataaa tattgataaa 240
   tgggaggtaa tatggcagca ccaaaagtat cattttcgcc tagtgatatt ttatttgggt 300
   tgctagaccg cattttcaaa gataacgcct ctggaaatat tcttatttca agagttgctg 360
   ttgtagttct tttgttccta atggcactaa tatggtacaa agggaatatt tttatggatt 420
   attacgtgag gtcgaaatat gatacttaca cagaagtaat tcaaaaagaa agaaatacac 480
   gatttgaatc tgcggcttta gaacaactac aaatagtcca cgtcacatca agggcggatt 540
   ttagttcggt gtattctttc agacctaaaa atctaaatta tttcgtcgac cttattgcgt 600
   atgaaggtaa gttacctagc acagtaactg aaaaatctat gggaggattt cctgtcgata 660
   aaacgacagc agaatattcg gtccatttaa gtggacttca ctttacttct aaaacagatt 720
   ttgctttctt acctaccaaa tcaaaaactc ctgaactggc gtatatgtat agttgtcctt 780
   acttcaattt ggataacata tatgcgggaa ctgtttctat gtattggtat aaggggtcgg 840
   atgtattaaa tgaagaacgc ttggctgcaa tatgcaacca agcagcaagg atattagggc 900
   gggctaaata attagttgtt agaatacatc gtcaaatggc gataaatatt ttcaaaacct 960
   tcattgaatt cactaatcag tgttttcttt tcttcggcag ttaaatttgt aattagtttt 1020
   ctgaacgaat tctgatttag ctgccgacca tcttttttaa ttccaatctc attcaagaat 1080
   ccgatgaaat ttgctcggtc ttcaacaata tcttccctag agaatttaat caaaatagaa 1140
   gcaaccgtaa taatttcacg tacaatttca atatctttat tcataacgtt ttattcattc 1200
   cattttgttg atatgagtat agtaccatta tcagtgttga ttgtaaacag ctttttctac 1260
   acttttaacg taatcagtgt agtcttcctg ttcatacgag tgtaaggcca agaacgtggc 1320
   taatagttca ttatagacta aagcctcacg ctcacgttct gcctctacta cgttctgcag 1380
   attatggctc atatcattcc ttataactat caatgtagtc ctgtatgtca aagaatgaca 1440
   tttcatattc tagggcatta agagcccgtt cttcagtata gtcagaacca aagacgatag 1500
   tgatttcacc aggcttcatg tgattgaaat caacgctaaa aggaatacga gaacatactt 1560
   cgtctgccat atctacgata acactacgat acacttctcg gttaacctga taaagctcta 1620
   cgagttcttc gcaacgaata acaagttcag tggcaggatt ttctttgatt ttagtaagca 1680
   tagtctttac ccttatgttt ttgtttgcgt ttagattgaa gttctgattt cttcttgtcc 1740
   ttatggacag aagccttatt gaaatcgtgt tttgctacaa gattgttcat aatgatttat 1800
   aacctcattt aaacatttat cggtaaaaga aaacttttta gccacagaac attcgatgat 1860
   gtgttctttc aaatctttag agtcagtata acccatctca gtaataaagt ggcgaattaa 1920
   agctttattc ttatcattaa cagaattaaa ataatctgct ttagaatcaa ttattctcat 1980
   catctaattt ccttaattga tattgcaacg agctaataat ttcttcgttc attttaatat 2040
   acatgtcacg caagaaaggt tttaacccat cacgaataat tgctaacgta tcacctttac 2100
   ataaattaac caaataagga tgatctaaag cgtcccaacc aggctgatta gcatacatac 2160
   catagttttc ttccatcata tgattaagac gtttgatatc ttggatacgt gattcaatag 2220
   catctttctg gagttgttta atattcatta ataaaggtcc tctgaataaa gttctttctc 2280
   acttccgcca cgttcaatac gtacctgtcc agcatacgtt gcaataatca ttgcttcttc 2340
   acgtgtccag taattagagt attggtcgat aaagccttgg tcatcaccac aaacttgttg 2400
   cgagactaat tgaggaccaa ctatatcaag tacttcagcc atatctttag agtaatgacg 2460
   aactccagga ataaccagag ttccaccgct cttcaattta aacctattag ctgcacatac 2520
   tataacacgt tgaacttttt ggtcattatt ccaatagcac gtttgccaac agatttcagg 2580
   aacttcattc attatatcat cagcagaata atcataaccg taggactgaa atttttctgc 2640
   tagactttca ggagtctcac gagacaaggc caaatctaat agctctaaac gttctttaaa 2700
   tgatttcatt taaaccattc cttaatacgt tgccaaatag atttttgtgc ttgtttattt 2760
   acaccaatcg aacgaataac cggttgtgac tgctggtatt ctttataatc ttctttatag 2820
   atttcgtaag ctgcatcaac aaatgaactg atcgcagcca tgtagttttt acgaaggcca 2880
   aatggcgcgt cattattttc acgaataacc gtatattggc ctgctttaat ctttacaatt 2940
   gtccctaaat aagccccgtg gtaccaaata tcccatcctt cctgagtagg ttctgcacat 3000
   ttacgaagtt cgttaataat attcagctta ttcattatgt attcctcaca gtacgttagt 3060
   tacaagggta atgatttctt tggacacatt agggaagtca atgtaagtat atttaccacc 3120
   gatcttaatc ttaacatcat tttcaagaga agtaaacagc tttgattcag tttctgacat 3180
   gttgtatgca aagattctaa gagcgccatc tcgacgaatt tcgatctgac gaatgcctag 3240
   aactcgttta gcgaatcgaa cttctaagtt actacggttt tcaccaattt ctttaacttc 3300
   aactttatct ttaatatttt caaaaacaaa gtttgctaat tcatgcattt caggagtaac 3360
   tccacgtgct tttctggtgt tacgtttctg aagaagttct ggagcatttt cttgggcaaa 3420
   gaggtctgca gctttttgga taatatccat tgcttcgccg gttgccacaa gtccatcacc 3480
   ggatttttcg actaaacctt tcttaatcaa tacaccaatg ttagagttaa caactgatgc 3540
   gttgtattcc gcttccagag cttcacggac attggcagca gtggtgaagt tgttcttaat 3600
   gatataaacc attattgcag cagttttttc attcagagcg ttttcagaag ctttgatgat 3660
   gtaagtaact ttagacattt tatttctcca aattagtatt tgttttgata ggtctataat 3720
   atcatgtttg aagcaaaagt aaactttatt ttgggctctt tttcaaaagc cctggtaaaa 3780
   tttacaaact catttcttta ataaggctaa taagttttgc ctttttagaa cgaagctttt 3840
   ctaaatgata tccgtttgaa ccgcgaacaa ctaaagaatt gatttcaact tctaagaaat 3900
   ttaattcttc aacaagtgct ttacgtggag cattcatacc aggtttaact ttaccgaaac 3960
   gagtagttgt gttttgggtt aatttcattt tagacatttt atttctccaa tttggttgtt 4020
   ttgttttgat aggtctataa tatcatgttt gaagcacatg taaactgttt tgtgagaaaa 4080
   aaatgcacaa aagggagccg aagctcccta atcatgtttg tccaaccaat acagccaaat 4140
   cactagtgca gctattgcac cgagaaatcc taaggctagg gcactcaaag tgcctccaag 4200
   tctttagtgt actctgtcac aacatcagta gacttccaat actcattttc ttctttctta 4260
   gctttagctt cttctgctag tttacgtgct tcgtcagaag tgatatggaa gatgttaaga 4320
   cctaccaatt tatcgatatg ttctttatac atttcgattt gtgctagctc ttcagtcaaa 4380
   gctttacgag tcttgccttg aatcacaatt tcgccactga taactttttt gatgaagtgt 4440
   gccttagcaa aagccagttt aaatgctttt tcagattcaa tgattttgtt atcaatacgt 4500
   ttctgaacgt aagtcttacg gacttcaaca aaatctttga ttaagtccac tacgttatcg 4560
   tagaccgtta atttaccctt ttcattaatc actgtgatgt tttgtgaacg acgttcgatg 4620
   agactaaaat ctttcatgat tttttcatga cgtagctctt cgtcatctgg aagcgagtac 4680
   tctttacgga acttcacctt aaacccaaag ccgtgttcac cacaatcatc ttcccatgag 4740
   attagacctt tgtcttccaa cgggtcaaga accttactca catacgtttc acggtcgaat 4800
   ttatatggga tttcagttat ctgcatttga gtgcgagatg tgaacttata tgttccgtga 4860
   atctcataac gcccatcaac ttcatgcact tcaccgcgga attccgggaa ttcaactttt 4920
   ggtttagtga catttttacc ttgaagagct tgtaatacag cctttttgac cgatttaaaa 4980
   ctatgaggca gaatatttgt tgcgtaaccg gttgcaatac cagagacacc gttcaaaaga 5040
   actgtcggaa taataggaag atagaatttt ggtggaacgt gttctttatc ttcatgagct 5100
   ggagcatatt cagtatcttt atatacgtta tagaaatttt tgcttacacg agcaaaaata 5160
   taacgagatg ctgcagcttt ttggactgtt cgagaaccaa agttgccttg accatctaat 5220
   agtggatagt tgttattcca tgtgttagcc atcaatgcac ctgcatcttg tgcagaagat 5280
   tcaccatggt gataacctaa atctgcaaca ccgcctgcaa ttgacgcaag tttatgaaat 5340
   ttttctttat taccacgagc caaatctaat gcacgatgaa ccacaaaacg ttgaaccggc 5400
   ttaaatccat caatcatatt tgggattgca cggttttcaa cggtgtacat cgcatatgct 5460
   aaggcttcgt tatcaataat actttttaaa ttacgagaat tcaattgcat attttcacca 5520
   tattaatgaa cgaataacca ttataacatc tgaaatgaaa agcactactt ggattagtcc 5580
   aaacatcaca ccgtagtaca gaatcgtaaa caacaacaag agcccgaagg ctcctgctaa 5640
   gattttctta atcattactc gccttttaac acgttaagaa gagctaagac cgctaaggta 5700
   ccttgtgata cggcctgttg aaataccgtt gtttcgcctg taactaaagt gttaacgaat 5760
   aaaaacgacc aacacaacaa tgcaataatc catgatacca ttttaattac ctcacaaata 5820
   aattaaaggc ctaacttaat aggcctgtat aaattagtaa ctctgcagaa taaatttgaa 5880
   attatcggcc agaagacggt tcatttcagt gagtgtttga tattctgatg tatggttgcg 5940
   agtgaatgcc agagccaatt gacctttacc ataaccagta gtcaggggtt tcatgttctt 6000
   ggctgaaaca aagattacgt catattcaat attattagtg cccatggtac gggctagttg 6060
   cgaacggcct tgacgaatgt gagaaagaat tgcatccaac ccttgtttag aacgttgacg 6120
   tcctacaaag aatcgtgctg ctacttgtcg ccagtcagat gaacctttaa caaagaagta 6180
   aaaaccttca cgagaacgaa actctttaga aacttcagaa ccgtattcgc cattacgaac 6240
   tactgcaaca acttcaccgc caactgccag aaggtcttta cgagttaaat atttggacat 6300
   gctaatttcc tcaattgatt aatgtttttg taatccatga gagcattata ctctgctctc 6360
   aagagtttgt acaattttat tcggctatat cagaacgttc aaaaagctct tcaacagtta 6420
   aacattcagt gtcatcttca ataaagaatc gagtccaaca cgttggggcg atacagttat 6480
   gagtttgtcc ggcgatagaa gctacacgaa taagctgctg accattattc agaacaaatt 6540
   tttcaccaat ttcaacatct ttaaaaagct tagccatttt gtgttcctca tgtttcagta 6600
   ggactactat accataatcc taccgtgatg taaacaatta aattacatta aaatatgtta 6660
   aagccagcca tgttccagtg aacgaacatg caatgtaaaa aacagtatca caaattgttt 6720
   ttagtacaat aagaagtgca tttttcataa gtacctcaac acaattgaaa atattataca 6780
   aatcattggg atactaagca accatattcc tatccacatt ttagagctca tatctcaact 6840
   tccgcgagtt ccatatatcc tttgttgata acctctttaa ggtctttaga aagaataaca 6900
   ttaaaatggt cgttcatatg cccgttaacc agacgagtta tttcggcttc agatgacata 6960
   taaggcacgt ccgagtccca atgagataga cctaattgag tataaatcat cccttcgtca 7020
   tctgcagtgt tgtacattga aacaaactca ccagtcactt tatgttttgc gtagaaaaat 7080
   ttaaatttca tgatgttatc ctcttttaga taggactact atatcatagt cctacttagt 7140
   tgtaaacact tttatgaaat taaagatgaa gataccaccc gttgtagttg ctcttgcgga 7200
   cgatttcttt taggtcttca tggtctccaa cgaactttat agaataagag aagacgtcag 7260
   gtgatgtttc tttgacattc aatataataa tacagtcagc gactcgatga ttaaggaagg 7320
   gccctatgtt gattccgttg ccacccggat aatcaccact gtatcctgtg gtaacagaaa 7380
   tccatttcga atccatctga tgggtcttta cttctacgcg tagaccacag tacttaggat 7440
   gagctaaaac gtcccatgca tacgtgtacg ggtcttctac gtcttcatta cctttattaa 7500
   catatccgtc tagccaattt gctactacat actctgcaaa gactgcaacc ttacaacgtc 7560
   gaacaacgtc ttcttttatt tgtcctggat cttgtttcaa cgagtaccga gcagtatcag 7620
   cgatcttaac cttcatttcg ctagtcaaaa actcattcga caggataaat gtcggaagac 7680
   tcttcagcct caacagtccc aagttcgtct ttaccataga tacctctaat gtgtagttcg 7740
   ccaaaataaa tgcggtcacc ttcttcaaga tattctgcat ctaccggtgg gatttcaata 7800
   acaacttcgt cacattctaa ccagccgtag tgtgcatcac ctgggtgttt taccaattta 7860
   gcacaataaa cggatttaca cttatgatga ccacgaagat tagcaatttt ggattctgat 7920
   acgtcaaatg gatgaatcat aattacccct aaagaaaaaa aaggggaccg aagtccccaa 7980
   ttattattct gcaaataatt cagtaacgtc tgaaattact tcataacggc aagtacgcat 8040
   cttagcatca ccgtagtcaa ccgggatgga tacaacatcg cgtggatgaa ctttaacttt 8100
   cacaacttta tctgagcttg aaccaaagtg gcgaatataa gatttagcac aaacatggag 8160
   accgcgagaa caggtttgtg tatcatcatc gttaacacga gtacgaggca ttgaaacaac 8220
   tttacctggt gagttatcaa atgtaccgct ataacaatct aaatagtctt tacgaactac 8280
   tttccacgca tagaaatacc catcttcagt gatttcaata tcgtttgcaa ccaagaagtc 8340
   gaataaacgt tgtacagctt tttcgcttgg gttttctaac aggttttcaa ggaacggcag 8400
   atagaattta aagtcttcgc ctttttccat ggaatcaata atacgattaa ccagtcctga 8460
   acgaatttct ataccttgat aaaataaact accaccttca atagtaacat taccatcaac 8520
   aaacttagta atagctttct tgatgttaat aagttcgaca gctttactaa ggtttcctgc 8580
   gaccagatta gctttaattt cttcaaaatt tgcatgactt gacggggtcg cattgtatgc 8640
   tacgcgtcct tcaataattg aaataaattt agatgacgcg ttccatacga tttcaggacg 8700
   atgtccgccg ataataggtt catcattttc tggaacttct tcgattacat aagaggcatc 8760
   gtttgcttca gggacaacgc cttctgcttt aaaattctta acaatcttac gaagagtatc 8820
   tgttgaaatg tcgaagtaat cggcaatttc tttacgagtc catttgcctg attcaaccag 8880
   gccgtaagct tctaactgtt cttcttggga caagcatttg atattgtaca taatgtttcc 8940
   ttatttggcc gcttccacgg ccttcatgaa tttaacgatt tgagcgacct gttctttaga 9000
   aagagtacca cgtcgtccca tgtattctga cacaatatga taattggttt cgaattcaat 9060
   aaccatttta tcatcattat cgtaagcatt atctttaagc ttatcgaaaa ttttagcaca 9120
   caattctaac ttcttattca ggtctgtatt agtagcacct gcgaatcgat tccatcccca 9180
   acgattattt actgttgaga gttttgcaaa atctttactg atttctttgt ttttagagct 9240
   aaaatactta ctaaggaagt taagctcttc attacgagta atatgattca aataaggctg 9300
   tgctctacga cttggtgata catactcatc ataatccact ttatcgatta gcgtaatata 9360
   gtcattaatt gatttttcga acaagcattc tacttcacca agtttacgaa ctttcttagc 9420
   aatctgtgga cgaactacag tgaactcttt aatacctgac aaatcagcaa tacgggtcaa 9480
   gaaattggag tcatagttca accaaccttt atctgcgtca agacattcag cgttagaacg 9540
   attacgaact atagcataac cttggatttc atctgcttca cttgccggaa taaagaggtc 9600
   ttctgatacc caaacatcgt tttcattctt aaagaatcga tgagcacttg gcgatttagg 9660
   acgaggctca ttagtccgtt tcctgacttc aatccatggc ttaacgattt gtaaaagctc 9720
   tgaagtcttg tacatgttaa tgttgtcgcc atcaaagata actttaaggt gttccagaag 9780
   tttcattgca tcttcgtctg tagggtcaac gaatagtacg cttttaccaa aaccacgtgg 9840
   aactttaccg gataatttaa cctcgtcttt agacatttca attgaattaa gagctcgcat 9900
   taatggaacg cgtcccttaa cgtcgtcaat aacaatttca acgtttttaa tgttaatgcc 9960
   gatcaggctg ttaattgaaa tggttgatga tgagctacca ctttcacaaa tacgacgaag 10020
   acgaggggat tcaacgatat cgtatacgac accaagatta caccactcag gtcccatttt 10080
   aaaacgtttg tacagctcag aataagttag ttgttcttta gagtatttca gtgattcaat 10140
   attagacccg gccttttcca tgtatttacg agcggtatag ccaagagaat caatttcgcg 10200
   gaacacatga cgtgggtatt tacaatcaat ccatttctta gaatcttcag cgaacaattt 10260
   agcatcgatt tctgcaacgc gtttatggat atttgctact gtacgtttat caaatgacaa 10320
   tgcttcacga gacggagcta cgtctagttc acccataggg aatttaatat aagctgtagt 10380
   gcatcgtgtc atcatccatg tttgttcttt aataacactt ccgattggat atacaatacc 10440
   gccataaaca gccatgatat taccacgttc accccatgga gcttccttgg ccaaatatac 10500
   atcatcaaat tctgggaagt atttaatatc tttaacacca cgaacttctg caatatcacc 10560
   tagaggacgc ataacatatg caatttcgga tgcaaacttt tcaaaatctt ccgggttaac 10620
   cggtacaact acttctacac ccgtacggtc atcaggaccc atcttatcta caaaggtcgg 10680
   cttaatctgt ggacctgtgt catcttggta gattacgtaa ccacgaactt caccattatg 10740
   ataagacgta aggttaaacg tatcagtata tgctaatgga gctttagagc cgagtccgaa 10800
   tccaccaatg aaatcgttac tcgatgtttt agttgaagca aaatatgagt tataaattcc 10860
   tggctcttcg tcattaccac gaatagtgaa atcactcata ccaggtccaa aatcacgaac 10920
   tacaaaacgt gggtctaaac gacctggagc ctgaacaata aacttatcag tgcaaccgtt 10980
   aagaatttgt ccatcgatac agtttgtaat taattcacgt acacaagcta attctttgtt 11040
   tgtataaagg tcattagaca aaatcttata aactttgcta ttgccttgaa tagtaaatgc 11100
   tgtgctttta ccgcctgaac caataatcgt ttctttagcc gtttcaataa tcatattttt 11160
   ctctcatcac aagttacgtt taaaaatttc tgctacttca agaagttctt ctttagtagc 11220
   tgtgtcggtt tgtattttat atcgaatttc cttaaagcgt tctttaaagg actctgctga 11280
   ctcgatatca aaaaatcttt ggataagccg gaattcccgg agcactgctt tatcaaaaag 11340
   ttccaagtcc acgttatatg ttctcataat attctcttca gtagattcct agggcactat 11400
   tactaaagac tatataatct atcatcttat gtgtaatagt gcctcaggcg attcaggatt 11460
   tacgctgtga agattttatc ccacatcgca acaaccgcat ctcggttata cttcttccat 11520
   actgcttcag cacgtttaac tcgagcttcg tatgtgtcag tgaattctaa tacagctttt 11580
   gtgtaaagcg caactacttc tttcttagga gtagagggat caatttctac acgcacagta 11640
   tctgggtcat attcgaatgt cgcgtgcgga taatccttaa aaactaactg aatcacaggg 11700
   acacctttag agatggcttc aatagaacta ttctctaccg tacccgtgtc aggacacgta 11760
   ttgataagtg ctttagctgt tttaagacgt tccatgattt cactgtgtgg agcatcaata 11820
   ataacaggat acttagccgg tggtgcccat ttactaggac aataaccaat accattttcg 11880
   cctagtgctt taatcgcttc aagagcaaca tcagtacgac gcattttagt gtcataacgt 11940
   tgtgcactaa taatcagatc agtcgccgga agaacttcag gtttctcatt caaacaaata 12000
   accggataat cttcagcttc aaacaacgga tttgctcgcc aataatcata atcaattttg 12060
   tcagtcagac ctgaagtcat ataacaatat ttgcgttcaa attcacggaa ggctttagta 12120
   ggagcataac tcttaccacc gttcttatga atttgaatag tctggtccca aatttgaaca 12180
   atgccacgag atacaggttc aaatggtgtt gccataaaat tcttatgaac tttagaaaga 12240
   cctgcgtctt taattgcctg aacggttagt ttggtcggtt ccattgtaac aacgaaatcg 12300
   tactcattaa tgccggctag ttcaacaatc ttacgagcca tagtataact catacctaga 12360
   ctgaatttag tcagatgacc aacaggatga tgattaacct tccaatcacc aaatgtgtca 12420
   ttatcaaagc cgatataatc aatatcagca ccgatggaca caagatattt caacacattt 12480
   aaatgtactg cttcaagacc accttgtaca cgttctgggt taaatgggac tgcgcgagat 12540
   gggataaaca taactttcat aattaagcct tattaaaatt aaaatgaagc tcccgaagga 12600
   gcttaggaca tccattcttt acgaagttct gaatcatcac ccatcagcat ttcaaagagt 12660
   tctttccaat tttcaggaag tttaactacg tcgtacacag ggttctgaat catttcacga 12720
   tactctgact tttcaaggga gcctaatccc ttaatgtatc gaatactatg cttaggcagt 12780
   gtatctttaa cttcttcata ctcagctaca gtataaaacc atttttgagt tttaccaatt 12840
   tgtgcaataa ttacaggcgt tttaacaaag cgaatacgtc cttgttcaaa caattcaggc 12900
   caattactaa agaaagcaag caaagcagga taaatacttc caaggccgtc atgatcggca 12960
   tcggtcataa ttgcaatatt acgatagttc gtattctctg ctttttcacc aagaataaga 13020
   cccgtgatag cacagatatc aaagagctct ttgttcttca tcatatcagc atatgacata 13080
   ccccagctat tcatcacctt accacgtaat gggaatccac cgtgtagttc acggtcacga 13140
   acatcaatga gatatccaat tgccgaatca ccttctgtta agaacaatgt cgtatctgca 13200
   tctttaccac attggttagc tttaatatgc ttatgaacct tagctttagt tgccttttta 13260
   gctgctttag tttctgcagc cttttctgct gccaatttac gagctaatgc tgcttcaaca 13320
   ataggcataa tcaaggcttc gtttttaaga agtgcctgag caattttctt agcgtctaat 13380
   tggatatgat tacgaatatc accatacgtt gacgttaaac gttctttagt ctgagaatca 13440
   aaacgcatgt tgctcatgtc tctaataaac atcaacattg tgagacattc tttaacacga 13500
   gctttagtga cttcaatacc cttatatttc tttttaatac ccggcaaaag atgttcgcag 13560
   atatcgtcca ttacacattc aacatgatgt ccaccgtttt tagtatggat gttattcaca 13620
   taagttaaat gacggaagcc atctggcgat gttgtgaatg ctatagagac tttatcattt 13680
   tcttgaatga tgttatcttc gccaaattgt ttagcaaatc gtttaaaatt accatcgacc 13740
   tttttaccat taaatgtgaa cttgatatca ggataaacga ctgccaatgt ttgaagtcgg 13800
   tctaaagtaa tatctaaata aatttgagat aaattatgct cttcaaatga agtgaaatca 13860
   ggagtgaaga ttactttagt gcctttacct ttagatttct tagaagacca tgatttgttt 13920
   tccattccgt ttgaacagtt aacagtgatt tcattttcgc catctgacgt gatgcctgta 13980
   aacaacgtag agaagatatt agttaaacta ctcccgacgc cgttcatacc accagtcttg 14040
   cgttcagagt catcaccaaa gttaccaccg gcctttggaa tagtccaggc tgctacaggc 14100
   ccaggaattt gttcacctgt ttggtcggta actaaacctt gaggaatacc acgtccgtta 14160
   tcttccaccg aaacttgatt atttttaatt tgcacatcaa ttttatttgc aaacttaaat 14220
   gatgtacgaa tagcttcatc aaccgagttg tcaataatct catcgattaa tttaaccaga 14280
   cctggaacat attcaacttg ttgatattta ccaaacaaaa aacgctcgtg ggcttcttta 14340
   gcagaagaac cgatatacat accactacgt ttcttaatat gttctacatc tgacaatacc 14400
   ttaatttcat ttttaatcat gttgtctcct cgttatgaag gtattctatc atccgaaatc 14460
   taaagcaaaa agggccgaag cccttattta aatttaattt tctgaagtgc atcaattgca 14520
   gccaaacgcc cttgttcact tttaactgta atgaccacat cgcctgatac gatcaccgaa 14580
   ccatcaacaa attctataga gccttttttg aaatcgacag ctgtatctgg ttcctgaact 14640
   tcttcaaaga atttaaattc tgcaccataa agcagaatat caccttgcat actatgaata 14700
   gttcggccat aatcgcctac ggcttctaca gcaatcattg aaacgccgta accaatcatc 14760
   tcttccacaa taaaatcttt ggacaataaa gtagtatgca gattagtatt aagacgttta 14820
   ccagtcgtat taagaattct caagaaatta tcttcttgac ctttgataat acgatatttc 14880
   ttaccaactt caaaaccttt aaaagccata attaaccctt cttaagtaag tcgtaaaaac 14940
   caccgttcac atgtttagga gcagaaaccc gacgagtaga cagacgatga cattcagggc 15000
   aaacatcgtt ttctcgttca gaaattcttt taattttttc atattcatga ccgcaatatt 15060
   cagattggca tttatagtcg tataacggca ttattattcc ttaaaatgtg ctttcaacat 15120
   ttgatacaaa gaccatgcct ggtcgttatt ttcgatagta attgtcatca ccggaaattc 15180
   agacaattcg tcaagttcac cagaatcttg ctcttcggac gcttgatacg gatttccaac 15240
   ttcgtcaaag aactctgctt cattcgtaga gagccagata aagttttcat caagaatatc 15300
   accgcctgtt gcgtatccaa ccatgccagt agatgtcaca attttagtag gacgaccaag 15360
   atggtcaaca tctaatactt taaatggttc catgccaaga cgtcgtgcat aaatcccatt 15420
   atcggtgtgg tccttaataa actcttcttg ggcacgttta tctttaaatt ggtaccatga 15480
   gttaacgcgg aatttattag ccattagtaa gtttccttcc aataagttgt accgttttca 15540
   gtgatttcac ggaagatacc atagatgggc atttcatcgc caagtttttc gtaaacgtaa 15600
   accacttcgg tgtagcataa aaattcggtc cagtttttct gtacaacaac taaatcagga 15660
   ttaaacagaa catcttcaaa ctcatcgatg tttgaatcag tagttacgtt tttaatgatt 15720
   tgacgtttca taatattctc ctcttgtttt gataggtcta tagtatcata gaccatagtt 15780
   gttgtacatc attttagcaa aataaaatca acttgttctg ccaatgaacg tctaaacatt 15840
   aattgacaac ttccgagtat gaaatcaggt gcttcataga ccgcgatgta gtatccttta 15900
   gctttcaaga gttcaacatc cacttggtca tcaaaccatg cataaaccgc gtcaacactt 15960
   tcaaatccaa aatagaaccc ttgagcccaa tcttcactca attcaaatgt gtactcaatt 16020
   ctatggtatc ttaagatatt ttgttcaagg agtttgtctt catttactgt aggacgatta 16080
   gggacattat agttgtcgcc gtgatggtag aagttacctg agagtattcc atgcttatca 16140
   aggaactccc atgtttcttc agagttgtca gtgccagacc aaccgtaagg tgagcgaggt 16200
   tctttattcc aagaggtttc ccaaattctg acggctttac cgtcttcgat aacaattcct 16260
   ggtaatgatt cacgttgagt acaaaaacta cgttctacgc ggtaaactaa catattattc 16320
   tcctctggtt gatatggtta taatatcaaa accagaggag aagtaaacaa ctttatgcga 16380
   aagcagctgg gctatatttt ttaatttgga attcaggaac taccatgaac tgaccagcga 16440
   aaacgatgtt ataaagctta acaccatctt ctacccaacg gtcgctaata aatccggcga 16500
   tttgcttagt tttccatggc gttccatcag ttgccgcagc aatagtgaat tcatggtctt 16560
   tttcaacaga aactgatctg catagtcccc atttgaacgc tttttcttct ggctcaatga 16620
   actggcttga gacgtagcca atattaccat tagcatcacg aactttaact tggttacgac 16680
   caaaaaccgg atcactcaca gaaagaactt caaccaactt cccggcaact ttagagcgag 16740
   aagcaacaga tacatatacg taatcacctg tagagagatt tacaatattc ataataattc 16800
   cttaagcttt acgaggagac acatagcgag tgatttcaga ttctttaaca actcggaagt 16860
   cacctaagaa tacgatattg tataactttt caccatcttc aacccattgg tcactaatga 16920
   agccgcacat aatatcagtt ttgtttggat aaccatattc gtcgatgaaa tcaaattctt 16980
   tatcagtccg gaattcaacg cctttgcaaa gggcccattt gaactgtaca ttatgctctg 17040
   tggattcaac tttttctctt gaagcaaaag cactactcac aggcgtttcc aagaactttg 17100
   cacgaacata agcaaatttg gtttcctgtg tcccaaaact aggaataata cgaactttta 17160
   cctcagaatc ttcatcttta acgccttctt taagttgaat acttacaact tcaaccagct 17220
   ttcctgcagc tttagaacga gatgcagacg atacacgctt aatttcacca atagtaatca 17280
   taatttctct ctctttgttt aagttgaatt cattatactc catccgtcct tggatgtaca 17340
   acattaatta aggatattaa ccacagcact acgtggaact acactaaaat cacctgcatg 17400
   aacaacgttc agaagttcaa caccgtcttc aatccattgg tcagttaccc atccatttac 17460
   tggacctgcg agcgggcgtc gaattgaaac atatttacac agcaaatcag taggctcaat 17520
   aggcttaatc agagtaccgt acatacaagc gatatccata tcaatacaga tgtctttaac 17580
   gggttctact tcttcaatca acagttcaaa gtctgtgctt ggaagggcgg tagtagaaat 17640
   tgtaccatat gaatttttaa tttctaagaa taaccaaggg ccttcagctt gtttgatgaa 17700
   gttaacaata gtgaaactat tgccttgagt ggcacctaaa atttcacaaa acttttcgta 17760
   agctcgcact gaagcactgt accagttgat ttgtttttcg tctttcagga tgtaagtagc 17820
   cgtagtattg atattcataa tgtttttcct gtttggttaa gatagaaaga ttatactcca 17880
   accatccttg gttgtaaact gattttatcg ctgttttaca cttatcgggt caggattctc 17940
   agggacatct tcaaggccaa gcgcataacg ctgagcatat tttaacatca aaatatcttt 18000
   agcacaatca tgaatactat catgggcaac gaatcctttt agcgtaccca tcggtaatgg 18060
   acacatactt aatcctcgtg tcaacgagta agcttcgatt gccgtacgaa tatcgcgttg 18120
   agcccagaat ttaaccggtt ctaatttatc agtatcaatc tctggttcaa gaacaccttc 18180
   atcacgatag agatcacgaa tcaaatcgac caagatcggg aagtcaaagc tcataccacg 18240
   gcaccacatt tgcgatttcc attggtcaac tttattagct cgacaataat ctaaaaagat 18300
   tttaatgcct tcaagcgtag tgacatcgtc ttcagacgga gccaggtttt tacgtgcttc 18360
   tgctgattgt tctttccacc atttcatagt ggatttagcg aaaactcgct tccctttttg 18420
   agaagctaaa ttaaatttaa ttcgcttacc tcgttgaaca agttcttcaa atgattcaac 18480
   aacttcaggg tcggaattat atgcaataac cgccaggtca ataacagccg cttttgatgt 18540
   attgccgaat gtttcaaaat ctataataac atcagtgctc ataattttct ctcaaataaa 18600
   atggacgtca aaacgaccac gagttgtgcc gacatacaat agctgtttag ctaattctga 18660
   atcggcataa tggatacacg gtgtataaac aaaggccgtg tcaacagaca taccctgagc 18720
   cttatggaag gttgatactg gtagtgcctt caccttagtg aacatacgtt tagcatccca 18780
   gaattcactc catggagctt taccaccttt attccagaat ttgtatgttt ctgctgtctt 18840
   agccaaaaac aaattgaatt tataaagctc ttcttcagaa gaaataacct ttatatcctc 18900
   gcggacgtat tcttcatcat ccccatatgt ttctactgta agattccaat atcgtatcat 18960
   atactcgcct gaaacaccac gtgctttaac gaatgtagac gtttctacag catctcgaat 19020
   acgaacgaat tgcccgttat tgaaaagcat ttcggtcata tttttgccat ctaacttata 19080
   cgtcttggta aacggctctt gtagaacaat gacttcatct ttaataaatg gcttttctgt 19140
   ttcgagaagt tttcggcgaa taattgaatt taacttgtct actgacttat ttgtgaatgc 19200
   catcattcga ttttcgaaaa ggtcttcagg agttttaacg acattaaaat agttcatcat 19260
   atatgattta aggtctgtat atccatgaac accgtgccca tcaactactt tatcataaat 19320
   ccatttgcca ttacgaatat cagtcgcaac atcaataatt ggcgcattgc tacgtttaac 19380
   ttctgtaagg ttgcattgca cgaagtcttt gtgagtgaag aaaggactta gatacgcagt 19440
   ggattcgcct gggtctacag gtcgaatctg acatcggtca ccaattccaa ttactgtgca 19500
   ccatttaggg atactagcta acattatttt aaacagagaa cggtcacaca ttgatgcttc 19560
   atcaaataac agtacacgac acaaggctaa gtcgggaacc tttttctgtt caaataacac 19620
   gttttcttcg tatgtagttg ggttgatttt aagaatgcta tgaatagtcg atgcttcttg 19680
   acctgcaagt ttgcttagaa cctttttagc agcgtgtgta ggggctgcta aaataatacc 19740
   tgattctcca gaagaaataa gggcttcaac gatgaatcta gtaagggttg ttttacctgt 19800
   accggcaggc ccattaatag ttacgtgatg tttcttttct ttaatagctt tcataacaac 19860
   gttgaaggcg tttttctggc cttcggtcaa atcatcaaat gtcatcgtaa agtccctgca 19920
   attggaatac taacaatacg gccagtatct agaattcgtt gatacaatct ttgcgtctcc 19980
   gcatctagtt taatgtgttt catttcaagc ttattaaacc agaacttacg cggagtttcg 20040
   accattctta gaactcccat ttttaaatct gttttatact gtgttttcaa agtatcgaaa 20100
   actttatctg caatatcctt tctaaaaaat actgcaggac gatgttcatc aagtggaact 20160
   gggacagtaa atccttccat atctcgataa actattgcat atacataaac cataattaac 20220
   ctttaatcag ttggaataat gatgcgctaa gtggtaggcc acgtttcaat tttaatttac 20280
   caatgaaacc aatcttttca aaacgttgca gaccttggat aactttatca tatgtatcga 20340
   aaagcgattc agcgtcagtt aatttagttt tagttttcca gtcagagcct ttaagaagaa 20400
   cgttgagttt agtaagttca tgtccttccg cttgatgtga gccgttaatg cgtgctttca 20460
   aagcaacaat tctaagttta gattcgttag ataaaggttg atttttcatc atgttcgcca 20520
   tatgcttaac acgattttca cgttttgctc ggatttttaa aatagtacca taaacctttt 20580
   tgatttgggc ccatttcttc tcgtcgagat taagtttctc tactttacgg gcagaaggat 20640
   tagcgatacg tcttgcaatg gcatcacgtt tctcaataag ttcttctaat gttaaatcag 20700
   aacgagccgt gttatatttt ttcttaactg ggataacatt acctttaatg tagccgatat 20760
   tgttatcaaa acgttctaag gataatttct caccttcaac agagttattg aaagattcac 20820
   ctgagtaagc gcagaccttt tgatcaagaa tgtttttaag gtaattaaag tctaagttaa 20880
   aatccttaga gcgtctcttc gcagaagctc tggtgtggtc aagacgacga tttattttgt 20940
   taattttatt atttgataac ataatatttt cctctgtctt acggacgtta ggtcatttag 21000
   ctgataggtc tataatatca tgccctcagg tagtgtacac tctttttttc ataaaatgct 21060
   ttaatttttt atgtgttata atggttctga gttccatccg gttattccaa tccagtcagt 21120
   aaccgacaat agacagattc cagattgtat aacgtccatc ttgaataata gaatctacac 21180
   gcagtatagt ccctataggg agcataaatt cgcattcgtc agatataagt ctagcattag 21240
   accgtcttgt tgcttcaggg aaagctccat tgaactcaca agatggcgca gccagaatca 21300
   tgttaagcat atgttcacgg aagttgaaag caaatggtgc atctttgatg cagaacatat 21360
   taaatgttcc ataaaaatta tatgaagcga agttacgtgc tacactgaac actgggatga 21420
   agctcattac tctatcaaat ttaataatac taccaatgcc tttatcatcg agtgtggtca 21480
   tagttttctt agagattcca cgatacattg tctcgggaac tgaagacgtc caatttctac 21540
   gaacaattgc acttaaatct tgttgaatgc tatcaggctt gttttccata cattgccata 21600
   gtacactttg ttcaatgtcg ctgaattgct catcgatttt gtactgaagc atatcttgat 21660
   aatctgggca ttgagcacga cgaaccattt cttcaatttc acactcgtta tacggcataa 21720
   acatttaggt ttctcctgtg gttgataaag gtatagtatc atcatcggaa gagtttgtac 21780
   actaaaaatt caacattttc aaatgaatga ttttgtataa ccgagataaa gggtcatatt 21840
   ctaagtcttc aatttctaca atctcaaatc tagagatacc tgagaccatg tattcttgtt 21900
   cagattcaat catatctagg acgtcaacac gtttttcttc cataacatgc ttagggttct 21960
   ttccggcgag tacaatgtta accatgtttt ggtagtaatc gaaaatgaac ggagcattcc 22020
   tgagactaag aatagtgtac gtttgatatt cccaactccc agagaactgt ctggccgtac 22080
   caaagtctgt tgtaaacgat gttactcgtc cgggagacca atgacatcca acagaaagaa 22140
   gggaaagacg ttctacttct tctggagtta caccacggta gagttctact ggtactgtag 22200
   aagtcatatg cttacgaaca agatggtcta attcatagtg gaaatctggg tcttccttat 22260
   aatcagtgca agcccagaga atgttttgtt cttgtttagt gaatttatca tcaattagtg 22320
   ctgcgagata ttcgcgttct tgaagttcag attgacatgt catgatagac ctccatttaa 22380
   agataagcct atcataacac gagaggaggg aatgtaaact actttttaag ctttcctact 22440
   aaaggcccat cagggccagg ttgttctaac atgaataacg atgctttaaa aagcaatgtt 22500
   aaccctttca ccattcgata ccatgtctca aagtcttttt ccttgatttc tagtaagaat 22560
   ttctctttaa atttctcttt catttccaag gactccatgg tggagggtcg tcataataag 22620
   aggcaaggtc tttcagggct tcaacaggta aactaaagcc gtgtcgttgt ataatactat 22680
   ctagttcatc aacaagttca ctatgatgca ctttaggagc cttgacttca ggctcatcaa 22740
   acatactcat aaggtccata ttattcacct aaataaaaac tagcaggttc agagaatgtt 22800
   tctagcgcta caccttttgt cttaaaaaat ttccaaatag ctcgttgcaa atggattaaa 22860
   tctgctaatg atgtaataaa gtattttttg gcaaaatacg tatggtctcg cttttcaaac 22920
   aggtctaaac gcttttcagg attcacataa accgcatttt ctgtggattc ataccctaag 22980
   tcctttatgc cttcttcaag actaaacgga gaatttttag aatgagtaat agctccaact 23040
   cgctctacac gaattttgta acctttagga atctgcaatt tgcagaattc ttctacgata 23100
   tattgcggat tatttagact tttgttaatg agaatagcta tttctagtgg agtcttatct 23160
   tctactggta tatgtttaaa tgacttggct tcaagcaaat acattttaaa taacatagca 23220
   tccattataa tttcctcgat ggttcaccca tataaagttt tacaatacgt tcgccttcat 23280
   cttcgacgag cttttcttca tgtttctgga attctttagc catattctct tcaagaacat 23340
   ctaaccaatc ttctggaccg acaccatcgt cccagctttt aaaccattta actatcttgt 23400
   tcattcgcag ctccatgagc ttgaaccaca atcagaataa cttgacccag aatcaccagg 23460
   ccaagacgat acgtatgaat gattataact tgaagagctt gaagcattaa cctgttttgt 23520
   ttttggacga ataacttctt ccatcattcc gtcaccaata tattcccaac ttttttcatg 23580
   tgtatcagga ataggcatca taccttcgcc tggtttaaca gcaaacaact tcttcagtat 23640
   cgtaagcatt aattgttcct cgaacaaaac gaacaccacc atcatcccaa cggatttcgg 23700
   cattaaattt gacgtcttca ccatccatca tgaatgagtg tacgactaca tcatcgtaat 23760
   atccaagacc gtatttcaac gaaacttctt gagcaatcaa agcacctaat gtgttattag 23820
   gtttaatcca acgatttaac atagtgttct cctcttgagt aagtaagaat atcttaacat 23880
   agtttttagt ggatgtaaac tctagaaacg aaaaaaggaa tcccgaagga ttccttaaat 23940
   tatttaacca cttactggtg taggggtaaa cattgcagcg ttcttagttt tccagtcagc 24000
   tgcatcagaa actacagtag aataagctgc cttttctgat ggaaagattt ggtaatgaga 24060
   atctgcaatc cgatgttcgt ttgaataaat ctcaaacggc acagaaactt ccataccttt 24120
   aacttcttta ccttcaccag caggatgcgt aaaggttttg atgtttacat aaccacccat 24180
   aattaactcc tttgttgttt aattacaggt gtatttataa tgaatttctt cgggacaata 24240
   aaatatccga atttagctaa tataacaaca ccaccgttaa ttattaccgg agaataatca 24300
   tctggagtat taaatttcca gtggagtcta gtattatatc catcagagcc gaatgcttta 24360
   ttagccggtc tcccggcttt ttgccatgca taaaaatcaa gtttcatttg caacccaatc 24420
   aaccatatgt agatggtctg ataattgact aacatgctga agctgattag acaggagctt 24480
   acaaataagc tcactgttat catttttaaa tagttggtca atacgtttct tatgagcttt 24540
   cttaagctga cgtttattta aacgagccat taataacctc ggtcttggcg agcgaagttt 24600
   tcagcattct tcaggtaata cagtttaaag atttcttcag ccgaaagacc tagaccttgg 24660
   aacatattca gaacgaaatg aagaatatca atcatctcaa atttaatttc aagctggtct 24720
   tcaggagaca gttcatcgat acgacgattt cggtattcag catgttgtgc tttccaaggc 24780
   ttccatacac tgctggcggc tttttcacca ttgctcatac caccaagtga agtcagcagt 24840
   tcacggaatt catcatcaat ataatctttc tgattacgca gccaatcaac aacatcacca 24900
   gcagtggcca attcatcagg atgtttgtta tgttcaggct tgtcattagc cagacgaacc 24960
   tgtaaagatt tctgcatatc aagcattacc tgcagtgggt ctttatcttc gtgaatcaga 25020
   gcattaaaat aagcttcttc tgctttatca acaccagaaa taagttgtga acattcgtta 25080
   aagtgtgcca ttatttttcc tttcaattca gtataagaaa gttaattcta acattaattt 25140
   tataaagcgt aataaataat aggtaactaa ggagatatta tgtttaaact tccatcgtta 25200
   atcaacgcta ttaaaggcaa taaaaagaaa actgctatcg ctgcaatagt tgtaggtggc 25260
   attatttcat ggaacttcgg gattgcacca ttgttagttg cccatggtat tatagttcct 25320
   tctgttccat tggatactgt agttgatttg gcctttgcta ttatagggct tgtttaaatc 25380
   ttcgaataac gagataatct cattttagca tgcaacccac ttgaaatatt ctgtttcata 25440
   tattctaaaa tttcttgtgg tgttgcattt tctttcatta ccatatcatt cacatccttt 25500
   gatctccatg gagctcggtc ccagaacatg acacgttcgc ctgcatcaac taatcgcttc 25560
   atacgagcta tagtatccgg atgcctaggt tcattatcca ttacccatac ccgacgttct 25620
   ttaaatggta ccacatctaa atctatcgag ccgcctgtaa tagcgattgc attgggtatg 25680
   aataaactat caatagggcc ttccattacc cagacgtctc cttccttaac acgttcaaca 25740
   ccataaattt ttgttgcaga ctcaaacgct ttaatagtga tatatttctg aggagcatct 25800
   ttgcgaagtg cacgtccttg aaatgactca gccttcccgt ctttattaaa aataggaatc 25860
   actaaacgcg gttctggaat ctctttctta tacgtaccag gttgaatctt gttgaccaat 25920
   ttaggccatt ctaatgtgaa ccataatctg ttccacgatt ctttaggaat acaccgagac 25980
   ttaacatatt tcacaatagg atgctcttca ggcagcttgt ccagacgaga acaagacggc 26040
   aaagaattta tagtcttttt ctccggttct ggtttatgtt caggaacctt ttcaatcttt 26100
   ggttccattc tagctttttc tttacgaact tccattagat attcacgata aagctcttcg 26160
   tcatactctt ttaagtatcc ggaaatcgta ttatgatatt gacagttata gcaatgcaca 26220
   ttacctggag taccgccata ataccatccg cgggctttca ttgcatctgt ctgagagtcg 26280
   ccacaaactg gacaacgaaa cgttaattta aatgtgcttg aattattgat ttgtctaaaa 26340
   cgaggtaggt gagaaaacgc acgataagca aattcattat ctatccattc agccatagta 26400
   ttctccagag gccttaaatt aataaggcca tcatatcaca acttattgat atttacttta 26460
   attcgctttc ttttcttgga aggaatttgt tctggacctt tatttacgac tgcacctgta 26520
   gttgttcctg atgcaatagc ttgagggctg ccacctgaat caccagccac catatcctca 26580
   aataaaggga gttctttaaa tatttctttc tgctgttctt ctgtaatgtt atagcgggat 26640
   gctacagcac tccaggcaga catcattgag gccattccat taagtcctgg aacagtgctc 26700
   atcattcgct tcattgaacg aactgaagca tgaaaaggtg tataagccgc tttctcttca 26760
   ggagtagaag gacgtttaag aacacttcca cgttcgtcga ttattttggc ttcgtacgct 26820
   ttccattctg taaaaggttt ttgcattagt cgaataaact tatatgcata aactgaatct 26880
   attgcagtgt taataatacc cataggtacc tcctgagtat atttaatcaa tcacaaagat 26940
   cgctttatca ccgttcgcat ctgtgacttc atatgtagtg tcttcatagt gtggaatatt 27000
   ccaaattaca ccatcattag cttttacgct aatatggcat tggtgttttg aaacaacttc 27060
   ataatcttca aaacttgtaa aaacactaat tttagaacca gtgcaaattg cagtagtcat 27120
   tttaacctcg attcataaat gcattaaata tttggtcatc aacagaaatt tctaattcga 27180
   cttcaattgt ttcagtcttt tcttcaggtg gtaattcgta tccacacact acaatttttt 27240
   catcgatatc aaaatataca gaagcaatac gattaatgtt actttcagtt aagtctaaat 27300
   caacatcaac atctttttga cctacactta aaggataact aatacgagtt attttattat 27360
   ctttaacaaa gagtccacca acatactctg tactacgttt gaagtttaca cgtttccgaa 27420
   ttgaaagata tgatacatct ttaccgtgag ctcggctcat aggacataat gaataactag 27480
   aataagagat gtcaaatcct acgccttcaa catgtgctag agtatcatga gtaaaccaat 27540
   tatagtcgta tgctaaatcc attaaattat aatatgtgaa aggcactgga ttaacatcat 27600
   atgttgtacc tttaaaatct gcccaggcga cgatttcgtt atctttaaca attacaaaaa 27660
   ttataccgat atggtatccg ttattatttt cattcggtgt tatattataa cattgtgcat 27720
   taggaaattt accatattta aatggcgcgt ttttgtgcat ataataaatc atattattca 27780
   cctgtgattt cggttacgat atctttgtta tcgaaattag ggtcacaata caaaacatag 27840
   ttgtactgca tgataccgcc agacttaagt tgttcaacaa ctttattaac catattaggt 27900
   cggtctcgtt tagtgatatt catgatatca gagaagattt gttgttcaac ctcttcttga 27960
   tttgcagtca ttgaccactc acagaggtct ttatcataac ctgctgtaac tggagccggt 28020
   gcacatgata ctgcaagtaa gattgctgcc aatgataact ttttcatttt aactccttag 28080
   ttgatttgat aggactacta tactatagac catctataat gtaaacaagt ttattctttt 28140
   tcgcgaataa taattacttg ttgaggctgc tgagcagatt tcttttcggc gacgatagac 28200
   caaattaaag ttccaatcca accaatcatc gtccagttaa agagtaaaga tacaaagaag 28260
   attgctgttg tgctttttgt gccacgaata agtgcgatta cccaaggcaa catgtaaatg 28320
   atgattgaac caatacctgc gattaaaact gctactgaac tagctacgaa taattccatg 28380
   ttaatttcct cttgatttgt tatgtttata gtatcttatt ttaactttgg tgtaaaccga 28440
   ttaattaaat ttgtttaaac gttcaataca ctggacgtag ttattcgcga tttgtttctg 28500
   ggaaggctct ttaccagttg tttcaacata gcgaatgaat gcgatttcag tagcctgtaa 28560
   gcaatgttct ttaacgcgtt gttcttttga caatgctaca gtatcagaat cacgataacg 28620
   ttgagcatgg gcatcagctt gaatcattcg attacctaca gtaaccgggt cttccacttc 28680
   aactttaggt tggttgtact gtttaacatt cgggttataa atcaattggc cgacattatt 28740
   agggataaag ccatgagcac cacatccagt taatccaaat accattgcta aagtgattaa 28800
   ttttttcatt tcaatttcct ctgtagttga tagaaagata gtaacacagt atgtagtgct 28860
   tgtaaacagc tatttttaaa attatcgaaa atcagcttca aaactttctt cgtcattaat 28920
   aaatgcttta actaatgcat tgttcacttc agcaagttgt accgcaaccc actcgatttc 28980
   ttcacgatca cctgataaca tgatatcttt agtgaacatc tcgccgcttt taaaccagcc 29040
   aataaaagta actttagtta atttgctcat atttcctcgc aaaatgccca ttcagcgtga 29100
   taaactgtag cacctttagt atcattcatt tgaactacat ctaccggaga aattactcga 29160
   tagagacgtg gttcacctcc ataagagcgt gctgctcgtc ctgcataaat ctttgctaaa 29220
   ccgatatctt cagtaaagaa tacacgatcc aagtttttct tacgtccagt ttcagacaga 29280
   actcctgttt cttcaggagg acaaagcatg ttttcgatgt tagctgaact acttgaaccg 29340
   tgataaaaaa tcttaaattt tgctttacaa tcgatggttt tcattttgtt ctccagtagt 29400
   tgataggtct ataataacac aactactgga gaagtaaact taaaaatgta atttagatac 29460
   atctgcgttg atttcacttt ggtttgcgtc atatgcaatt aaacgaaccg ctttagatgg 29520
   gtcattcaat gcatagaaat gatggaacat tgctcgaacg atatcaccat ttacgagttc 29580
   tacctggata ggagaacctt tttcgagttg gaaatgttca taagctttga tggttcgcag 29640
   ataatcaagt ttttgatttc cggctccaac gatttgtacg atacgtttgc ccatgttttc 29700
   catacgagat acaatagatt taggagcaag aataacgtag gttgccagcg gagaaactac 29760
   ttcaacagat gtgttttcgg tgattgcagt aaaaattgct tgagacatat tcataatgtt 29820
   ttcctcttgg ttggtgtaag tacatcatat catgcctaga ggaagatgta aaccccgaaa 29880
   ggagaccgaa gtctcctttt tattaaaatt ttaactcgtt ggctaagcta tctaggtcag 29940
   cacgtgttgc tttggcttta cctacgcgtt ccgtacgatt cacctctgcc tgacgcatct 30000
   gagcacctgc gttttcattt actgtgttag gtgtgttcat gccttcttgc tcaatctcaa 30060
   tccatttctg gttaccttta cgtacaccca tcaggaattt attccactta ttcttatcac 30120
   catatcgtga tttgatttgt ttgataagtt gttgttcagc ttgtgcaagt tcttcggttt 30180
   caattacagc caacatgaaa tctgctgttg ctggtaaacc tgctgattct gcaatatcac 30240
   tcatgttcat gtctgaagca tcccatgctg cacgtcctac ttgagctgca gtccatagaa 30300
   ctgtttcaga ttctactgct aatgcacgaa gttcttctgc gatagcttta actaatgtgt 30360
   agctattttc agtgtaaact ctgatacgac aagacccaca aatacctaag taatcaacca 30420
   tgattacaga tggcacaaag ttcttcttaa gttttaactc attaagcaat gcacgaaatg 30480
   tattagcatt agctccacca gtaggatact gtttaactac taaacgtcct aatgttgttt 30540
   ttgaacgcca tttttccatc ttggctttat attcagcata agatacgtta ccatcatcga 30600
   tatcatcgag tgatacgtca agcatgtttg catctatacg cttagcacaa acttcttcag 30660
   ccatttccat ggaaatataa agaacattat gtcctgtttg aagataatct gcagcaagtg 30720
   aacaaaggcc taaagattta ccaacgttaa caccggccat taaaacgttc agtgtacctg 30780
   tttctgcgcc acctttggtg attttattaa gaatgtttaa tttgaacggg accttacgtg 30840
   ctttatttag ataagaaagc cagcgtgctt catagtcttc catccagtca tgaccaacat 30900
   aactatcaaa cgaaattgaa agtgcttgac gcataatatc aggaatagct cctacgtcag 30960
   gcattttctt gttacgttgt tctggaggta gttcagcatt agactgaatc tcaatgattt 31020
   tggatgtagc attatacatc gctttttgct ggacatattt ctcagtttct ttaaccaacc 31080
   attcgtggtc ttcaggagta tcagccaatt tttcaattag gtctgaagtt cctttatatt 31140
   cggcttcagt taaagaactg ttatctaaag ccacctttaa tgcattgata gatggcattg 31200
   cattatattc gttaacgtgg ctctttataa ttttaaagac attttttgcc ggtccacgct 31260
   caaaatactc agaatccata taaggccaga ccttagaaaa ataagcctgg tcgtatacga 31320
   gatgagacaa aataatttct accacgataa ctccttaaaa gaatttaaat tttttcttgg 31380
   ttcgttcatt aaaagcttgt tgtacttgca ttgtaacaca tttttcaaca tgtggtgcaa 31440
   gttctgcttt tctgtcctgg tcaagaactg caaagtccat tacgatttta ccatcaaccc 31500
   agtctagttt tgtaatatag actatatgat gggaaccgtc ttcaagtgta atcagaatct 31560
   cttgaatgac attttccatt gctgaacgga taattttaag agattcatta aacactcgtt 31620
   ctttgcgaat gtcttcccct tccgaagaag gggattcatc aacgatttct agatttaaat 31680
   catcgagatt attcatcgaa atcttccatt tcgtcaaggt cgttttctat atctgctgca 31740
   gacggagcat cagaagcttt aacaggaact ttagttgttg ctttgcaatt aataaggtcg 31800
   ttaacagcgt catcaacttc tttaatagaa gagatagcac ctagtttata tttagtttca 31860
   attgcatctc ggaaaggttt gtgtttaaac aacggtcccc agaattctac acaatcagta 31920
   gctttagcac gccacgattt ctcttcacga atcatttcac ctgtttcttc gtcaaggaat 31980
   tcgcgagcat accaaccggc tttaggctta acaacaaatc caatttcagt tgccatttct 32040
   aacaagccac tgaatggatc aataccgcca tcaaaattaa ccgtgattgg gaaagtagat 32100
   ttctctttaa ctgtacgaga tttttctgct ttcaacgtga agtcataacc ggtcaattct 32160
   gtaccttctt taacctgacg tttagagata aagaatacag tgttagcaga gtaaagaata 32220
   cctgtaccac cacccataat ctctttagga tatagtccgc cgatttccat tgctgtatgg 32280
   ttgattgcaa cacacggaat atctttaata gtcagataag gggtcacaat acggaacaga 32340
   gatttaagtg ccttagcacg ggacatatca cctacgactt tctcgttcaa tgcatcttcg 32400
   gtttctttct tagatgcagt attaccgatt gaatcgataa agataataac tttatcaccg 32460
   cgttcaatag cgtcaagctg attagtcata tcaactttaa gttgttcgac agattgaatc 32520
   ggagtatgaa ctacacggtc taaatcaaca cccattgaac gaaaataaga ttctgaagca 32580
   ccaaattctg agtcatagaa caaacagatt gcatctttat atttcttcat atacgctgca 32640
   accatagtta gtccaaacag cgttttaaag tgtttagaag gtgcagcaaa aatagtcaag 32700
   cctgattgca atcctgcatt cagagcacca cctagtgcaa tattcaacat cggaatacga 32760
   gtagggactt catcacgatt attaaacagc ttagacttag tcaagtctgc agtcatttta 32820
   gaagtagaag ctttaatcag acgagatttt aaatcagaca ttgtattttt ccataggcat 32880
   cattatattt tactcatgtt taaaagataa aacgattata tactaagggt tatacagcat 32940
   ttaaaacttt aaacaggctc ttaacgagcc catttgatga tttctgtaat caaaccatta 33000
   cgttcgcttg cattaaatcc tataactttg ttagcaccat catgattaag taattgatat 33060
   gtttggattt tgttatgaat tgcaaactct tcaaaatgtg aaatgactgt attaaaaccg 33120
   aatgaagaca aagtaataag gtcgtcttca aaactttcag tcatttcgtt acgtgttaaa 33180
   ataattgtga acacatcatg gttttggcaa agacgaacat agtcctgttc ataaacgtca 33240
   agaaccgagt ccgcatcttg tcgaatacgt ccataaaccc agttactaat atatccacgg 33300
   tctaacagat aaatcttagt agggtctaaa taattcaaca tcgtttcgaa acagccaact 33360
   tcatttcgag acttaacatc gaaacgccca tcaactgttc gtttagggaa atcaatcttt 33420
   acatatcttt cactaatttc cattatgtca ttaatgaaag ttgttttacc agcattatcc 33480
   ggcccgtcta ctaaaataat tttagccatt ttattcctcg attacgacat cttcgtattt 33540
   aggtatatta tacggtgaag aaattaaagc attaaagtag atgaactcac caagacctgg 33600
   atggaatact tcttcaggat tttcgaaata ttgaatcttc ggacgtcctt taagaatttg 33660
   atagtactca gttttagttg gattaaaaat tcgaatcagc gcttcttcag gatacttatt 33720
   tctgtcgaaa ctattatttg gatcagttat ataaagcggt tgacctaatt gaattgcgca 33780
   ttcaaccact tgttcaaaac gatagcatgg gtcactaatt cgaaatggat gaaaaatacc 33840
   atctagatga ataggagcca aatcacctac aagacgctgc atgatggaag gacgtataac 33900
   tttatgcgtt acgataatct tattaccatc tgcacctgcg ttaaccagca cttctttttg 33960
   acgttcattc aacacagtag tttgaagtga acgattaaca gacaacatgt cggtttttaa 34020
   gaattcatca atataaaaac gtggtttatt agggtctgca gtgatattga aattgaattc 34080
   tacatcaaaa gcgccgtcat atccagtgat atctgtgatt agaaaatcac aatcataata 34140
   agcaattaat gaatcaacca caaattcatt ttcagaccaa aaatatttac gagtgttata 34200
   cgcgttatct ttataccata atggaacaaa cttaaattct gggaaaagac gttcacattc 34260
   tttaatatca gatgcattac gtggatatgt aatcacatca cccggattag cacggtgcaa 34320
   atgaagttgg aagttgccat ccttaaggat agcatactgt ccagtttcat aagaccgcat 34380
   actcaagata ggtacgcgga aaatagccat tagtcaagga atcctttaac ataatcaaaa 34440
   tcacgttcaa acacatgggc acttaccatg gtatgagtat aatcaccaac ttctacacca 34500
   cattcgttag cgatataatg aagtaatttt ccttggagat aaaaatccaa ctgcataaca 34560
   acagcacagt tctgggaacg catatgacag tgggtataaa gcttaccgtc acgaatataa 34620
   taagtcactg agtcagtaca tggatattca agcgtttcgt ccgaatcaag caaagcctga 34680
   tcagaacttt ctagaatttg gaacacaact cttcgggagt taggcttctc tttaagttct 34740
   tttagaagag ctggtaattg ggccgcaatg cgaggaccat agaaagtgtt gaagttcgcc 34800
   ggcaaaacat ctgattttgg tttagaaata aatttagcga cattaggata tgccttaaac 34860
   gcttcttctg cgttggtacc accagaaatc ataaacttcc agaagtcttc ggcatattca 34920
   taagagatgc ggttaatacg tgggtcggtc attttgaatg atgatggagt gtcaacaact 34980
   actgtcatag acccaatttc attacaacgt ccaatacgag aatcagttac gaattccggg 35040
   ttttctaaaa tttcacgatt gactgcttta aatgcggttt gaaaatctgc ggctttgata 35100
   tatttcattg tgttatgttt tcccatgttt acctcaatac aatttattat atactcaatt 35160
   cgtaaagctt atttgaattt cttggcaatg ttgatataac gtaaagcttc agaatctata 35220
   cttccaagaa cactatgaag gaatgcatca gtgtagtatt ccaattgaac aggttcttta 35280
   atcactttag ccgctttggt ttcaaacagg cccataaggg cctttttaag aatttgttcg 35340
   atagtgttca gtttcatagt caaaatctct tcgttaagtt tataattaaa actttttcta 35400
   gaaatcattc cgggtacctc aaagcgtcat cagagttagt tatatcaata atagatatgg 35460
   agttttctgc taacccgttt ttagataatt gacgtttata atacctttca caatatgtac 35520
   acgttgaccc acagatttca ttagcacaat caaaatctgg atgtctgaac cacctttttt 35580
   ctaaaaagcc atctaacaaa gaggtttcaa tatttactgt atgtgaaaac tcagtctcat 35640
   tttcaagagt gaaaatagtc tctaaaggct tccacagttc taacaaatta ccttcaaact 35700
   tttcagaagc gtatgcttca agaactttca aaatgtagtc tgtgctacct gtacgtcctg 35760
   aaactttgaa ttgagtgata ccaatatcat tataaagttt caaatcttga ggacgaacaa 35820
   aacgagttct cagccagtta aatgggtctg tatcacgagc tttaatacag tgctgcattg 35880
   gatatccatt taaggattta gcatcatcag cggtaacatc agtactatgg aaaatatagc 35940
   aagagtcacg atagctacaa tgtgttgtat accctttacc agcattagaa cagaactcat 36000
   ttacaagaac ttcaaatatt atattgttct tattgcaaaa ctctgctgca cgcttcagga 36060
   aagaaactga acggttctta tgaattccgc aacagacctt tttgatgtta tattggtcgt 36120
   gcaagtattt gatttgagtt acagcatcga catgtagaat tgttgaaact tcaatctcaa 36180
   tgtctttatt gacttcacga acaatttcca taacaacagg gttagcgata gtaatacgcc 36240
   atacgcctat tgaccaaaga tactgaacat aatcctgaat agctttcttc ttccactcga 36300
   ctagttctct ttttgaacca ggatttatcg tgttgagtgt atagttaaag caaataccta 36360
   attcgttaca acgtttaacg taatcttcta acatcttttt gtcaacgtca ggaagcctaa 36420
   aatcaggtct ggctgctaca aaagccattt ctcttgtaga gccgtatact tcgtttatca 36480
   agctattagg ataagtttta ttgagctcta cgattttatc aagaagagct aaatcaaaat 36540
   tcgttccgat tttaaaagta ttcatttttt gcatctcgcg ttaattgatg cgatggtttt 36600
   tgcaggtctt acttaaatta ttatagattc ggtttatatg atttaaatgt ttcgaattcg 36660
   cctttattca taagagcttt gaattcgtca ggacgcatag ctttgtcgtc gataatccaa 36720
   tcatatactg gtttatgtgt tagcaaatta tggtacttca agccaatgtt tgccaagttc 36780
   tgaatcagac ttgggagaat gtcaattgct atacgaccag gacctactga cttcatacca 36840
   cgagcagtgt aaagcgttat ggtatgccct gcatcataca gggcattaat ggcagacacc 36900
   atttcgacgt ctggtttgaa gttcacgtaa tcacgattgt tattccactc tgttatacaa 36960
   tcatcgacat caaaacaaag attaagcatg ttttctacac ggtgcattag attcgttccc 37020
   attgatgagt gatatcgcca ccgattggac gataggaaac accagtttct ttatcagtta 37080
   gtacccattt accaggattt tttgtaatca gagtagccgt tacaggttct gctacgtcat 37140
   aactaataga gccgtcttga agtacacggc ccatgttatg taattgctta gtcagaagtt 37200
   ccataccttt ataatatgcg gcaaccgctt taagtggatt attcttgaaa taaccaccta 37260
   atgcaatcca gataattgct actgccaaat gatgatactc gttaaacgaa ctcatctttc 37320
   catatttgta aactaatgga ttgatgttaa tagtcacatt acaagttgtt tcatcaatag 37380
   taaactgacc ccaggtcgga ttagcattga attcatcata acctgaaaca gcataaagca 37440
   ctttagcttc atcatacaat tttggcccgt aaatcttggt ttcaccaaaa tatccacgag 37500
   ggtcgataaa cttgacttcg ccgttatcgg taatcatagt attactgaag ttcgggtcac 37560
   catgaataac agagtactgt ccctgagaac ggttatgata acgaataaga tgttctaaag 37620
   cttgtttcaa cattggcttc agacgtccaa tcttagtgta gttcacatgt gtgattttac 37680
   caaatgaatc aatcaatggt tgaatacttt cacaacgatc aataactttc gtatagaatt 37740
   ctttagtgaa atcacggcga acagtttcag gacttacaaa ataagtgtct gtgctgaatt 37800
   tcagtgcatc taaaattgca tcaacaattt gtggacgagc aagactcgat ttggatttaa 37860
   tatattcaaa tgccggctta cctttaatgc gttccatttc aaagttattt tcattaacag 37920
   aaactaactg aggaactgaa tctgatttca ctttaagata ccatgaaatc tcatctttct 37980
   gaagagctcg gccctgttcg gttaatgcga atttagttac agtttcttca ccaatttcaa 38040
   ctgcattaaa actacgatta agttcacgaa cttcatgagc tttttcaagt ttaggcatat 38100
   cacctaaatc aattagattc ataagctctg atttattgaa tgcgctgccg taaaggaatt 38160
   ctacgaaatc acgtcctttg cagtattcat gaatttcctc atcagtagaa cccatataga 38220
   attcccagtc tttgaactga taaattccta caacattacc acctgttgaa cctacgttag 38280
   tgatattttc accgtcgaaa ttatatcgac attcgtcacc cttaacatag attgcgttaa 38340
   cattccaaga ccatgaacca aaatcaggaa taatatcaca ccagttaaca attacattat 38400
   ggccatctaa atctttgtgt aatttagcaa gagcataagc agaaccatac gcttcatcaa 38460
   ctgtacgaat agtcacattg aacccttctt gctcacaata agctcgaaca gtttcagcaa 38520
   attttgaatg aactactaca ataatttcgt ctgcgccaag gtcagaataa attgtataca 38580
   aattgctcag aatagtgtct tgtttatagt taacaagcac cttaggaata tggtgagtaa 38640
   ttggatataa acgagttgct aatccagcac caagaataac agcttttttc atgttgaagt 38700
   cctcattggt aagtaggtgt attgtacact attctagtta aagcattcat actttgcctg 38760
   tagaccaatc ataatccatc caattacaag tgttggaatc attaccggag ccgttaagct 38820
   ccagtagata gtcatgacaa gcatcaatag taaaagtcct attgttattg ctttcattgc 38880
   tcgcctcgta acatggcacc tagtgcaccg cctggatgga acttaaggaa atcattagag 38940
   gtaaatccac gttcagatga aaggttgata gcgaacgtat cgataagcgc taacaacaat 39000
   gtagtagaca tcgtcggagc taaagagttc tcgtcaactt ctaccgcgat accggtacag 39060
   aacgtataat caaaaagagc ttcgtattct tgtggaatgt cagggttaca atgtaacaga 39120
   atctgtttaa cctgaggacg aatcatacga agatgtttag caacacctat catttcttca 39180
   gtcttaccgg aacgactgat gtgaactaat acatcattag gtgcaataaa tcctgcgtca 39240
   ccatgagaat aatgcccagt gttcaaatac atgctaggaa tacctagtga agcaaatgtc 39300
   tcagaagctt tagttgcaat attagcattc ttaccaacac ctgtaataat tactcggctt 39360
   tcataattac tcagtccagg gcgtcgaaga gtttctagaa tagcattata acgagcaggg 39420
   ttttgactaa ttactttggc catagcggcc aaagaagaag cttgttttat gattgcgtca 39480
   atagcgatag taataggagt agttttcata tttctctcat ttaattagtt tactgtccat 39540
   gacattatat caaaaatcga acatatcgaa cagagatgct ttcttctcgt aatcgagttt 39600
   cgctgctgat gtgaatcctt caagtggttt aataaatgtc ttctcaagga gaacagtgta 39660
   gtccatccaa tgaagtacgt cgtctttaat taaatctgtg atttcagtac cagaaggcca 39720
   tgcgatacat ttatcaccga atgggtttcc atcacgtaaa ggcagaacgt ataccttttc 39780
   gccttctaca acttgtggag catcaatatt acctttgata gctctgttat atgtcagaat 39840
   tccacgaata tggaacgggc atttgggacc agggaatcca ccaacgtcat atttagcaat 39900
   gttattcgca gaagataccg acgcgatgct aatataattc aattgacgga attctttttc 39960
   aaactcttta aaatattctt gtaatgattc ttcaccttct tgaagcatac gacgaataca 40020
   ttctttaaga gctttctgta ctgctttagg ggtcgaagat ttctgagtct ctagacccat 40080
   gattttgagt ttaggctcag cgtaacgagt accttccata tcccacacgt ttaatgcata 40140
   acgtttctta ccagtccaga atccaccaat acctttagaa ccgagcggag gaccagcgat 40200
   agcttctcgg tccatgaaca ttaagtgttg tttattgttc atgtattcgc acatttcacg 40260
   gaaacctcta tcaatagcag gttccatacg ttcacgagcg aacttatcta agaagtctac 40320
   ccaatggttg gtatcacgga atttagattc accaacctta tcgataattt tatcagcaga 40380
   tacgtaaata gagtctgtat caccataaag aacgaaagct tcaccttccg taccacaaac 40440
   ttcgttcaga tattcattaa ctttacgttc aatccactgt aaagccattt gcccaaatgt 40500
   tgtgattgca gtagcattac gcaaatcata ataacggaac catacgttac caagtgcacc 40560
   ataaagcgag ttgatgagca atttacggtt aatctgtgca gtcatacctg caacttcagt 40620
   acgttgagct ctaaacaaca tttcgttaag agatttagca gacaactttt taatcttttc 40680
   tttaatctca tcgctgaagt cgaaacgata atcaacatct aatggttcgt caacagaaag 40740
   attaggatta tgtaaggctt ctttaattat ttcaccatta cgttgagctg caagcatgta 40800
   acctttatgt tctttacgtt gattaaagac cttagtgatt tcagttggaa ctacaccgtc 40860
   acggtcttta taatacatca tgccgttagg agagcaactg tacacatctg aaggacgttc 40920
   agcgattgcg ttaatataat catgcaatgg agctacttta aacgttcctg ctattgtttc 40980
   tgggctaata ttcacttggc gaataatact tggatacaga gatgtaaggt cgaaactcat 41040
   tacatatttg tatcgatttg gaataggttc cttaacaaaa gcgccaggat aaggttgaac 41100
   cgggtgagaa cgaccttgtg gaatcacctt gttctgctct ttaaggctat taaaaataat 41160
   agcatcccat gttttaattg ggctaaacac agattgaatc tgtatcttag cataataacc 41220
   catgtccaag ctcaagttaa tgaactggcg tttagcatca atttgcaata cacgatatac 41280
   gtcgataatg ttataagaaa tatatcgttg gtgattgctt tcacgaagct tagaaatagg 41340
   gccgtcatat ttcagtttac cgacattcaa ttcaaattct gaaatgtaat ccagagaata 41400
   cgacggttga ttggtaaaag agaatttttt gtaaaggtca atgtaatcaa gaacagagat 41460
   accgaacaat gtaatgattt cacgagaacc atacatgttt tcgataactt taacacgagt 41520
   tttacgatgt ggcgacaacc gtttcgcagt agattcgcca aaaatattct tgattcggtt 41580
   atacacatat ggaatatcaa atgactcaac gttccatcca gtcaaaatga caggagtttt 41640
   ctgttgccag aagttgagat attccatcaa caattctttt tcgttatcga acggcatata 41700
   aatgatttta tcgataattt cagatggaac ttcatcacca ccttgttctt gaagcttagc 41760
   cgcaatttcg atagaccatt cttctacatt gccatatgga gaattcaata gatcaaatac 41820
   gtagaacctg tcgtcaattg agtcataatg ggtgatagca tcaatcggat gttttgcttg 41880
   tgacggctca gggaacccat ccggggatgt tacttcgatg tcgaagttag ccacacgaat 41940
   ttttgtatgg tcgtatttga tttcatagtt ataagtgtca gacaaatacg ccaatttgaa 42000
   atcgtccatg ccaagtgctt caagtccgat atcttccatg cgtttaatcc attgggaggc 42060
   atcacgcata ttagcgaaca acttacgagt acaaggttta ccgtagatat cgaaatattt 42120
   agtagcctga ctttctggac aatgagcaaa cagtgatggt ttatattcta cttcacgagt 42180
   acgttcacgg ccattagaat cgatgtaacg ttcaaaaatt gaatcaccaa tctgttcaac 42240
   cgttaagtaa aattctttca tttaatttcc tttagacgag ttattgtctt ttgtttgttg 42300
   atgaacttat tatactccaa atgggaccga agtcccattg ctttaattac caattgtata 42360
   ttttgctttc aaagtccagt catctttctg tttaaaagag ataacacgga agttattagt 42420
   caggccaaac agatcttcac gagcagagtc ttcaataaca atcaatcccc agtcttcaag 42480
   caactgagca atcgaatcac gtcgctgata gtcttcacca tcaatatcaa cctgacgtcc 42540
   gtccatacgc aacatttctt taaagtggac aatatagtac ttaccttgct tttggaggat 42600
   atgacaagac tggtaaagga ctttatcttt gttattagcg atgcccatac gggtcaaagt 42660
   ctctttgact ttaagaaaat cttcagggtt cttcaattta atttcaatca tttttaccat 42720
   tccaatgcta attttttaaa ttgtttttgt tcttttacgt ttttagtaac ttctttgaga 42780
   aattcatcag tgaccatggc cttcagttct ttaaggacta ttggcaactt gttatttttt 42840
   tcaaggagcc gcttatactc ggtagcatcg ttcatgttga ttgtgtaatg tttcatcaac 42900
   agtttgataa ctagtaactc tgaagtgtct tcgactagtt tagcccattt agagaatcga 42960
   cgaccacgtg gaattgccgc catcatatag ttgaagtgcg cttcatcact taattcagaa 43020
   ccgactaagt tcatcatgta aacggctggc atacattctg ggaactggga taatgagttc 43080
   tcaaccataa attttgagta gtcgacctga gcaattgaca tagatttctt ttcattaata 43140
   gcattaatga ttttaaagaa ttcattctca ggtttttctt taaacatatc agcacatttt 43200
   tgaatcgcat cagcgtcatt agacttccat gcaatttggt gctcgttaag ctggacgtca 43260
   tcatcgaata agttcatatt atttccaagt cagttcacag gtcagttgaa gaagcatata 43320
   cataacatgt aattcaatat tacttgctag tccatgaaac ttattatttt cgcctgcaat 43380
   ttcgtaaagc gaaataatac ttttaccagg tgcaacttgg tcataacatt ctgaaactaa 43440
   tttatcgatg aaccatgaat aatctgcagc atattttgga gctaatgcac gaagctgttt 43500
   gatatctttg ttcttcattg cttcgataac atcagagact gtgccacggt cattagttac 43560
   gatagacaga atacctgcat caagaacacc tttagacgag tactggtcta attggccaat 43620
   tgtacgacga aagtctggga agttcttttt aaccagagct gcaacaactt tcatatcagc 43680
   gatttcgata ttttcgttct tacagatttc aaccatacga tgaatcattt tcttcatcat 43740
   cgagatttta tcttcttcag tcggacgacc gaattcgata actctacaac gactacgtaa 43800
   tggttcaata attccatcaa tattgttagc agtgataata atagagcagt tactagagaa 43860
   ttcttccata aacgtacgaa ggtgacgttg tgattcggca aggccactac ggtcaaattc 43920
   atcgataaca ataactttag gcttgccttc cattgagact gaacgagcga atgcagttaa 43980
   cgggccacgc acaaaatcaa tcttacaatc agaaccgttg acgaacatca tttcggcatt 44040
   gatatcatta cataatgctt ttgctacggt tgttttacct gtaccaggag aaggagagtg 44100
   caaaataata tgaggaagtt ttcctttaga tactaaagac ttgaatgttt catggtcata 44160
   cgcaggcaga atacattcgt caatggaaga aggacgatat ttctgttcta gaatgtgttc 44220
   tttggaattg atagtaatca tgtaattttc ctcattatca ttaatctgga ggatttctcc 44280
   tccataatcg tactacatta aacgtaaagc tttgacttag aagtcgtgag tagaatcggc 44340
   ttccattgca ataacataac ttacttgaga gctttcaaac ttggctgcaa ctttatcacc 44400
   agctccccac aacattactt tatagttacc tggctggatt ttcatattag ccatattgat 44460
   tacgaagtta aagttgttag aaccatcata atcggtaaga gtcaaagaat acttaggacg 44520
   agttagtcca gaatcttcaa ctttattgta accattgata acaattttac catctttatt 44580
   tgtaatagca attgtatcaa tctggagacc acgtgaaact cgaagcaatt gttgaaggtc 44640
   ttcagcttta atttcagtaa tcacagaagc caccgggaat tgaattggct tattaggaaa 44700
   aacaatggtg ctcttgtcgg cagcaggcca ataaaccgtg gaacgtgtat ctgcaatttt 44760
   aatattgcca tcagtgtgca ttgaaatttc agcatcgtca gacactagac tcaaaatgct 44820
   taagaagcta ttcaaatcat aaagtgctac gtcaaaatca atttcatcag agatatttgc 44880
   ttccgcataa gtggtgccat taactgcacg ggtcataata aatttacctt gactcagcag 44940
   aataccagag ttaatagagg caaagttttt cagaatagcg atagtatctt tagacagttt 45000
   cattttattt cctttcaaga caattcaaag tttataacaa atttattatg cagcaaccat 45060
   ctgcttttta atagcttcag gcagtgattc aatatattct ggaattttag aagcaacaat 45120
   ttcgccagct gcaattaatt gttcgtcggc atcaacacag tagaaatctt ccagacgctc 45180
   agaaccatct ttatcaagaa cgatttttcc acctgcttca atagaatctt tatagtgctt 45240
   acgcatcaga tgaatccaca tatcagatac atcgttatca ataaaaccat aaatcataaa 45300
   acgagtttct tgtggcaaag attgaagctt tttagcactt gcgtcatcac aatgcaggac 45360
   ccatgcttta cgaacacgat tatggttatg tgggttagag tcagtacgga tagaagtaac 45420
   tttcagatca gcagcagtga aacctttagc agtagtaaac atattatttc tctcttattc 45480
   gtagaagtca gtaactgtcc atgcggaatt gcgattagac attattttat tatactcatc 45540
   tttaaaagca gatttgagca tttgttcagt aacaatatgt tcttcacctg aaaacgtatt 45600
   ggtcaaaaca tattttttaa tttcaggaac aacgagaaga tgttgtccgg ttgaatactc 45660
   catgttgttc tccatttaat ttgatgtggt cattataacc acatcgtgtt aaagcgttta 45720
   tgaaacagtc atcacagtaa atcgtccaac tttactcatc tgaagatgtt gtccataacg 45780
   ttgagggtca tggtctcgat gagagataat gaacacatta gtattttgta aacttccaag 45840
   aatggtatct atagccttaa cagcttcaga atcaactgcc ccatcaaaaa cttcgtcaag 45900
   aataagagtg ttgattttaa caccagaaac tttttcagca atatctcgcc atgtaaacaa 45960
   caatgcgata tctatacgtg ccttttcacc ttgactaaat gaagcataac taaaatcttc 46020
   acgaccacgt gattttatag attcattgaa ttcttcgtca atagaaaaca cgtaatcggc 46080
   ttccatgatt ttaagataat ggttaatctg cttgttgaac agaggcacat attttttaat 46140
   gatagcacct ttaataccag aatcttttaa catatctgtg ataataccac gatggtattt 46200
   ttcaagaacg atatccgatt ttgttttaat gattttatca agttcttttt gaagcaaagc 46260
   tatctcatcg gcatggttga taaattccgc tgaggcttgt tctattgcag ctttaacttt 46320
   cttagcttta tcaatagtgg caatcattgc ttgtttcttg gtacgaatat cagatgcaag 46380
   gtcctgttta gtcttaacat tagctcgata ttcgtccact aggacactca gattatctct 46440
   gtggcaggta agttgctcga atgagtgatt acattcatga agcttatcgg taatcttaga 46500
   gacaataggg tcaccctggt gcaactgtga tgcacaagta ggacatgtac cgccatcatg 46560
   atacatctta ataactttgt tatatgagtc aattttagat ttaatcaaga atgcttcttg 46620
   tcctatcttg ttgaatgcct cagtagggtc ttcgtcaaga acgatattaa gtagacgctc 46680
   gttagcttct tcaatttctg cttttaatga ccgagcttct ttggctaggt cgtcatacat 46740
   attctgtaga cgcgctacat tatcgccaga taatttcttt tgacgttcga tattctcatt 46800
   ataaatctta atttgttgga taataccatc tttcttggca tcaagaactt gcccctggga 46860
   attaagttct cgaacttgag acttattgat tttatccatt tcagcgagtg ttccaacttc 46920
   taataagtct tcaacaagct ttctgcgagc aggggtgcta agagccatga aaggggtata 46980
   ccctgccgtc ccaagcacaa cgatctgttt aaaagacgcg tatgacattc cgatgctacg 47040
   ttcaaattct tcttggaagt cgcgactgct ggcagattca tcaagacgaa caccgtcaac 47100
   ggagatttcg aaaatattgg gcttttggcc tcgtttaatg aagtattttt tatcatcgaa 47160
   ttccatccaa agctcaacta agagctcttt cttgttcgtg ctattaatga tttgtccttt 47220
   cttaacatca cgaaatggtt taccgaaaag tccgaaagta atggcttcta acatcgtaga 47280
   tttaccacca ccatttttac cagtgattaa tgtcttttgg actttatcta attggatatc 47340
   aataggattc ccacctaccg acatgatgtt ttgataacga acacgattta atttaaatga 47400
   tttcatatgt attctcttcg aatgaagtcc tctacatatt ttaacatgtc atctctatca 47460
   gcaaaatatt catctagttc tgcatacata acaacacctg gtgcgtactc acaggtaaca 47520
   tacattgttc caaagaaacg aggagttgtc tcctcgtaat tctcagtcca ttcaacgata 47580
   ccaatataac cgtcaccaat actaaattca tgacgatgga tttcatagtc agtcttcgtc 47640
   attatacgca tcctcgatac atgcttcagc cattttaaga atagagccag gagtatcgtt 47700
   gtccattata ttaaagctaa attcaatagc ccagtcggta cagacatgaa cggctttaac 47760
   ttcattgcct tcaacgcgta atggctcaat ccaatacttg atagtgttct cgaatccatc 47820
   atcatcttta acgtctattt caacttcaag cccaagccca gcattgttaa attcatctaa 47880
   agtcattgtg ttgcctcaat ataaagttcg ttggaatatt tgaccagcgc ttctctgtct 47940
   tcgtctgaga tatctggaat cgcgtcaata tattctttaa tgatatcttg aagtgattta 48000
   atctcgattt cctggtcgtc atcagattca acactgttat caactttaga aaccatacga 48060
   agagagtgaa ctactttttc aagttcagat tcaaatttag tcaactctga atcaatttct 48120
   gatactataa cacgtacggc tagattagta taatcagaat aattgatttt gcctttaaac 48180
   ggataagtga ttcgtcgatg ccatgtcgtt tcattaggaa taaattccat acgttcaaca 48240
   tcagtgtcaa atacccagaa tccacgtggg tcgttctcgt cacctgcagt aagggtccat 48300
   ggtgtcccaa tatatttgac gttagcagct tcagagattg tgtggaagtg gccggaccag 48360
   acttgtttat aggtctttaa gaaatcaggt tcaagcccat gagacttcat tcctttatag 48420
   aaataaaagc cattcagttc ccagtgccca acacaatacg cagcagaaga agtcttgata 48480
   tgctctaaga tttcacctgt attttcttca cacatccatg gaacgatatc aatcagacat 48540
   ccatcaaaat ctacagtagt tggatgttcg taaatagtaa agttttcaaa ttgagttaac 48600
   aattcagaaa cagtgttagg tgttattgta ttcttaaacg ccatgtcgtg atttcctatc 48660
   gtgacatgaa cgtgaatgcc tgctttagaa atcatatcaa cgatttcacg attgaattcc 48720
   attgtacgat gtgtaattgc tttacggacg tcaaaccaat caccgtattg aatccaagtt 48780
   ttaatcccgt gtttcttaga gtattcaatt gcttgacgaa taccatcacg ttgaatatct 48840
   tgaacccatg ggtcgtcttg tttgactccg atatggaagt cgcctaagtg caaaattttc 48900
   atattatttc ctcattagtt aattcattct atcatccgtt tgtaaagcaa aaaaggaact 48960
   ccgaagagtt cctatccaca tagtacaccg tacagaggct catttggact tttaaatcga 49020
   atgggcttgt acgtaatagt tccttcggct ttagctttat cataagactc tttaaaacgt 49080
   ctcattcttt ctgctttgcc tttacggatt ttatcgaaat tttcagaagt cgggatgttc 49140
   atcatgcata agcacctctc ggaaaatgcc taaatcgatt tctctcatcg tttccaccat 49200
   ttcttaactt gaaatgaatc aacccacacg agttgttttg ggtctgcctt tacaataggc 49260
   ggacaaattt ctgtaataga accatcgtga tattcctttt cagaaatttt aacacaaaga 49320
   ggaccaccca atttaacggt gaaccctgcg ttcagcttca gacgtttaaa ctttgtttga 49380
   actagcgcca ttaatgatgt cctcaatagc ctgacgagta gtaacccact ggattttaat 49440
   taacttgtca ttatatggtt gtcgtagaat atcccgagat ttcttaatag cgtattcgta 49500
   agcttcgaag ttgttttgaa ttgctgcttc ttgagcatgt gcataaagac ggtttagttc 49560
   aaaacgattc tttttaagaa tcttttcgga ttttttacgt gcttcagctt catttttctt 49620
   ttggatttct tgtagcattt tagctactgc atcttctgct tctttatttt caataatctc 49680
   ggtagtcata ttatctcatt aaattaaaat aaaaacgtcc gtcctcttca atacagaact 49740
   cgtactcaac tggagtccca tttatcgaaa atgacacgtc atgtgggttc aaagggtcga 49800
   tgtcaaaatt aattttaaat tctcgaccca acagttttac caaataaggg attgcttcgt 49860
   caaaaggttc ttctaagtag ttagagatat caattgtcat cctcatataa aaaatccaaa 49920
   ctaggcgaat cgctaacaac ttccttcttt tcagccccag gagctttata agcggattct 49980
   tcataatgcg tcattttatc gtagatgtct tgaataaacg tttcatctac caacgctacc 50040
   atatcatcgt catgactgtc gtagacatta tgaacgaagt aactatattt ctttgcaact 50100
   tccttacgtt cttttttgat acgttggaca aaagcattaa aacaagctcg agttatgtat 50160
   gcatgtgggt tattgtactt agtttcgtcg aaattatgaa gtcccttaat agaggcttca 50220
   atcccatctg caatcatctc ctgcttccaa gactgggtat atcctgaaaa gttgaaacgt 50280
   ttggacaggc cttcggcgat aagcataatt gctaaaccga tggtatcatt ctgacgaata 50340
   attttgtttg ggtctttatt atttaagagc tcctttttcc actcgattat agcagcgaga 50400
   agctctttat tattcacata gttattttta ggtttttgtt cagtcataag tacctcttgg 50460
   ctcaattcat aggtctatta tatcataata tttagagacc tatcttaaag cacgagatac 50520
   tatcggagca aacaataagc ctggaatact ttttccatta aacgtttgaa ctcatagata 50580
   tcgatataaa gctcggttaa tccagctcca taaagaaaat ctaaaaagtt ctcatgtgtt 50640
   tcgttatagt cgccttcgtt taattgagat acatgagttt cccaaaaacc aacaccttta 50700
   acgaatggaa aatacgtgac tttcatgtat tcatcaacat aaaattcgcc cagcttttct 50760
   tcagttatgc tgattcgagt taatttcatt ctagttcctt tgctgcttca agagcaacat 50820
   ccagcgaatc aaattggtca acgaaatcaa tattcaacgc atcgtcaacc gtagcatata 50880
   accaccattg tttgaattcg tattctagaa taaatccagg cccttcaatc tgaggaagac 50940
   ctagactatt tgtaccaact tcgtaaccag ctagacgcag gtcattaatc aaagtttctg 51000
   ttaatacgct cattttgccg ccttaacaat tttcaggtca acgaattttt tcttacgttg 51060
   atttttcata cgacggatag tcttttcaga gatttcatgt ttctgataag ctttagattc 51120
   aacaccaaat gcagcaacgt caaattcaga aagaatatat tgcactagaa tttcacgaat 51180
   agaattacgt ccaattttct ggtcgtgttt ttgcatgatt tcataaagac ggaattccca 51240
   tgcgtcaaga acttgaggag taacaatatt gccggattcc agaagagcaa caactttatc 51300
   gaaagcgaaa gaattaacta cgtttttgat tttagtagac atattatttt cctcggttat 51360
   tttattatcc gatgagagaa ttatactcat ctctcaagag tttgtatatt atttttaagc 51420
   atgaaaagta agtaatttgt atcacgtgga gtaatatcaa cacccatatc aaagatgcct 51480
   ttatagattg ggtatttgtt ttcggttgta tcgaagctga aactatttcc atcacaacta 51540
   acagtaatag catctgttaa aatgtcatac tcaatcctaa caattgaatc cccaatttta 51600
   acgtcgaggt gttttttgaa tttgataaaa tctaattctt gggccatgat attttcctca 51660
   ttttaagacg ataggaagat aataacatgt cttcctatcg ttgtacatta ttaaccgaac 51720
   tttttagcgc ctttccagcc ttcgtacata gctgcacaag tcaatagtac agcgtgctgg 51780
   cattctttgt atttgtaaat ccagaaccag cgtaagaagt cttcatcctc aacgccgttg 51840
   agtttcatat tggcaaagaa ttcggcacgt tcttcacgaa gcttttgcga taacatagat 51900
   tcaggattca ttttgtattc cacgccccat tttcatcgat gaacttaatc cagtcattta 51960
   cagaccattt agttgtatca ccctttggat taacatttaa agtatataat ccttgcttaa 52020
   aaagcattcg tttaatattc ataatttcct caactgtaac gataacattc gttagcttta 52080
   cgtttggctt cagtatgaga agtattataa tcatactctt ctttactgta aacatctttt 52140
   ttcaacttac gaacctcttt gcggagttgt cgattcattt cattcttgac ttcagaatca 52200
   acacctttca tacgtttcca tttatctgag cggaagatgt tttcaaccat gtctttgtgg 52260
   ctaacaccgt caggagcttt acgcttacca aaatagtcgt aatcgcgaac tttcaaatct 52320
   ttacgacgat atgttttacc cataagtacc tcagaggttg attgatacaa tgcgtttgat 52380
   aaaattcact tcacttactg aaaggtcttt aagaccgatt gattctaaac gctgagtaag 52440
   cgtcaacatt gggatatagt cccattgagt tttagacgtt tgataagtac gaacgccatt 52500
   agcaatttca acatgcaaat ctgaaatggt aacgaatacc ggtcgctcat cattaacttt 52560
   aagggttaga acaactttct ttgaatctaa actaatcata gtttaatttc cttcgcgatt 52620
   gaacagaaga taccacggtc acaaagcatt tcaccaattt ctggatattt gattgaacga 52680
   taccaaacat ccaggcttga aattctggtt atccgtactg cattaccatt gacagaataa 52740
   tactggtcaa ccttaatttc tttatcttca attgtcattt tgttttcctc tatagcagat 52800
   ggtttaatct taacatatct atcgaggatg taaactgttt tatgaaaata tttttgattt 52860
   gagaggggca caaggagata gtgaagtgaa gattccaatg gattatatta gaaatgagat 52920
   aaagaagacc aggcggcacc tggtcaatct tctatgatat cagagtcgta tcctaatgaa 52980
   atcaaggact ttttaaactc tgcaatattc ttttgtttct tatcatttaa aaatatgaca 53040
   ggataacgaa tattaagtga agtgaatcct gcacgtttag caagagatac gattaaagga 53100
   cggtcatatt caatcttacc attattagtg aggactttat aaaatgtata aggagcattg 53160
   agctccttta aaagttttgt aacagtataa catcctggac aacgtcctac ttcttcaggg 53220
   atgccgtaga tttcaatctt attctttaag ttcgagtttt gttccacgag aaataattcc 53280
   ttgataagcc caataaggag gattaacaga atcctcaccc gagttttctt caggaaaata 53340
   aacctggata acaaatccag atgcatcaaa ggtaaaataa accgtaggca gttcacctaa 53400
   caattcggca cgagcattaa tttcttcgag atctcgttca cgtccagccc aaaaactagg 53460
   atttagacta cattcgtaaa aataatagtc attaccgaac atatcaaacc ccttccaact 53520
   ctctaccacg taccgctcaa aactaatcat aattaagcct ttttatcaag aacagaattc 53580
   agtttgttag taattttgtc cagacgctca ttgaactcac tagaagataa gcccttttct 53640
   ggagaaatta aactaatcac gaaaaataca ccagtaaaag gaataagaaa aatagctcca 53700
   actgccataa acaaaaagaa cgttacagtt gtaagaaaat cagctaaacc tttacgaaat 53760
   ttatacatat ttacccttaa ttaattaacc aagcgttgat aagcaaacca tattgcgaat 53820
   aaaattctgg accaaaatga aaaatcatat catttatagt atccataatg tagttcaatt 53880
   taatcatgtt tccacacccc atcggtattt gaccaaagtc gctgattatc tgatcctcgc 53940
   cacagctttt tggtcggaag atttttctca tacttcccat caataataac atcaacatat 54000
   ttaagcattt ctagctgttt aatatcttca aacttatatc ctgtccacaa ccaaatgctt 54060
   ttattaggat aaagattttt aatagtttga actacagagt gaataacatc tcggttgtca 54120
   ggatagagag ggtcacctcc ggttatagtc aatccttcta tataatcatt attcaaacat 54180
   tcaattaatt gttctagtgt ttcaccagtg aatggaacac catttctagc attccatgtt 54240
   gatttattat aacacccttc gcatttatgc aaacaccctg taacgaaaag aacgacccta 54300
   caaccagggc cattaacaaa atcgcaagga taaaatctat cataattcat tggtgtttaa 54360
   ccctatgcat gatttcttta tttttaccga gattaaatcc gcgttcgttc ggatttccca 54420
   aatagccgca tgttcttctt atggtgttca tctttttagg atcagtttct ccacaaactg 54480
   aacaaacaaa tccgttttca gtaggagtca tttcatgagt acttccacat gtaaaacatt 54540
   tatctactgg catattaaca ccaaaataat ctaaatgttg tgcagcataa tcccagacag 54600
   cctcaagtcc ctttaaattg tttttcatat ctggaagttc aacataagaa atgtgaccac 54660
   ctgtcgcaat gaaatgatat ggcgcttcac gagaaatctt ttcaaacgga gtaatatttt 54720
   cttctactga aacatggaaa ctattagtgt accagccttt atcagtgaca tcttttacac 54780
   ttccatattt ttctgtatca agtttacaga agcgatagca aaggttttca gcaggagtag 54840
   aatacaagct aaaagcaaaa ccggttcttt cggtccactg cttaagacga gcattcattt 54900
   tagttaaaat ttcttgtcca atatcacgac cgacaagaat attcaattcg tgaataccga 54960
   tgtatcctaa agacactgaa cttctaccgt ttttaaataa ctcaattata tcatcgtcgg 55020
   gtttaagacg aaccccgaat gcaccttctt ggtaaagaat aggagcaaca gtcgctttaa 55080
   ctccttttaa ggaactaatt ctacacatca aagcttcaaa gcacagatcc attcgttcat 55140
   tgaataattc aacaaatttc tgttcattga actgtgttcc aatataagaa tctaatgcga 55200
   tacgaggaag attcagtgtt acaacaccga gattattacg tccatcaaga atttcattac 55260
   cagtcgaatc tttccatacg ctcaagaaac tacggcagcc cattggagaa actggaactg 55320
   aagaaccggt aatagcttta ttattcttaa cagaaataat gtcaggatac attcttttgc 55380
   ttgcacactc tagagcgagt tgcttaatat catagttcgg atcgtcttta taaagattaa 55440
   cgccttcttc aacaaacata acaagcttag ggaaaatagg agttattcca tcacgaccaa 55500
   gtcctttaat gcggttttta agaattgctt tctgaatcat tcgttcagtc caatcggttc 55560
   ccgtaccaaa tgtaattgtt acaaaaggcg tttgtccgtt tgaactaaag agagtattta 55620
   cttcatattc ataagcttgg aatgcgtcat agacatcttt ttctgttttg gattgagcat 55680
   aattcagaat gtctggtatt tgccattttt ctgcatcttc aacatgttta gcgtatgtac 55740
   gtttaacata aggagaaaga actttatcga cattcgcaaa tgtagttccg ccatactgat 55800
   gtgaagcaac ttgtgcagta atttgtgcca taattgcagt agcaacacca atcgatttag 55860
   gcgtttcaat ttgagcattg cccaatttaa atccgttctc aagcatcccc tttaaatcga 55920
   ctaaacagca attggtaaac ggaagagcag gagaataatc aatatcatgc atgtggataa 55980
   ttccactttc atgcgcattc ataataaaag acgggaccat atttttggca atgtgcttag 56040
   acacaatacc agccataagg tcccgttgag ttggaaaaac acgagaatct ttattagcat 56100
   tctcgttcaa aagatcttta ttagttttat aaattaatcc ttcaatttct ttttcaattt 56160
   tcattttaaa ctctttctaa gctgcttttt gaatgaagct attaattgtg ttttagtatc 56220
   agattcatta tattcaaatc ctctttgaag catctcagtc atcatttcct ctttacctag 56280
   tcttgagaac tctttagttt tgtctggaac aaaattagga tggatattat tatcagaata 56340
   atcttgtttc aaataagcaa gaagagcttc aagccattcg aggtagtcga catcacgacc 56400
   tttaagacca gaacggttga acttatgctt catttggcct tctgctgcgt tgcacagatt 56460
   acagagtaat ccacgaactc taccggcttt tggcccgttt aactcgtggt cgtggtcaag 56520
   atggttacct tgaacatcag ggtctaattc acggtggcaa ataggacact taccgttctg 56580
   tgcatcataa aatttttgtt tttcttcttt atattttgcg cctgataata acatgataaa 56640
   cccttacctt aaatagataa gggtatttat aatttagtaa atgttataaa agttacgatg 56700
   agaacgttta gctttgcgaa gctgacgttt agtaggacga actgtaaatg gttgtccaga 56760
   gaaaaaattc ggatcggttt taataagcaa ataccaatca cgcttgccat tttttaataa 56820
   acggtacaat aatttaactt gcataataat ctcttcccat ttccatatcg attaattcat 56880
   ttattgaagg ttcatcataa catggttctt cgtcgttgta aactgtttcg acgtctggct 56940
   cgacagtctc ccatcgagca gaataccacg gtttaacccc ggagtttatt gagctcttca 57000
   taatcaatcc aggattctag ccctgacggc aggtcacatt caatgttcca cagaagctct 57060
   tcaatggctt gcaggcgatt atattcttca acggtaatgg taaccattgg tgcaaccggt 57120
   tctgcctttt taaccatcat atcgttcatc aggacgtaaa ggtcatcacg aacgacagtg 57180
   tcgttccaac cccatgattt ggcttcgtaa gctaatgatg aagggagcac atttaaaact 57240
   tcaataatag ttggagcatc aggaccaagt tcattggcac aaacttcgta tacatcttgg 57300
   aaatctttaa gatttaatga atatgtcatt taaaatattc tcgcagttta ttgttctacc 57360
   caggcacagg aaacttcgaa atcaggacca ccgtattccc agtgatattc accaacagga 57420
   atccagtcac cgtgctcgtt ttcttcatag tcctcagcct caaggccaac gaatcgccat 57480
   tggacaccga tgattcgatt acccattaat atatgaaatt caccccatgg attatcgata 57540
   gcatattgga tatcgttctt agcacgagaa cacatttctt gatataacaa agcttctaat 57600
   tcgccctgat cataagggcc cgggaaagtt aaatcaatac tcatttaaaa tattctcgta 57660
   gttggtcaaa tccaccaata tgacttccat caggagcaaa tacttgaggc atagtcaatc 57720
   caacttgtga ttcacgtcca agcttaacaa gaagctcagc aattttctca tcatcaaaaa 57780
   cacctttttc tggcataaca ttaatgaatt cgaatggctg tttcttaaca gtcagcaatc 57840
   gtttcgcatt atcacaataa atgcatttat gaatagtaga gtcatacccg tacactttaa 57900
   acattatttt tcctttcatc gattaaatat gcaatgccaa atacaataca tgtaataaaa 57960
   cctgtgatac agatagaaac caacaacact gtaccaaaga accaaatcaa gttatcttta 58020
   gtagtttgtt tgatatccat ctcacgaatt tctatagcta tcgtatcacc tttattcaga 58080
   agcgtataaa acgacggtga tacacgcctg tcaaaaactc gtccttgctc atctttatag 58140
   actgcaataa attctaattt agaatatttt cctgtagatt ggcctgagat taattccact 58200
   acctccacag gaattacttt agtttccatg taagtattct gactgttcca aatgaatgga 58260
   ataacggcaa aaactaaagc tgcgataaaa caacgaaggc tatatctcat tataatattc 58320
   tctcaaattt gtggaagcat ttacggcatt tcaatttcaa gttatcagtg gtccagttaa 58380
   ccagttggac ttggatagtt ccacattccg ggcatggatg ttttgctgct gatttggcag 58440
   cattttcacg tcgttcaacc attttcatta tttcgttcca tgctggagga acgaactctt 58500
   cttcaccgtg gattttatct cgctgaacaa attcaatctt tgaactatcg tcaataaact 58560
   taaccaagcc atgattagtg ctcatatcaa tatcctctaa aagacgtagt ttcattataa 58620
   cacctcaatg gcatttcgta aaccgtccgc tttcgcattc agagctttta acgatttagt 58680
   ctgttcttta atctgggctt cgacttcttt cagacgagat ttgagatatt cacgttcttc 58740
   gctcaagttg tcaacttttt ctactttagg ttctttaata gtcttaagtt tatactcgtt 58800
   agggttcttc agcttaattc gggtgcgaga actgtataac attgaaccgt tatcttcgga 58860
   ttcgtcgaac gtttcaacaa catccatagt ctctaacaat gtaataagtt tataagacaa 58920
   tgaacgaacg gttttgttat gcttattaat ttcttcagct ttcatggtac cgagattacg 58980
   tgagtagtaa taaggaacct ggatatcaat tgataatgat gcgttaccat taccggtcgg 59040
   tttgaatact gcagtaaccc aatctttaac atgaacttta tttgtacgac catcaccgcc 59100
   cgtgaataat ttacgagaat gtttaaaaag catattcact tggttattga attcacaagc 59160
   aaatttatca gcgaatttag tttcaggaat ggtagagaac caatcagcta aatgagtctt 59220
   tttaacatca ttaacaatag tgaattcaac caaatcagtt ttagatgtca cagatttaat 59280
   aaagctcggt ttcataaagt ttttaatgtt atcgcgagct acagtagaag ataataaacg 59340
   agaacgttta aaccattgaa gcagtgtccc taatgaatga gaatacgcat tctgagtatc 59400
   tttacagata tgattttctt ggcctgtgta tgtaccaaaa tcgcgaagaa taccacttac 59460
   tacatctcgg tgtggaacat caaatcctag tgcaccaaca gcgcgctggg catgaacgtt 59520
   agcgataaga ccggcaaagt attcaacgta atgtgaacgt gtcataatat tttcctctcg 59580
   ttaagataga tttattatac tccaaccatc cttggttgta aactgattta tgattttaac 59640
   gcattcattg cgttaaaaag tttagagtac tgcaactgga gattcgttac aacactttga 59700
   gcatcacgaa gcttagcttg tgctttgtca agttcaacgg acagttggtc tacagtagcc 59760
   ttgatttggt tgtaatgata atcttcggcc ggaagaaggt cattagaaac atttagtttt 59820
   actgccgaca gtttgcacac atttggcaaa cctttaaaca taccgggttg tgtcggagaa 59880
   tatgcgctta gaacctctgc attaccgact ggtgttgctg tgatattgcc ttcgaacgtt 59940
   gcattttcct ttttaagatt ttgatagtat tcagagtctt tgtgccacca tggcgcagga 60000
   acctttttac caatagatac acgagcctgg atagcgatat ggcccttatt cttgataggg 60060
   tcgataatat taagctcacg agaataaatt gctttaatct ggtagatatt atcacctaaa 60120
   acttctaacg cttggtggaa tacacgagca acgttatcgt tcttaggttt atacgtgaga 60180
   gtgaactcgt tctcactgat aaccatgatt tccaacttat cgtaagtata tttcaaatgt 60240
   ttcatgttaa ttgcttgagg acgaatacga gaagcaatgg ccatcagatt gttaaggtta 60300
   ttccggaact gcttagcgaa atattgtcct tcaccgccaa tacaaaaatt acgaagtttc 60360
   tgtttggcta ataaacggct gaactcacca tttttaatga actctttaac gggcgtgtta 60420
   gtaacacatt caaattgagt aaacatacga gaagtaatcc cataatacga acagatttcc 60480
   tcaatctctt caggagtctt tacaggcata cggataggat taggtcctgg agtatatgcg 60540
   ttcgaggtaa attctttgat aagttctcta cgtgtaatag ccattttata ttcctcatgt 60600
   tgaaaaattt attatactcc aaccatcctt ggttgtacat cattccttaa gaagttttac 60660
   tacttgaata agattagctc gttctgtttt atttttaccg tttaagcctg ctaatcgtga 60720
   gatttcatcg tcagtttgtt gaatagttac attaagttca ttaatcaaag cttcaaagta 60780
   tttgatttgc tcagcatgtt ttgattcaaa tttctgaacc ggaataacgt cttggttcag 60840
   ttcatcaata aggtctttaa gagcatcttc ttggttgata tcatattcaa cttcttttat 60900
   ttcttcaacg agctcgtctt tatatctaac gtcttttagt cctggagctg gtcctgaaat 60960
   ctcttcttta agagtacaaa catacccaat acggtatcca atgccgtctc gcttgatatc 61020
   aacttcgtcg ataaatccac aatgtttaag accagagccg attatgtcaa aatatccttg 61080
   acgtcgtcct tcaaacgtta cacgaactac taagacagtt ttaccaccaa ccttttgaag 61140
   ctcacattta taaaagtctg ttgaacctga ataacgtgga tgagttttaa agttcatctg 61200
   acgatctagg cccatttgta aacgtgctgt tacggccata aaacgtgttt ggttctcgtt 61260
   aagatgagca acaatccatt tcaaaggcat tttacactgt tcatgaccga acaaatcagt 61320
   acgtcctaga ttacgcagga tattgttaga gttaatcatc gacagaacgt taagtttatt 61380
   cagcgcgctg aagattccat cagccattga ttttccataa cctttacacg ctttaataag 61440
   tgctcgcggc agattagtat agatttcaga cggcatatga aaatcaaatt tactagtcag 61500
   taattgatga acttggatag caatttgctc ataattaagt gctctgcgat cgttatggtc 61560
   tttaaaccac actttaatct gtgaagcaca taaaccgtta atttcatgtt ttaccgcatc 61620
   agatgtaatg ctcataacta tttcctcaga ttgaattgga actcaaaacc attataaccc 61680
   gtccttgggt aaagcgttta tgcgtgaacc gtctttaaac gttcttcaaa tttccgaaga 61740
   gtactaatac gttctgcttt ctgttttgta tagtttgcaa tacgttctga agtgagagcg 61800
   tatcgttcgt ttactgtttc tttcatgaag tcagggattt cacgagaagc tttaatttca 61860
   tcaaacttgt cgagtactgc ctggtcttca gcgattaaat tttcatacat ctcgatatct 61920
   ttcttgataa actcgatatc ttcctgagtt actttggata aacgagatgc tttatccttt 61980
   ttgtactgct cgttagtttc acgagaccag aataatgtac gttttttgtt agtgtttttg 62040
   tagacttcca caattccaac tgaatcgatg ataacaatcc aattccagcg agatttgaaa 62100
   atctgacctc catccacggt aatctcgtta ccataagcaa cgtcattaaa gaacttgctc 62160
   tgtgcttcag atttaaattt accgtcgtta taatttacca ggttgaaaat atctttagcg 62220
   ttcatgtttt acctccagta gttgataggt ctataataac ataaccatag accttgtaaa 62280
   caactaaaag aaactttttt cggtattttt tccactgaac caaacagact tcgggatttt 62340
   ttgtaatttt tcagatttaa gtttctcttc ctgttctttc ttatatacac tcttgtcgta 62400
   ctggagcatc tctatggcat tcttttgcaa atgatctaag gtaattaagg ctttagtttt 62460
   tcctttcaag gaagagtcct ggcgaacttc ctcgctatat ttcatcgtag aactaaccat 62520
   acgatgtgca tagagttcga ctgcggcttt aattttttgc ataaaatcca taacatatcc 62580
   tctttgattt gtacatcatt atttttaaat atttctatgc cctcagtagg ttcctggtgt 62640
   ccttctttga ggtaagccat atgattattc atctttatgg cttttgttac ctcagaactt 62700
   cctataagct tatttcagta aagtaacttc cgagatttct tcccaaccat ctttatcagt 62760
   tgaaataaaa tctgctacat aaagagccgt acgtaatgat acattgcgaa gacgagatac 62820
   gttagccttc atccaggaca atgctttata cgtttctgca tccgtcagac cacgtttctg 62880
   catcatgtca gtagataata ttacatcttc aactcgaacc ataatctctt cattagagtg 62940
   tactccaagg tcgagataca ctgaacgaga tactaaagct tgaaggtgtg gagcaagttt 63000
   cgaaccccgt tctaactctt tatcgatatc aacgtttgta atgaaaacga tagtaccttc 63060
   aaactcaaat tctttatcaa tacccttatc ctcgagataa gatgacgcag tgctccaaca 63120
   aactttgcgt ttatctccgg tatctaatgc tgctttaaga aggttcagga tgtccatatc 63180
   tgagaatacg tcaacgtcat cgattaagag aacgctgtct tcatcacggt tgagccaaag 63240
   ttgttcgtaa aggccaatac tggagatttt accattaatg cttttgtatt tgatgaaacc 63300
   attatcatta gcattattaa gtgctttatc cagagaatac gtcttaccaa taccagctgc 63360
   acctgaaata atgagtgaac gaattttgcc atcaataatg ccattcgtca tcatgttcat 63420
   tactttaaag cgtttgttga tacgagtttt catatcttca gtgctttcaa caactgcaac 63480
   tggtgacgtc tcaactcctt ccattacgat gtctgatttg aagacccata ctccacgttc 63540
   agaaccatca atcataacga aaattttacc gtcacctaag tgggaacatt caggattaag 63600
   ctttgccggg aaccatgtct ttgtggcaac ataagaacca gagatttcgt tattacgata 63660
   gatacctttt ttgatttgaa cgttgaacat tttattctcc aaatttactc atttcgtttc 63720
   gataggtcta taataacatg tttaaagcaa aagtaaacaa ttatttgcga cgagattttt 63780
   tggtaagagg ttgaaagaac actgctttac cggcaggagt caaagtgact cgtaatgcat 63840
   taccgattcc caaggatgct ttaaccagac ctttagattt cagttcaccg aatgcttgac 63900
   gttggtctgc attacgagtg ttaaccataa gacctttatc accagcttca cgaatcattt 63960
   ctaacatttt ggttttcatt ttgttttctc ctcttgtttt gataggtcct ataataacat 64020
   atcctaaaag catgtacact actttttaaa aatattttga ccccctaaag ggcccgaagg 64080
   cccttatcat ttatagtatg aattgtggtt ttctaagtag acttgctcaa ggaagtcatc 64140
   ccagaaactc atatctactt tttgctgcat accgttctta gcggcttgtt ctgcggcatg 64200
   ttcaacttgg tcaacgatat cttctaagaa ttcctgtacg gttttaaacg tatgtttacc 64260
   aagcttaaca tcgagaataa atggagcatc ctgaagcggg taaaccaggt cgccggtttt 64320
   gtaaatttct aagagctgaa gtccaccacg gcatgcatga ctcaatgctt tccaatcaat 64380
   accttcgtta gcttctgcct tacgagcacg ttcaccatag ttctcttcca gtttaaccag 64440
   gatggcattg aactctttat tggttaacgt gtcttggaat ttacggtcta acagattata 64500
   gaatgtttgc ttacctgttt tctcgttttc ggtttcaatc cacgaagcaa attcacctat 64560
   aggcagaaca ttcttcatat gtgataaacg aattactgca ccgttgacaa agtattgtcc 64620
   ccattcatta gaacgtttaa gggcttggcg gagaacagcc agacgagaac ccttgacgcc 64680
   atatttagaa gcttgcttac ggacataccc taaataagat ttcatgttag tggtatagaa 64740
   acgagatctg ttatcttgaa tatacttcca tacatcaggc aaatcagatt taactactag 64800
   actcggtgga gtgtgaagca tatctaatgc gacggtttca ccatctgcag ctagtttaaa 64860
   gaaatatttg agactataca actcatggtc tacgtcgtct tttgtgttct tagaagatgt 64920
   gttattcgtg ttcttgctca tatgttcttt aacgttaccg gtcaaaatat cacgagcatg 64980
   agggacataa atttctttaa aatcaacgtc agattcagga gtagacgttc cataaaggtg 65040
   gctaccaaag taagccttaa caatcgtttt cattatttag ccttatattc aagagtagga 65100
   cagacttttt ctttacgagc tttaaggtat tcacctatta atgatttctt acgttcaata 65160
   ggctcaagtc catttttaat tcgttcttgg ttttgcgcaa tgagctcaat ttctttataa 65220
   tacttccgct ctctatataa gtcgattagc cacggtatgc ctaatactaa aactatagaa 65280
   ataccaacac ataatgcaat gaataaaaca cctacaccaa ttgaggccgg aaccaatgac 65340
   cataaactac taaagacaaa gaatttagtt aaaatttcat ggccaacgat tgtggccgct 65400
   gctaaaatcg caaatccaat aatcgaaaca aacaataccg ggagaaacgt tttccaaaaa 65460
   taagcacaca acgttttcgg acgattccat gtactattaa acgcggcata aattttaaag 65520
   tgccaagagt tttcattgat aatcataata attccttatt tcaaagttgg gataatagaa 65580
   tcgagataag ctttaagagc aactgcttca tctttgtcta atgtaacgat atgttgacga 65640
   aggtcatcaa tttggcgaat aactacaaca tcgccatctt cataagaagg attaacgcct 65700
   aggaccgttg tatagtcagg gcggtcctga tttgtaataa aagcattacg gcatttagcc 65760
   cattgaagtt tggccttttt ggctgtttca gattccatta attctttcac cttagtgacg 65820
   aatgttttcc aattattaat agaaaggcat tcatcttctg tttcgatatt aaccccgcta 65880
   taaagaccgt gataaaaacg aacttgaagg tcttcatcga tgttaattac agatgcatgg 65940
   ccgggcggat tgagtttcaa aatcttgtcc agttccatag gaagaatagt aacagaatca 66000
   tcttcttgtg tcagaacata cacataacca cgtttttcga tacataaatc accaaacaac 66060
   tcggttgttt caattacaat tttcatatta ttcaccaatt aaagtttcaa caaaattcac 66120
   caaatcattc agacaagcat caacctgttt ttcataaaag tcttcgttat catttactgc 66180
   atgataagct acatcattca aatctgaatc ggcttcacgt aaatcacgga taaggtccat 66240
   caatttatta gcttgttcac gggtcatttt accatactcg tttcaataag attcactatg 66300
   ccgtttacca tgttataagt aaacatcgga taattgtatt gtactacaac ataagcgata 66360
   agcaaaagtt tcattttgtt ctccgttcgt tttggtagga ctactataac ataatcctac 66420
   caagatgtaa actaccgatt gtgttttaaa tgctgataaa gttcttcaac ttgtttaggg 66480
   tccaaaacaa ttaggtcatt ttcttggcga agataaatcc atcccttatc accagggcca 66540
   tttttacaaa acatcaccac catatcatca tcttcagcgc tttcaggtaa aagttcaagt 66600
   acaccgtctt cagattttcc agtataatcg taaagcataa tatcctcaca taatctcgtt 66660
   aacatacgtt tgcaattctg tgtgggcgtc agccatcttt ttcattgcct tattgatatc 66720
   aatatcgcca tcttcgcacc catgggcatt ctcccaatca gatacggcat tagcgtactc 66780
   ttcaatcaaa aacattaact tagaaatttt ttctttacgt gacataacgt tctcctctag 66840
   taagtaaaga ctactataac acaacctgcg gagaagtaaa ctgatagata caaaaaagcc 66900
   ccaaccataa ggaaggggct agtaaattag atagatgtag gctctaattt tattttcatg 66960
   aactcagcaa attgtctggc agtttttatg ccttgttcct ggcattcaaa tgcatcatca 67020
   caggggctat ttttgtaatg ctttgccata tacttataaa atttttcaga attctcaaca 67080
   ttttgaattt cgaacaaagt atatacgttt ttaatccctg attccataaa gttatcaaag 67140
   tatgggtcaa catatgcagt gccaacaata ggagtcgttg ctgcaaaaaa ccccagcatc 67200
   gccacaatgg ctatcgctct taaggccata taggcctcct gttaattttg gccagtattt 67260
   atttttcgcc tttggacggc cttaacatct gcgaggtatt tttccagctt tcgcttgcgt 67320
   gtataatcag ctttagaacc agtttgacga atatcctcgt ttaaatgaga aatagaatgt 67380
   cctaattctc ttcggatttc gtccaattgg gttacagtaa gattacgaag ttgtctttcg 67440
   tttaattgct gcatatatat acctcttggt tagatattag tatttataac aaccctgtca 67500
   aatcgttcca atgcttccgg cataatgaca cgtatctatc ggtatcacca atttcaattt 67560
   gtccaccatc tgtaattgga agtccatctt ccgtgactct agctaccatt gtggcttttt 67620
   taccgcaatg acagacacct ttaagttcaa caagcttatc ggcaatagcc attaatgctt 67680
   gagaaccttc aaatagatta cctttaaaat ctgttcgtag tccgtatgcc attaccggca 67740
   cattgtatac gtcaacaata cgactaagtt gatacacctt ttcagtagtt aagaactgag 67800
   cttcgtcaac aaatacacag tgaatatcct tttgtgctgc agcccattta taaaattcaa 67860
   agatatccat gtcttttgta attatattgg catcctgacg aataccaata cgggagacta 67920
   tttcaccttt tgaatcgcga gtatcaatag ccggtttaag aactaataca cccatgccac 67980
   gttctttata gttatgtgcg gcagtaagta atgaagcaga cttaccagcg ttcatagcgg 68040
   cataggtaaa aattaaacta gccataagag tcccttatag tgaattcacg tagtcaatga 68100
   gttcttgttc tttcctgtca aatgattcca tcaatgcttg atattcttcg tctgttaaag 68160
   gagcatattc atagtacgca ttagtggctg cttgctcatc caataggtca tggataagtt 68220
   cgaacagttt agttttctct tctttagtca atttcattta ggttcacctt ctttaacgaa 68280
   atctgggtaa gggtaaaatc ctgagtaaaa cccttcacct ttatgattgc tacaaccttt 68340
   gttcgaacag taacaccaat aatcaaaatc acaagccatt tcgtcattgc ataaagcaaa 68400
   aactacaggc cattcgcatt tttcgcattt agcacctgta agaatttttg tttgactcat 68460
   aaccatttga cctgcatgca atattcttcg acatgctttt tacgaccttt cttatcaata 68520
   aaggtatatt ccataaagcg tccatgataa cgacgttcaa catcgtgagg ggtgttaata 68580
   ggacacttag tctgacgaat ttcttcccat tcacgaataa taactgcttt attctttggg 68640
   tcatacgggt gaggataatg cacattcata acaatggttc ccaatcaaca atcacaattt 68700
   ctagtttagt ggaatacgta tctaaaatct cttcgatgac atcccagttt ccaccaccaa 68760
   tacctgcacc aattcttggc atataaatta caggctttgt tatcttattt tttccgaatt 68820
   catttaattc tattatacaa ttcattaaag cagagtactc aaaattaggt cccggctcaa 68880
   actgtgtata aagattaaag caataagctt tattggtctt aaaatatttt tcataaaccg 68940
   aataagtccc gagtttagac tcgtctccga attcggtttg aagtttatcg gcttctaaaa 69000
   tttttggata agcctttgcc aattgacctg ctacaccggc acccattgta tgaaaacaat 69060
   tgcacccatg agcaatatta ttacctttag aaaacagggc gacaatatcg cccttgatat 69120
   agtttttaat cacttgctac aagtcctcta gtataagagt caactagacg gtccaccata 69180
   gaattgcatg ctgccttatc tttaggttcc gccaccgagc attctaatat ttgccatttt 69240
   ttatagtatc ttttaattaa ggtagacgcc ttgaacctac tagacgcccc atctttttct 69300
   ccgtctttat acgcatataa caatttttgt gaaaaatcta ctttacactc ttgactttta 69360
   ccacaataat cagacgccgt tctgtttgta tactccgtta catcagtata tgtagtatta 69420
   gcaagagcgt taaatgaaac caagccaact attaaagcta acattttggt cattctttta 69480
   tttccagtag tttatttgat ttaaggtaat tagccttttc taggacttca gaagcatatt 69540
   tacttccagc acttgtcttc caccccgcat tatatgagga tatagctttt cttatatccc 69600
   cgttatgtat atttaaccaa taagaaaatt cgatataagc ccatctggcc gaattagacc 69660
   gcttagatag cattttctta atttgggcat cagacatatt ataaccaagc tggttaattc 69720
   ttgccctcat tgttggtaga tagttttgga acattccata cgcatggtga cctttcttat 69780
   ctttaagatt aataccggca gatgattctt gccatagtat tgcggccatt atgtatccaa 69840
   gtcctttctt atgcatattt tcatgcgttt taaattttcc gtccttagaa aactgttccc 69900
   caaactgata agcataattc aagttatcga gttggacatt actaaaagta tgctcggagc 69960
   tatgtgctga taacgaaaga gcaaatagac cagcgagtag tgcttttctc atgtttacct 70020
   ctagatttta atcactgctt tagaagcttt tcctggtagt cgtttactgt taataatagc 70080
   cattctacat tgaagagtct tcattttgaa tgttggattt tcgcgacaga ctgtaagtcc 70140
   taaccaaaga tttccatcag ttatttctaa acggcctggg cgatattcga caccttcgac 70200
   aaaatcttca tcaacatctg gacgtgccgg cattgataaa aattctgcaa cttctttaac 70260
   atgatttttt actttatgaa ataattcaaa aacgtaattc tcatctattt ctctctggat 70320
   ggcacgatct aagagatgtt gagaatactt gatatgaaag cctggtagtc caagagactt 70380
   tgcagcatct tttataccgg catttatttt gcggaactcc gtttcaaaga ttcgacggag 70440
   tttattacgg cggataaaaa cttccgaatt gatagtcatt ttgatttctc ctctagttga 70500
   caggtctata gtaacacaac tagaggagaa gtaaactact ctttgaaagg aattaacttt 70560
   ttaactgctt tagatacttc gtcacgaacc tgatggaagt tcttgagctg ttcaaggcgt 70620
   tgttcgtaat acgcaatctc atcttcttca agacaatcgt cagcgtcttc tttcagatat 70680
   gccttgtact cattaaagag ggcccatagg cggttctcaa agtcatcaag gctttgaatt 70740
   ttcttgtgct tcggaacaag gccaaaagga ttgctttcag ttatgcgtct agatgcttca 70800
   taatgagttc caaagcctga taggatttga gccattagta tttttcctca ggtcgaaata 70860
   ctgcacgaca ggcccacatg ctggcttctt ttagatgttg cttagcatac ttaagagcct 70920
   gtactgcatc catatcttct ttggtttgtt gctctgtttc attttctttc aagatatatg 70980
   cttcttcaat cagagcatca aacaacaatc caagacggac ttctgcatct ttaatagcgt 71040
   tcactttacc gattttatcg tcggtatgtg gcttgtagcc tttaatatct tcaatcgaca 71100
   taaggaattt cctcatcggt ggcaaggtca tattcgacca attggatagg ctcattaata 71160
   ccatatccac gtacagtgat atcagttgcc ggatagtttc cagtaatatc acccattcta 71220
   aatgcttcaa tgaatgattg ctcgcctgag caactaaacc aggcggcaaa acattttaaa 71280
   acatcttcag acccttctat aatcaattta gccattatag actctcagta aaggtacgag 71340
   cgataacgtc acgctgctgt tccggagtca gagagttaaa gcgaactgca taaccggata 71400
   cacggatggt cagctgcgga tatttttccg gatgcttaac tgcatcttcc agagtttcac 71460
   gacgcagaac attaacgttc aggtgttgac caccttcaat tttaacttta ggttgatgtt 71520
   caatttcaat ttcacgggca ttcaaaccgt agaaaatttc cgggtctaca atagcgtctt 71580
   ctttaaaagt cttagaggca ataatccggg cttgagtacc gtcttcaaaa taaatagtgc 71640
   ctttgtgtgt gccttcaaga atttgatatg ctttcatata acctcaatta gaaaataaat 71700
   ttatccaagt tttttcttta atcaaaaatg ggtcggaatt aaacgccatt aaactttgag 71760
   tgattaatcc cttaaatggc ccatcaataa attccatttt gaaatgcgga atactagtca 71820
   taaaccgtgc attaggagca gtacaaagaa cctgctgccc tgcgaacggg cctttagtca 71880
   aacgatatcg tttaggttta aaatggtaaa tctcataatt ttctacaaga gcccttttat 71940
   tgcatactgg gctgttataa caatattcaa aaacttcaat agtggtcatc attgcattcc 72000
   aatcacaaaa aattgaggac gagcattacc gccgatgcaa tatttgcttt tgcaacagtg 72060
   ttggactgtt ttaatatgga cattgtaaat cttatccata tcaggagctt taataggctc 72120
   gtcaattatg tacagaatac gagaaagcgt taatccacgg aatttagaac cttcattgcc 72180
   aataaaacta cggacagaat cggtaaataa acggaatcga atatcatcgc tagaataacg 72240
   agaaaattct tttttaatgt tgccagcaga aattttagca taggctgaag tattagaaag 72300
   aacaataact gttccaccgt catacagcca attaacagca aaattagtta ctgcagtcga 72360
   tttacctgat tgtctgccgc cgtctaaacg aagtgtacag tactgcttga gtacgtcttc 72420
   aaatggtggg gcataaccgt tcttacaaat ttcttctact ctagcatcag aatggtgtgt 72480
   aaaagcattc atcagggata gataaggacc agttaaaaat gttctcattt agtttctctt 72540
   ttaggttggg ccattccgtg gcgcatgaat tgtccatttc tgtatttacc cctctaagta 72600
   attatgaata tactatcaca attctaggag aaagtaaact gttatttacc tttaattact 72660
   ttagctgctt cgatagccgc ttgctgaagg tcatccatag acataccgaa tttagaagca 72720
   aagttatcaa ttttcttttc gacagcattc aaaggcttgg cctgtttacc ttcattagcg 72780
   ccaggaagag cgagaatacg ctctttgtca atataaaggc ttacaagttt cttacggtct 72840
   ttatcggaca aatcatgaaa tgaatgggcc tttttattta tagcggcttc taatttacct 72900
   gcactcactc gcgcttcagt aataaattct tgatatgttt tcattttttg tttccttgtt 72960
   gttttgatag agtaattata ccatattaat acttaagcgt aaacaattta agctttaccg 73020
   ccagaaataa ttccgaagtt gttaaaatcg tatccaatat cgtcaacatc tactttagga 73080
   tttactacaa cataagcaat ttcactacgt tttgcatagg tgttgatttc tttaccatcg 73140
   ccacctaaga aagaataccc attacgtttt aatgagccaa agtcttcggc atcaccaatg 73200
   aaagctttaa ctttaaattt aggacctttg gcatctgctt caatgataaa ttcttgatac 73260
   attttcattt ttccttagta aattgattta ccatagtatt tgtaccaagt aggacgttga 73320
   gcaatttttt catctaagcg tgcttgactt atcgcaacgg acgcttcgga cgggacataa 73380
   ttattacgaa attcggcagg gatgtcggaa atatcctgga ctgtcgtgtc cttgattttg 73440
   aaaccacgtt tcaagcattc agcgattagc tcgatttgtc gtaaacgtaa aaattcaagt 73500
   ttgtcgtaaa agaatgttac gtgacctgtg cctaagataa atgttggaga gattttaaaa 73560
   tcctttacac gtttaccgtt ttggacatgt ttacggactg cactaaatac gcgtggtaat 73620
   tcacgatact cagccataag atgttgatcg gtcagttcag aaacaagtgt taaattgata 73680
   cgagtcataa tttcctccaa gtagttgatg aatgtatagt atcaaaaccc ttggaggaag 73740
   taaacactat ttttctgaag cgcctgaaat atatttttca agacgttgtt tcatcagcga 73800
   gccgttctta gcgatttctt ccggtttatc gcacatacgt aacaaggctt caagcttagc 73860
   gatatcgctg gtacgttcaa atttgcgagt atctatctta ctagcaagtt cagaaatacg 73920
   tgctaataga ctttcggctt caaccaaagc tgcacctagc ttagttgcaa atgtgtcagt 73980
   agcttcgtta agaatagatt ctgataaaaa ttcactatat gttttcatat atacgctttc 74040
   caagttcctg ttttaaatgt tgagattacg cgtttcgcgc gattaggtgt ctgacgatac 74100
   catttagatt gggcgagatt tactgccgct tcatcccaac gtttctgttg aagcatacga 74160
   agagaattag taaaaccagc aacgcctgct acacccattt ggaagaccat attgaccaat 74220
   gcacatcgac gaaccgcgtc taaagaatca tataccggtt taagtttagc attacccaag 74280
   attccgcgaa cggctttatc gacgtcttca ttaaatagct tttcagcctc atcaagggta 74340
   attgtaccgt tgcatttacg accgatcatt ctgtcaagtt cagctttagc tacggctaaa 74400
   gacgggttct ttgttactaa atggcctatg ccaatcgtcc aaaagccttc agtgtctttg 74460
   tataaagtca gtctaagacc ttcgtcatta cgaagcattt caaatatatt cataatacct 74520
   cccacgttta taggaggtat ttattactta taggatggta gtgagcacct tatataatga 74580
   tttgcccatt acatattccc attgagaagg tttaattaaa gcgaatgcat caaattcagg 74640
   aatctgtcgt ccgtccggga atgtatgata tgaattacaa acactgtttc tgaattgctc 74700
   gtgctctaca gggatggtat ataggaacag atgaatattc ttattgtctg agtaatgatg 74760
   ttcacctagg tctttcagga aggcagggtc atattgtgta aacccagttt cttcttcgca 74820
   ttcacgaata gcggcttcga taggagattc gccaggttcg acacggcctt taggaatatc 74880
   ccaccgatga gctaatgcac ctttaggacg agaaccagtt acacgaccca tgaataattc 74940
   tttatctttt gtcatgaaga taataccggc tgatagttct ttcactttat ttttcttact 75000
   catcatctcg gtcccatgat ttggccatta attcggacat aaattcttca atacgcgttt 75060
   ctttagatgg actttcacta ttagagaccg caagtttgta atctgcaata ggactgaaca 75120
   aatcaaggtc catattaaga tcgtctttga ttttttcttc tgacacatct ttatcatatg 75180
   tgtacagata actatcacat gattctgatt taatgtaaat tgctatactc atctcaaatt 75240
   cctgcattcg gtaataaaac gttcaacttc attattcttg gcctttatct tgtctttacg 75300
   ttcctgtaaa ctacttttaa caagattaat aatgatgcct atagtacctc caccaataat 75360
   accaccaata atacttacga tagtagatgc taataagata gcgataatat ctggtatggc 75420
   aaaggcactt actttagtac taaaaaacca taccgacggt acaaatataa tccatcctgt 75480
   tatccattga cacttattga aaaacttttc aagatcagct tgagttgttc gtattacttc 75540
   atcgataggt tgatatccat taatgtatct gttcacgata atacctgctg gctggtcgat 75600
   tttaaattct tgacaaatac gtttcactaa gtaggatgga catcctgtcc aatctactga 75660
   atagtatgaa ataccttcat tgccgtcgtc aatattagac caatatgttt tgttaatcat 75720
   taccagcctt ccatagggaa ttccagataa acttgggcat tgacacgaat ttcattggta 75780
   atctttttaa ctcggtctgt attacgagat acacgtccga accaacgcca tccattacta 75840
   attgcacgag aaccagtgtg ccatgtttgc caatcaaact gaagaagagt acggtctgga 75900
   gcttcccatt tctctagttc acctttttca attttttcga ggacttcttt gtgccattgg 75960
   cgatagataa gttcgccatc aggaatctga ctgaactctg cagttcctgt tgcaaaatga 76020
   gtaggacaca cgtcagcgtt aacaagacca agaatatgtt cagaatggta acgagggtta 76080
   tcatagtcag gttgtcctgc agtaataaag tgttggccga ctggaatatc agggcgagga 76140
   acatcgtcat ggtgatatcc aggaatagca ggataccaac caggcatcag catatgaacc 76200
   cgagaatcaa atacaacatg ttgattagtc caatcaattg ggagattggc aataaagcta 76260
   cgagtaatag gaccgccgtt atcccaagca aaactcaaat cacagttaaa gaacattggt 76320
   tcatttttga tttggtcatt ttgtacgtta tgagcaaaac cacctactgc acgagcttga 76380
   ctattaaaca ctttagaact attcatatta ttttcctcga cattctttaa tgaaattttc 76440
   tagattggta gattcaatag cttttttacg agcttctgcg tgttcttctt cagacaattt 76500
   acatttataa ttaacccaga cgttatggtc aaaactagtc gtggcatata ataacaaagc 76560
   cccgacgaat aataaaaatg gtgataagaa tacaaaacca ggaacaccat ccatatcgta 76620
   agccttttgt aatactgcaa cagaaagagc tacgtaaaaa agtgatacaa atatagttaa 76680
   aagaccaatt cggttggttt cgcctaattt cataatattc tcctcatgtt ttaataggac 76740
   tactataaca tagtcctata attgtgtaaa cagttttgcg aaattatttg aaaggtttta 76800
   ccatatggaa attttgttta aaacgtttta cataatcgtc atgtcctaag ctttggtaca 76860
   tacttacaac tttgctgtag ttttcccaag cgtattcacg gaaccagcct gacgtgattg 76920
   aagtacagac tttttctaat gccatcataa aactatcacg tggatggatt tcaaagttat 76980
   taggaatctg agaacgttct aacgctagta cacatgattc ttcgtatacg ccggctaatt 77040
   tgaccatttc tggtaatgat tcgaacttct cgcgtgaggt catgacttca gaaccatctt 77100
   tcatataaaa tgtatacgct ggacggtctg ttagtgcaat ggcttcgtga atagtatcat 77160
   ggtcatacgt atagatgtca tctttaaaga atgcgctttt agaagtgtca agaactgggt 77220
   gagcatagtt caaggtttct ttctggcgtt tcagcataat ttcagtaagt tcatcgttca 77280
   gtgtaacacc tttaccacga agatactgaa tatggttcat agtctttaaa aagaatggat 77340
   tatttttctt gaaacgatga gacatcttaa ttgcaagaca catctcagga gttgcccagt 77400
   aaaacccgtt caaacggtct ttagtcaaat ttttcttggc gtattttaaa aggtcataag 77460
   aagacgtgaa atcttcagga gtgagttcac cgactaattc acggacatta gtgattgccg 77520
   gaacaatata tgcttcaaaa tgagtttcgc gtccattatg gaagcatttg aaaacttgga 77580
   catcagggtt atcttggtga acatatacac ccatcatttg atttttgaat acattccaag 77640
   aaccttggtc cgcaataaag tcccagtcac tatttttgat agcgctagaa tcaataagac 77700
   cgtgatagtg aagtgcacga gaaccaataa ctaacatcat aatattttcc tcgaatttag 77760
   attacttaaa tttcgcgaca tgcgatatta tattcatccc agaggtcgtc atgtgaagcc 77820
   tctactaatg gaacgattgt gccgtttatg tcttcattat aagggcaaat tgtacagtgc 77880
   gcatcttcga atcggatgcc tttgtgttca gattttttat aacgcatatc gaatatcttt 77940
   gcaaactttt cacgagtaat agttttagca actacttcgg ttcctatact ttcgtaataa 78000
   accattgttg tctccattta atttgatagg actactataa catagcccta tcttgttgta 78060
   aactgtttta gcaagaataa ctagatgata cccagccttg aacagtaata ctattatttg 78120
   ataacgagat tgcgtcctgg agattcagac ctgcattatc ttcaatccac aatgtaactt 78180
   cttcacgctc ttcggttgca atctggaaca ggttttctag agcagctacg gccgtacgta 78240
   cagcacaatc taattcacga gacatatgat ttccttaaca agtgtgagaa gatgcaaccc 78300
   aaccaccatc ttcaaggctc aagttgtagt cgtattggta aacgtcggcc cattcagcat 78360
   aaccgttttt tgtacaacgt tcaagatcgg ctttaagatg tcctggtgaa tgataggttc 78420
   cacccatgcc ataagctgga tacaagttga atgttgtgtt ttgtttatta gcaatttctt 78480
   tggcagcctc aatagctgca tagatttttg ctacagcttc agccagttcg gtttgttctt 78540
   tagacataat gattccttag cacaacgcag aagacgaaac ccaagcacct tcagtaataa 78600
   cgtaaccatt accttcacgc ttaacatcgc caccattatt ttcaatttca tctttcatcc 78660
   actgggatac gatttcaccc ttaggataat aagtacgacc gctaccataa tcacctacag 78720
   aaaatgatac tgcatattgg tctgcgatct ttgttgcttc ttcttcgatg cgttcagctt 78780
   gattcagaag tttataaatt gcatctgaag cggatttggc atcattaaac tctggtactt 78840
   taatttcaat aatcttactc attttctaca ttccttaata aagttttcga ttgtttcatt 78900
   ttcactttag ctaggagttc attataacta atcatttgag aagcgtaaac agcataaatt 78960
   cagttaaggc ttttgcatct tatttcctta acgaattcca tagcttcttc atattcatac 79020
   tttttaacag cttcatcata ctgagcttgg gcttttgcgc actgaacttt ccattctctt 79080
   aatcgggctc tgtgatgacg tccttggtac caatatccca tccagttaat aggcattaat 79140
   aaaaacggta caataagtgg tgcgacgaac attaatgaaa atgctgcatc agacgatata 79200
   acttcactag aatctaaaac ggctcctata atcataaaga ttaaaaatgc taaagctaaa 79260
   atagggccaa atagtacttc atttgggatt aatttacgct tgtattcata cgcccagggc 79320
   ttacttggca tgtatagggt tggctttgac atattctcta cattccataa taaattgctc 79380
   aaggattaaa ttgttagtga aattaggact aacaaccata gtttcataaa gcgcatcaat 79440
   agcacttaaa gcatcaactc tcttaagcaa aggcttgcct tcatacatag tttcagaatc 79500
   gacgattaac gtaaagtagt attttttatt atcgactaga acttctgtat cgatataaaa 79560
   catattagac ctcagaagct tatctctaaa accataataa tcttcagagc gttcgctgat 79620
   atattcccat tcagtgttta aacaacaaac agaccacgta ggagcaacga aatcagtgaa 79680
   tctaatatca tggtctgcaa ctagtttagc aatagctccg atatcatgaa tcataatacc 79740
   tggggctaat aaagattttc tgctatggct agccgttttc tcggtggtct cataagaaga 79800
   cgaccagtac catttacgtc cggttgcaac atatcgtaat gaatcaacta aagacgtagg 79860
   tggatttacc ttatctcgga actcttcgaa cattgagtca atatcattat tttggtttcg 79920
   catgttaact cctttattat ccaaaaggag gaccggagtc ctccgtatta ttttatttca 79980
   aagagttaat atattcttgt acatcagcag aagtggtttc gacgccggct ggagtgccat 80040
   tgaacgtttc aacacgagtc agagtgtctt cgatatcaac cttagtcagt gcagcaattt 80100
   caatcacatc atcagccgta gagataccaa gggcattggc tgctcgagtt tcacgaatgt 80160
   attcgagttt gattgccaga ttctgacgag catcatcaag ttcaacaacc ttacgagcaa 80220
   tttcaattcg catagcggca taaccgtcag cttttgtatt aagttgttcg gcagttcgac 80280
   gatacagaag accaagctta gcatgcatgg taacatcttg tccttcagcc aggagcttac 80340
   gaatttcacg ttccttagaa gcggcttgct tgttcttttc attaacaagt tcgcgaatac 80400
   gtttttcttc attaatagat ttaacagaag ccgtttttag ttctttgatt tgggtaatca 80460
   acttatctgc agctgcgtta tactgttctt cgacagtcag atttttagcc atagcagtac 80520
   ccagtttagt gcggatgaat tcaacaagtt tcttcagtgt gttcatatta ttacctttca 80580
   attggtggtt tattatccaa tgagagcatt atactctgct ctcaagagtt tgtaaattat 80640
   tttttaatca aattgacata aaacttggcg tcttccgggt gcatgccttc gcagtattct 80700
   tcaaccatga acttcttaac agcttcataa ggaccactca gattaagctc agttgtccaa 80760
   aacttggaat cttgggctga atcgatacga acttcagggt gtcggttgcg aataacttct 80820
   tcggtgtatt cataatcaac gacatcaata ctaactttag ccattttgtt tctcctctgt 80880
   agttgataag tcctatagta tcaccatcat tggtgtttgt acatcattat ttttaatttt 80940
   tccaacttaa caacttgtag tgtccttcat tcatacgcgg tttatcccaa gaaatactaa 81000
   tatatgattc gccatcaata gaagtcgccg ggtttttctc taccgtaaga ccttcacttt 81060
   ccagccattc aatagtattt gcgcataaat ctgaagaaca cctcatcgga aactggcaag 81120
   atttttcacc tttagatgca gcgttataca tcttttctaa aatacgcttt tgcacgtttt 81180
   taaaaatggt ttcagcagat tcttcagatt ttttattcag atcagtataa agactcataa 81240
   tattctcctc attaataggg ctcataatat ctcaatcata agcccatgta cgcatttatt 81300
   tcatattatc gaaatattct tcagcgattt cgacattatc atggtaaact ttagaagaca 81360
   gtttaacata accttctgcc gcgaacatat taatcacaac ctttacagta taccattgtc 81420
   cgtcttcatt acccattact gcgtaagttt caaacatcgg atggtcaggt ccaataactt 81480
   taatatcatt caccgtacgt ccgaaatctt ctggaacgca tttcataaag aagttgaaca 81540
   gttcaccgta attatccatt tcattctcca aggtttttct gtatcggtag ttgatagttt 81600
   tatagtacca cggaagaaca gggatgtaaa cagtttttgt gaaaaaaaaa tttttaaaaa 81660
   gttttgtgga attctagggc agggagggga aatcaaagga tatgataata tattataaag 81720
   ggcagaaact aaatgatgcc tagagaggtc gggaaaggcc tagataccaa aaagccctat 81780
   catttagata gggctttaaa attatttacc tagtttagtt attatagctt cggcagcagc 81840
   ttttagctta gatgcagtgt taatacgatt tttaatttca gtatacgcat cgccaagaac 81900
   atcaaggcta tcagaataaa cacttacact attgcgttcg ctatttgaaa tagaaccttt 81960
   agttttacga tcaaaatcag tgtacacgtc ttgaagatca cgggctgcct tctttgcatc 82020
   atccaatgca tcaagagctt tattcaaagc attgacgtat ttttttagtg tcaaacatat 82080
   tcttagaaat tttaacaggt gcacgttcag gagtagctaa ggcggattta ggaacctgtg 82140
   tgccagatgc cagtttctta gaaattttga aaaatgcttt attgaaacta ttttcttgac 82200
   tcatcgtaaa agaactagta tcgatgttat acatttcaat agctttagct ttccacttaa 82260
   gaaaatcgcc acggtctttt ggatccaagt ggaagaatac tttagtaaga tctgcttcag 82320
   atggtaattt tgctgcttcg gttaaaaatt cggcgtaggt tttcatttaa aatccttgaa 82380
   ataatttatc ggttggttat taattattta ttactttggt attatcagta acagcattct 82440
   ctgagagatt caaactaaaa agccccaacc ttttggctag ggctaagaat gttatttgat 82500
   ttgtttagca gaccaaatgc ggtctttaat aagtttttgg atatcttcaa catactcaag 82560
   gctatgagcg tgaggattat ctttgaagct atgtgctcgt gccagtttct ggccttcagt 82620
   tttgattacc aaaagttctt taagaattgc ttcatactta gaaataattc ctttggcgat 82680
   atttttttct tgagctgctt tagggcccgc ttttggtgct ggtgctttac ctgtagcttt 82740
   ctggaacaat tcacctgatg cagctaggct tttaaaaact cggctaacag tattagcatt 82800
   agtaaaacct tcggctttaa aatctctaat aaaacggaaa cgttcttcat cagaggcgtc 82860
   tttgtaagaa tatttacctg cttgaattgc tgcaatcgca gcggctttta catctgaact 82920
   agattgttca gtcaaaaatt ctgcatatga tttcatgtta cttttcctaa gttaattaac 82980
   ttatctattt attattatgc aaaaaagccc caacctttcg gaaggggcta agccttgcgg 83040
   taaccttgtc ggggttccac ctgctaaggc aagtgtttgt acgaaacgcc gggattcgaa 83100
   cccggttatt aagcagttga cgctactcaa tatttttaaa aggccatatc tcgaccatat 83160
   ccgaacgttc cgtcaaaaac gctactcggc ttacggcaaa gatatttcct cgaatcgata 83220
   attctgtgcg ccgtttctgc tgtgatgtaa ggggatatta acgaatcata aagatttatt 83280
   aagaccagtc cttactcagg gaacatcagt ccgacgactt accggtagcg acccggcttc 83340
   ttattttggc atatcatcaa tttgctctcg acgccgacgc catagattaa tcgcttcatt 83400
   aacacattca gcgtttggta tatgacggca ataatataat gattcacgtt tctgaaaccg 83460
   atgttgcaat gggatttcgt ttaaaaggtc tttacgatca aagtattcaa aactagttaa 83520
   ccctgaacgg tttttaatga acttccaatg tccgaattca ttttgaacat agatgtaatc 83580
   ggcatgctga tacagcgaat taatcaatcg atgagaaata actgtatcat attcgttaaa 83640
   ataaaacgtt aaatcatgaa ctaccagata aatattgcct ttcatatttt cctcacttaa 83700
   agttggtcga gacagaagga ttctaacctt caacctacgg attagaagtc cgttgctcca 83760
   tacaattgag ctatgcctcg aatatatggc ccagaccaga ttcgaactgg taacctttcc 83820
   cttatgaggg gactgctgct aaccattgag ctacagggcc taaataactt tctattatga 83880
   aaggcacttt agagcacctt tggtaataga gggtatgaat taataataac acataattct 83940
   taaagcaaat caatcatttt aacggttggc aaaacgattt cttcttcaat ataagcaaat 84000
   tcgacacgtt caactacacg caagaaatcg taatcatagt attcagaagc atagccaaga 84060
   atatcaataa gtccttgaga tatctcatcg cctttcacaa agaacaggtc gaatacgcgg 84120
   gcttcgtctt cttcagattt atcaccaagg tctacttcga tgccaaagac ctttttaata 84180
   aattccggtt ggagttggtc tgagaacagt ccttcaacca gatattcgta gataataagc 84240
   gagcaacgac cgaatggtgt attaccgcaa taacgttcgg ctttggtgct agaatgacgg 84300
   ctcttaaatt tcttcaagag atatttaaac tgttggagct catgctcttc cacttcaaac 84360
   aatgttttgg ttttatagtt gtcaccatca ctttcatagg tagtgacatc aattacgtag 84420
   cccttaggaa ttgtactgcc taatcgaata ttcatttaat caccgtacca tattaatgta 84480
   attatattca atgcaacgta atttcaaagg attcttgaaa atatcatatt caagagggcc 84540
   tttttctgtt tcaataaaga aatcaaagtt aactgtgtta aatttatcat cttcacagag 84600
   taatttaacc tgtgaagacg aacggtcaat gtaaaccttt tcaacttcaa aatatgttaa 84660
   aatgccataa tcatcgatca atgctttagc tgcacgttga tcgctttcgt atccattctc 84720
   gacagatttt actttagcat aaagaatcat gagtgttcca ccacttcaaa agtatgtgca 84780
   tttacaatct ggtaccagtc aaaattttct tcagcatttt tgtagatttc acgaagagct 84840
   tcaacagttt ttcctgtagc aacaagatca tcaataccac caagaggata acagtcataa 84900
   ccagcaaaca gaaggaaatt aatagtttct gtaggttctg gatgcagtgt accattgtca 84960
   tcgactactt caaagaacat ataaccaggc gatttagatt taatccaagc ccatgcttga 85020
   gccgctgaat caaatggttt gcgtatcaaa cgacaatcag ggtctttaga aggatttgct 85080
   tcaaaatccg catatacacg atatttcatt ttactcacct tttgcaattt tatccataac 85140
   gatagcgatt aagccgatta gaaatgctac taccaatgag atagaatttt ctgttgttgt 85200
   cagtagtcca caaatgaatc ctacaaacat cgaaaaacta aaagccattg aagcaattac 85260
   tacagcaaca ttacgaatca gttcagcgcg tttcatttta ttctcctcag tagttgatag 85320
   ggtaatagta tcaccaccct atctaaaagt aaacggtttt ataaactatt ttattccgcg 85380
   tgatgcaatc gtattctgcc gtttaacacc agtttgttta aagtcatgac tgtcgatttg 85440
   atcggtgcga gcaactccga atgatgattt aatcttagct ttagatcctt tagttataga 85500
   aataaacatc tcggaagacc ctttcttaaa gtctgatgca gatacttcaa caccgttctt 85560
   agcaagtgac ctcagaattg catccgcgta ctcatcatca agactcgctt gagtattaat 85620
   aacaaatgtc tttggtgcag ccgcttcatt gataaattct ttataggttt tcattttatt 85680
   ttcctaatta attttgatag gactactata acatagtcct attagcatgt aaactactta 85740
   cagaagggtt tttgcaataa ttttaagctg agctacagaa atatgcttag ctttaaacag 85800
   tgcagtctct tcagccggag agataacaat agtagcgcca tctttcttag cagaccatcc 85860
   atcccctaag taaacagtac cttttacttc ttcgccgtca accagactaa tcatcggttt 85920
   accctcacgt cctttatttg ctttaataac ttcggaagta agggttgctt cagtcagagt 85980
   agaaacgtta gcatcaggtg atataaattc tttataggtt ttcattttat ttccttgagg 86040
   tcgtttattt gataggatga tagtaacacg ccatcatcct tatgtaaact gttttgtgaa 86100
   attattttag atcttcggcc caatctagtt ttaagggttc tttgtattca cggtcgagaa 86160
   taaccggaat agctacatca ccactgaatg atagttggcc aacgttatat gataacgtta 86220
   tatgtggagt gtagtcgtcg aaatcatgag tagcacctaa agctcgtgcg tactgatgac 86280
   gacatctaag atattcggaa tcaagaacta aaaccagtac agcaccgtcg tcagttttcc 86340
   atacctcaag gtgtcctgat tcggcaacag aataactacc actagatgta gtatatggta 86400
   tgtttacacg tgaataacaa attgttgaat gaagcttttc acgtggtact ggattaggaa 86460
   ttcttaattt gcgctggagg tcttccagcg catcaagtgt taattctgaa aacttagctg 86520
   cgacgtagag gccttgtgaa aagtcctcaa atttcattat tcttcttctg tggtttcagc 86580
   tttaacaagt tcacgtactg cttctaccag gtcttccagt ttaacagatt cacttgtaat 86640
   accgaccagt tgagcaatct gagctagggt attctgaaga agtttagaat cgtcagaaat 86700
   acgagcggcg gcatcttggg tatcaagaat acgagatttc agaattacga tttcagactg 86760
   cagtttttca atagtttcat tggacatttt attaccttaa gattttttca attttagaat 86820
   aaagctcttc taacgagccg tcgtttgtaa taactaagtc gccttcttca ataggcaacc 86880
   cagcttctgt aatatgttta tcagtggatt ccgtaccaga gcgaaccaca tgaataactg 86940
   tagcacccat cgctctagca gtttccattt catggacttg gcgcgtatcg gtcacaacgt 87000
   agtaatcgaa atcacttcca atataatcca tatagtttaa tgcaaaaagc ttaacccaat 87060
   acatacggtc aaataaatta acgacaacat ccgtaccgag ggcttgcata agacggcgga 87120
   tcgaccatgt gtcgttgata ttatttatag cttcacgcag ggcttcatat ggtttaatat 87180
   ctagatatga atatccacct tctaacgatg ataatacttt aacattatta attggtaaat 87240
   attgactttg taaataaatt agcgcttctt ccaataattc aataacatcc aacttattga 87300
   gatttaatgg tgtttcacgg tcgtatccaa tgccttcaaa gtattcgtat ttcaactctg 87360
   tgaatacatc aggattttca gcatgtctac gtccccaggc tattgctaga gcgtctttaa 87420
   ttggatatgc taattggtat tttacggatt taaagtttga attgatgtaa tcagcagtag 87480
   tgtctttacc actacgtttg atgccactta aaaagattag ctccataggt tttcctcata 87540
   taatttataa gtgattataa catacggagc tagaagcgat taacgcaagg aagggtgtct 87600
   agaaatggat ttagattcgg ctgcttgacg aggctgttga gatacaatga cttcgccgtc 87660
   tttttctatc gtcatatacg cgtagtgaat agttgctacg cacgtaacgg caggatcaga 87720
   atcttcagtg taactaaatt caatttcgct caaatcagaa atccatggtt tatagaaatt 87780
   tgccgtcaaa acgacttcag tcttattgtt gtttaaaatg tgcaaagtaa caaactcagg 87840
   accatcggca tgggctgtat tatgaccggt tatatagtta ttagtgctga gcatccattt 87900
   atacatgttc atccaggacg tcatgtcttc gtcaactata aatcgtacaa taaaagggtc 87960
   atattcaaat gtggtgcctg cacgcattgc acgacctagc cccattgtgc ctgtcacagt 88020
   ttcaacaaca ggaattctca cacctggcag tggagccgtt tgggcattga gcacaaaacc 88080
   tgaagttcgt gtactatccg ggatatccac aacaaagttt gttatatttg tttggttaaa 88140
   aatctgtctc gctggacgag tcataagcac ctcaaaaacg ctttaataaa aatgcagtat 88200
   aatggtttta ggtctcttcc attaatctgg ttcaacagaa tatcagaaga acggaagaga 88260
   atagaattaa gatttccaaa cagtattcgc agagaaacgt tttccttttg acataaattg 88320
   ctgagtaggt aacatgatta tgttagccca gtctttagga gcgatttctg ttattgttcc 88380
   tctaatgtgg cctggcaggt atgctttaat cattttgtcg gcaccttgaa atcctttcac 88440
   cttgctccag tctatcttca actttgtgtt attagtaata gtagttgtgc tagcatattg 88500
   cttcagcaat tcttcaagaa actgttgacg tgctttaggt gggatatagt gcaagtttaa 88560
   cccgtacatc aaattatgtt tacctaatcc taaataaata atcaaaggat atcggtccca 88620
   gtatggcaga gtatctttat gtttagcatc atacacgtaa gtataaattt tgcctggcgt 88680
   aggcttgact actttatgac cttttacact tttcttaatt gtttcaacaa accacttaga 88740
   tgatttgttg tttactgctg cgccttcatt tctaatcttt tctcgtattg aactgcgaaa 88800
   cgagtttatc atcagaagtt gacgttcttg cttattgagt ttatttttag gtttagattc 88860
   aagcgtttca attttctgtg cagtcttaat agatgatgca tatctagaca ttgcagatgt 88920
   aaacgacgag tattttattc cttttgattc tgcaaatgct tttgcagtga ttcctttctc 88980
   tttagctttc ttgtattcta atcctatctc aatccatttt ctttcattca tagactgagc 89040
   aatcttaggg gcttgtacgc cttcagatat aatttggaat atgctcataa ttaccccttg 89100
   tatccgagtt tcctaagtcc gtcttcggtt agaactcgaa attttatatt tctcttttct 89160
   gcgacagcag aagcagcttt ccatttgtcg cagttcacag accaggtgta tatttcgttc 89220
   atataacgtt tctttgctgc ggtcgtaaga tttacaggct taataggcgg ttgagtttct 89280
   ttctttggtt ttatttctac aaagaactct tgacctgatg catctttcat ccatatgtcc 89340
   atatagtatc gacgtttctt accttcagca ttacaaaaat atggaattac ggctgtttca 89400
   ctaccccatg caataatttc cggattttta tccaaccatt cgaaaaagaa tttttcccag 89460
   tttgaacgat acgttatttt ggtatggtct ccacggtatt tttttaaatt ttttgggacc 89520
   catttaccag agtaggccat gttgtcctcc ttataaatag tattattatt tataccaatt 89580
   tcatattgga aggagactat cttgttattt acattcttcg acccaatagg atatagtgct 89640
   aaaaccatta ataagaacgc acctactatt cctatgacag acatatttcg taactataaa 89700
   gagtacttta aacgtgttgc gaccaactat agattacaaa cttattatat taaaggttct 89760
   ccacgccctg aagaactagc aaacataatt tatggcaacc ctcaattata ctgggtcctt 89820
   ttgatgtgta atgacaatta cgacccgtat tatggttgga ttacatctca ggaagcggcg 89880
   tatcaggcat ctattcagaa gtatgcaaat gctggcggag accaaattct ttaccatatt 89940
   aacgagaacc gtgagaaatt ttacaattta gtttcatatc cagacgagcc tttagtgtgg 90000
   tatgataaag gtgatgaagc tcgtaagtat ccgcaatata aaggaccttt agctgcagta 90060
   gatacttatg aagcagctgt actagataac gaaaaacttc gtaaaataaa aattgttgca 90120
   aaggaagata taaactcgtt tatcactgat ttgattcgtg agatggagat tgcataatgg 90180
   aaatgattag tagtagcctt aattggttcg ttggtgttgt tgaagacaga atggacccat 90240
   tgaaacaagg gcgtgttcga gttcgagtcg taggacttca tccagcgcag agagcacaag 90300
   gtgacgtaca aggtattcct accgaaaaac ttccatggat gactgttatt caacctatta 90360
   cgtctgcatc aatgtcaggt attggtggtt ctgttacagg tcctgttgaa ggaactcgag 90420
   tatatggtca tttcttagac aaatggaaaa caaacggtat tgtactcggt acatatggtg 90480
   gaattgtacg cgaaaagcct aatagactcg aaggtttttc cgaccctaca ggtcaatatc 90540
   cacgacgttt aggtaatgat acgaacgtat taaaccaagg tggtgaagct ggttattatt 90600
   cgaattctaa cgtaattcaa gacaacaact tggactatgg tataaacccg gatgatacag 90660
   atttagcaaa tattccagaa gataatgacc ctaattttac aataacagaa atgttacgtc 90720
   gtgatgaagg tcttcgtgat aaagtgtatt gggaccatct agggtatcct acagtaggta 90780
   ttggacacct tatcgtaatg gaaaagaccc gagacatgac tcgaattaat aaattgttat 90840
   ctgatcaagt agggcgtgaa gtaacaggaa atcccgggac tattacatta gaagaagcaa 90900
   cagcgttatt tgagaaagac cttgctaaga tgcagaaaga cattcgttct aattctaaag 90960
   taggtcctgt ttatgctaaa atgaacaggt ctcgtcaaat ggcattagaa aacatgtgct 91020
   tccaaatggg cgttggtggc gtagcaaaat tcaatactat gcttaaagcg atggccgcag 91080
   gtgattggaa aactgcatac aaatctgggc gtgattcatt atggttccaa cagactaaag 91140
   gacgtgcttc acgcgtaaca acaattattt taactggtaa catggaatca tatggcgttc 91200
   ctgttaaaac accaccttca ccaggagtcg gtgccgattt agttactaga aataccgatc 91260
   cagaagaccc ggccggtcct cctgttccat tagattcgcg tatccttttt aaagaacctg 91320
   aatcaagtta tcgtggtgaa tacccatatg ttcatgctat ggaaacagaa agcggtcata 91380
   tccaagagtt tgacgacact ccaggtaacg aacgttatcg cttagttcat ccaacagggt 91440
   catatgagga agtttctccg tctggtcgtc gcactcgtaa aactgtagaa gacctttttg 91500
   acatcactaa tggtgatggg aatttcctgg tttctggtga taaacttgtt aacgttggtg 91560
   ctaacgaaat atattataac atggcggacc gcctccatca aatagatggc agcgacacaa 91620
   tctttattcg tggcaaccaa gttaaaactg ttgaaggcga tggaactctt tacgttaagg 91680
   gcaatatcaa aatagtcgtt gacgggaatg cagatattct tgttaaaggc gatgctaaaa 91740
   ctcaagttga agggaatcat gactatactg tcaatggtaa tgttaaatgg actgtcaatg 91800
   gtaatgttga tatgactgtt gctggtgatt ggtcggagac aatgactaca atgagctcaa 91860
   tagcttcagg acaatatact gttgacggat ctcgtattga tattgggtaa tatatggcac 91920
   aaatacttcc tttaaatacc gacttaggag aagacatgga aggggcgtct attgacgtcc 91980
   tttttactcc gcagttagaa actaccgaaa ctttggtgtc aataaatata atagattatg 92040
   aaccaacaca aggcattaca gttgacggta atcacttata tggaacatat gaaagcgtat 92100
   ttagcttttc ttcagatgca ttgaaatatc gattaaacga tgattttaaa actgcatctt 92160
   catgggaaga tttaccacaa gaccaaagca ctcaactata tttgtggaga gctcctcaga 92220
   atcttcgtaa agtgtttagt tatacggttg aaatgattta taactaccaa gaagaaagtt 92280
   catcaggcgg cacaagaagt gactctggta cagaacctcc accggctcca gtacaaaaaa 92340
   ctcttactaa ggtttataca aaaactatag taggaaattg gagcaaatgg gctcaacaat 92400
   tacgaaacta tgtttatgcg aggccataaa aatgtctgga ttaagttacg accagtgcgt 92460
   tacaacaggt catgaagcat ggcctcctac agtaattaat gcttctcaat ctaaagtatt 92520
   caccggcggt attcctgttc ttgtagcagg agaccaaata acacctcata cagaaattaa 92580
   aaaaccgtac gagactcatg gtggtgttac tgaacctcgt acatctaaag tatatgtcac 92640
   cggtaaaaag gccgttcaaa tggctgaccc aatttcatgc ggggacactg tttctcaggc 92700
   ctcgtcaaaa gtctttataa aataggaatt aaaatggcaa acactcctgt aaattatcag 92760
   ttagtcagaa cagcgaatgc tattcccgaa atttttatcg ggggcacatt tgccgaaatt 92820
   aaaacaaata taatcgaatg gctcaatgga cagaacgaat ttcttgatta tgattttgaa 92880
   ggttcacgtt taaacgtatt gtgtgacctt ttagcatata atactcttta tatccaacag 92940
   ttcagtaata gtgcagtgta tgaaagcttt atgcgtactg ctaacttgcg cagttccgtt 93000
   gtacaagcgg ctcaggataa tggatatctg cctgcatcta aatcggcagc acaaacagaa 93060
   attatgctca catgtacgga tgcgttaaat agaaactata tcactattcc gcgtggtact 93120
   cgtttcctgg cgtatgctaa aggtacttct gttaacccgt ataactttgt ttccaccgaa 93180
   gacgtcattg tagttaaaga caaaaataat caatatttcc cacgtcttag gttagctcag 93240
   ggacgtattg ttcgtactga attaacattt gataaattaa aacctatcat tattcgtgat 93300
   aagaacattg accgtaattt ggttaaattg tatgttgacg gagcagaatg gattaactgg 93360
   actcgcaaat cgatggttca tgctggttct acttcaacca tttattatat gcgagaaact 93420
   gttgacggga acactgaatt ttattttggt gaaggtgaaa tctctattaa cacgtcagaa 93480
   ggtgctttaa catctaacta cattggcggc cttaaacctg tccagggttc taaaatagta 93540
   attgaatata tttctactaa tggtgctgaa gcaaatggtg cagtaggatt ctcttatgct 93600
   gatacattag ctaatatcac agttattgga attaatgaaa acccaagtaa taaccctgac 93660
   ttcgttggtg cggatggcgg tggcgaccct gaagatattg aacgtatccg tgaattaggt 93720
   acaattaaac gtgaaactca acaacgatgt gtgactgcga ccgattacga cacattcgtc 93780
   tctgaacgat ttggttctat tattcaagcg gttcagacat ttacggattc atctaagcct 93840
   gggtacgcat ttattgctgc taaacctaag tctgggttat atttgacttc cgtccaacga 93900
   gacgatatta aaaattatct taatgagtat aacttaggga cgataactcc tgttgttatt 93960
   tctcctgatt atttgtttat taaaatgaat attcgcgtta cgtatgcatt gaacaaatta 94020
   caggaatctg aacaatggtt agaaggccaa attattaata aaatcgaccg gtattatatt 94080
   gaagacgtgg aaatttttaa ctcgtcattc gcaaaatcta aaatgctaac gtatgtcgat 94140
   gacgcagata tttctatcat tggttcgtct gcgactattc aaatggttcg cgaggtacag 94200
   aacttctaca aaacacctga aacaggtatt aaatacaata accagattaa agatagaaca 94260
   ttagaatcta acgtgttttc atttgatagt ttacgcgttg accctgaaac ggaagctact 94320
   attaaatacg acgttcgcat tgtaggttca gatagaaatg accgtggcat tggacaagtt 94380
   atcattggcc cgtttgcaga tggcgacgtt atagaaaatg cttatattca accatatact 94440
   ggtgacgatt ttaataaact tcatgttacg gacggaagaa acaaatatta ctctataggt 94500
   gaagtaaatt atccggctga ttcaatttat tggaatatag ctaaaattga tttaacttct 94560
   gataggtttg aagttcaaac tattgaactc tattcagacc cagctgatga tgttatcttt 94620
   actaaagacg ggtcattaat tgtatttgaa aatgacttac gtccacaata tttaaccata 94680
   gatttggagc ctatttcaca atgacagtaa aagcgccagc agtcacgagt ctcagattat 94740
   ctaaattgtc cgcaaaccaa gttcaaattc gctgggatga ggttggcgct aatttctact 94800
   attttgtgga aatagctgag accaaaacag ctaacgggga agcaattccc cgtaatagct 94860
   atagatggac taatttagga tataccgctg ataacaattt ctttgagtct ggtttaaatt 94920
   cattaacaac atatatgatg cgtgtggcta cagcagcgga gggatttgag cagtctgatt 94980
   ggagatacac cgaagagttt gaaacatttg aaataaacgc ttacacattc caacatatga 95040
   ttgaaatgca attagctaac gagtttattt ctgaaaaatt tacaaaaaat aatacaaact 95100
   acgttgattt taataatgac acgatcatgg cagcactgat gagcgagtcg ttccaattca 95160
   gtcctgcata caccgatgtg tcatcgatta gaaattttat aattggtgaa aatcagtatc 95220
   atgaaatcca ggggcatatc caggatgtat gtaaagatat aaatcgagta tatctaatgg 95280
   aatctgaagg aattctttac ctttttgaaa gattccaacc tattgtaaaa gtgtctaatg 95340
   ataaaggcca aacatggaaa gcggttaaat tgcttaatga ccgtgtagga tatccacttt 95400
   cacgaaccgt atattaccaa tcagactata cgacgtattt gctcggctat gataagattt 95460
   tttacgggcg taaatcatct gatattcgtt ggtctgctga tgatgttcgt ttcagttcac 95520
   aagatgtaac atttgctaaa atcggggacc aattaaattt aggttttgat gtagagattt 95580
   tcggtacata cgcgtcatta ccaggaaacg tatcacgtat tgcagaagct attacttgta 95640
   atgatgacta tgtttatatt gcggccagag ataaagttcg cttcgttaaa accagtaacg 95700
   ctcctattga ttcagatcca ttatctccga cctattccga acggttgttt gaacctgaga 95760
   cgtttacaat aacaggcaac cctaaagcag tttgttataa aatggattct gttggtggaa 95820
   gaatattcgc tcttattatt ggtgaagttg aaaacgttaa tgacgaccca agaacaaagc 95880
   ctattttgga ttccgttgat aaaggtgtat acgttttaga ccatgacgct ggaacattta 95940
   aaaggatatt tggtaatacc gaagaagaac gccgtcgtat agaaccggct ttcaccaata 96000
   tgtctactga tggtgttgag ctttatatct cttctagtaa ttttaaattt ttagaatctg 96060
   atattgttga cgacccagaa acgcaatcta agtacggact tttgggagca gttaagtatg 96120
   aatatcctag agaatggctc gcagataaac attaccatat gatgattttc gcatcaaacg 96180
   aagaaagtaa ttgggaaact ttttcaccaa ctcctatgca gtattatgcg gaacctttct 96240
   ttagttattc tagaaaatca ggcacacgtt cttggattaa taactctgat agagcggtag 96300
   tgatttattc tgatttgctt tacacaaagg tagtggaaag ctaccctagc acttcacctg 96360
   atcgtaaagt tcacgaatac tggaatgatg gcgattgtaa gattgttatg ccgaatatag 96420
   aattcactgg atttaaaaaa tacgcatctg gtatgttgtt ttataaatca tcaggtgaaa 96480
   taatttcata ttacgatttt agttatcgtg ttagagataa cgtttctata atttggaagc 96540
   ccactaacgt atttttgacc gcctcattac aaaaccaaga aaaagaaact tcttgggttc 96600
   ctgttgaaga gacaggtcta gctgacccgg atttacgccc attactcact acaatgatgc 96660
   cggagtctta tctgttggat aatacaaact ttgaagcatt ttgtgaagcg tatattcaat 96720
   atctttctga tggatacggg acccattata ataacttatt gaatttgatc aaaaataaat 96780
   acccacgaga agaacacgca tgggaatatc tttggtctga aatttataaa cggaacattt 96840
   atttgaatgc tgagaaacgt gatttagttt ctaggttctt tgagtccaga agttatgatt 96900
   tttattctac taaaggaact gaagcgtcgt ataagttttt gtttaaagtt ctttataacg 96960
   aagacgttga agtggaaatt gagtcatctg ctggaacgga gtacgatatc ataattcaat 97020
   ctgattctat aagtgaagat ttggttggac aaactatata caccgctaca ggtagatgta 97080
   atgttacata tttagaaaga agctattcta aaggtaaatt acaatggact gtgactattc 97140
   ataacctttt aggtaggctt ttagtcggtc aagaagtaaa agccgaaaga ctagcagatt 97200
   ttgaaggtga aatagttcgc ggtattaaag gtaaagaact tgcccaaaac actatcgatt 97260
   attttaatcg tggtagagct tattacgtca tgaaaataaa gtcaaatctt ccatcctctc 97320
   gttggaaatc tgacgtacta cgttttgtac atccggtagg atttggattt attgcaataa 97380
   ccttattaac aatgtttatt aatacaggat taacgcttaa acatgttgaa actattatta 97440
   ataaatataa aaattataag tgggattccg gactgcctac ggaatatgca gaccgcgtcg 97500
   ctcgtttaga ccctcaaggt aatgtagaat tcaatcctgt tacaggtgaa gtaatttatg 97560
   atgctggtcc ttatgccggt atcgaatacc ctttacctgc aaattataac gaagaaaatg 97620
   ataactctat tttccaagga caattaccgt ccgaacgacg caagccaatg agcccattat 97680
   ttgatgcatc aggtacaacg ttctctagat ttagagagct tgttaatgaa agattgttag 97740
   ataacgtggg aaatcctaga gacccgatta atccaccaca ggttaaatta gatgaatgat 97800
   tcaagtgttg tctatcgttc gatagttact tcaaaattta gaactgaaaa aatgttgaat 97860
   ttctacaatt caataggtga tggtgataat aaaaacacca tctttataac gtttggtcgg 97920
   tctgaaccat gggctgctaa tgaaaatgag gtgggcttcg ccccaccgta tccaacagac 97980
   tcggttttag gtgtaacaga tatgtggact aacatgatgg gtttagttaa agttatccca 98040
   tctatgttag actcggttat tcctagacgt gactggggag atattcgtta tccggaccct 98100
   tatacgttca aaataaatga tatagtcgta tgtaatacag ccccatataa cgccactgaa 98160
   gtcggagctg gatggttagt ataccgttgc gtagatgttc ctgatgttgg gatgtgttct 98220
   attgagtctc tcgacaacaa agaagaatgt cttaaattag gcggtaaatg gactccatct 98280
   gttaggtctt taacccctcc tgaaggccgt ggagacgctg aagggattat cgaagttggt 98340
   gatggatata tatgggagta tctttatgag attccacccg atgtgtctat aaaccgatgt 98400
   actaatgaat atatagtcgt tccatggccg gaagaagtta aagaagaccc tgctcgttgg 98460
   ggatatgaaa acaatctaac ctggcaacaa gatgattttg gccttgtgta tagagttaaa 98520
   gcaaacataa ttcgttttaa agcctattta gactcagttt atttccctaa tgcagcactg 98580
   cctggtaata aaggatttag acaaatttct attatcacaa acccgttgga agctaaactc 98640
   cgtcctaatg accctaatat aaaagctgaa aaggattatt atgaccccag agatttacag 98700
   cgtcattcgg gtgaaatgat ttatatggaa aaccgtccac ctattattat ggcaatggac 98760
   caaaccgaag agatcaatat tttgtttatg ttctaattta gggagacttc ggtctccctt 98820
   tcgtgtataa atagtataaa ctattaagga ttagccacat gtatattcaa actccaaaac 98880
   aattgattga cgttggcgaa attggtaacg cttctacagg cgatatcctt tttgacggcg 98940
   gtgttaaaat aaacaatgat ataaatgcta tttacaatgc gtttggcgac caacgaaaaa 99000
   tggcaactgc taatgggaca ggaccaaatg gacaaataat ccatgctact ggatactacc 99060
   aaaaaggagg tcctactgat tatttcaccc ctgttccagt aggaagtaga cacgacatag 99120
   atgcttccac cggtggtgtt attgttactg ttgctagagg tgaactcggt gattccgtgg 99180
   aatttataaa ttcaaacggg tcaatttcgg taaacaaccc attaagtatc caagcattag 99240
   attctattaa aggtgttgca ggtaatttag ttataaccac accttatact aaagttactc 99300
   ttcgttgtat ttcttctggt acaggcggct caatatggga ttattctaca gaaagtatgt 99360
   ttagtcatac cgaaattcct gtagacggga catggaatat tatttcagac tatgtcaata 99420
   ttcctctatt ttataaaact gaatataatg ctgctaaact tttggttaca tgccaatctg 99480
   ccaatggcag aaaaattaaa tcatgcgaaa taaatattct aatagataca attaattcaa 99540
   gagttatttc aaccgaatac gcagtgatgc gcgttggtaa tgataacgaa gaagatgaaa 99600
   ttgcgaatat tagtttttcg ataattaaca attttgccac tatgacagtt tcttcacata 99660
   taaatggtct tcgagtggca gctaaagtta tttcaactca gaaaattagg gtcgctcaat 99720
   aatgaaacaa aatattaaaa ttgggaacgt cgttgatgac ggacaaggcg attaccttcg 99780
   tcgtggtggt gcaaaaatta atgaaaactt tgatgagttg tattatgaat taggcgatgg 99840
   cgaagttcca tattccgctg gtgcctggaa aacataccat tcttcagatg gacttaattt 99900
   agctgctgaa tggggtaaat catacgcaat tgatacttca acagggcgtg tttctataaa 99960
   tttacctaaa ggaacagtgg aagattataa taaagtaatt agagcccgtg atgtattcgc 100020
   cacgtggaat attaaccctg taacattaat tgctgccagc ggtgatacta ttaaaggttc 100080
   ttccagccca gtagaaatca atgtgcagtt ttcagactta gaattagttt actgttctcc 100140
   tgggcgctgg gaatacgtta aaaataaaca aatagacaaa attactagtt ctgatattag 100200
   tagtgtagct cgtaaagaat ttttagtcga agttcaaggt caaactgatt ttcttgatgt 100260
   atttgggtct gtttcttata atgtgaataa tatccgtgta aaacatcgtg gtaacgaatt 100320
   atactacggt gatgcgttca gtgacaatag cgactttggt tctccaggag ctaatcccgg 100380
   agaaatcgtt gcattagatg gtaaaaacat ccgtttaaga caaccatgta atattggcga 100440
   caccgtacaa attgaaacct ttttggatgg gattacacaa ttacgcagtt cttattctcg 100500
   ccgccaaata cgtattttag attcaaaatt aacaacccgt ccttccttag aaggaagtgt 100560
   atatgtcgct gatttgtcaa ctttgaaatc tattcctttt tccgcatttg gagttaatcc 100620
   ttcagaaccg gtcaatacca attctttaga agttcgtttt aacggtattc ttcaagaatt 100680
   agctggtact gttggtttac ctatgttcag atgtgaagga gctgacgcag aaacttctac 100740
   tgagtgttca gctttaggtg ggacttgggt tacatctaac acagattatt ctattgaata 100800
   tgatgataac gatgcaacta tcgcacgtgc tttagtcttt gaccgtaagt ttgaagacca 100860
   agacattatt gatattacat ggtttaataa cgatttaggc actttattaa gtatcgatga 100920
   cattttagat acaactgatg aaaggtatgt cgcacaaggt gcaaccgtaa atgtgacagg 100980
   cgatgtggct ttgaccgatt tcaataatat cggatggcct aatgtagaac ctgttccgac 101040
   atatagcagg gaattctcgt ctattgcaaa tatttttaac actatttacc ctgtcggtac 101100
   tatttacgaa aatgcggtga accctaataa cccagccaca tatttaggat ttggctcttg 101160
   gaaattatgg ggacaaggaa aagtattagt tggatggaac gacgatatta gcgatcctaa 101220
   ttttgcatta aataataacg accttgattc tagcgggaat cctacacata ccgccggtgg 101280
   aacagtcgga acaacgtcta acacactaac taattctgat ttacctccta ctcaaactga 101340
   cgagaaagtt cttatttccg atgagaacgg tacaattatt attggtggtt gtcaatacga 101400
   ccctgatgca gaaggtccta tatacaccaa ataccgtgaa ggccacgcca caacgaatag 101460
   tacacacgta ccgcctcaag ctgtaaataa tattcaaccg tcaattaccg tataccgttg 101520
   ggtaaggatt gcataatgac catacttagt actaaagcgg gagtaatatc ccgcgaagca 101580
   gattttttag gatttagaaa aaatcctact caaattgata ttttaaacaa ccaaggcgta 101640
   ggttctgtaa ctatttctca attggcaaaa ggattctatg atgataacgt agaatctgca 101700
   ataaacgatg ttcataatat tgctagagca gatgtaggaa cagttacaat taacacctca 101760
   ggtgtatctc ctgaaggaac ttcacaggta gattattggt cctttagtgg aagtgtaact 101820
   gaccctagtt tgcccgatgg aagccctgtt attgtaaaag tttatggact tcctgttaat 101880
   gctactgtag gtatgaccgc cgacgaattt gttgtacaat tacgtactac tctccggaat 101940
   gcgatttccg accaattggc tattgcagag tataaagatg acccaactgt tgggactaaa 102000
   ttacaaataa catatttaga taaccaaaga cacgtactcc caacgtattc ctcttatggt 102060
   atcacggttt ctcaagaaat agtgtctcag gctaagtccg ggtacggcac ttggaattta 102120
   ctaggtgcgc aaaccattac tcttgacaac catatttcac ctacaacctt ttattatttt 102180
   gtgagaaccg catgagtaac aatacatatc aacacgtttc gaacgaatct gtatatgttg 102240
   aatttgaccc ggtagggtca aattttgata gttcaataac caatgtccag gcagccttag 102300
   cctcaattag cgcgtatggt gttaaaggtg ttccggacgc aagtgaagca gaaaaaggtg 102360
   taattcaatt agcgacagaa caagaagttc ttgatggatt taatagtact aaagccgtta 102420
   cacccgccac attaaatgca agacttcagt atccgaacgc gtcagaaaca caatacggtg 102480
   taactaaata tgcaacacaa gaggaagcca ttgccggcac tttagacact gtttctatta 102540
   ctccgcttaa attagaccaa actattgata acacgttctc tactcgttat tccaccgaaa 102600
   caactaatgg tgttattaaa attgcaactc aaaccgctgc acttgccggg tctgatgata 102660
   ccacggcaat gacaccgctt aaaactcagc aattagcaat aaaattaatt tctcaaatag 102720
   ctcctaataa cgacccggct tcagaatcta ttaccggtgt tgtgcgttta gctacggtgg 102780
   ctcaaacacg tcaaggaact cttcgcgaag gatatgcaat ttccccatac acctttatga 102840
   attctgttgc aacacaagaa tataaaggtg ttatacgtct aggaacacaa gcggaaatta 102900
   atagtaattt aggagatgtt gctgtaacag gtgaaacgct aaatggtaga ggagctaccg 102960
   gttctatgcg tggagtcgta aaattaacga cgcaggccgg tgttgcaccc gaaggcgata 103020
   gctctggagc attagcatgg aacgcggatg taattaatac acgcggtggg caaactatta 103080
   atggttcttt aaatttagac catctcacag caaatggaat ttggtcacgt ggcggaatgt 103140
   ggaaaaacgg tgaccagccg gttgcaacag agcgatatgc atctgaacgt gttcctgttg 103200
   gaacaattca aatgttcgca ggtgatagcg ctcctccagg ttgggtttta tgtcatggcg 103260
   ggaccgtttc tggagaccaa tttccggact atcgaaatgt ggttggaacg agatttggcg 103320
   gtgattggaa taatcctgga attccagata tgcgcggcct ttttgttaga ggagctggta 103380
   caggtagcca tattttaaat aatcgcggac aagacggcta tggaaaggat agattaggtg 103440
   taggatgtga cggcatgcat gttggcggtg tccaagcgca gcaaatgtca tatcataaac 103500
   acgctggtgg atggggcgaa tatcaacgtc acgaggcacc attcggagca tccgtatatc 103560
   aaggttattt gggtactaga aaatactctg attgggataa tgcgtcatat ttcacaaatg 103620
   acggcttcga attaggtggt caccgtgacg caacaggcac tcttaaccgt gaaggactta 103680
   tcggatatga gacccgtcca tggaatatat ctttaaatta tattattaaa gtccattact 103740
   aagggtaaaa aatgatcgaa ttaaaaagtc taccatacgt tgatggccct cctgatgagg 103800
   gtcaaaaacg tttaaactgg attaaaaatt cagaagaaat aactggcgct gatacgttat 103860
   acggttctga aggagtaatg aaccgtccaa taacagaagt tcaacgaaat gtagaaacaa 103920
   ttaacgataa cgttaagact atcgcagaat ctttagatac cgctaatgcc gatattgtca 103980
   caattaaaag tatcttggat gtttctggtg acgtagatgc tttagcacaa attggacata 104040
   acactgatga tattgaagta ttaaaacaca ctgtaaattc acacagtgtt gatattttaa 104100
   acactgaaga aaaactagat gatactattg ctaatatcgg agtagttaat ccagaaacag 104160
   attcagtgta tcgtactgtg cgtaacgacc ttctttggat taaaaccgaa ttagggcaat 104220
   atactggaca agatattaat ggtgtcccga ccgaaggtaa tgaaagcacc gggatgaaac 104280
   gacgtattat tactaacagc tcagtgttgg ttgaccaagg cgttcgttta accgagttag 104340
   aaaataaatt tgccgattct gatgttgggg ctctgacaac cgaagttgaa aacctccgtc 104400
   aagaaatagg tcctagacca tcgttaacag taccggttta cactcgttta tccggtattg 104460
   attcatccat ttctatccaa actagagata ttgcggcatt aaaagatttt gttggatatc 104520
   ctaattcaac cgctattaaa actcaagtcg aggccaatcg tctttctata tcaacaataa 104580
   attcagacat taactcgcct ggcggtatta agccacgttt gacaacgtta gaaactacta 104640
   taggctctcc agatttacca actactcttc agggtaagat caaattaaat accgattcca 104700
   tttcaggtat taacacagtt ttaggtgttg attcttctag tggtttacgt tttaatgtag 104760
   catggcttaa tcaggtggtt ggcgttgatt caaatggtgg acaacctgaa ccggctggtt 104820
   cacttttata taggactcgt attcttgaaa ctggtgttac cgacctaggc aataacattc 104880
   aaaacgttca aactgaattg ggaactaatt cgtctggtat taaaggacaa gtaactagtt 104940
   taaataaact tataagcggc accaacccaa acggtcaaac catagaagaa cgtggtatat 105000
   tgccaacagt taaaaaccac gacacgacaa ttacggattt aactacgcgt gttaccactt 105060
   tagaaacaga ccttgctgca gcagaagcag aaattcaagc ccttaaagag gctggatata 105120
   tcaaagatgc tccttcagac ggtaaattct atgttcgtaa agatggtgct tgggttgaac 105180
   ttcctacagc ttaatgaaaa gggccttcgg gccctaaagg atttatatgt caggttataa 105240
   tgcacaaaac cctaaagaat taaaggatgt aatattacgt cgtttaggcg ctcctattgt 105300
   aaatgttgaa ttaactacag accaaattta tgattgtatt tccagagcat tagaacttta 105360
   tggcgaatac cattttgacg ggttgaataa aggtttccat gtattttttg ttgatgacga 105420
   agaaaaatac cgtcacggcg tatttgatat gcgtggttct aatgtatttg ctgtaacaag 105480
   aattataaga acaaacgttg gctctattac ttcaatggat ggtaatgcaa cttatccttg 105540
   gttcactgat ttccttttag gaatggcagg tgtttctggt ggtatgggta gtaattgtgg 105600
   taaattctac ggtccgaacg cttttggtgc cgacctcggg tattattctc aattagtatc 105660
   ttatatgggt atgcttcaag atatgattgc tcctcttcca gatttttggt ttaactctgc 105720
   aaacgagcaa ttaaaagtaa tgggtaactt tagacagaaa gatgtcatta tcgttgaatc 105780
   gtatgttcgc tcttatatcg agacccataa gatggtagga aatacagtag ggtacggtca 105840
   ggtcggtcct cgtgattcat ggtctatttc tgaacgttat gataatccag accataattt 105900
   agtcggacgt cgagttggtg aagatcctgc aaccaaacaa ggcgcatata acaaccgttg 105960
   ggttaaagat tatgcaactg cattagctaa agagcttaat ggacaaattc ttgctaaaca 106020
   tcaaggtatg atgttaccag gcggtgtcac tgttgatggt gttcgattaa ttgaagaagc 106080
   tcgtgtagag aaagaagctt tacgagaaga gttatattta ctagaccctc ctgcaggtat 106140
   tttggtaggt taatatggca acttatgata aaaatctttt cgccaagcta gaaaatcgtg 106200
   gtgggtattc tcagacaaat gagaccgaga ttttaaacaa atacgtgaac ttcaacaaat 106260
   acgaaaatag tcaaacccta gccgatgttc tagtggcaga aagcatacag atgcgcggta 106320
   ttgaatgttt ttatgttcca cgtgaatatg ttgccgtgga ccttatcttt ggtgaggacc 106380
   ttaaaaataa atttactaaa gcatggaaat ttgctgcgta tctgaactct tttgaaggtt 106440
   atgaaggagc gaaatcgttc ttcagcaact tcggaatgca agttcaagat gaagttacat 106500
   tatctattaa cccaggtctt ttcaaacacc aggtcaataa tcaggagcct aaggaaggtg 106560
   atttaatata cttcccaatg gacaacagtt tgtttgagat taactgggtt gaaccatacg 106620
   acccgttcta tcaagtaggt aaaaatgcga ttcgtaaaat tactgcaggc aagtttattt 106680
   actctggtga agaaatcaat ccggtgcttc agaaaaatga aggtatcaat atacctgaat 106740
   ttagtgactt ggaacttaat cctgttagaa atcttgacgg tatccatgac attaatatcg 106800
   atgaatattc tgaagttgag caaatcaatt cagaggcaag tgaatacgtt gaaccatatg 106860
   tagtagttaa caaccgtggt agacaaaatt caccatttga cgatgggttt atgaattaat 106920
   aaataaataa gataaagcaa acaccggtcg aaaggccggt taggagaaat gatgtttggc 106980
   tatttttaca actcgtcgtt caggcgatat atcacaatga tgggcgattt gttttcaaat 107040
   atccaagtta accgtcaatt gtctacaggc aataaattaa ttagagttcc tattacgtat 107100
   gcatctaaag agcactttat gatgaaactt aataaatgga cctctataaa ctcacaagaa 107160
   gatgtggcta aagtcgaaac tattctacca cgtataaatc tccaaatggt agatttcgtt 107220
   tataacccga cttttaaaac gaatatattg aataactctc ttttgagtaa atcgactaag 107280
   gatattgtag accaatacaa cccatcacca attaaaatga tttttgaact gagcatcttt 107340
   actcgttacg aagatgatat gttccaaatc gttgagcaaa ttattccata ttttcagccg 107400
   cattttaata cgactatgat tgagcaatat gggcaggata ttccatttga aagggatatt 107460
   aaagtcgtat ggatggcagc agcaatggat gaacaaatag acggtgataa tttatctcgt 107520
   cgacgtttag agtggtcatt aacatttgaa gtaaacggat ggatgtatcc tccggtaggc 107580
   gcagctgaag gccttattaa aacgacttat cttgattttc atgctaatga acgagacctt 107640
   caaacagccg ccagtgtttt tgaatcagtt gatacagaaa ttaaaccgag agacgttgaa 107700
   gcacaagatt ggaacggtga agttgaacaa acttatactc atgatattcc aatcccgact 107760
   ccaccaaccc ctcctggtcc tagaaaacaa taagaggtaa atatggaagg tctagatatt 107820
   aataagcttt tagatatctc tgacctccct ggaatatctg gggaggaagt agaggtatat 107880
   gctcctttac aattggtgga agttcagagt aatcctcaaa accgcacccc ggatttggaa 107940
   gatgactata gcgttgttcg taagaatatg cacttccaac aacaaatgtt aatggacgct 108000
   gccaagattt ttcttgagac ggctaaaaac gccgattctc ctcgtcacat ggaagtattt 108060
   gcaactctta tggggcaaat gactacgacg aacaaagaaa tactgaagct tcataaagat 108120
   atgaaagaaa tcacatctga acaagttggc actaaaagtg ctgctccatc tagccagatg 108180
   aatatccaaa atgctacagt gttcatgggc tctccaacag aattaatgga agaagtagga 108240
   gatgcttacg aggctcaaga agctcgtgag aaggtgatta atggaacagc cagttaacgc 108300
   attgaatgat aatcacccat tgaatgaagg tgacaaagtt gttattctac cgcctcattt 108360
   agctgaacgt aaagaagaag atggtatcta ttggattaaa tcccaatggg atggtaaatg 108420
   gtacccggaa aaatttagtg attatctacg tataaacaaa atagttaaaa ttcctaataa 108480
   ctcagataag cctgaattat ttcaaacata caaagataag aataataaac gtacacggta 108540
   tatgggttta ccaaacctta aaagagcaaa tataaaaact caatggacct atgaaatggt 108600
   cgctgagtgg aaaaaatgcc gtgatgacat tgtatatttt gctgagacat attgtgctat 108660
   tacccatatc gactacggta caattaaagt acaacttcgt gactatcaac gtgatatgct 108720
   taaaataatg tcttctaagc gtatgactgt ttgtaacttg tcacgccagc taggcaaaac 108780
   gactgtagta gctatttttc ttgcccattt tgtttgcttt aacaaagata aagcagtagg 108840
   tattcttgca cataaaggct caatgtcagc ggaagtatta gaccgtacaa aacaagcaat 108900
   cgaattactc cctgattttt tacaaccagg tattgttgaa tggaacaaag gatctattca 108960
   gttagataac ggttcctcaa ttggcgctta tgcttcatca ccagatgcgg ttcgcggtaa 109020
   ctcattcgcc atgatttata ttgacgagtg tgcatttatc ccaaacttca tcgattcgtg 109080
   gcttgctatt caacctgtta tttcttcagg gcgtcgatca aaaattatta ttacaaccac 109140
   gccaaatgga ttaaatcact tttatgatat ttggactgca gcggttgaag gtaaatcagg 109200
   ctttgaacct tatactgcaa tttggaactc ggttaaagaa cgtttataca acgatgaaga 109260
   tatttttgat gatggatggc agtggtctaa acagactatt tcagcctctt cattaacaca 109320
   gttccgtcag gaacacacgg cggctttcga agggacatca ggcacactta tttccggtat 109380
   gaaattagct atattagatt atattgaagt tacacctgat agccatggat ttcaccagtt 109440
   caaaaagcct gaggaaggtc ataaatatat tgcgacgtta gactgttctg aaggacgtgg 109500
   acaggattat catgcaatgc atattattga tgttacgaca gataaatggg aacaagttgg 109560
   tgttctacac tctaatacta tttcacacct tatactccct gatattgtgt ttaaatatct 109620
   aatggaatat aacgaatgtc caatttatat tgaattgaac tcaacaggtg tttctgtcgc 109680
   taaatcgctt tatatggacc ttgaatatga aaacgttatt tgcgattcaa tgaatgattt 109740
   aggcatgaag caaagccgta gaactaagcc tgtaggttgc tctacattaa aggatcttat 109800
   tgaaaaagac aaactcaaaa taaaccatag ggctactatt caagaattca gaacgtttag 109860
   tgaaaagggt gtatcttggg ctgctgaaga aggttaccac gacgacttag tgatgggtct 109920
   tgttattttc ggatggttgt caacgcagca gaaatttgca gactatgcgg ataaagatga 109980
   catgcgcctt gcatcagaag tattctcaag agaattacag gatatgaatg acgactacgc 110040
   cccggttata tttgttgatt gtgcaagtaa ttcggcagaa tataatccat cagcacatgg 110100
   cctgtcaatg gtataaataa aataaagcaa attaagagga attaaaatgg cacttctctc 110160
   tccgggcgtt gagctcaaag aaactaccgt acagagtact gtagttaata actccactgg 110220
   taccgctgcc ctggctggaa aattccaatg gggacctgca ttccagatta aacaaatcac 110280
   cgatgaagtt gcgttggttg atatgtttgg tactcctaac acagacaccg cagattattt 110340
   catgtctgct atgaacttcc ttcaatatgg taatgacctt cgtgttgtac gcgctgttga 110400
   tcgtgatact gctaagaact catctcctgt ggcaggaaac attaatttta ctatttcttc 110460
   ggctggcaca aactacagag tcggtgataa agttgtagtt aaatattcta cagatgttat 110520
   cgaaccagac ggtgaagtta cttcagttga ttctgacggt aaaattttga atatctttat 110580
   tccttcaggt aaaattatcg ctaaagcgaa agaaatcggc gaatatcctg aattaggttc 110640
   aaactggact gccgaaatgt ctgggtcttc ttctggtctg tctgcagtaa ttactattga 110700
   ttcagttgta atggattctg gtattctgtt gactgaagtt gaaacttcag aagaagctat 110760
   tacttctcta actttccaag aatctattaa aaaatatggt gtcccgggtg tagttgctct 110820
   ttatccaggt gaacttggag accagctcga aattgaaatc gtatctaaag cagactatga 110880
   caaaggtgct tcagcccagt taaaaattta tcctgatggt ggcactcgtt attctactgc 110940
   taaagcaatc tttgggtacg gtccacagac tgatgaccaa tatgcaatta tcgttcgtcg 111000
   caacgacgcg gtagtccaga gcgtagttct ttctactaaa cgtggtgaac gagatattta 111060
   tggtagcaat atcttcattg atgatttctt tgctaaaggc gcaagcaatt atattttcgc 111120
   tacggcacaa ggttggccaa aaggtttctc tggcgtaatc aaactgaatg gtggcttatc 111180
   atctaatgaa acggttgaag ccggtgattt aatggaagct tgggatttgt ttgctgaccg 111240
   tgaatcggtt aatgcacagt tgtttattgc tggttcttgt gctggtgaat ctttagaagt 111300
   tgcttctaca gttcagaaac acgttgtagc aattggtgat tctcgtcaag attgcttggt 111360
   tctttgctcg cctccacgtg cagctgtagt tggtattcct gttaatcgtg cagttgataa 111420
   tctggttgat tggcgtacag cgtcgggaac ttacactgat aataacttta atataagttc 111480
   tacttatgct gctattgatg gtaactataa atatcaatat gacaaatata acgatgtgaa 111540
   tcgttgggtt ccattagctg ctgatatcgc cggtctttgt gctcgaacag acaatatttc 111600
   tcagccttgg atgtctccag ccggttataa ccgtggacaa attcttaacg ttattaagct 111660
   tgcaattgaa actcgtcaag cacaacgaga tcgtttgtat caggaagcta ttaaccctgt 111720
   tactggtacc ggtggtgatg gatatgttct gtatggtgat aaaactgcca cttctgttcc 111780
   ttcgccattt gaccgtatta acgtacgtcg tttgtttaac atggttaaaa cgaacattgg 111840
   ttcggcatcc aaatatcgcc tgtttgaatt gaacaacgca ttcacccgtt cttctttccg 111900
   tacagagact tctcaatatc tacagggtat taaagctctt ggtggtgtat ataactttaa 111960
   agttgtttgt gatactacca ataacacccc agcagttatc gaccgtaatg aatttgtagc 112020
   tactttctac ttacaacctg cccgctcgat aaattatatt acgttgaact ttgtagcaac 112080
   agcaactggt gctgatttcg acgaactgat tggtgctgta ggtggctaat aagacgcctg 112140
   atgggctatt tcggtagccc atataaatat actatatcgt ttaaaaatta attctatggg 112200
   catccggtta atcctaagcc catattacta cagaggctaa tatgtttgta gatgacgtaa 112260
   ctcgtgcctt cgaatctggt gatttcgctc gccctaacct gttcgaagta gaaatctctt 112320
   atcttgggca aaactttagc ttccagtgtc gtgctactgc tcttcctgct gcgatcgttg 112380
   aaaaggttcc tgtttcttat atgaaccgta aaattaacgt ggccggtgac cgtacattcg 112440
   atgattggac tattacagta atgaacgacg atgctcatag tattcgtcag aaattcgttg 112500
   attggcaagg tattgcagca ggacaaggta atgaaattac cggtggtaaa cctgctgaat 112560
   ataaaaagac tgctatcgtt cgtcaatttg ctcgtgatgc taaaactgtc actaaagaag 112620
   ttgaaattgt tggcttgtgg cctactaacg ttggtgaagt atctcttgac tgggattcga 112680
   ataacgagat tgaaacgttc gaagtaactc ttgctcttga ttggtgggaa taatctggtt 112740
   ggggagaaat ccccagcttc ccgtgatgac ggagaagtcc atataaatat aactataatt 112800
   cccattcgga gaatacaatg aaatttaata tcttaagttt gtttgctcca tgggcaaaaa 112860
   tggacgaacg tgattacaaa gaccaagaaa aagaaaattt agaatcgatc accacaccaa 112920
   aattagacga cggcgcgaaa gaatacgaag tatctgaaaa tgaagcacag caaacatata 112980
   atgctatgtt tcagagaatg ttcggtagtc aagaaccagg gctcaaatca acccgtgaat 113040
   taatcgatac gtatcgaaat ttgatgacga actatgaagt ggataatgcc gtctctgaaa 113100
   tagtttccga tgctatcgta tatgaagatg atacagaagt cgtttctata aatttagaca 113160
   acacaaaatt tagtccaaat atcaaatcaa tgatgttgga tgaatttaac gaagtgttaa 113220
   atcatttatc tttccaacgt aaaggttcag atcattttag acgttggtat gtggattctc 113280
   gaattttctt ccataaaatt attgacccta aacgccctaa agaaggtatt aaagagcttc 113340
   gtcgtttaga cccacgtcaa gttcaatatg tccgtgaagt tattacaacc actgaagctg 113400
   gtgttaaaat agtcaagggt tataaagaat atttcattta tgacacatca cacgaatctt 113460
   atgcttgtga tggtcgcata tatgaagctg gcacaaaaat aaaaatccct aaagccgcga 113520
   ttgtttatgc ccattctggt ttagttgatt gttgtggtaa aaatatcatt ggttatttgc 113580
   atcgggctat taagccggcg aaccaattaa aacttcttga agatgctgtt gtaatttatc 113640
   gtattactcg tgctcccgat cgtcgtgtat ggtacgttga tacaggtaat atgccttcaa 113700
   gaaaagcagc agaacatatg caacatgtta tgaacacgat gaaaaaccgt attgcatatg 113760
   atgctaccac aggcaagatt aaaaaccagc agcatattat gtcgatgacc gaagattatt 113820
   ggttacaacg acgtgatggt aaagcagtaa cagaagttga tacattaccg ggtgcagata 113880
   atactggaaa tatggaagat gtacgttggt tccgtaatgc gctttatatg gctttacgta 113940
   ttcctattac ccgtattcca agcgaccaag gtggtataca gtttgatgct ggtacttcta 114000
   ttacacgaga tgaattgaca tttggtaaat ttattcgtga gctgcaacat aaatttgaag 114060
   agatattcct agacccgctt aaaactaatt taattcttaa aggaattatt acagaagatg 114120
   agtggaatga tgaaataaat aatattaaga taaaatttca tcgggatagt tatttctcgg 114180
   aattaaagga tgctgagatt ctggagcgtc gcattaatat gcttcagatg gccgaaccat 114240
   ttattggtaa atatatttct cacagaacag ctatgaaaga tattcttcag atgtctgacg 114300
   aggaaattga acaagaggct aagcaaattg aagaagagtc gaaagaggct cgtttccaag 114360
   accccgacca agaacaagag gatttttaat ggacgattta attcaagcta ttaaatcaaa 114420
   cgacctcgtt gctactcgaa agttttttga aagtgcaatg gcagaaaaaa cggttcgttt 114480
   gattgaagca cgcaaagcag aaatcgcttc tcaatttttg attgaaggcg aagaacctga 114540
   agaagaagaa aagaaagcta aagcttcgga agacgacgct gatgaaggcg atgacgaaga 114600
   cgaagatgat gaggacgatg aataatgtat cttatccctg aatcttatga attggtactc 114660
   gaaaatgtcg aagcacttat tcctgaagca cagggccgaa tcgatgcatt gtcttctgct 114720
   ctcgatattg acgatataaa tactattatc gagaacatgc ttgaaactga aacggattta 114780
   gctgttgcaa tggcttccat tattaatgaa gagcagttaa atgagtttat cgttaaacat 114840
   gtttcttctc gcggtgaagt cacccgcact aaagaccgta aaactcgtga acgtaacgcg 114900
   ttccaaacaa ccggtctttc taaagcaaaa cgtagacaaa tcgctcgtaa ggtcgttaaa 114960
   gctaaaaaag ctaacccttc aggccaagtt aaaggcatgc gtaagcgtaa aaaagctctt 115020
   aaacgtcgta aagcattagg attaagctaa tgaataaacc cgagttactt attgaaactt 115080
   ggggtcaacc cggtgagatt attgatggcg ttccaatgtt ggaatctcat gatggaaaag 115140
   attctggctt aaaaccaggg ctttatatag aaggtatttt tcttcaggca gaggttgtta 115200
   accgaaataa acgtttatat cctaaacgtg ttttggaaaa agccgtaagc gactatatta 115260
   aagaacaagt cgcaaccaaa caagcccttg gagaattaaa tcatccacct cgtgcaaatg 115320
   ttgaccctat gcaagccgct atcattattg aagatatgtg gtggaaagga aacgatgtat 115380
   atggacgcgc tagaattatt gaaggcgacc atggtcccgg cgataaatta gctgctaata 115440
   tacgagctgg ttggattcca ggtgtttcct ctcgtggtct tggttcttta accgacacca 115500
   ataaagggta ccgtatcgtg aacgaaggat ttaaattaac cgtcggtgta gatgccgtat 115560
   ggggaccttc tgctccagat gcttgggtta ctcctaaaca aatatcagaa tcagaaaatt 115620
   cggtggaaat caccaaaaac agtgctgatg aagcatttaa agctctcgca gagagtttaa 115680
   aagcattata aataataatg taatctaaac cacaggacac taaaatgatc aaagaacaac 115740
   ttttgaaaga agctcagaat atcgagactt cagttgctct taatagtgtt ttcgaatcag 115800
   tagaactgtc tccggacgtt aaagctaatt tcagcactgt attcgaagcc accgttaagc 115860
   aggaagcagt caaactagct gaatctcata tcaaagctat cgccgaaaaa gccgaagaag 115920
   aagttgaaaa ggctaaagaa gatgctgaag agaaagctga taaaaaattg gctgaacaag 115980
   cttctaaatt cctggaccat cttgcaaaag aatggctcgc agaaaaccaa atcgcagttg 116040
   ataaaggcat taaagctgat ttgttcgaat ccatgcttgg tggtctgaaa gaactgtttg 116100
   ttgaacataa cgttgttgtt ccagaagaat ctgtagatgt tgtagcagaa atggaagaag 116160
   agctggccga acataaagaa gaaaccgctc gtttgttcga agaagttacc aaacgtgatg 116220
   catatatcaa ttacgttcag cgtgaaaccg ctattaatga aagcgtaaaa gacctgactg 116280
   aatctcagaa agaaaaagtt attggcctgg ttgaaggtat ggattattct gacgcatttg 116340
   gtactaagtt aactgctatt gtagaaatgg ttaaaggttc tactaaagaa gaagccgcta 116400
   ttaccgaaag tataaataca gttgacaatg acgctgccgg tcttaatttt gttgccgaag 116460
   cagttgacac taccacaact caggtagaac agaattctaa tgtaagttta tatgcgaaag 116520
   tcgcatctcg tttctaattt aaaggttaac acaaatgact actatcaaaa ctaaagctca 116580
   gctcgtagac aaatggaaag aactgctgga aggtgaaggc ctgccggaaa tcgctaatag 116640
   caagcaggct attatcgcta aaatcttcga aaaccaggaa aaagatttcg aagtatcccc 116700
   tgaatataaa gacgagaaaa tcgctcaggc atttggttct ttcttgaccg aagctgaaat 116760
   tggtggtgac catggttata atgctcagaa catcgcagca ggtcaaacct ctggtgcagt 116820
   aacccagatt gggccggccg ttatgggtat ggttcgtcgt gctattccta atctgatcgc 116880
   ttttgatatt tgtggtgttc agcctatgaa cagcccgacc ggtcaggtat ttgctctccg 116940
   tgcagtatat ggtaaagacc ctatcgctgc tggcgctaaa gaagctttcc atccgatgta 117000
   tgccccagac gcgatgttct ctggtcaggg tgctgctaag aaattcccag ctctggctgc 117060
   tgacactact accgttgtag gtgatgtcta tactcacttc ttccaagaaa ctggtactgt 117120
   atatctgcaa gcttctgctg tcgtaacact tgattctggt gcaactgatg cagctaaatt 117180
   agatgcagaa gttaagaaac aaatggaagc tggtgcactg gtagaaatcg ctgaaggtat 117240
   ggctacttct atcgctgaac tccaggaagg tttcaacggt tctaccgata acccatggaa 117300
   tgaaatgggc ttccgtatcg ataaacaagt tatcgaagct aaatctcgcc agctgaaagc 117360
   tgcttactct atcgaattag cacaagacct ccgtgcagta cacggtatgg atgctgatgc 117420
   tgaactgagc ggtattctgg ctacagaaat tatgctagaa atcaaccgtg aagttgttga 117480
   ttggattaac tactcagcac aggtcggtaa atctggtatg accctgactc ctggttctaa 117540
   agctggtgta tttgacttcc aggacccgat tgatatccgt ggtgctcgtt gggccggtga 117600
   aagctttaaa tctctgttgt tccagattga taaagaagca gttgaaattg ctcgtcaaac 117660
   cggtcgtggt gaaggtaact tcattatcgc ttcccgtaac gtagttaacg tactggcttc 117720
   agttgatact ggtatttctt acgctgcaca gggtctggct tccggtttta ataccgacac 117780
   tactaagtct gtatttgccg gtgtacttgg tggtaaatac cgcgtataca tcgaccagta 117840
   tgctaaacag gattacttca ccgttggtta caaaggcgct aacgaaatgg atgcaggtat 117900
   ctactatgct ccttacgttg cacttacccc actgcgtggt tccgatccta agaacttcca 117960
   gccggtaatg ggctttaaaa ctcgttacgg tatcggtgtt aacccgtttg ctgaaagttc 118020
   cctgcaggct ccaggtgctc gcatccagtc cggtatgccg tctatcctga acagccttgg 118080
   taagaacgct tacttccgcc gtgtatatgt aaaaggcatc taatcttaat tgattaaatc 118140
   ttattttggg agacttcggt ctcccatttt tggttttatt tctgtgcaat ttcgatgtaa 118200
   gttttcaaaa tattttgcca attaggcata ccttcttgga catatgccgg agaaccgtct 118260
   ttgtttttga atgatgcaag tttatcagtg gcccaataac ctttagctcg agcttcagaa 118320
   taaactttca tccatacttt gttgaaggca ttaccattaa cttttgcacc atatttttct 118380
   actaacttaa ctgcttcagc ttccagaact tttaaagtat ctcgtaagaa acgtgcttta 118440
   gtcattttgt ttactcctct gtagttgata agtctatagt atcacatacc aaatacgttg 118500
   taaacaatct ttataaataa tctatatcac ataaggaaaa aatgcaatga gtaaaatcca 118560
   aaaattattg cgtgaatcta caacgtctac tagcaactca atcggtcgcc caaatctcgt 118620
   tgctttgact cgcgctacga ctaaattaat atattctgac attgtagcaa cacaaagaac 118680
   taatcaacct gttgctgctt tttatggtat taaatatctt aacccagaca acgaattaac 118740
   gtttaaaact ggtgctactt atgcaggtga agctggatat gtagaccgag aaaaaattac 118800
   cgagctcaca gaagaatcaa aagtcacact aaataaaggc gattttttca aatataataa 118860
   tatcgtctat aaagtgctag aagatacacc atttgcagat attcaagaaa gtgatctaga 118920
   attagctctt cagattgcgg tcgttcattt aaaggtacgg ttattttcag atgcagctgt 118980
   gacaagcaaa tttgaaagct ctggtagcga aatttctgat gcaagattcc agattaataa 119040
   atggcaaact tcagtcaaat ctcgtaaact taaaaccggc ctcacggttg aattagcgca 119100
   agatttagaa gcgaatggat ttgatgcccc taacttctta gaagatttgc ttgctactga 119160
   aatggctgat gaaattaata aagatattct tcagtcttta attactgtat cgaaacgtta 119220
   taaagttact ggaattactg acactggatt tattgacttg agctatgcgt ctgctccgga 119280
   agcaggtcgt tcgttatatc gaatggtgtg tgaaatggta tcgcacattc aaaaagagtc 119340
   aacttatact gcaacgtttt gcgtagcttc agctcgtgca gctgctattc ttgctgcttc 119400
   cggttggtta aaacataaac cagaagatga taagtatctt tcacaaaatg cttacgggtt 119460
   cttagctaat ggtttaccgc tttattgcga tactaacagt ccattagatt atgtgatcgt 119520
   tggcgtagta gaaaatatcg gtgaaaaaga aattgttgga tcaattttct atgctccgta 119580
   tacagaaggt ctcgacttag atgacccgga acatgttggt gcatttaaag ttgtagttga 119640
   tccggaaagc ttgcaaccgt ctatcggttt attagttaga tacgctttat cagcaaatcc 119700
   ttacacagta gcaaaagacg aaaaagaagc gagaataatt gatggcgggg acatggataa 119760
   aatggcagga cgttcagact tgtctgtttt acttggtgtt aaattgccta aaattattat 119820
   agatgaataa aaaaaaaagg gaccgtaagg tccctttttt cgttatgata ctaattcaat 119880
   ccatgcagga cgcaatacat cttgcaccat tcggactaat tctttcttaa caagattcgg 119940
   attatcagat gatgttaata caataccttc acgagaagtt tcttccagaa tatcttgaac 120000
   agtcaatccc atcactttgc caaaatcttt cggagttact gtcccaatct tagaaatcac 120060
   attgttaaca cgatttaatg taacataaca agctagaaca tcaagtaagt ttttgtcaat 120120
   ttcggttaat ggaacctgag ttttaatagg cttatcagac tttttctttt cactgaattt 120180
   agagttcttg cacttaatcg ctacacgagt accgtttggc agccatttag ggaaacaagg 120240
   cttcagaaca taaccttctg cagtattatc accaataaca ttagcatcaa atacgcaatt 120300
   atttgcttcg acaaggtctt ccgatgccgt agcgttataa gcggctaaga cggaatcaag 120360
   gtcgtttgga atcgtgataa gagcatcaaa tgttccacgt ccaagcattg gtgccatttt 120420
   aaaaccaaac gtattacaga aatcttgcat ttcgtaatca ctcatgtaat aagtatcacc 120480
   gctttcagtg tttatgatga tatcgaatac atagaaatct ttttcaccat agtcaacacc 120540
   tttctgaatt ccaccaccag cgaattcacc aaagacttga tatgtcaccg gcactgaagt 120600
   tgaaatactg cccatgactt cctgaacggc tttaatagct ttatcatact tcttgagtac 120660
   gatttcgtaa ccataaaaat cttctgctgg aagaataggg cctgtgcgct tggcgcatgt 120720
   gacgttgtca cgttcaataa ttaaactaaa gttggtcccg tggattttct cacgtgctac 120780
   ccaaacacca gtagtcaaac cattagtgta gagtttttca ataaacttag agttgtagtg 120840
   gttttcaaga ctgctgtatt tcttaaacat aaatcaccat tataaataat aatatcaaaa 120900
   taattagcag agactctatc tccgacccga tgtttaattt gaatggatag aacctagatt 120960
   cattatacca tcaaaaaatg taaagcatat tagaacttga tttctatcca atgcccgtca 121020
   cgttggtcat gattaatttt gtactgaaag ccgttatcac gaagccattg accgacttgc 121080
   tcggtgatag ttaaatcatt ctttgatgga tagtacgtga attgtgtctg gcctttgtca 121140
   gcggctttct ctgccatcac agagaagtta gtaataaaat cactcaacaa attagcttta 121200
   atggccagag ccctttctct taattcctgt gcaaaactca tttcttcacc tttttattaa 121260
   tcaaacgttt acggtataca actttttctt ttgctttaaa ctttgaccga gctgtctcgg 121320
   ctttagtcca tagtaatcca acttctttag gttgtaaacc aattttttga gcaatttcta 121380
   cgaatgaaaa tcccgcctca tgtaatgtat aaacttcaac ttcagctctc ataatcaatc 121440
   ccgtattatt tggtttgtgc cattatatac atcaatctca agttgtgttt tgtatgcttt 121500
   aaaaacgaag aaaggggccg aagcccctta cgattatgga tatgtataga tgataccagt 121560
   ttccaaagca gttttatgga tgatgtatcc attacgtgat tcttgaacat caacttctgg 121620
   atagtctttc atcatcttct gaagcgtata acgatgcaag taatacttac taccaggatc 121680
   gtcaattttc caatctttac cttcttcagt cattttttgg atttcatcca taacccacaa 121740
   accgcaccag atgtaagctg aagtacgatg tggaagagga tgaacataag gtagttcacc 121800
   ttctggctca attggagtct gttcagcaat aacaacttca gcggttgaac taattgtctt 121860
   tgaagtataa ttatctttag ttaatactgc ttcaacttta attgtatgag tacccgccga 121920
   ggaggtatca atactgtaag tattagtaga attactttct gctacgttgt ctttcttcca 121980
   agaatacaca atattagtat cacctggagc accagtaaca gatgctgtta aagttgcagt 122040
   tccaccaaca gtagtattaa ctgatggcgg agataaagaa atggtggcac tattaaatga 122100
   cgcattatta gttactactg ttgcttctgc tgaaatttct tttgtaacat aatttgttgc 122160
   agaaacagat acagtgcatt taagagtttt acttccgacc tcggttggag tgaatttata 122220
   cgtagcagaa gttgcatccg gaatattact gtcatccatt gaccactgga atgatgtagt 122280
   agcacctgaa ggtgcaccag agacatttgc agttaaagta ataggaacac caaagaccgt 122340
   agtcggagac tcaggcgtaa tttttagtgt tgaagaatta gcctttttat ttacagtcaa 122400
   agaaaccgct gatgattcta ctgttttatc aacgtaatca gttgcagtca ctgtgacgga 122460
   gcaagttata gacttagttc cttcggatgt tggcgtatat tcaaaggttg attgcttttg 122520
   accatcaaca gcaatcccgt caactttcca tgtataagca atagcagcac cactaggttg 122580
   attgcttaca ttagcagtga atgttgttgc tgttccaatt tctactgccg ccggagaagc 122640
   cggagtaaca gcgacggtag aattattttc tttcttatta acagtgatag ttgttgacgc 122700
   ttcagctatt tccggatctc ctgcggaaag tgtattagtt gcaaccactt taattttttt 122760
   ttgttcctgc cggagaagca agagtataat ccattgtagc agccgtagac tcctgtgcta 122820
   tatcatctac agtccagacg tatgtaatag taccatctgg agtttcacct gcaggtgtag 122880
   cagtgaatgt ttgtgaacct ccaataaccc ctgtaggagt taaaggagat aacgtgacag 122940
   tgaatgtcat aagttatcct tattttaatg ttatgaaaga agaattacga gtttcacgaa 123000
   ttagaactga tccatcgcga ttaatataat aaattaagct aaataatgtt tggtgagcag 123060
   aagcgtgcac aaaactagtt gggcgagatt tccaatctgg ggtttcggct atccattgat 123120
   aaatccacca aggaacagta acatatcctg ggcctttgcc taatttaatg agtgtaggac 123180
   taaaattttc aggcagaaca aattttactt ctttatttac ctctgtctca ggctcttttt 123240
   cagattcaac aacctgagta attgcttctt cgaatttatc atggtcgaat tctttttctt 123300
   gttcgacaac ttctaatact tcaacttgtt ctgttggaga atcaaacaac agaatagctt 123360
   cttctttaac ttcttcgttg gtgaaattaa caccgtccac atcatctgcg gcagtaatta 123420
   aatcggtgat agataatcca tcagtttcag gtaatggttc atcagccagt gcgttaagag 123480
   cttcttcgat atcaataaca atattatcga acgattttgt tttcttaaca ctaatgccaa 123540
   attgttccgc atattcaaac agtttagctt tggcttcctt tttatcagaa agagcacgta 123600
   actcttcaat atagtcttta tcgatcatgg tatttcctca ttataaatat aactatattt 123660
   attacatggt aatttgacat gactgacatt aaagtacatt tttatgattt tagtcacgtt 123720
   cgcattgaat gcgacgaaag tacgttttac gaacttcgtg acttttttag ctttgaagcc 123780
   gacgggtata aatttaaccc aaaatatcgc tatgggcaat gggatggtag aattcgactt 123840
   ttagattaca atcgtttgtt accatatggt cttgtaggac aaataaaaaa gttctgtaac 123900
   aatatgagtt attctatatg gattgaccct aaaatctttg agactgaaga ccttaccagg 123960
   gaagattttg acgcgtggct ttctaaacaa gaaatttatt caggcaacgc caaaatcgaa 124020
   ccacattggt atcaaaaaga tgcagtatat gaaggtctag ttaatcgtcg tagaatttta 124080
   aaccttccga catcagcagg taaatcacta atccaagcac ttttagctcg gtattattta 124140
   gagaactatg aaggtaaaat tctcattatc gtccctacaa cagcattgac aacccaaatg 124200
   gctaatgatt ttgttgatta tcgcttgttt agtcattcga tgattaagaa aattggcggt 124260
   ggtgctgata aagccgacaa atctaaaaac gacgctccaa ttattgttgg cacatggcaa 124320
   acggtagtta agcagcctaa agaatggttc tctcagttcg gtatgatgat gaacgatgaa 124380
   tgccacttag caacaggcaa aagtatttct tcaatcatct caggattaaa taattgcatg 124440
   tttaaattcg gtttatctgg ctcattaaga gacggaaaag ccaatgtaat gcagtacgta 124500
   ggtatgttcg gcgaaatctt ccgtcctgtt agcacgtcta aattaatgga agacggacag 124560
   gtaactgagc ttaagattaa tagtattttc cttcgttatc cagacgaatt cgccaccaaa 124620
   ttaaaaggta aaacatacca agaagaagtt aaaattatta ccggactaaa acgtcgtaca 124680
   aagtggattg ctcaactttc agtcaaattg gctaagaaag acgagaatgc ctttgtaatg 124740
   tttaaacacg ttacacacgg taaagaaata tttgaagcta ttaaagagct tggatacgaa 124800
   aaagtttatt acgtatcagg tgaagttgat actgaaacac gtaacgcact taaagtcatg 124860
   gcagaaaatg gtaaaggcat tattattgtc gcatcatatg gagtattctc taccggtatt 124920
   tcagtcaaga atcttcatca tgttattttt gcccatggtg ttaaatcaaa aattatcgta 124980
   cttcaaaccg taggtcgcgt gcttcgtaag catggttcta aagctgttgc tacagtttgg 125040
   gatctcatcg atgacgcagg tgtaaaacct aaatcattaa atactaaaaa gaagtatact 125100
   catcttaatt acttgttaaa acacggtatt gatcgtatcc aacgttacgc tgatgagaaa 125160
   tttaactatg taatgaaaac aattaattta taagggcttc ggcccttagg agataaaaat 125220
   ggtacttgaa tttaaacaat ttttatacga agcatctatt gacgaattca tgggtaaaat 125280
   tgctagttgt cataccctag aagggcttga agaattggaa gcttattata agaaacgtat 125340
   aaaagaaacg gaattaaaag atacagatga tatttctgtc agggatgcct tagcaggtaa 125400
   gcgtgccgaa cttgaaggcg acgatgagga tgtggaagaa gacttctaat taaaaaaaag 125460
   gcccgacctt tttggaaggg ccaaaaacca taggcaaaaa aggtaacact atagtaaaag 125520
   ttgggtttcg ttcaattgct cttctgaact ttctgaaacc ggcaattgtt gtacataatt 125580
   ataacaagga ccaggatgtg ctggaccttt gtctgtttca acaaccaatg cagaatcgat 125640
   tggagtttta cagacaacac aaatcttatc tgacatgatt gcctcctaag ttgaaattac 125700
   atatctattt ataccactcg catgcacata tcgataccca tatcttttgt ataaaaatac 125760
   tcgtcgatat acccagcaaa cttcccttta atataaagga cgtcttcacc acaagggtgg 125820
   tctgccagga tacgaatatc ctgacgtcgt agacgatact tttccattaa gaatttaatt 125880
   tctgtttcaa attcttcatc attattaaag gcatcttcaa ttgaataacg cattattgcc 125940
   ccgcttcaaa ttctcgcata tcctggatat gtttaagtgc aaaactacga gacttaactg 126000
   cgtcaagtgc accactacag aactcaagta aaattcccca gtattgtaaa gtagtctcta 126060
   ttttaagcac atctttatct gcggccagga cagttttcat ttcagatttc tcatatcggt 126120
   ccatactgaa ttcatcacca tcacctcgtc ccgagtagta gtctaatctt gcttttagag 126180
   cagttttctt ctgagtctct atacgcaaca tttctttacg aataaatgag tgtttacgca 126240
   gccatttgct gtataactta acattattcg ccgtctcata ttgcaacttt gtcatatcaa 126300
   tagctaagtc tgcgtctaac tcttcctgta aatcttccag cttcataata ttctctcttt 126360
   taactaataa tctcccggct gtatcggaag gacctaaaac cattatacat aggttgcctt 126420
   gaagcattac actctattgc ttgacgctaa ctgaagtttg atttgttcga tatcatcagg 126480
   gttatctacg actgaaaatc taatctctac tataactgta tagtcatcat atactggcgt 126540
   tacacttaca gaaagtttgt cgatgcgtgg ctcatagtta cgaacagctg cctggatatt 126600
   acgctcaata gtatcggctg taagtggtgt catgttctcg aaaagttcat caaccaaatc 126660
   gcatccaaat tcagggtcga aggggcgtga gccttttcta gttgtcacta tgccaagaag 126720
   actattttta atagaacgaa gtcctacaga acgagcaacg tccttgttcc aatccatacg 126780
   catttctggg tcgatatctg aataaagttt attgatatta gacattatag taactcaaag 126840
   aattctttga ggcctcgtat tgtgtgaacg tgtgacgccc cgcatttggg acatttaata 126900
   ggggttgcca gataaattga tggcgacacc aaaagatttt taatattgat catgtcatct 126960
   tctgttataa ggccataaag gtcgtttatt tctgcttcag ttaaatcttc aacagggatg 127020
   gactctccat tgacaaaaac agcctcaata catgaagcaa tcattaacgc gatattttta 127080
   tcatcaaata atttaggttg acggaactta attttaatac cactgaacga ataccaggga 127140
   tcggcctgtt catctaattg agtgtgcaat aaattcatat agacttgaaa tgaatgccca 127200
   caggaacaat tccaggtgtg ttcttgattc acttcaccta atgaatgtgc ccacagattt 127260
   accaacaata actcggcttc ttgtttagtc aagtttttag cgttagagca attagtgata 127320
   atattgttca ctgctgattc aatagtaccg gtagcccgag cacctataag tcctaaatat 127380
   tcttttaaag taaaggctct acaattcact tctttatcgc ctaaacgtac ggtaaacgta 127440
   tatttgtaca tgttaactcc tttattgtgt atttataaat aatagaaaag gagtaattat 127500
   ggctaatata attcgttgta aattaccgga tggtgttcat cgtttcaagc cgttcaccgt 127560
   ggcggattat agagactttt tactagttag aaatgatatc gaacatcgtt cgccacaaga 127620
   acaaaaagaa ataattgctg atctgattga tgattatttt ggcgagtacc ctaaaacatg 127680
   gcaaccattt atattcctgc aagtttttgc aggctctata ggcaaaacaa aagttccagt 127740
   tattttcact tgccctaaat gctctaaaga aaaaactgca ccatttgaga tatatcaaaa 127800
   agaactagtc gaacctgaac ttgatgttgc tggtataaaa atacgttttt ctttccctga 127860
   aaagttttat gacaacaaag cattaatgat aagtgaaaac ataaaagaaa tatattataa 127920
   tgatgaatgg tacccatgga atgatttgac tgaagagaac caaatccaag taatagaagc 127980
   aatagacatt gactcgctcg agaaggttat tgcttctatg aaccctatta atttgaccct 128040
   tagattaggt tgttgtgaac gacatgttaa aacatatact gatatcttag aggtttttaa 128100
   attgttagtt aaccctgatg agatttttac attctatcag ataaaccatt cactagtaaa 128160
   gagtcaatac acattggatt ctattatgca aatgattcct gttgaacgtg gtattgcttt 128220
   aacattagta gaaaaggacc ataaaaaatg acagtattcc aacgtccagg atatcctaac 128280
   ttaagcgtta aattatacca agattattcg gcttggcaag aaaatagata cgttgaactc 128340
   gcagcaacaa taacaacttt aaccatgcga gattcgctat atggaagaaa tgaagggatt 128400
   cttcaatttt atgacactaa aaacatccac actaaaatgg acggtagaca gatagtccaa 128460
   atctctgttt caaattcaaa cacacctctc caagttagaa ctcgaattta tggatgtaga 128520
   cattattctg tgtctgtgga ctcaaaaggt gacaatatta ttgcaattga gcttggaaca 128580
   atacattcta tagagaacct taaatttggg cgtccattct tcccagacgc aggcgaatct 128640
   atacgtgaaa tgttaggtgt catatacaaa gaccgtacgt taattactcc gccaataaac 128700
   actattaatg cttatgttcc tgatattcct tggacaagca catttgatga ttatttggca 128760
   tatgtcagag aattaggact tgctacagcg agtgataagt tcgtattcgt atggcaagat 128820
   attttaggcg taaacatgat tgactacgat actcttattg gtcaagaagg cattaagatg 128880
   attgtaggtg agccaaatac tgtaggtcag tatattcaag agcttgagta tccattagta 128940
   tgggatttta cctggatgac taaagccaac caatttacaa gagaccctat taaaaacgcc 129000
   acgatattcg cccattcatt tttagataca tctattccgg ttattgttac aggtgatggt 129060
   gataatgcta ttcttgtgtc aagatccggc ggatattccg aaatgaccta tcgtaatgga 129120
   tttgaagaag caagtcgtct tcagacaatg gctcaatacg acggttatgc taaatgcact 129180
   actacaggta attttaacat aacgcctgct actaaaatta tatttgttga ccaaaagaac 129240
   caattcaaat ccgaatttta cgtggatgaa gttattcatg agttatcaaa taacaactca 129300
   caaactcatc tgtacatgtt taccaactca atggtgttag aacctgttaa cccagttaag 129360
   gttaaaaatg aacttaaatc tgattctacc tctaaagaaa ataattctac cacagtctaa 129420
   taaaaaggta atgattccca aattagggct caaacattat aatcttttaa aggatgttaa 129480
   aggtcctgat gaaaacatga aaatcttagc tgattctatt tgtaaaaata tgagtcctgc 129540
   tgattttgat tttgcatgtc tacatatatt ggaatttaat aacaaactta agtctgaagt 129600
   tgaaaaagac ggattcacat ataaactaga tgacgtctat gtatgccaac gaacagaatt 129660
   ccaattccaa ggcaatactt tttattttag acctccaggc aaatttgaac aattcgctac 129720
   aatcagtgaa atgttatcaa attgcttaat aaaagttaat gacgaagaga aagaaatttc 129780
   tttccttgag atgcctgctt ttgtaatcaa atgggcagaa gacctttcga caactattgc 129840
   tattccaggc cctaatggtc ctattaaggg tatagcagaa attattggat tgcttgaatg 129900
   aaacctgaag aaatgaaatc catgcgtaga aataaggtca ttgccgataa taaaccacaa 129960
   aaagtagctg caacagcagc taccgattca ttagaagcat tgaatgatat ttcatcaaaa 130020
   ctagacgatg tccaagccgc gtctgaactc acatcgcaaa gtgtagaaga taaaggcaat 130080
   ggaattattg aatcaattgg agacttaaag aattcgacag ataacactgc tgaaggcaca 130140
   gagcttatcg cagaagttat tgaaaaacaa actgaagtca ctaaaagtat aaacgaagtt 130200
   tctagtgcaa taagctctaa gttagaccga ttagctacat tattagagca aaaattacaa 130260
   acatctaccg ctattcaaaa tacaggaggc acctctcttg aagtaattga gaacgcgata 130320
   cctgttaaag tggttgagaa cgaaacttct gatgagttgt ttaaagcatt acctacacct 130380
   gaaaagatta ataataagcc agacgaagat ttcttcccgg tcccggttca agaaagtgct 130440
   aattctactt ccgattctaa aggcggtatt agctttaaat tgagcgataa aattgcaatg 130500
   ctcactaaaa cagttcaaac cgggtttaat aaatcaatat caatttcaga cagaattgct 130560
   ggaatgcttt tcaaatacac tataacggct gctattgaag cagcaaaaat ggccgcactt 130620
   attcttggta ttgttatcgg tattgattta ttgatagtcc atttcaaata ttggacagac 130680
   aagttcactt ctgcctggga tttatttgat gaaaacttta ctaaattctc cgatgaagct 130740
   aaagagtggg gtaaattctt aagcgatata ttcacttcta ttgactcgat aaaacaattg 130800
   tgggaagctg gagactgggg tggtttaacc gttgctattg tcaaaggtgt aggaacagca 130860
   ttaatgaact taggtgaact aattcaatta ggaatggcta aactctccgc gtctatattg 130920
   cgtgctatag gttttggcga taccgcggac gaaatagaag gacgtgcttt ggaaggattc 130980
   caagaaacta ctgggaacaa attaaagaaa gaagaccaag agaaagtcgc taaatatcaa 131040
   atgaagcgtg acgatgggga attaggaacc gtttctaaag gtcttgatat gttacaacgt 131100
   ggcaaaacat tcgttactaa ctgggttcgt ggtaatgaca ataaagaaga gtttagtaca 131160
   agcgatgagc gtgctgcgga atcagcaaaa ttaaaagagt tacctgaaga ggagcgtaaa 131220
   gaggcgtata taaaagctaa tgaaactcgt gcggcactag ttagatttga ggattatatc 131280
   gataaaatcg atatgactaa tccggaaaat gctaaaaacg tcgagaagtc ttatgctgat 131340
   ttaagtaaac ttattaaaga ccctgaactt aataaaactc ctgttgttaa aaaggaaata 131400
   gatgctagat ttgaaaagct aaataataaa atggctgaag ccaaaaaggc tcagacaaca 131460
   gttaagcctg aatcttcgac taaatctcca gaagcaaaac aagttcagtc aatagagaaa 131520
   ggacgtgctt ctgaatctaa acaacagcaa ccggtagcgg cgattagtaa cacaaataat 131580
   gtcgtcaaaa agaataccgt agtccagaat atgacccctg taacaagtac aacggctcct 131640
   ggaattttcc atgccaccgg tgttaattaa ggattattat gattagtgta aaagaaatag 131700
   tagtagatgc aaaggattta aataaagcaa tcccagtctc agaaagtgct gggcaaagca 131760
   ctaaaaccga aacaactact aaaacttatg ttgctcaatt tcctacagga agagcagccg 131820
   gaaatgactc cacaggagac ttccaggtaa cagaccttta taagaacgga ttattattta 131880
   ccgcgtacaa tatgtctgca cgcgattcag gatccctcag gaatttacgt ccggcatacg 131940
   ccgggacttc atctaatggt atcatatcag atttaactga taacgtgaaa gatgcagtga 132000
   ctaaattctc aaatggattg cttcctgcca gagctaataa gtctactata aataaaactc 132060
   ctgttgctaa tatacttttg ccacgttcca aatctgatgt tgatacaact tcgcatcgtt 132120
   ttaatgatat cggtgatagc cttattacta aaggtggagg tactgctaca ggtgttttaa 132180
   gtaacattgc ttctactgca gtctttggtg cattagattc tattactcaa gggcttatgg 132240
   ctgataataa cgaacagatt tatacgactt ctcgtagtat gtatggcggt gctgagaacc 132300
   gtactaaagt ctttacatgg gatttaactc cacgttcaac tgaagacctt atggctatta 132360
   taaacattta ccaatatttt aactatttct catatggcga gaccggtaaa tcacaatacg 132420
   cccaagagat taaaagttat ttagacgaat ggtaccgttc aacgtttatt gagcctatga 132480
   caccagacga tgctgtaaaa aataagacgt tatttgaaaa gattactgca tcattaacaa 132540
   atgtattagt tgtaagcaac cctacgattt ggatggttaa aaactttgga cacacatcta 132600
   aatttgacgg gttaactgat gtatttggcc catgtcaaat tcaaagcgta cgttttgata 132660
   aaactccaaa tggacaattt aatggtctcg ccgttgctcc taacttacca tcaacattta 132720
   cgttagagat aacaatgcga gaaattatta cattaaaccg ttcttcactt tatgcgggga 132780
   ctttctaatg tactctttag aagaatttaa taaccaagct ataaatgcag atttccaacg 132840
   aaacaatatg tttagttgtg tatttgctac tacaccgtcg accaaatcgt cttcactaat 132900
   ttcgtcaatt ggtagttttg cttataataa cttagggttg gattccgatt ggctaggatt 132960
   gactcaaggt gatatcaatc agggcgtaac aaccctgatt acagcaggaa cacaaaaatt 133020
   aattcgaaaa tcaggtgtaa gtaaatatct gattggagca atgagtcaac gtacggtaca 133080
   aagcttgtta ggtgagttca cagtaggagc ttatctgata gacttcttca atatggcata 133140
   taataataca ggtttaatga tttattctgt aaaaatgcct gagaaccgat tatcttatga 133200
   aacagatttc aactataact caccgaacat ccgaattacc ggtcgtgaaa tggacccgtt 133260
   agtaataagc tttagaatgg attccgaagc atctaacttt agagcaatgc aagactgggt 133320
   aaactctgtc caagaccctg taacaggtct tcgtgcttta ccacaagacg tcgaagcaga 133380
   tattcaggtt aatttacatg cacgaaatgg tttaccacat accgccgtca tgttcactgg 133440
   atgtattcct gttagtgtct cttctcctga gttaacgtat gatggtgata atcagataac 133500
   agtttttgac gtaacatttg cttatcgcgt tatgcaatca ggtgctgtaa atagacaggc 133560
   cgctttagaa tggctggaat caggacttat tagtagtgtt tcaggaatgt ttgggaataa 133620
   tcaaaatgat tctgggctag gaagtgcagt atcaagactt tctagattag gtgggactgc 133680
   tggaggtgtg tctaatataa acacaatgac aggtatggtg aattctacat caagagtttt 133740
   aggtctataa gaaaaaggga gacaacgtct ccctttaggg gtttatttac ggaatgaaag 133800
   aaatgctggt gctcggaatg gagtatcatc tttagagtat ttaactaata cgaccattgg 133860
   tacctctgtt tctaaatgac ctgtcagtac accgataaaa atttgatcag tgtgttcagg 133920
   gtcacgaaag tttttaagag gttccatagc aatatggaat gcacctggga atactcgatt 133980
   caattcgtca atcacagctt cgttaaaaac atcatccgct gggatagcat cttcaactac 134040
   tagttcttga ccattaaagc gaagcataga tttcataacg ttttcctcat gttgttgata 134100
   ggatgatagt atcacacatc catgtgcttg taaactatcc ttgaacaaat ttaaacggaa 134160
   ccgattctaa atccattaaa gatgctaaca gtttcaatcc tttaggagca ctagtttttt 134220
   ctacaaatga ggagaacgat accggctcat ccgattcgat ttcaccagtc atagcaacca 134280
   cctcgcctgt ttccatgaga acatcaccat cgtaaatgac agcttcatcc aactggtctg 134340
   aggacatgac ttcagcttga ataaatttaa ggttggattc taaacctgca ccagtgtcag 134400
   atgacgcatc tgtaatttta tttatctgca agagaatgcc tctaggcaaa ataatttctt 134460
   gctcattagg gtgattactc aactgtcctg gataaatcac gttaacttta tgggcgccgt 134520
   caatagccca tccaacgtta acacggattt gttctggtgc atgcatagct gttcttactt 134580
   gactaggaga aatagtaaca ccttcgtcat tattgtcaat gttaagctct ttgcgaactt 134640
   cttcaggagc aagtccaagg gcaacgttct ctttaaaccc gccgaaaata ataggagcta 134700
   gtgaagtaga aacatagttt ctgaagtaga ataccttatt tttaaccatt gcttcgtaaa 134760
   taggcattct gatagactga gctctataca acgttagtcc ttcaggtaaa cggtctccat 134820
   ttaaaaacgc cgaatcaaga ccatcaatag cgcgtttaac ttcatcttca tctgcaacat 134880
   cataaaaatc cggtttataa cgtcctaaca acatattatt gatatcagta tatgcagaag 134940
   aggcatattc acgaatagct cgtttttcgg caatagagta ttgtttaggg tcccgattca 135000
   ttgttatatt aaaggtgcct ctggccgatt ctcgtgcata agtatacatc acattagaca 135060
   agaacgctga tttacgttgt ttccaagttt tttcagatac ttcagctaaa acgtcatcag 135120
   ccagttggaa attatctcgt ctaaaatctt caaaccattc gtctctaata gagtctgcaa 135180
   tttcgtttgc agcttcaaca aaagcagcta atgctttaac agatgttgtt ttttcggttt 135240
   taattttctt agcaaacttt tctaaagtcg gcttaataac actaccataa gaaacttcat 135300
   cagcaaatct aaaggattca tcaatacgat tataaatctt attttcgata tcagcaacta 135360
   aagtgccttt tgctgtagac gatgcttggg ctagtacgat ttcatatgct tcaggaagta 135420
   attcagctgt agctgggcca ctaacttctt tagcagaatt ttcgtatttc tggaatagct 135480
   caccttcttg gcggtcagct tctaaagatt gacttgcggc aatagcgcga cgagaaattt 135540
   tagtacgagc aataacaggc ttatctttac gtttttcttc tacagcagca atagaaccag 135600
   cgatagccgt ttctttggtg accttttcgc cagtcttttt actcacataa acttcaccaa 135660
   catcagaatc tactttcgtg tacaactctg tgttaatgtt agggatgcct ttaatatctt 135720
   caatgttggc atttttacga accatcatca catatgtgtg tttacctgtg aattgataca 135780
   ttgcagacaa tactttaaaa cgaccaccag ttttagtaga aactaaacga gcaagaagag 135840
   tttgaagttg ttgtccacgt cctttcaatt tcttggtagg gaatcggaac aaaactgcat 135900
   ccatacgaag agctttaacc tgttcataca ctgtattgaa aatggtgttg attgcatcaa 135960
   taggagcagg accaagacca tttttcaatt cagcgagatt acccttttca gaaagactca 136020
   taataacgac atgagcgtat ttgtcgccaa gtttaacttg ttttacagca tcgccttcgg 136080
   aaaggtaaga gaataaacga actacgatgt ttgtatcgat atcaccaatt ttccagactt 136140
   gtggaacttt agctttaggg ttcaaattga cgacaggaag actgccttca gattcaaaca 136200
   cttcatttaa ttgttcagac atagtgtttt ccttttagta ataatttata tttatgccaa 136260
   aaagagcccg aaggctctta atccacaatc ggaagcaaag tacgataaac ttcaaaatta 136320
   gggccttttt cgtacttatc aaaattgtcc ataaaaatat tataggaatc gttattagga 136380
   aaatctaata caatttggga acaagaggtg gaaacatcac caccatcttt attagcgaca 136440
   actactgttt ctaaataagc tttcattata aacctgtctg ggaccacggt ccaaatacgt 136500
   cttcaaatga atccgcttct gttttatcaa aacggaaccc tttaataatt ggaaggaaaa 136560
   tacctacagt accttcacgg cctttagaat gaacccaacc attacattcg caatcagcaa 136620
   tacgtccaat caatttacct tcacgagctt cagccattaa tcgttcacgg tctaaatcat 136680
   gacgctcatc taaaggaata agaatcttta caccatcaac tgttttatga gttgtatctt 136740
   taaaccctga accacaatcg gttgtaatac gacgacaacg tgatacgagt tcgacaccac 136800
   ctaatttgtt agggtcttta gagtgttcat agtatcctac aacttctaaa gcgatatcaa 136860
   taacttcttt aaatttaatt aggttcttag agcgtttgtt ttcccaatat gaatcacggt 136920
   ttttaaggat aataccttca agaccttggt caacatattt tttataaacc actttagctt 136980
   cgtcaagatt acgaactaac tgattttcaa ttggctcaat tcgattaaag ccttcagcca 137040
   tattttcaag agcagcaaaa cgaacatcgt atttctgacc tttaattttg ccatcagaat 137100
   agacttcatc taaaggaaca tagtcccagg cttgaagaac cataccttcc gcttctttag 137160
   gagaaatagt cccttgaaga gatttatttg ctaaaccgtt tgaagtgcta cggtctgcaa 137220
   cttgtacttc ttcggattct tcattatcgc caaataagaa cgacaggtca tttcctgagc 137280
   tcacagcctt tttaatatca aatgaatgat aaactaattc gccgtcgatt aaaaccccat 137340
   tagggtgtct ttcacgggct tctttagtca tttccattag ttcatccgct aatagagtaa 137400
   gaccatggta ttcgttacca gcacgagaaa agaattgaac accatcatca cgaacttcag 137460
   cgaaacaacg agcaccatct gcttttaatt gggcgaatgc cggccattta atatttttag 137520
   taatcaattt ctcatcatat gcagatgcta acatctgtgg ttgcaattga ataagtccag 137580
   gccacacctt attcgcgata gacactgaag cacctacttc aaggtcgcgc atcattacac 137640
   gacgaaggac ttctacatca tcaggtttac cgtcagcgat ataacccatg agctctttaa 137700
   ttgcagcatt accagtgagt ttacgagtgg ctaatgtgaa ctcaataaaa tccaacatgt 137760
   catctaattc aagaagacca tacgcttgag acctttcacc tggcccaggc catttcttaa 137820
   tataatactg gataccacgt gcatacgtta aacgatacac acgttcaagt aatttgttgt 137880
   ccttattttt cttaaggatt tcctgttttg ttttagtaga accgatcgca gctatttggt 137940
   ttaaaatatc taaaatcatt tgatatcctc gattttggtt acggttctat tataagccac 138000
   ttcatctaga agcgatttca gcgttttctg ataagtggta atccatttac cgagtccttc 138060
   aacataatat tctgaaatgt agatgaaccc ttgttcaaca cactctttaa atatttctgc 138120
   gtcttttact gaacgaaaca ccggccagtc tcgaaagaac atgttaacat ggccgtagtc 138180
   acgtgtggtg tttttgccat ctgtaatatc atgaactaca aacataatta gcccttaaca 138240
   atttatagac caagtccatt tagacgaatc taaaggaaag ttttgaatcg tccaccatgg 138300
   ctgcatatcg gttggtcctg gggttcgcca ggtatatgga gtacgtgttg tcgaggggcc 138360
   accaatttga ttaggccatg cacctggact tgttgcacca gattctttaa gcaaatcggc 138420
   caatcgtttt aaaaggtctt cacgatctga atttgaatta tgctctggcg gaaccaatcg 138480
   agattcaata tcatcccacg tgtgtacacg ttgtgcagtg cgaggaatat catcacgttc 138540
   accacgagcc aaccaataaa tcggtacatt aagaatttct gccgcatggt cacagtgatg 138600
   agctaaatcg tcaacgtaac aaatcacgtt atattttgtt ttagctagtt cgaataattg 138660
   ttcctttgaa gaatcatgac cacacatcat tacttcagag aaagcgcctg ggaacaaagc 138720
   attcaaatta aattgtctat tcaatcgagc atcaattgaa tcacctagcg ccgtcacggc 138780
   tacaaaatta tagtcttctt ttagtttatt gataactcga agagcatctg aataaggcgc 138840
   taagtaacga ataaaatcag agctattata tttttctatt aattgtttac caaggttatc 138900
   gtcggtattg aatagtttac caggagaaat aaatttttcg tcctgaatca tttttaaaat 138960
   atgttctaat ggaagattat atttttgggc aaaataagga agtccggatt gccaacttaa 139020
   acatacacca tcaatatcag tcaaaatagt aggcttcata aagagtctct taataggttt 139080
   aacacatcaa taaattcagc ttcagttagt attgtatcat cttttgttag gtggctagcc 139140
   atgctgtgct tcaaaatttt tcctttagag gcttgtaagg catcacgaaa ccccttattt 139200
   tggatcgcgg cttcaaaata tgcatttgtg tataattctt tccacgcttc ggagtatctt 139260
   gaaaacggaa ctcctaacca gaagagagtt ccacggtcct gagctcttgc ataagacctt 139320
   ccagcttgct gtgcagccaa tccagacaac ccaaatatac gtctttgttg ttcaacattt 139380
   ttcaccttac atccttggag aaatccttca agacctccga attgaatacc atccataaca 139440
   aaaggccatt gagcaaagtt acttaatgca catgacggcc acttgaaatt acttcttatt 139500
   tctaattcag acatctttaa cacttataat ttcaacatca gcccaatggc cgtaactagg 139560
   cagacggtct tcaatagata aaccaccgtc atttttaagc attttaattg tcttgataaa 139620
   tggttcatcc tgtggatgaa taggcgagca gtcagttaca cgataagtaa cttcgatcat 139680
   ttctgtacca aacaatttct ggattagttt attcaacatg tttcaaccct ttaataggag 139740
   ataactcttt acccatacgt atgtcagaag tcccatcaac ccattgaaca gcatacgctt 139800
   cattgataat tacggcggcg gttttaaatt catggagaac tttaaaaatc atcccaggga 139860
   ttccaacacc ttttaattgg actgtttgtg ctattaaaaa tttcatattt ttccacttgc 139920
   taaagaaatc attatactct aatccgtcct tggatgtaaa ctacttaacg aaattaattt 139980
   ccaccggatg aacaacaggt tgagcagtga tatattgagt aattgctttc atgactagag 140040
   cttcagaacc aggatttttc tcaacgaatt cacgtgcttg gttatgagct tcgagttcac 140100
   tcaataatcc ggaacgttgg catccacggt cttgagtacc ctgttcagat aaaaagacga 140160
   cgatgtattt ttcatcgtca tacggtttag acggttgagc aaatgttcct ttacttccaa 140220
   aaggagagtt atgacgccac atcacgccgt gaggagtttc aaccttcgta tattcgtgcg 140280
   ggccattttt cttaatttct tgatgtggaa cttcatagac cttagggtta ggacattcaa 140340
   taaaatgctc atctaaatcg gtcgagaata ttaaagtaaa acaattactg aatttaccaa 140400
   cacctgcctg ataaatttta ccttcagaag ttctaatttc acaaaccgag ccagcatcag 140460
   taacttctaa aacttcaaaa ggctcgataa ggtcatatga gaacatgttt ttaaggcgtg 140520
   gagtaatatc cccattttta aaattcattt tataaaaagt attaacttta aacataattt 140580
   cctcactggg agcttcggct cccaattaat ttagatatca aattcattaa gaactacatc 140640
   aaagattgct tcgagatgtt caggtttagc tcggttactc aggatatgac gaatccaggt 140700
   tttaaccaga agtttacgat taacaccatt ccagcaagga tgagtaccta aatcgcgctg 140760
   acggaaatca tcatcttaat gcaattttaa aatttgaacc ttccatcgtg attgaaaccg 140820
   tgatgccatt ttcaaatcgc atataaacat aattaggagt catgcactgt tcgatttcac 140880
   aaactgtacc gttttcgtgt ttccataagc aaataacttc agaagaacca gcgataccgt 140940
   tagaaacata tttacgttcg aagttgatgt agttcatttt attctccaga tgtttaagtt 141000
   gtttgttggt acaggtatat aataacatat cctgtaccaa agtaaactgt tttatgcaac 141060
   tttaatccaa ttgtctaaat ttaaaacacc tttggctttc atttttttcg atcaggttat 141120
   ctgctgtaat gcgtccattt tgataccatg taacatcttc agagtaacca gtttcttcgt 141180
   caacatatgt ttctgagcga attttaccgg taataatagt ttttaattcg tatgtgtcac 141240
   ctgtgaaaaa gtttgctaac acaattttcc aagagaaatc atttgaagga ccttcagggg 141300
   tcatacaact tacgaactca aattgctctt tgtataacat ccactggaat tctttaattt 141360
   cgatagcttt ataaccgtcg tggtctttac atttgatgta tgagtttaga ttgattttca 141420
   ttttattctc cagtttgttt tattgattag gtggtacaag tctatagtaa cacatcctac 141480
   aggagagtaa acatcttttt aaactttttc tgccctaaac gcacaaaagg gagaccgaag 141540
   tctccctaaa attacgcaac aacctttcca aaacgagaag catcatctcg aagaactgca 141600
   cgtgctctgc gcatgatctt ctcaactgtt tgattaatac gagagttcga cccacgcttg 141660
   tatccagcgc gtttagaggt accatcaact ttcttttcaa ctgctttctt tgctttagct 141720
   tgttttgcca ttataaattc tctttaaatg aaaatgcagg acttattgac attgcctgcg 141780
   caagccctca tgggaacata ggttttggat atttaacgac aggataacca taaacccgtc 141840
   atcataccaa ccacgactta gacagataac ctttttctga cgcgtgatag tacaaaatac 141900
   attcaagagg tacaccgtaa aactgccggg gtcttaaaac tataatgatt cgcaaatcat 141960
   taatcagaca attcgacggc tcctcgattt aacttacttc agggtaataa caaaatgacg 142020
   tactgcttta cgagctgctg aagccaaagg cttagcatat ttcagttcat cttttgcttc 142080
   cagttcagca gccagagtag cctgagcagg attcagatgt ttgaaatatc gcaggatttc 142140
   cagtgcttcg gcttcaacat caattgaagc gccgtagttt tcgtgaccgt tattccatgc 142200
   gttgcgttgc agatcaagag cgtgttgtag ttgtttaatc attttaaatt ctcaattcga 142260
   gataatattt agtagccacg ttcgtctgaa tttcaccttc tttcgacaag tctctcagtt 142320
   gtaggctacc gcttagaaga gtgttctctt catatagtta tttatatcac ttataaagac 142380
   acggaatagc tttatagtgg catgttacga atttctgttt aatttcttta ggttgtttaa 142440
   gtcccaaagc cacaaatgga tgtggaacat ttcgaatttg tccaactggt agtggagtca 142500
   aatcaccgac ttcaacaaaa ccttcaggaa catcaggacc tacagaatga actacacata 142560
   gttcaggaac ttcaccttga acacgtttac caatcacaag tcctgattcg gtaacttctt 142620
   catcacctgc ttgagcgggt tcagaaacca gaatgacata ttcaccaaca gcacgaattg 142680
   ggagttgtac ttcagacatc ttatttcctt gttgtttgac gatagattaa taataccata 142740
   ctattcgtaa agcatattac ataagaagca aacgttcgac cggagtacca tcaaccgaaa 142800
   ttactttatc aaggcggaaa gaacgccact gacctaactt ggtatcgaat gctcgaactg 142860
   attcacgagg ttcataacga acttctgttg ccggattgtt attttcatat ttgacttcaa 142920
   gttcatcgcg cgaagcaaac atagtgcgta catcaccgtt agccttttca aatacaacac 142980
   aatgaacgcc tacagacaga atagttttaa tacgttcacg aagaaccata gtttcttgtt 143040
   cagttaaaat catattattc accacaaaag tttcggattt gttcccaatt cagagaacga 143100
   agattattac gagagtactg aattacttca ataccagctt gttttagaat gtctgcccaa 143160
   ttttcaggag atctgtcgta taattctgcg tatacgacct ttttaatacc tgactgcgta 143220
   atagctttag tacaatcagg acatggagaa agcgtcgtat ataacgtagc accttcaata 143280
   gaattacctt tacgagcggc aaacaaaatt gcattcaatt ctgcatgaat ttcattatta 143340
   gcagaccatg cactatgagc agctcgatgt tctttggcta aaacaaaatt atctacccgt 143400
   ccaaatgctg tagtgccttc tttatgtcca gcaacgatga ctgggattgg tttattcttc 143460
   aaccaaccat tttcagatgc atgttcgcaa cagttaacgc cacctgcagg tgaaccgtta 143520
   tatccagtag agataatgcg tccatctttt tcaatgacgg caccaacttt ccatgaacaa 143580
   cattttgatt cttgagaaat cagatatgca atctgaaggt atgtgctagc tttcattatt 143640
   gaaccttatt aatataagtt aacagctctt tagtttgttc ccaactgatg cacgcatcgg 143700
   taacggaaac gccataggac atattatcag aaattttttg atttccttca tgtaggaacg 143760
   attctatcat aattcctttg accaaatcat tagctgctaa atttttacca ataccaattt 143820
   gattgctata atgcccatca gcgtttgcat ggctacagtc aaccattaca taatgattca 143880
   gacctactgc ccttgctttg ttagaagctt cttgaatatc agaagaatga tagttcgggc 143940
   cgttggtccc gccacgcaat acgatatgcg tattctgatt accttcagct tcaacaatgc 144000
   ctacagtacc atcgacgtcc atacccatat accgatgagg ccaggcggca ctatacatcg 144060
   cgtctgtagc tactttaata gaaccattgg ttgcgttctt gaagcctacg cacatcggta 144120
   gacctgaagc aatttcacga tgtgtctgac tttcagtcgt acgagcgcca atagcaaccc 144180
   atgagaaaat accagaaaga tacttaatcg tgaatgggtc taatacttca gttgctaatg 144240
   gaagtcccat acgtagaagt ttacgacaca atgttcgtgc aactataaga ccgtggttca 144300
   tatcaaaact tccgtcaaga tatggatcat ttacaagacc tttccaacct acggtagttc 144360
   gaggcttttc aaaataaacg cgcatcacta acagaacatt aggaagacga gtttgaagtt 144420
   cagccagacg tttgccgtat tcaaccgcag caatagggtc atgaattgaa caaggaccta 144480
   caacaataag tttgcgtggg tcttcaccgt tcataatcga attaacttgt tcgcgatgag 144540
   aagccacttg tgatgcgtaa gcttccgaca aaggaataat ctcagccagt tcttttggag 144600
   aaataagttt tccgacgaca ggtaacataa caatccttag gagatataaa cagattctga 144660
   ttcaacaata cctttaaccg gattagacag gctaagttgg tagtgctcag atttatgacc 144720
   attctcgata gtaatgagat agctctcatt atagtgttcc atatactcga gttgcttcaa 144780
   caagaccatc actgcctaat ttaagattag taatttggtc accatcttta ggattaacga 144840
   ttatgagaaa agaacgtgga gattcttgaa taactcgtaa tgttgcatcc tggaaacgtt 144900
   cagacacttt attgatgagt gcttgagcaa attcttttac tttaatctga aattcgtgta 144960
   ctgtaattgg attttcactt agcatttcaa tttcctcatt tgttggtaga gttatagtat 145020
   cacaactcca ccatgttgta aacttaaatt gcaaaacatt taatttcttg tgaatcgaaa 145080
   tcaacttcac actgaattac tagttcagca tcaagaccaa acacaacaat cacatgagcc 145140
   gagctgggca acaatgtaac agacacagaa tccacttggt caggcaccag agtattcaaa 145200
   acgtagagta gttcagagtg gaccttaagc tcggtagcag tatccagctt atcaaagaaa 145260
   tgatttgcga taatctgact aaaaacgacc tttacaattt cagaatattt agggaacata 145320
   attacctcag tgtatagtat ttactttaac tcgaacaaca aattgctcgg tcggttcgcc 145380
   tacaggaaca aactctacat gatattcgcc tttgtaacga gtattcaggt cgttacgaat 145440
   ttcggtaagc ttgttgatta atgtagaact catattaagt ccaactagtt tacgaagcat 145500
   tttgtatgct tcatcttcga tttcatgatg tttatcgtac atagttttca tttaaccatt 145560
   caattgcctg gtcgacgtta tcaaatatac caccgtccaa acgttcttca tgctcatagt 145620
   ccagcacatc aataccgaac tgtccattta ctaaaggcca ggctacaaac aaatattctt 145680
   tatgctgttc gatggcctca atcacttcat ataatcgttt cattaaaaat caccatggtt 145740
   aacttgccaa cactctaaac caatacgacg ccacatttcc acaacctggt tacggtcatc 145800
   aacggccaat ttcacatcat aatacggagc aatacaattc caaaagagtt cttccttaac 145860
   aacatcgtcc ttacgagtgt caccttgatt acgttgaatg tgcatttccc atggaataga 145920
   aaccgtgtcc atccattttt tagttgcttc gtaataacac attgaatctt ctttagttcc 145980
   agattcacga ccacttacag taataattgt atacccggcc tgatgaagca tcttaacata 146040
   ttcgacaacc atagggttcg gggcatctgt ggatagttta tccaactcat aaggtccacg 146100
   agctacatgt agagctaaag tcccgtcaag gtcgaagata accgctttag gtttacccgg 146160
   agttccttta taaaccggaa gtcctttgta ttcacgcata cgagaataca tcgaacgtag 146220
   aacatcaata ggaacggcct ttgtgccacg tttggcatta cgtttaacca attcagtcca 146280
   tggaacatcg aacacttgat atacaacttc atgaccccac tccttagcgt attcttccca 146340
   gacaagacga cgttcaggat ttaagttggt gtcagaaata ataacaccct tagttccgtc 146400
   ttaacagaga atcatatgtg ccgcgtcgtg ttgcatataa gttacgatac tttctttctt 146460
   ctttgtgtat ttgtactcgt cgcgttcttc gtggcccatg acggattgac gataatcgtc 146520
   gcggttgata ttaaagaacc caggattctt agcaataaat tcacgagccc aagtgctttt 146580
   accagaccca ggacaaccta cagtcaaaat aatctttttc acagttttac tacctcttca 146640
   gcaaattcaa attgtccacc aagaatttta gcaatttcac gtcctagttc agcacctttc 146700
   ttgcaatctt gcatagtcca agactcacca ttctgtgatt tacgaagagc ccgtgtattg 146760
   atatctgcta caagcgtatg gattaactta tcaattttgg cttggtccat tattttattc 146820
   ctaagaaaat ttcaagaata cgaatgttat gctcgcgccg gtctttatta atttcagcaa 146880
   cgtttttatt aattgacgta cttctaacgt ttgaagaaat aaatttaatc atatgacttt 146940
   tgagttgttg caaatccaac cctttttctt tagcagcttt acgaagagct ttacctgctt 147000
   catctagtgc ctttgctgca tctttgtcat aattgccaag tgacattcca cgacagatat 147060
   caatatactc atcagaccgt ttgatgtaat tctctagtaa tgttttcatt tagatttcct 147120
   caattatcca taggagcatt ataatctgct cctgagagtt tgtaaactat tttactaaag 147180
   attcaatata ctcgtgaaga gctttagtcg ctactgccca cttattctga tattcatttt 147240
   tcagattagc acgagactga actaacaatc ggtcattagt cggaggcaaa gattctaatc 147300
   gttgaatctg gcgcccataa atcatagccg ccatttcaga ttcatagata agccctttaa 147360
   tgtattcatg ctgttcttta gtcattggca tttttcctct tttaaactac tacgataata 147420
   acataacata gtcttctggt catgtacata tctttttacg tcgtttaacc agatacgaaa 147480
   ttcttgagag tcttcaaatg gcattccaac ccatggatta ccatcaataa cctttacttg 147540
   ccatttctga ttatattcaa caatagattc aggccatggc ggatgtaaag tttctttagg 147600
   gcttaagggc acatcctggg cacatccagc caaaatgcca atagataata caactgcagt 147660
   taatttaatc attctgagag cttcctgagg tcttctgcaa aggaatcgaa ggacctgttg 147720
   atttgttttt cgaccaatcc tggcttactt gccaccacgt tcttcttctt cgaatcctga 147780
   cggagctttt cattttcagc cttaagttgg tcaactaaaa cacggttctt ataagtcatt 147840
   tcttcgattg actgatattg aactttgaag tcgtctaata ctttagcatt gtttttagca 147900
   gcctctttaa cagaagctaa ttcagtattc aacgaatcaa ctttgttgtt taagacaact 147960
   attgttaccg tagaaactag agcaaaagct gctactatag cataaatcgg atttacttta 148020
   agcatgtttg aataatctca atgatttcgt ctcgggataa tccattgata agaactgttt 148080
   taggcccatc agtttggatt ttgtaggtct caaacattac acacaactct tctgctgtat 148140
   gatatggact tgagattccg aggcggttga atttgtcggt aagagggtcg caaataatgt 148200
   agaattttat acctgcgata tgtacgtttg gttgactaat attaatgaac acattagcgt 148260
   catatttagt caaattgttt tgaagaaatt ctaccatagc attaactgct tccggcattg 148320
   cttcacgttt ctcttcggtg taacgtgttg aatattcctt ttgctttaac ttctttttag 148380
   catttttcgc tagagtagta cgaaggtctg taagataacc gactgcacgg cctttcttaa 148440
   agactcggat tccatctgta gaatcaccaa atgctgccac aaccatatcg ttagtaatca 148500
   attgcatatt cataattttc tcctcgtgtt gttgataaaa gccatagtaa caccatcctt 148560
   ggtgtttgta aaccattaat atccttcagg gatgaaattt ttatgatttt taataaagac 148620
   tgattctaga gcagtcataa tttgctcttt tgaaccacct tggtaaagat tcataatgat 148680
   gccaaataac caaggtgtct gtgcaccctt acaaacagcc tgagcttcta tagcatacgt 148740
   tttacgatct tttcctttgt gcttatcata tgtatctaga cagatataaa atgagcggtc 148800
   taagaaatcg agatatgctt tttcgaagag ctcgaccttt ttaaatgaaa actcatcatc 148860
   tgcatacatc gctttaaggt catctgaagc accgttcact atagcaagaa acaacttttc 148920
   tgggctagag attgagtctc gagtcgtatg aagagcaaca taccaatcag tcttcaattt 148980
   gaaatgagac ccgtctttca ttacggccac atacccttca atatttgtag aagacttaac 149040
   atctgatgtc cagtcacctt ctggtacttc aaatcggtct acaagatatt tacgaaaaac 149100
   agggtctaga taaatgtcat catactcgat gtattcacct gtgttgttat cgcgaatatt 149160
   caaaagaatc aaacgttttt cagggtatgt taaaacaatt ttatttgttg gagcaacgta 149220
   ttcgaagtta gcagtgaacc cgtcattaca aagttctaat aaacgatcag ccaagttttt 149280
   atggtcgatg tctaacaaaa tactcgtggc cgatacagcc tggtcggatt tgattgaccc 149340
   tttagatttg aaacgaacag tcccaccatc aagatatgtc gagactaacg aaccgtcttc 149400
   tttagtcatc agatatttta catcatcaag gttaattgac aatgtaaacg ggttctcatt 149460
   tagattaaag aatttttcca ttggacgaga agcaatacgt ataggagttt caccatccat 149520
   ttcgaacata ataccacgac actctaacgc gtcaggcaat aaccaatctg aatatgaagc 149580
   aaagttatat gagaaaatac gatacttttt accgattgga cttacatcat ccgagtagaa 149640
   aaatttacgg tcggatgacg atttacacag agataacaga ttataataaa gcttttccat 149700
   tttgtatcct cagttagttt tttttaaatt gtaccacaat ccttgtggta tgtaaactac 149760
   tttttcttat gttggatatt ccaaggcggg ttgaactttt tgataaaaag tggttcttca 149820
   aggtccattg ttgcgatggt cattgaacca agttcgttgg tgactgaaag gtcaaaacat 149880
   tggcgggccc agaattcaac ctttttgcct tgcattaagg catccaaaat cataagtgat 149940
   tttcttgaat ctgaagtctg gtcttttcga tttatggctg ttcgataata gttaatgcgt 150000
   ttctttaaat tcttggtttt accaatatac acaagttcgt catctatagc aatagcgtat 150060
   atgacatttt tcttattagg ggcatcaatg gttttaattg ttgcatcttc taatagctcc 150120
   aattctatat atttaataaa ggaatattcg gcggcaatat ctttcataat aaagtgggac 150180
   cgaagtcccg tccttagaaa tattttttgt atgaatttat tacattttgg tctacatcat 150240
   tatcaatttg tgcgacaaga taagaactga tttcaacttc ctgaggagct gcttgaacta 150300
   aatcagagtt cagatattca cgaatccaag gatacggatg cttggtcgga gcatcagtga 150360
   ttgggcatgg caaaccacac tgtttcatac gagacacggt caagtaatca acaaaagcac 150420
   acatactctg cgtattgata cctggacatg taccatcttt aaaaaggtgt gcagcccatt 150480
   ccttctcttg actatttact tccataaaaa tgtcaactgc ttcttgttca cattctttag 150540
   caatttgaac ccactcatca ccgtcagtac cagattgaag ttgacggata atgtactgag 150600
   tacccttgag atggagttgt tcgtcacgag caatgaactt cataatcttt gcattgcctt 150660
   ccataatttc catattttta tggaagttga aagtacatgc aaatgatacg taaaaacgaa 150720
   tagcttctaa ggcgttgata acatgcaaac agagataaag agacttcatt aggtcatgtt 150780
   tacattcttc catgtgcata agactatttg aatatgcatc taaatcatat tcattttcgg 150840
   aaaaacactt aaaaatttct ttagcatttt cccattcacg ggttttaatc agaacgtcat 150900
   cataataacg tccaatggat tcagcgcgtt tcataatagc ttcatctaat acaatttcat 150960
   caaatacctt cgatggatcg gtataaagat ttcgcataat atgagtatac gaacgagagt 151020
   gaatagtttc actgaatgtc catgttgcga cccatgtatc taatgaaggg tcagaaatca 151080
   gtgccataag taccgcagat ggggccctgc cttggataga atctaataat gattgatact 151140
   taagattgtt tgtaaagatg tcctgttgga actgaggaag cttattgaac tgcgcagagt 151200
   ccaacatcaa gtttacctct tctgggcgcc agaaaaacga caattgtttt tcaataagtt 151260
   cttcgaaaac tttatgacgc tgaatatcat accgtgcgag gccaagacca gaaccaaaga 151320
   acatcggttc attcaaaaca tcgacttgtt gtgtgttaaa aactgtactc attatatttt 151380
   ctcacttagt tattaactca tccatgagta aattataatc aaaagtctta aagcttacaa 151440
   gcagagcaat cttcagcttt aggtgtttct aattcatagt catctgtacc tgacccatca 151500
   cgagtgttat gatagtagag gttttttccg ccaaagtacc agaagtaaag cagatcgtca 151560
   agcattactg acattggcac tttacctttc tcaaatactg cagggtcata gtaagtatta 151620
   gcagaagcag attggcacac ccatttcaac atgatagcaa cttgggtcaa ataaggttta 151680
   ttacctttct tagcaagttt ccacgcatag tcataaagac ccatgttgtg ttcaatatta 151740
   ggaactactt gacggaaatt accttcttta gattctttaa tacttactgg gccacgaggt 151800
   ggttcaatac cgtttgtgct atttgagacc tggctgcttg attcgcatgg catgagtgca 151860
   gacaacgttg agttacgaat tccgtgttct ttgagttcag ctcgaagttc ttcccagtcg 151920
   cagacgtagt tcggggctgc gatttggtca atctttttat tgtaccagtc gataggtaat 151980
   tcgcctcgag accatttagt gtctgaataa tactcgcaag gtcctttttc tttggcgagt 152040
   ttgattgaag ctttgataag gctatattgc aatctctcaa acaattcatg agtcaaatcg 152100
   ttagcgtctt catatgaagc aaagttagaa gctaaccatg cagcatagtt cgtcacacca 152160
   acaccgagat ttcgacgctt tttggctttt aaagcttctg gaaccggata gtcttgatag 152220
   tctaacaggt tatcaagtgc acgtacttgt acttcagcaa gttcattaat tttatcttgg 152280
   tcttgccaat caaatttatc taatacaaaa gcagataacg tgcataaacc aatttcagca 152340
   tcagggccat tcacatcatt agttggaata gcgatttcac aacataagtt actctgacga 152400
   attggagctt tttcacgaat aaatggagtg tagttattcg tgttatcaac aaactgtgga 152460
   taaattcgag cagtaccaga acgttcagtc atgaacaatt caaagagttc tagtgctttg 152520
   attcttttct ttctgattgt tgggtctttt tcagctttct cataaagctc acggaattta 152580
   tcttgatctt caaagaacga gtaatatagt tcgcctgaca tttcgtgagg gctaaataaa 152640
   gtaatataat catttttgcc aaggcgttcc atcatgaggt cattaatctg aattccatag 152700
   tccatatgtc gaatacggtt ttcatccacg cctttgttat ttttcagaac taataggttc 152760
   tctacttcta agtgccacat cggataatac gctgtagcag caccaccacg aataccacct 152820
   tgagaacaag attttactgc agtttggaaa tgtttccaga atggaataac acccgtatgt 152880
   cgaacttcac ccataccgat cttagaacct tctgcacgaa gcataccaac gttgataccg 152940
   ataccagctc gtttagaaat atattcaata attgatgctg aagttttgtt aatagatttt 153000
   aacgaatcac cagcttcaat aactacacaa ctagagaact ggcgagttgg agtacgagca 153060
   cctgccataa ttggagtagg caacgaaact tgatgagtag aaacggcatc ataaaaacga 153120
   ataatatgtt tcaaacggtc atgagtttcg tcttgatgta aggccatacc aatggccatg 153180
   atagcaaact gtggagtttc ataaatcttc ccagttgttt tatctttaac caggtatttt 153240
   tctttaagct gcatggcacc agcataagtc agatcaaaat ctcgctcatg tttaatccga 153300
   gaatcaagat aaatgatttc ttctgcagaa tatctagaca acaactcagg atcatatttg 153360
   ccttcattaa cacaatacga gatatggtcg ataaaagcag gaggttcaaa ctgaccatac 153420
   acatctttac gaagtgcaaa cattaatcct ttagcggcaa catattgata atcaggttct 153480
   tcgacagaaa tcgagttagc tgcaacttta atacaaatct tctggatatc tttggtgctc 153540
   atcccatcaa ccagatgagt cttaatttct tcatacaatt cgtatgggtc gatttgtgtt 153600
   ccttcacact gccaagttaa gacttgaata attttctgtg catcaaaatt ttgggatacc 153660
   ccactgctct tagttacttg catatattcc tcagtatagg ttgatagaca ttactccaaa 153720
   cgggatagta ttatcccgtt aatttattat acacttttaa actgaagcgt taattattct 153780
   tagcaataat ttctttgtat tctttaggtg tcaagactgt gatattgaaa taaaccaacg 153840
   gttctttttc cataccgtaa ccaccacaaa attcacgata ttcataaatt cgttcgtgtg 153900
   aatgacaaat gaatcgttca cctttaactg tgatttcact atttggtgaa gggatatcca 153960
   gttcggtagg atttttcatt tcaaaaattg tggtgccata tgaattaata aaacgtaaaa 154020
   tcatttatac cgccatttta gctttgatag ttggatgaga ttcgtaattt ttaagaatga 154080
   aatcactcgg attcatatgc tctgttaccc aatgaagctg taatgtcgtg ctccaatcag 154140
   agaatccttc aggccaatca attttaagtt cacacagttc tttaggctca cgacgaagaa 154200
   cttctttgca ctgctctaca tggttgctgt agatatgcgt attgccgcct gagaatacta 154260
   aatcacccgg gataagatta cacatcttag ccacaatatg cgtcaaggca gcgtacgagg 154320
   caatgttgaa cggcaggccc aagaaaacat ctacactgcg ttgataccat tgtaaatcaa 154380
   gatgaccatt acgcacatta aattgataga acatatgaca aggaggaaga gccatttggt 154440
   caatttcggc agggttccag gcagaaacaa tttgacgacg atcagttggt aattttttaa 154500
   tacggtcgat tgtttcaact aattggtcca caccaccgaa atcacgccat tgtttaccat 154560
   atataggacc aagttcacca ctatgataac caaggtcttt tgcctgattt tcgtaattat 154620
   cgtcccaaat agttttacct tcaattagac taccatgagt acgaagacgt aattcattaa 154680
   cattagtaga accggacatg aaccacagta actcggcaat acaagctttc catgctaatt 154740
   tcttagttgt tactgcaggg aatcctttag tcaggtccca acgtaattta gttccgaaca 154800
   gggcaattgt tcctgtgcct gtacggtcgt ctgtttcgta gccattttct aggatatcat 154860
   taatcaggta ttggtattgt ttcatttata tacgctctca gtaatttcgg tgagttcatc 154920
   gattctgtag taatgagatt ctaccataga acgttgaagc ataatatctt ggatgaaatc 154980
   tttatcaagt tgaacatcgg aattaacacg acatttctta atgattttag tcatcacaat 155040
   ttcatcggca taaggcagtg cttgttttaa taaggcagga ccgccgataa cagatacatc 155100
   aacatgttca gtagaaagtt taaacggtac gtctgttgaa gggctgaaca agttaatttc 155160
   actaccagtc accagggtaa taaattctgc ttgggtgata tagaaccgtg caagttcatc 155220
   agttttagtc ctaggcagtt ttctggccat atcagcgaca accacatgag tacgatctgg 155280
   tagtagacga ggtaatgatg caaaagtctt agcacccata accattacag tgtttttagt 155340
   gcgagcaaca aagttactca tatcttgttt gatatgcttc catggtagtc catcacctaa 155400
   tccaaaagca tattccagtt tatcatcgac tgttttagat ggtgcgcatg cgaatactaa 155460
   tttaagcatt tttatttcct cagatttttc ttaacaactt tccaatcagc tttgaacgat 155520
   tcaacgtcag agtggcaaat ccagaatcct gcactttcac catcttcgta gagtggacat 155580
   ccatcacatt cttcgttcca acccatttca cgtaaagctt cttcagcttg ttcaaggagt 155640
   tcagcgtctt taccaacgat gttgaagtac catttacctc taacttcaga atctttgatg 155700
   ctctggcgtt gcaatctcat tttattctcc ttcacttttg atttgatagg gctactataa 155760
   catagcccta tcttgttgta cactactttt taaaagtttt ttgcaaaagt tcgatgattt 155820
   cgtcgacgtt ggtttcatct acgatacaat ggatttttgt tacacctaaa gttatacctt 155880
   cgttagtagg ttgaggcttt actaaatcta cctcagtaaa gtatttgaac tcgtcaggat 155940
   aaatctcaaa atatgtttca atttcaccac cggcagaaca tacagtacca tcttccatag 156000
   ttacacttac tacgtttgac gtatcgctat cataatcagg ttccattccg tttacaacaa 156060
   aactaggtcc gtggagttca atcaatttta acatcttagc gttattgtgt ggtgcttctt 156120
   gaacaaagtc atctttaaat aaagggttga tgatataaga ttttccgatt tccattttga 156180
   tttcctcatg ttgttgatag gtctatagta acacatctag ggaagaagta aacaactttt 156240
   tcggcaattt acagatactc aaaagggaga ccgaagtctc ccgatttatt atagaccagc 156300
   taacaggtca tcgaggtcgc catcatcaga ggatgaggta gagctcatga agtcatcttc 156360
   agtttttgca gaactaaagg cttccatgtc tttatcaaaa tcgtcaaggt cagaagcaac 156420
   tttatctgca acagaagctg ctgcggctgc tgcaccacca agagcggcag taccaagaac 156480
   cttagcgaaa gattcattta atttttcaaa ggatttgaac tggtctttag ccgtcaaagt 156540
   agtaaggtct accatctggt catatagttc tttctggaat gcttcgtcat taatacgagg 156600
   aatttcagat tgacccaaga atttagattc gtcgtagttg ctaaaaccag aaacctgctt 156660
   agctttcaga acgaagttgg caccttcaaa cgggcaagta acatcaactg gagtttcacc 156720
   catttcggta tcaacagcaa tcattgcatt gattttgtcc caaatctttt taccgaaacg 156780
   atatttaaat actttacctt cgttatctgg agcttgaggg tctttaacaa ccagaatatt 156840
   agcccaataa gaagttttac gtttcagttg agaatattca gttttgttgg tattgtacag 156900
   gtcattttta ctaatgtact gacatacagg acaagagtcg taatcaccat gtgtagaaga 156960
   gcaattttca atataccatt taccattttt cttgaaaccg tggttaacaa gaattgcaaa 157020
   tggtagtgcg tcatcagttt ttgctggcag gaaacgaatt accgcttgac cattacctga 157080
   tgcatctagc ttcagcttcc attcgccttt atcttctgaa gagaaacctt tgttaccgtt 157140
   cagtttagcc atttgagcag cgaggtcagc agtagattta cgtttaaaca ttatatttac 157200
   ctttatttag attatttaat ttatttacag ttggttgcta cgacacttat acctcgtagc 157260
   tggtatgatt tatttataaa gtaattttaa cacacaagaa ataaagctta atacttacat 157320
   gatttgattg tttctatgaa caatttctta gcagcatttc catcgatttg aagtattttt 157380
   cgatatgcct ttaatttggt agaataattt tgccagacta aattgtcggt gagttcgtcg 157440
   tgcttatcta taatattcaa gaatgaatcc aacaacaaga aggtttcaaa tgaaattata 157500
   ttgctttgta gcaatttaaa tatgtagctc gactgaactt tattattata atcaaatatc 157560
   tcttgaagcg cactaacttc cactttctta ctgaaatagt agatgttacg tacatcttca 157620
   gcaaacgtct ccttggcttg tttgagacgt ccaatatatt ccctatagaa aactaatgca 157680
   tcagcgtctg atatctcacc aatccacgca tcttggttag caactaagtt actgatgaat 157740
   atcaaggtga gttcctttaa cttatatttc tctgctagtt tctcaaagaa atatttatct 157800
   cggcgttttt gatacgcatt atccgaaaca cgcatgcacc agttatattt gattacgtca 157860
   taccgaccat taaaatgttg tttgcacatc aagtatagtt tgtataccga ttttccgtta 157920
   atataacgtt ctccatcagg aggcatgcgt attttaatca taataagaaa tctaacgtat 157980
   tagttttttc accacgagca actgatgggc gaagcatatt ttcgtcaatt gcttcgcttg 158040
   taattttttc aacaattcct gtaggaataa atttagcaaa ttgtgtttct ggaatagaat 158100
   tttcttctaa gaaagcagtt gttgcttcta aataagacat accaaattgt tcaaccatag 158160
   attcgataat gaatccgttt tcttggcggt caagcaattt agcgatatca tctttatcat 158220
   gttttactgc taattcttgt tcagataaac cggcctcatc aaccggttta atatcactta 158280
   gtgaaaactg tgtcataaag ctcgactacc tcttcgtttt cagcttcaaa ttggtcacga 158340
   gcatctttat gataaagagc taacaaacga ttaaacattt ttccatcgac accaagctct 158400
   tctttagcac gagtacgaat gtctttaatc agttcattat aaccagaaat tttaagtttg 158460
   ttatcggaag cttccttgac tagtttagcc agttcttcac catgcacatc ttgattaaat 158520
   tcaactgctt ctttcttagc catgtttacc tcagaattca ttaattacac ttgttaattt 158580
   agaaagaccc gattttacaa agtatgaata aatcttgcct ttaggttgtt gtttatatga 158640
   gttataatac tctataatgg ttgaagcaat attatcaggg atataatcaa aatcaatgag 158700
   aaccaaattt tcttggtacc gtttatattc ttctgcactt aataatactt cagcttgaga 158760
   acggtcatta gcaattgctt caataatcgt tgttttcata ctcggagttc gttcgccttc 158820
   aactcgagta aaccagaaat caccacgaac tcgaacagac gcaacaccgt ctttacggtc 158880
   gcctttaaga attttagtca tgcaatcaat ttcggcagaa ccattcttaa ttttaaccca 158940
   ttttttctgc ggcggcgacc actgtttaac gttagggtat ttgtgtaatt gtgtaaagtc 159000
   accatctgat gcaacaatac acaccttatg accggctaat gacaaatatt tagttaatac 159060
   accgatatgg tcatctgctt cgtatttgtc aatatccatt acaacgtatg gcatatattt 159120
   cttaatctca tcaacgactt tatggagtgc agtaaaatat ccttcccaat cccactttga 159180
   agcttcacga tcggttttac gatttttctt ataatagtaa gcaaagtcac gacgccaata 159240
   tccagaagta gcgttatcca tgcacaatac aaattttgta taaccttgct tacggaacat 159300
   cactacgttt ttacgaattg aattcaagac tacatgacga accatcggaa cagtaatttt 159360
   atcaccatct tcaaagttgt ttaatgcagc tgccaatgca atgttactaa agtctgcaag 159420
   cgcaatacct tctttgtaat cttcatccaa catcatttct aaatccatat gaacctcttg 159480
   ttcagttagt caacttagac catagtaaca tactagttcg aaagcgtaaa tagtctttta 159540
   ctcatttata aatagtaaaa tagaacacat aatgataaga ggactaccat ggttgacatc 159600
   aaacgtaagt tcagggccga agatggtctt gacgcaggcg gtgataagat agttaacgtt 159660
   gcgcttgctg accgcacagt tggcaccgat ggcgtgaacg ttgacttttt ggtgcaagaa 159720
   aacaccgtac agaattatga cgatacgcgc gcgtatacaa aagattttat tgttctttat 159780
   gataatcgtt tttatcaagc tttaaacgat attccagctc cggcaggccc tttctctttg 159840
   gctaaatgga aggcaactcg tacagatgca gaatggacta cagttcaagg cggagatttc 159900
   caattatcag taggtaactc tattgcggtt gatacttcag ctggcactga tattaatttc 159960
   actttgcctc aaaatccttt aaatggcgat acagttattt tgtctgatat tggcggtaga 160020
   gtaggctatg taaaagttca aattactgca gaggctcaaa gtattgtaaa ctttagagga 160080
   caacaggttc gttcagtttt aatgacgcac ccaaaatcta aaatggtctt catttttagc 160140
   aaccgcctat ggcaaatgta tgtttctgat tacgaacgta atgcagtcac agtaactcct 160200
   gctaagccat atcaggcgca acctaacgat tttatcattc gacgttttac atcagccgct 160260
   cctattaata ttacattgcc tcgcaatgct aataatggcg atattattaa tttagttgat 160320
   ttagacaaat taaacccgtt atatcacaca attgtcaaaa cttacgatga tacaacttct 160380
   atacgagaag ccggtgttca tgtagcagaa ggacgtaata ctgcggaagc attctttgtt 160440
   tatgattctg ctaatagctt atggcgtgtt tgggaaggtg accagaaatc tcgtttacgt 160500
   atagttcgta atgatactga tttacgccct aatgaagaag ttcttgtttt tggaactaat 160560
   aatgatacaa tctcgacggt aaatcttact ttgcctacag atattttgtc tggtgatact 160620
   gttaaaatat cgttgaatta tatgcgtaaa gggcaaactg taaaaattaa agctgctgaa 160680
   ggcgatacaa ttgctagcag tatttcttta ttacagttcc caaaacgttc agagtatcca 160740
   cctgatgcac aatgggtttc tgttagtgag ctggaattta atggcgatac ttcgtatgta 160800
   cctgtacttg agttagctta tatagaagat tattcatctg aaacaaaata ttgggtagtt 160860
   caacaaaaca ctgtaaccgt agaacgagtc gatgcatcga gcaatacaac tcgagctcgt 160920
   ttaggtgtta ttgctcttgc ttctcaagcg caagcaaatg tcgatttaga aaatgctcct 160980
   ggtaaagaac ttgctattac tccggaaaca ttagcaaatc gtacagcaac tgaaactcgt 161040
   cgtggtattg ctcgtattgc aacaactgca caagttaacc agaataccga ttttgcattc 161100
   caagacgact tgattatttc tcctaagaaa ttgaacgaac gtacagcaac tgaaacccgt 161160
   agaggtgttg ctgaagttgc aactcaggat gaaactaatg ctggcataga tgataccact 161220
   attattactc ctaagaaatt ggatgcccgt caaggttctg aagttttatc tggtattgta 161280
   aaatacacat ctacgactgg tactacagcg gctactgttc gtggtaatgc tgggactaac 161340
   gtttataaca aagccgtaga taatttaact atttctccaa aggctcttga ccagtataaa 161400
   gctaccccta ctcaacaggg cgcagtaatt cttgctattg aaagtgaagt tatcgctggt 161460
   gaatcacaaa ccggttgggc taatgctgta gtgacccctg aaacactaca taagaaaact 161520
   tctactgatg gacgtattgg tttaattgaa attgctacgc aagcagaaac taacactgga 161580
   actgattata ctagagcagt aacgcctaag acgttaaatg ataggaaagc tacggaagga 161640
   ttatccggca tagccgaaat tgctacgcaa gttgaatttg atactggaac tgacgatact 161700
   cgtatctcgt ctccactgaa aattaaaact cattttgatt cttctgaccg taccagtgtt 161760
   aattctgatt ccggacttat tgaagaagga accttgtgga accattatac tcttgatatt 161820
   tctaaagcaa atgaaacaca acgcggtaca cttcgcgtag cgacccaggc agaatctaat 161880
   gcaggaactt tagatgatgt tcttattact cctaaaaagc ttttagggac taagtccact 161940
   gaaacgtctg aaggcgtaat taaggttgct actcaggctg aaactgtaac aggaacttct 162000
   gctaatactg ctgtatcacc taagaattta aaatggattg ttcaatcaga accatcatgg 162060
   actgctacta cggcaattcg tggattcgtt aaaacttcat ctggttctat tacatttgtt 162120
   ggtaatgata cagttggttc aacacaatct ttagaatcat atgagaaaaa tagctatgcg 162180
   gtatcaccat atgaattaaa ccgtgtactt gctaactact taccgttgaa agctaaagca 162240
   gctgatagta atttgttaga tggtctagat tctcttcagt tcatccgtag agacatcgac 162300
   cagacggtta atggttcttt aagtcttact aaacagacca acctgagtgc tcctttagta 162360
   tctacaagca ctgcttcttt tggttccgaa gcatctgtta ctcgtagatt aactcttaat 162420
   gattctagcg gttctgaaat aattttcact aaaggaaccc aatctcttag taataaagag 162480
   aatttcgttg ttagagcatg gggtaatagc gctacagatg gtgcccgtga tacagtattt 162540
   gaagcgggtg acgaaaccgg ataccatttc tattctcagc gcgctgctga taatagagta 162600
   tcatttaata ttaatggaac actttattca acaggtattg tttctacaaa tggattaaat 162660
   gttacaggtg tttctacctt tacagggcct attagtgcta caggcgaaat tgtttctagt 162720
   tctcctattg cattcagagc tattaatggt aactatggtg ttatgcttta taatgctggt 162780
   aacagttctt atattgcatt aactaactca ggtgaccaga ccgggacgtt taataactta 162840
   cgcccgatca cgattaacaa cgccacaggc ttagttcgtc ttgacaatgg tgttcaaatc 162900
   acgagtggtg caacaataac taccggtggg ttaactgtaa atagcagaat tatttctaac 162960
   ggcgttaaaa cagccactgt ttataccgat aaaccaacag cttctactgt aggtttttgg 163020
   tctattgaca ttaacgattc tgctgtatat agccaattcc ctggatactg gacgcgtgat 163080
   aacaaaggta accgtgacca agaaattaaa tatcctggta ctttgactca attcggcaat 163140
   agcttagatt cgctttatca ggattggatt tgttatccta caggtgcaaa tggtggtagt 163200
   attcgttata ctcgtacctg gcagaaaaat aaagatgctt ggacttcatt tgcaatggta 163260
   tttgatagtg gcaacccacc ttcacctagc gatgtcggtg ctatcccatc tgataatgct 163320
   gttattggaa accttactat tcgagacttc ttacaattag gaaatgtgag aattgttcca 163380
   gacccggtta acaaaacagt taaatttata tgggttgaat aagaggttat atggaaaaat 163440
   ttatggcaag gtttggacat ggatacgtcc aaacgccgtt tttatcggaa agcaattcag 163500
   ttagatttaa gctaagtata gcgggttcat gcccgctttc aacaactaat ccgtatattt 163560
   tgttccaaaa cgagccttta gggttgcagt cttttggtat tggactaaat gttagggtga 163620
   taaatccgga aaatggaact atagttgata gtaaattata caattttgcg cctacaaata 163680
   atgcaacttc agctgcattt atttcgtttg ttaatacata cgcagataat ttcattttcg 163740
   catttatttc taataacaaa tttaatttgc caccggaaat aattgaatgg tttaaagctg 163800
   ctgggagttc agttataccg tcaattgaag ttgctagtct tgttgatatt tcatattcag 163860
   cgttttatgt ttcaggtaaa aatactattg cattagaaca cataaaatac agtaataaga 163920
   aaacaatttc tgattacgca actccattag atattgtata tgactctatt gctgatatag 163980
   gtgctaccgg ctatcctaga cgtacatatg aagctctaga aacgttttta tctcctgttg 164040
   gaggaactaa caatgaaata aaaaggatgc ctacgtcatc ccttgttact cctatcgcaa 164100
   actatggatt aaagcctaca gattttcttt atttgaaatt tcaattaatg gctgatgaag 164160
   aattattaga ggaaggaacc acaagacttt caattcgttt ctttaagtct ccatctagct 164220
   ctcctatatc atctaaagat ataaactttg atggaactgc cggggagtgg aagctatatg 164280
   aagaatacgt tgaaatacca gctgaagctg atggctttac agtatattgt taccgtacgg 164340
   cctcagtcgg tcaaggtgga ctaagaaatg ttattttcac tgaagtatca tgtaacggaa 164400
   gtatagcaaa acccgctgag tttggtataa atggtattcg tgttaattat attgatgaat 164460
   cgttaaccgg taatgatata atggacttgc ctactcagtt atctaacgac acaggtaagg 164520
   tatttgggca ggaatttaaa gagtacacag aataaaggag acttcggtct ccttttcgcg 164580
   tataaatact ttaatattaa taaggagaca cgaaatggcc gatttaaaag ccggaagtac 164640
   agttggtggt tccgtcatat ggcatcaagg caattttcct ttaatgccag ctggcgatga 164700
   catcctatat aaaacattta aattatacac cgaatataac aagccaaaag cggctgataa 164760
   cgatttcgta tctaaagcaa atggtgggac atatctcaaa aaggtaatct ttaacgaagg 164820
   tattgcagtc aaaacagcag atgaccaaac gaatggtatt ttttctggtg gtggtgacgc 164880
   cgcgacattc gaccaaacaa acatggatat tgtttcgtgg tatggtattg ggtttaaatc 164940
   atctcaaggt acaggtgaaa gaactgttgt aattgatact cgcaccggta atataagttc 165000
   taaaggcgtt attgaagcat ctcaattcag agccacaacg ttagcacctt taaataatta 165060
   cgaccttact cgcaaagatt atgttgatag tcaaataaat acagttaatg ctaatgcaaa 165120
   cagtcgtgta ctccgttcag gagatacgat gaccggcgcg ctgaccgccc caaacttttt 165180
   ctcacagaat cctgcatctc aaccctcgca tgttccacga tttgaccaaa ttgttattaa 165240
   ggattctgtt caagatttcg gctattatta agaggactta tggctacttt aaaacaaata 165300
   caatttaaaa gaagcaaaac tgcaggagca cgtcctgccg cttcagtatt agccgaaggt 165360
   gaattggcta taaacttaaa agaccgtgta ctttttacta aagatgacca aggaaatatt 165420
   attgatctgg gttttgctaa aggcggtagt attgacggga atgttattca tacaggtaat 165480
   tataaccaaa ccggcgatta tactttaaat ggcaccttca ctcagacagg taattttaat 165540
   ttaactggta ttgctcgagt aactcgtgat attattgccg ctgggcagat tatgactgaa 165600
   ggcggtgaac ttattacaaa aagttcaggt acagcacatg ttcgtttttt cgatggcaat 165660
   agccgcgaac gtggaatcat ttatgccccg gctaatgatg gattaactac acaagtactt 165720
   aatatcaggg ttcaagacta cgccgctggt agcgaaagca tttatgcatt ttcaggcaat 165780
   ggacaattta tttcacctga agtatcggca cggaaatcta tgtcaactcc tcagattttg 165840
   actgacaaag tcattacaga cgggaagaag gccggtgatt atgacatcta ttcattagca 165900
   aataataatt ctaacacaga taaaaataat ttacgtgtcg tacgtaccga cccggcggcc 165960
   gcaatgctcc atgaaatttg tgaaaataac ggcatcagtt ggtattctgg ttcaacccct 166020
   actgattaca tgttgtcatt ttcttattcc ggtgggcttc aagcaggcca ttcaattgca 166080
   gtaggtatgg aatcaagtcc tatgacatat tcagccttag gtaaaggttc tattgctatt 166140
   ggcgataatg acaccgggtt aaaatggcac caggatggat atttccatac agtaaacaat 166200
   ggaacaagaa ctttcatcta cggccctgca gaaacacaaa gccttagaaa aatggttatg 166260
   ggttattctc cagatgggct tcttatgaca acgccaccga cagaaaacta tgctttggct 166320
   actgttgtta cttaccatga taataacgca tacggtgacg gtcaaactct tttaggatat 166380
   tatcaaggcg gtaactatca tcattatttt cgcggtaagg gtactacaaa cattaatact 166440
   cacggcggtt tgttagttac tccaggtaat attgatgtta ttggcggttc tgttaatata 166500
   gatggtagaa ataattcttc tacactaatg tttagaggca acacaacagg atacagctcg 166560
   gttgataata tggatattaa agtttggggt aatacgtttg ttgatccaag cggaggtatc 166620
   cgtaaaaaca ttatggaaat ttctgatgca actagctgga tgagctatat tcaaagactt 166680
   actactggcg aagtagaaat gaacgttaat ggttcatttg aatcatctgg tgttactgct 166740
   ggagatagag gagttcacac aacaggtgaa atttcatctg gagcagtgaa tgctcttcgt 166800
   atttggaacg cagattatgg agccattttt agacgttcag aaggaagtct tcatattatt 166860
   ccaactgctt atggcgaagg caaagatggc gatattggtc cacttcgccc atttagtatg 166920
   gctttagata ctggtaaagt tactattcca gatttacaat caagttacaa tacgttcgct 166980
   gctaacggtt atattaaatt tgttggtcat ggagcgggtg ccggcggtta tgacattcaa 167040
   tatgctcaag cggctcctat tttccaggaa attgatgatg atgctgtaag caaatattat 167100
   cctattgtta aacagaagtt tttaaacggt aaatctgttt ggtctttagg taccgaaatt 167160
   gaatcaggta cattcgttat tcatcatctg aaagaagatg gttcacaagg ccatacatca 167220
   agatttaata tggatggtac agttaatttc cctgataatg ttctggtcgg tggtggtgaa 167280
   gctgctattg ctcgtaatgg taatattttc tcggatattt ggaaaacgtt tacttctgca 167340
   ggcgacgtaa ctaatattcg cgatgcaata gctactcgtg ttgccaaaga aggtgatacg 167400
   atgaccggca ctctttggat taataaagat gctgctggaa tagttcttaa tcctcctttg 167460
   gccagtgatt catcatttat tcgttccgat acggccgggg tcaataattg gtatattggt 167520
   aaaggcggtg ccgacaatgg tctaggtttt tacagttatg ttacacaagg cggtgtatat 167580
   ataacaaata acggagaaat atcactttct cctcaaggcc aaggaacatt taattttaat 167640
   agagaccgcc tccatataaa cggtacacaa tggaccgcac accagggagg tggttgggga 167700
   aatcaatgga atcaagaagc accggtattt gtagattttg gtaatgttgg taatgatagt 167760
   tattatccta ttattaaagg aaaatccggt attactaatg aaggatacat atctggtgta 167820
   gattttggta tgcgacgcat tactaataca tgggcacaag gtattattcg tgtaggtaat 167880
   caggaaaacg gttacgaccc acaagctgta tatgaattcc atcataacgg cactttttat 167940
   gctccaagct tacttaagag cagtagagta tcagctggtg gtggtgaccc tgcatggggc 168000
   gggccgtgta ttgtacttgg agataacgat accggtttgc tttgggaaaa cgatggtatt 168060
   ttcaacgcat atgcaaacgg ccaaggtgtg tttagtttta gacctggttt agctcagaca 168120
   ttcggtgatg ttaacttcca ctgtaatgca ggtatgtatg ttcgtgataa cattgatgtt 168180
   aacgacgttt atattcgttc tgatattcgt tgtaagtcgg aaattaagct tattaagaac 168240
   gctcaagaga aatctaaact attgggcggt tatacttatc tgcttaaaaa ctctgttaca 168300
   gacgaagtta aaccgtccgc aggtttaatt gctcaggaag ttcaagaagt attacctgaa 168360
   cttgtttctg aagataaaga gaccggactg cttcgtttga actataacgg tattgttggt 168420
   ttaaatacag ctgcaataaa cgagcataca gatgaaatca aggaattgaa atctgaaatt 168480
   gccgaattga aagcattaat taaatcattg ataaaataat aaaagggcct tcgggccctg 168540
   gaggtttata tggcagtagt aggaatccct ggttggattg ggacttcagc cgttgctgaa 168600
   acggggcaaa gatggatgac ggctgcttca agggaacttc gtttaggaaa cccttcatgg 168660
   atgtcccaat tcgcgggccg ttcaagagaa ataattcaca cacttggagc agaccataac 168720
   tttaatggtc aatggttccg agatagatgt tttgaggcgg gtagtgcgcc tatagtgttt 168780
   aatatcaccg gaaatttagt atcatatagt aaagatgttc cattattctt tatgtatggc 168840
   gatacaccaa atgaatatgt tactttgaat attcatggtg gagttcatat gtggggacga 168900
   ggtggtaacg gtactgtaaa cggaaaccca ggcacaaatg gcggcgatgt aatccaaaat 168960
   gatatcggcg gaagacttcg tatttggaac tatggcgtta ttgcatcagg cggtggcggt 169020
   ggcggtgcag tgtcattaca gaatagctgg gcgccaaatg ttacagcagg tggcggcggt 169080
   ggtagaccat ttggcatcgg cggtggcggc gttaattggc cgggtggtaa tgc        169133
<210> 5
   <211> 5213
   <212> DNA
   <213> Artificial Sequence
<220>
   <223> synthetic pUC57.HR.Fluc plasmid sequence
<400> 5
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtaagct tagattgata aagaagcagt 420
   tgaaattgct cgtcaaaccg gtcgtggtga aggtaacttc attatcgctt cccgtaacgt 480
   agttaacgta ctggcttcag ttgatactgg tatttcttac gctgcacagg gtctggcttc 540
   cggttttaat accgacacta ctaagtctgt atttgccggt gtacttggtg gtaaataccg 600
   cgtatacatc gaccagtatg ctaaacagga ttacttcacc gttggttaca aaggcgctaa 660
   cgaaatggat gcaggtatct actatgctcc ttacgttgca cttaccccac tgcgtggttc 720
   cgatcctaag aacttccagc cggtaatggg ctttaaaact cgttacggta tcggtgttaa 780
   cccgtttgct gaaagttccc tgcaggctcc aggtgctcgc atccagtccg gtatgccgtc 840
   tatcctgaac agccttggta agaacgctta cttccgccgt gtatatgtaa aaggcatcta 900
   aacgcgttat aaataaatac tagtagataa ggaggatttc gaatggaaga cgccaagaac 960
   atcaaaaaag gcccggcacc gttctatccg ctggaagacg gcaccgcagg cgaacagtta 1020
   cacaaagcca tgaagcgcta cgccctggtt ccgggtacaa tcgcctttac cgacgcccac 1080
   attgaggtga acatcaccta tgccgagtat tttgagatga gcgttcgtct ggccgaagcc 1140
   atgaaacgct atggcctgaa caccaaccat cgcattgtgg tttgcagcga aaacagcctg 1200
   cagttcttta tgccggtgct gggtgccctg ttcatcggtg tggccgttgc ccctgccaat 1260
   gacatctata acgaacgcga actgctgaac agcatgaata tcagccagcc taccgtggtt 1320
   ttcgttagca aaaaaggttt acaaaagatt ctgaatgttc agaagaagct gccgattatc 1380
   caaaaaatta tcattatgga tagcaagaca gattatcaag gctttcagag catgtacacc 1440
   ttcgttacca gtcacctgcc gccgggcttc aacgagtatg atttcgtgcc ggagagcttc 1500
   gaccgtgata agaccatcgc cctgatcatg aacagcagtg gtagtaccgg cctgccgaaa 1560
   ggtgtggccc tgcctcatcg caccgcatgt gtgcgcttta gccacgcccg cgacccgatc 1620
   tttggcaatc agatcatccc ggacaccgcc atcttaagcg ttgtgccgtt ccaccacggt 1680
   tttggcatgt ttaccaccct gggctatctg atctgcggct tccgcgtggt gctgatgtat 1740
   cgcttcgaag aggagttatt cctgcgcagc ctgcaggact ataagattca gagcgccctg 1800
   ctggttccga cactgttcag ctttttcgcc aagagcaccc tgatcgacaa atacgatctg 1860
   agcaacctgc acgaaattgc aagcggcggc gcaccgctga gcaaggaagt tggtgaagcc 1920
   gtggcaaaac gcttccacct gcctggcatc cgtcaaggct acggtctgac agaaaccacc 1980
   agcgccatcc tgatcacccc ggaaggcgat gataaaccgg gtgccgtggg caaggtggtt 2040
   ccgtttttcg aggccaaggt tgttgacctg gacaccggta aaccctgggg tgttaaccag 2100
   cgtggtgaac tgtgtgttcg tggcccgatg atcatgagcg gctacgtgaa taacccggag 2160
   gccaccaatg cactgatcga taaagacggc tggctgcata gcggcgatat cgcatactgg 2220
   gatgaggacg agcacttttt tattgttgat cgcctgaaaa gtctgatcaa atacaaaggc 2280
   taccaggttg ccccggccga actggagagc atcctgctgc agcatccgaa cattttcgac 2340
   gcgggggttg cggggctgcc tgatgatgat gcaggtgagc tgcctgccgc cgttgtggtg 2400
   ctggagcacg gtaagaccat gacagagaag gagattgtgg attacgtggc aagccaggtg 2460
   accaccgcga agaagctgcg cggtggcgtt gtgttcgttg acgaagtgcc gaagggcctg 2520
   accggtaaac tggacgcccg caagattcgc gagattctga ttaaggcaaa aaagggtggc 2580
   aaaagcaaat tataagccgg cttatttctg tgcaatttcg atgtaagttt tcaaaatatt 2640
   ttgccaatta ggcatacctt cttggacata tgccggagaa ccgtctttgt ttttgaatga 2700
   tgcaagttta tcagtggccc aataaccttt agctcgagct tcagaataaa ctttcatcca 2760
   tactttgttg aaggcattac cattaacttt tgcaccatat ttttctacta acttaactgc 2820
   ttcagcttcc agaactttta aagtatctcg taagaaacgt gctttagtca ttttgtttac 2880
   tcctctgtag ttgataagtc tatagtatca cataccaaat acgttgtaaa caatctttat 2940
   aaataatcta tatcacataa ggaaaaaatg caagtcgacg gcgtaatcat ggtcatagct 3000
   gtttcctgtg tgaaattgtt atccgctcac aattccacac aacatacgag ccggaagcat 3060
   aaagtgtaaa gcctggggtg cctaatgagt gagctaactc acattaattg cgttgcgctc 3120
   actgcccgct ttccagtcgg gaaacctgtc gtgccagctg cattaatgaa tcggccaacg 3180
   cgcggggaga ggcggtttgc gtattgggcg ctcttccgct tcctcgctca ctgactcgct 3240
   gcgctcggtc gttcggctgc ggcgagcggt atcagctcac tcaaaggcgg taatacggtt 3300
   atccacagaa tcaggggata acgcaggaaa gaacatgtga gcaaaaggcc agcaaaaggc 3360
   caggaaccgt aaaaaggccg cgttgctggc gtttttccat aggctccgcc cccctgacga 3420
   gcatcacaaa aatcgacgct caagtcagag gtggcgaaac ccgacaggac tataaagata 3480
   ccaggcgttt ccccctggaa gctccctcgt gcgctctcct gttccgaccc tgccgcttac 3540
   cggatacctg tccgcctttc tcccttcggg aagcgtggcg ctttctcata gctcacgctg 3600
   taggtatctc agttcggtgt aggtcgttcg ctccaagctg ggctgtgtgc acgaaccccc 3660
   cgttcagccc gaccgctgcg ccttatccgg taactatcgt cttgagtcca acccggtaag 3720
   acacgactta tcgccactgg cagcagccac tggtaacagg attagcagag cgaggtatgt 3780
   aggcggtgct acagagttct tgaagtggtg gcctaactac ggctacacta gaagaacagt 3840
   atttggtatc tgcgctctgc tgaagccagt taccttcgga aaaagagttg gtagctcttg 3900
   atccggcaaa caaaccaccg ctggtagcgg tggttttttt gtttgcaagc agcagattac 3960
   gcgcagaaaa aaaggatctc aagaagatcc tttgatcttt tctacggggt ctgacgctca 4020
   gtggaacgaa aactcacgtt aagggatttt ggtcatgaga ttatcaaaaa ggatcttcac 4080
   ctagatcctt ttaaattaaa aatgaagttt taaatcaatc taaagtatat atgagtaaac 4140
   ttggtctgac agttaccaat gcttaatcag tgaggcacct atctcagcga tctgtctatt 4200
   tcgttcatcc atagttgcct gactccccgt cgtgtagata actacgatac gggagggctt 4260
   accatctggc cccagtgctg caatgatacc gcgagaccca cgctcaccgg ctccagattt 4320
   atcagcaata aaccagccag ccggaagggc cgagcgcaga agtggtcctg caactttatc 4380
   cgcctccatc cagtctatta attgttgccg ggaagctaga gtaagtagtt cgccagttaa 4440
   tagtttgcgc aacgttgttg ccattgctac aggcatcgtg gtgtcacgct cgtcgtttgg 4500
   tatggcttca ttcagctccg gttcccaacg atcaaggcga gttacatgat cccccatgtt 4560
   gtgcaaaaaa gcggttagct ccttcggtcc tccgatcgtt gtcagaagta agttggccgc 4620
   agtgttatca ctcatggtta tggcagcact gcataattct cttactgtca tgccatccgt 4680
   aagatgcttt tctgtgactg gtgagtactc aaccaagtca ttctgagaat agtgtatgcg 4740
   gcgaccgagt tgctcttgcc cggcgtcaat acgggataat accgcgccac atagcagaac 4800
   tttaaaagtg ctcatcattg gaaaacgttc ttcggggcga aaactctcaa ggatcttacc 4860
   gctgttgaga tccagttcga tgtaacccac tcgtgcaccc aactgatctt cagcatcttt 4920
   tactttcacc agcgtttctg ggtgagcaaa aacaggaagg caaaatgccg caaaaaaggg 4980
   aataagggcg acacggaaat gttgaatact catactcttc ctttttcaat attattgaag 5040
   catttatcag ggttattgtc tcatgagcgg atacatattt gaatgtattt agaaaaataa 5100
   acaaataggg gttccgcgca catttccccg aaaagtgcca cctgacgtct aagaaaccat 5160
   tattatcatg acattaacct ataaaaatag gcgtatcacg aggccctttc gtc        5213
<210> 6
   <211> 4162
   <212> DNA
   <213> Artificial Sequence
<220>
   <223> synthetic pUC57.HR.NanoLuc plasmid sequence
<400> 6
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtaagct tagattgata aagaagcagt 420
   tgaaattgct cgtcaaaccg gtcgtggtga aggtaacttc attatcgctt cccgtaacgt 480
   agttaacgta ctggcttcag ttgatactgg tatttcttac gctgcacagg gtctggcttc 540
   cggttttaat accgacacta ctaagtctgt atttgccggt gtacttggtg gtaaataccg 600
   cgtatacatc gaccagtatg ctaaacagga ttacttcacc gttggttaca aaggcgctaa 660
   cgaaatggat gcaggtatct actatgctcc ttacgttgca cttaccccac tgcgtggttc 720
   cgatcctaag aacttccagc cggtaatggg ctttaaaact cgttacggta tcggtgttaa 780
   cccgtttgct gaaagttccc tgcaggctcc aggtgctcgc atccagtccg gtatgccgtc 840
   tatcctgaac agccttggta agaacgctta cttccgccgt gtatatgtaa aaggcatcta 900
   aacgcgttat aaataaatac tagtagataa ggaggatttc gaatgaatag ctttagcacc 960
   agcgcctttg gccctgttgc ctttagcctg ggcctgctgc tggttctgcc ggcagcattt 1020
   ccggccccgg tgttcaccct ggaagatttt gtgggcgatt ggcgccagac cgccggttat 1080
   aacctggatc aggtgctgga acagggtggt gtgagcagcc tgtttcagaa tctgggcgtg 1140
   agcgtgaccc cgattcagcg cattgtgctg agcggcgaga acggcctgaa aattgatatt 1200
   catgttatta ttccgtatga gggtctgagc ggcgatcaga tgggccagat tgaaaaaatc 1260
   tttaaggtgg tgtatccggt ggacgaccat catttcaagg tgatcctgca ttacggcaca 1320
   ctggtgattg acggcgttac cccgaacatg atcgactatt tcggccgccc gtatgaaggt 1380
   atcgccgtgt tcgacggcaa gaaaattacc gtgaccggta ccctgtggaa cggcaacaag 1440
   atcattgacg agcgcctgat taacccggat ggtagcctgc tgtttcgcgt gaccattaat 1500
   ggcgtgaccg gctggcgtct gtgtgaacgc atcctggcct aattaattaa ttatttctgt 1560
   gcaatttcga tgtaagtttt caaaatattt tgccaattag gcataccttc ttggacatat 1620
   gccggagaac cgtctttgtt tttgaatgat gcaagtttat cagtggccca ataaccttta 1680
   gctcgagctt cagaataaac tttcatccat actttgttga aggcattacc attaactttt 1740
   gcaccatatt tttctactaa cttaactgct tcagcttcca gaacttttaa agtatctcgt 1800
   aagaaacgtg ctttagtcat tttgtttact cctctgtagt tgataagtct atagtatcac 1860
   ataccaaata cgttgtaaac aatctttata aataatctat atcacataag gaaaaaatgc 1920
   aagtcgacgg cgtaatcatg gtcatagctg tttcctgtgt gaaattgtta tccgctcaca 1980
   attccacaca acatacgagc cggaagcata aagtgtaaag cctggggtgc ctaatgagtg 2040
   agctaactca cattaattgc gttgcgctca ctgcccgctt tccagtcggg aaacctgtcg 2100
   tgccagctgc attaatgaat cggccaacgc gcggggagag gcggtttgcg tattgggcgc 2160
   tcttccgctt cctcgctcac tgactcgctg cgctcggtcg ttcggctgcg gcgagcggta 2220
   tcagctcact caaaggcggt aatacggtta tccacagaat caggggataa cgcaggaaag 2280
   aacatgtgag caaaaggcca gcaaaaggcc aggaaccgta aaaaggccgc gttgctggcg 2340
   tttttccata ggctccgccc ccctgacgag catcacaaaa atcgacgctc aagtcagagg 2400
   tggcgaaacc cgacaggact ataaagatac caggcgtttc cccctggaag ctccctcgtg 2460
   cgctctcctg ttccgaccct gccgcttacc ggatacctgt ccgcctttct cccttcggga 2520
   agcgtggcgc tttctcatag ctcacgctgt aggtatctca gttcggtgta ggtcgttcgc 2580
   tccaagctgg gctgtgtgca cgaacccccc gttcagcccg accgctgcgc cttatccggt 2640
   aactatcgtc ttgagtccaa cccggtaaga cacgacttat cgccactggc agcagccact 2700
   ggtaacagga ttagcagagc gaggtatgta ggcggtgcta cagagttctt gaagtggtgg 2760
   cctaactacg gctacactag aagaacagta tttggtatct gcgctctgct gaagccagtt 2820
   accttcggaa aaagagttgg tagctcttga tccggcaaac aaaccaccgc tggtagcggt 2880
   ggtttttttg tttgcaagca gcagattacg cgcagaaaaa aaggatctca agaagatcct 2940
   ttgatctttt ctacggggtc tgacgctcag tggaacgaaa actcacgtta agggattttg 3000
   gtcatgagat tatcaaaaag gatcttcacc tagatccttt taaattaaaa atgaagtttt 3060
   aaatcaatct aaagtatata tgagtaaact tggtctgaca gttaccaatg cttaatcagt 3120
   gaggcaccta tctcagcgat ctgtctattt cgttcatcca tagttgcctg actccccgtc 3180
   gtgtagataa ctacgatacg ggagggctta ccatctggcc ccagtgctgc aatgataccg 3240
   cgagacccac gctcaccggc tccagattta tcagcaataa accagccagc cggaagggcc 3300
   gagcgcagaa gtggtcctgc aactttatcc gcctccatcc agtctattaa ttgttgccgg 3360
   gaagctagag taagtagttc gccagttaat agtttgcgca acgttgttgc cattgctaca 3420
   ggcatcgtgg tgtcacgctc gtcgtttggt atggcttcat tcagctccgg ttcccaacga 3480
   tcaaggcgag ttacatgatc ccccatgttg tgcaaaaaag cggttagctc cttcggtcct 3540
   ccgatcgttg tcagaagtaa gttggccgca gtgttatcac tcatggttat ggcagcactg 3600
   cataattctc ttactgtcat gccatccgta agatgctttt ctgtgactgg tgagtactca 3660
   accaagtcat tctgagaata gtgtatgcgg cgaccgagtt gctcttgccc ggcgtcaata 3720
   cgggataata ccgcgccaca tagcagaact ttaaaagtgc tcatcattgg aaaacgttct 3780
   tcggggcgaa aactctcaag gatcttaccg ctgttgagat ccagttcgat gtaacccact 3840
   cgtgcaccca actgatcttc agcatctttt actttcacca gcgtttctgg gtgagcaaaa 3900
   acaggaaggc aaaatgccgc aaaaaaggga ataagggcga cacggaaatg ttgaatactc 3960
   atactcttcc tttttcaata ttattgaagc atttatcagg gttattgtct catgagcgga 4020
   tacatatttg aatgtattta gaaaaataaa caaatagggg ttccgcgcac atttccccga 4080
   aaagtgccac ctgacgtcta agaaaccatt attatcatga cattaaccta taaaaatagg 4140
   cgtatcacga ggccctttcg tc                                          4162
<210> 7
   <211> 5163
   <212> DNA
   <213> Artificial Sequence
<220>
   <223> synthetic pUC19.HR.OpLuc.KanR plasmid sequence
<400> 7
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagctt agattgataa agaagcagtt gaaattgctc gtcaaaccgg 2280
   tcgtggtgaa ggtaacttca ttatcgcttc ccgtaacgta gttaacgtac tggcttcagt 2340
   tgatactggt atttcttacg ctgcacaggg tctggcttcc ggttttaata ccgacactac 2400
   taagtctgta tttgccggtg tacttggtgg taaataccgc gtatacatcg accagtatgc 2460
   taaacaggat tacttcaccg ttggttacaa aggcgctaac gaaatggatg caggtatcta 2520
   ctatgctcct tacgttgcac ttaccccact gcgtggttcc gatcctaaga acttccagcc 2580
   ggtaatgggc tttaaaactc gttacggtat cggtgttaac ccgtttgctg aaagttccct 2640
   gcaggctcca ggtgctcgca tccagtccgg tatgccgtct atcctgaaca gccttggtaa 2700
   gaacgcttac ttccgccgtg tatatgtaaa aggcatctaa ggcgcgccta taaataaata 2760
   ctagtagata aggaggattt cgaatgttta cactggcaga ctttgttggt gactggcagc 2820
   aaacagccgg atataaccag gaccaggtgc ttgaacaggg tggcctttcg agtttgtttc 2880
   aggcgttagg tgtgtcggtg acccctattc agaaagtggt gctgagcgga gaaaacggcc 2940
   tgaaggccga tattcatgtt attatcccgt acgaggggtt atccggcttc cagatgggtc 3000
   tgattgaaat gatcttcaaa gtggtttatc cggttgacga tcatcatttt aagattatcc 3060
   tgcactatgg caccctggtg attgatggcg tgaccccgaa tatgattgat tatttcggcc 3120
   gtccgtaccc aggcattgca gtatttgacg gtaaacaaat caccgtcacc ggtaccctgt 3180
   ggaatggtaa taaaatttat gatgaacgtt tgatcaaccc ggatggcagt ctgttatttc 3240
   gcgtgaccat taacggagta accggttggc gtctctgtga aaacatttta gcttaagcga 3300
   tcgcataact tcgtatagca tacattatac gaagttattt gacaggctct gtattacgtt 3360
   tctataaata aggaagagta tgagccatat tcaacgggaa acgtcttgct ctaggccgcg 3420
   attaaattcc aacatggatg ctgatttata tgggtataaa tgggctcgcg ataatgtcgg 3480
   gcaatcaggt gcgacaatct atcgattgta tgggaagccc gatgcgccag agttgtttct 3540
   gaaacatggc aaaggtagcg ttgccaatga tgttacagat gagatggtca gactaaactg 3600
   gctgacggaa tttatgcctc ttccgaccat caagcatttt atccgtactc ctgatgatgc 3660
   atggttactc accactgcga tccctgggaa aacagcattc caggtattag aagaatatcc 3720
   tgattcaggt gaaaatattg ttgatgcgct ggcagtgttc ctgcgccggt tgcattcgat 3780
   tcctgtttgt aattgtcctt ttaacagcga tcgcgtattt cgtctcgctc aggcgcaatc 3840
   acgaatgaat aacggtttgg ttgatgcgag tgattttgat gacgagcgta atggctggcc 3900
   tgttgaacaa gtctggaaag aaatgcataa acttttgcca ttctcaccgg attcagtcgt 3960
   cactcatggt gatttctcac ttgataacct tatttttgac gaggggaaat taataggttg 4020
   tattgatgtt ggacgagtcg gaatcgcaga ccgataccag gatcttgcca tcctatggaa 4080
   ctgcctcggt gagttttctc cttcattaca gaaacggctt tttcaaaaat atggtattga 4140
   taatcctgat atgaataaat tgcagtttca tttgatgctc gatgagtttt tctaaataac 4200
   ttcgtatagc atacattata cgaagttatt tatttctgtg caatttcgat gtaagttttc 4260
   aaaatatttt gccaattagg cataccttct tggacatatg ccggagaacc gtctttgttt 4320
   ttgaatgatg caagtttatc agtggcccaa taacctttag ctcgagcttc agaataaact 4380
   ttcatccata ctttgttgaa ggcattacca ttaacttttg caccatattt ttctactaac 4440
   ttaactgctt cagcttccag aacttttaaa gtatctcgta agaaacgtgc tttagtcatt 4500
   ttgtttactc ctctgtagtt gataagtcta tagtatcaca taccaaatac gttgtaaaca 4560
   atctttataa ataatctata tcacataagg aaaaaatgca atgagtaaaa tccaaaaatt 4620
   attgcgtgaa tctacaacgt ctactagcaa ctcaatcggt cgcccaaatc tcgttgcttt 4680
   gactcgcgct acgactaaat taatatattc tgacattgta gcaacacaag tcgactctag 4740
   aggatccccg ggtaccgagc tcgaattcac tggccgtcgt tttacaacgt cgtgactggg 4800
   aaaaccctgg cgttacccaa cttaatcgcc ttgcagcaca tccccctttc gccagctggc 4860
   gtaatagcga agaggcccgc accgatcgcc cttcccaaca gttgcgcagc ctgaatggcg 4920
   aatggcgcct gatgcggtat tttctcctta cgcatctgtg cggtatttca caccgcatat 4980
   ggtgcactct cagtacaatc tgctctgatg ccgcatagtt aagccagccc cgacacccgc 5040
   caacacccgc tgacgcgccc tgacgggctt gtctgctccc ggcatccgct tacagacaag 5100
   ctgtgaccgt ctccgggagc tgcatgtgtc agaggttttc accgtcatca ccgaaacgcg 5160
   cga                                                               5163